# FASCICULE DE BREVET EUROPEEN

(11) **EP 3 562 500 B1**
(45) Date de publication et mention de la délivrance du brevet: **19.07.2023**
(21) Numéro de dépôt: 17825875.2
(22) Date de dépôt: 27.12.2017
(51) Int. Cl.: A61K 38/22, A61K 38/28, A61P 3/10, A61K 47/34, A61K 9/08, A61K 9/00

(54) **COMPOSITIONS SOUS FORME DE SOLUTION AQUEUSE INJECTABLE COMPRENANT DE L'AMYLINE, UN AGONISTE AU RÉCEPTEUR DE L'AMYLINE OU UN ANALOGUE D'AMYLINE ET UN CO-POLYAMINOACIDE**
ZUSAMMENSETZUNGEN IN FORM EINER INJIZIERBAREN WÄSSRIGEN LÖSUNG MIT AMYLIN, EINEM AMYLIN-REZEPTOR-AGONIST ODER EINEM AMYLIN-ANALOGON UND EINER COPOLYAMINOSÄURE
COMPOSITIONS IN THE FORM OF AN INJECTABLE AQUEOUS SOLUTION COMPRISING AMYLIN, AN AMYLIN RECEPTOR AGONIST OR AN AMYLIN ANALOGUE AND A COPOLYAMINO ACID

(30) Priorité: 27.12.2016 FR 1663438; 07.12.2017 FR 1761806
(43) Date de publication de la demande: 06.11.2019
(73) Titulaire: Adocia, 69003 Lyon (FR)
(72) Inventeur: DURACHER, David, 69004 Lyon (FR); MEIFFREN, Grégory, 69330 Meyzieu (FR); SOULA, Rémi, 69330 Meyzieu (FR)
(74) Mandataire: Tripoz, Inès
(86) Numéro de dépôt international: PCT/EP2017/084674
(87) Numéro de publication internationale: WO 2018/122278

(56) Documents cités:
- WO-A1-2013/104861
- FR-A1- 2 985 429
- GONZALEZ-ARAMUNDIZ JOSE VICENTE ET AL: "Polypeptides and polyaminoacids in drug delivery", EXPERT OPINION ON DRUG DELI, INFORMA HEALTHCARE, GB, vol. 9, no. 2, 1 janvier 2012 (2012-01-01) , pages 183-201, XP008172801, ISSN: 1742-5247, DOI: 10.1517/17425247.2012.647906

## Description

L'invention concerne les thérapies par injection d'amyline, d'agoniste au récepteur de l'amyline ou d'analogue d'amyline pour traiter le diabète.

L'invention concerne une composition sous forme d'une solution aqueuse injectable, dont le pH est compris entre 6,0 et 8,0, comprenant au moins de l'amyline, un agoniste au récepteur de l'amyline ou un analogue d'amyline et un co-polyaminoacide porteur de charges carboxylates et de radicaux hydrophobes selon l'invention et des compositions comprenant en outre une insuline (à l'exclusion des insulines basales dont le point isoélectrique pI est compris entre 5,8 et 8,5). L'invention concerne également des formulations pharmaceutiques comprenant les compositions selon l'invention. Enfin, l'invention se rapporte également à une utilisation des co-polyaminoacides porteurs de charges carboxylates et de radicaux hydrophobes selon l'invention pour stabiliser des compositions d'amyline, d'agoniste au récepteur de l'amyline ou d'analogue d'amyline ainsi que des compositions d'amyline, d'agoniste au récepteur de l'amyline ou d'analogue d'amyline comprenant en outre une insuline.

Le diabète de type 1 est une maladie auto-immune conduisant à la destruction des cellules béta du pancréas. Ces cellules sont connues pour produire l'insuline dont le rôle principal est de réguler l'utilisation du glucose dans les tissus périphériques (Gerich 1993 Control of glycaemia). Par conséquent, les patients atteints de diabète de type 1 souffrent d'hyperglycémie chronique et doivent s'administrer de l'insuline exogène afin de limiter cette hyperglycémie. L'insulino-thérapie a permis de changer drastiquement l'espérance de vie de ces patients. Cependant, le contrôle de la glycémie assuré par l'insuline exogène n'est pas optimal, en particulier après la prise d'un repas. Ceci est lié au fait que ces patients produisent du glucagon après la prise d'un repas, ce qui conduit au déstockage d'une partie du glucose stocké dans le foie, ce qui n'est pas le cas chez la personne saine. Cette production de glucose médiée par le glucagon aggrave le problème de régulation de la glycémie de ces patients.

Il a été démontré que l'amyline, une autre hormone produite par les cellules béta du pancréas et donc également déficiente chez les patients diabétiques de type 1, joue un rôle clé dans la régulation de la glycémie post-prandiale. L'amyline, aussi connu sous le nom de « islet amyloid polypeptide » ou IAPP, est un peptide de 37 amino acides qui est co-stocké et co-sécrété avec l'insuline (Schmitz 2004 Amylin Agonists). Ce peptide est connu pour bloquer la production du glucagon par les cellules alpha du pancréas. Ainsi, l'insuline et l'amyline ont des rôles complémentaires et synergiques, puisque l'insuline permet de réduire la concentration de glucose dans le sang alors que l'amyline permet de réduire l'entrée de glucose endogène dans le sang en inhibant la production (sécrétion) du glucagon endogène.

Cette problématique de régulation de la glycémie post-prandiale est assez similaire pour les patients atteints de diabète de type 2 traités par l'insuline dans la mesure où leur maladie a conduit à une perte très significative de leur masse de cellules béta et par conséquent, de leur capacité de production d'insuline et d'amyline.

L'amyline humaine a des propriétés qui ne sont pas compatibles avec les exigences pharmaceutiques en termes de solubilité et de stabilité (Goldsbury CS, et al., Polymorphic fibrillar assembly of human amylin. J Struct Biol 119 :17-27, 1997). L'amyline est connue pour former des fibres amyloïdes qui conduisent à la formation de plaques qui sont insolubles dans l'eau. Bien qu'étant l'hormone naturelle, il a été nécessaire de développer un analogue afin de résoudre ces problèmes de solubilité.

Les propriétés physico-chimiques de l'amyline rendent ainsi son utilisation impossible : l'amyline n'est stable qu'une quinzaine de minutes à pH acide, et moins d'une minute à pH neutre.

La société Amylin a développé un analogue de l'amyline, le pramlintide, pour pallier le manque de stabilité de l'amyline humaine. Ce produit commercialisé sous le nom de Symlin^{®} a été approuvé en 2005 par la FDA pour le traitement des diabétiques de type 1 et de type 2. Il doit être administré par voie sous cutanée trois fois par jour, dans l'heure précédant le repas afin d'améliorer le contrôle de la glycémie post-prandiale. Ce peptide est formulé à pH acide et est décrit pour fibriller lorsque le pH de la solution est supérieur à 5,5. Des variantes d'analogues sont décrites dans le brevet US 5,686,411.

Cet analogue n'est ainsi pas satisfaisant sur le plan de la stabilité lorsqu'une formulation à pH neutre est envisagée.

A ce jour, il n'existe aucun moyen permettant de stabiliser l'amyline humaine afin d'en faire un produit pharmaceutique. Or, il serait avantageux pour les patients d'avoir accès à la forme humaine de cette hormone physiologique. Il serait également avantageux de pouvoir formuler un analogue ou un agoniste de récepteur d'amyline à pH neutre.

De plus, il y aurait un intérêt à pouvoir mélanger en solution aqueuse l'amyline, un analogue de l'amyline, ou un agoniste de récepteur à l'amyline, avec une insuline prandiale puisque ces deux produits sont à administrer avant le repas. Cela permettrait d'ailleurs de mimer la physiologie puisque ces deux hormones sont co-sécrétées par les cellules béta en réponse à un repas afin d'améliorer le contrôle de la glycémie post-prandiale.

Or, compte tenu du fait que les solutions d'insulines prandiales ont un pH proche de la neutralité pour des raisons de stabilité chimique, il n'est pas possible d'obtenir une solution aqueuse répondant aux exigences pharmaceutiques en termes de solubilité et de stabilité.

Pour cette raison, la demande de brevet US2016/001002 de la société ROCHE décrit une pompe contenant deux réservoirs séparés afin de rendre possible la co-administration de ces deux hormones avec un seul dispositif médical. Cependant, ce brevet ne résout pas le problème du mélange de ces deux hormones en solution qui permettrait de les administrer avec les pompes conventionnelles déjà sur le marché qui ne contiennent qu'un réservoir.

La demande de brevet WO2013067022 de la société XERIS apporte une solution au problème de stabilité de l'amyline et de sa compatibilité avec l'insuline en employant un solvant organique à la place de l'eau. L'absence d'eau semble résoudre les problèmes de stabilité mais l'utilisation d'un solvant organique pose des problèmes de sécurité d'utilisation chronique pour les patients diabétiques et également des problèmes de compatibilité avec les dispositifs médicaux usuels, au niveau des tubulures, des joints et des plastifiants employés.

La demande de brevet WO2007104786 de la société NOVO NORDISK décrit une méthode permettant de stabiliser une solution de pramlintide, qui est un analogue de l'amyline, et d'insuline par l'ajout d'un phospholipide, dérivé de glycérophosphoglycérol, en particulier le dimyristoyl glycérophosphoglycérol (DMPG). Or, cette solution requiert l'emploi de quantités importantes de DMPG qui peuvent poser un problème de tolérance locale.

A la connaissance de la demanderesse, il n'existe pas de moyen satisfaisant qui permette de combiner en solution aqueuse une insuline prandiale et l'amyline humaine, un agoniste de récepteur d'amyline ou un analogue de l'amyline afin de pouvoir être administrée avec des dispositifs conventionnels.

Le pH de formulation acide et la fibrillation rapide sont des freins pour obtenir une formulation pharmaceutique à pH neutre à base d'amyline et de pramlintide, mais aussi un frein pour combiner l'amyline ou le pramlintide à d'autres ingrédients pharmaceutiques actifs, en particulier des peptides ou des protéines.

La demanderesse a remarqué que, de manière surprenante, les co-polyaminoacides selon l'invention stabilisent des compositions d'amyline, d'agoniste au récepteur de l'amyline ou d'analogue d'amyline à un pH compris entre 6 et 8. En effet, des compositions comprenant de l'amyline, un agoniste au récepteur de l'amyline ou un analogue d'amyline en combinaison avec un co-polyaminoacide selon l'invention présentent une stabilité accrue dans le temps, ce qui est d'un grand intérêt pour le développement pharmaceutique.

La demanderesse a également constaté que les co-polyaminoacides selon l'invention permettent en outre d'obtenir une composition comprenant de l'insuline prandiale et de l'amyline, agoniste au récepteur de l'amyline ou analogue d'amyline, ladite composition étant limpide et ayant une stabilité à la fibrillation améliorée.

Une méthode classique pour mesurer les stabilités des protéines ou peptides consiste à mesurer la formation de fibrilles à l'aide de Thioflavine T, encore appelée ThT. Cette méthode permet de mesurer dans des conditions de température et d'agitation qui permettent une accélération du phénomène, le temps de latence avant la formation de fibrilles par mesure de l'augmentation de la fluorescence. Les compositions selon l'invention ont un temps de latence avant la formation de fibrilles nettement supérieur à celui de l'amyline, d'un agoniste au récepteur de l'amyline ou d'un analogue d'amyline au pH d'intérêt.

Les compositions selon l'invention telle que définie dans les revendications, présentent une stabilité physique, et éventuellement chimique, satisfaisante au pH désiré.

Dans un mode de réalisation, l'invention concerne une composition sous forme d'une solution aqueuse injectable, dont le pH est compris entre 6,0 et 8,0, comprenant au moins :
a) de l'amyline , un agoniste au récepteur de l'amyline ou un analogue d'amyline ;
b) un co-polyaminoacide porteur de charges carboxylates et de radicaux hydrophobes Hy, ledit co-polyaminoacide étant constitué d'unités glutamiques ou aspartiques et lesdits radicaux hydrophobes Hy étant de formule I suivante : 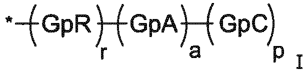 dans laquelle
   - GpR est un radical de formules II ou II' : 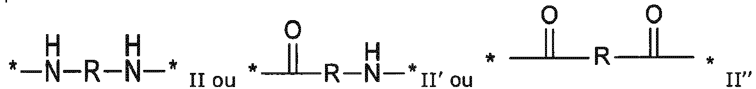
   - GpA est un radical de formules III ou III' : 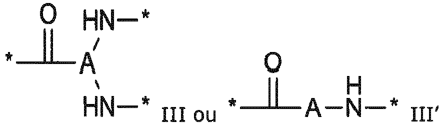
   - GpC est un radical de formule IV : 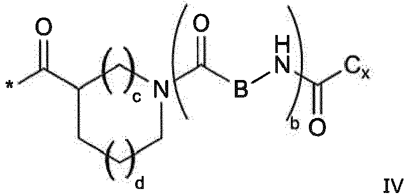
   - les * indiquent les sites de rattachement des différents groupes;
   - a est un entier égal à 0 ou à 1 ;
   - b est un entier égal à 0 ou à 1;
   - p est un entier égal à 1 ou à 2 et
      ∘ si p est égal à 1 alors a est égal à 0 ou à 1 et GpA est un radical de formule III' et,
      ∘ si p est égal à 2 alors a est égal à 1, et GpA est un radical de formule III;
   - c est un entier égal à 0 ou à 1, et si c est égal à 0 alors d est égal à 1 ou à 2;
   - d est un entier égal à 0, à 1 ou à 2;
   - r est un entier égal à 0 ou à 1, et
      ∘ si r est égal à 0 alors le radical hydrophobe de formule I est lié au co-polyaminoacide via une liaison covalente entre un carbonyl du radical hydrophobe et un atome d'azote en position N terminale du co-polyaminoacide, formant ainsi une fonction amide issue de la réaction d'une fonction amine en position N terminale du précurseur du co-polyaminoacide et une fonction acide portée par le précurseur du radical hydrophobe , et
      ∘ si r est égal à 1 alors le radical hydrophobe de formule I est lié au co-polyaminoacide :
         ▪ via une liaison covalente entre un atome d'azote du radical hydrophobe et un carbonyl du co-polyaminoacide, formant ainsi une fonction amide issue de la réaction d'une fonction amine du précurseur du radical hydrophobe et une fonction acide portée par le précurseur du co-polyaminoacide ou
         ▪ via une liaison covalente entre un carbonyl du radical hydrophobe et un atome d'azote en position N terminal du co-polyaminoacide, formant ainsi une fonction amide issue de la réaction d'une fonction acide du précurseur du radical hydrophobe et une fonction amine en position N terminale portée par le précurseur du co-polyaminoacide;
   - R est un radical choisi dans le groupe constitué par :
      ∘ un radical alkyle divalent, linéaire ou ramifié, comprenant, de 2 à 12 atomes de carbone si GpR est un radical de formule II ou de 1 à 11 atomes de carbone si GpR est un radical de formule II' ou II" ;
      ∘ un radical alkyle divalent, linéaire ou ramifié, comprenant, de 2 à 11 atomes de carbone si GpR est un radical de formule II ou de 1 à 11 atomes de carbone si GpR est un radical de formule II' ou II", ledit radical alkyle portant une ou plusieurs fonctions -CONH₂, et
      ∘ un radical éther ou polyéther non substitué comprenant de 4 à 14 atomes de carbone et de 1 à 5 atomes d'oxygène ;
   - A est un radical alkyle linéaire ou ramifié comprenant de 1 à 6 atomes de carbone ;
   - B est un radical alkyle linéaire ou ramifié, éventuellement comprenant un noyau aromatique, comprenant de 1 à 9 atomes de carbone ;
   - Cₓ est un radical alkyl monovalent linéaire ou ramifié, dans lequel x indique le nombre d'atomes de carbone et :
      ∘ si p est égal à 1, x est compris entre 11 et 25 (11 ≤ x ≤ 25) :
      ∘ si p est égal à 2, x est compris entre 9 et 15 (9 ≤ x ≤ 15),
   - le ratio i entre le nombre de radicaux hydrophobes et le nombre d'unités glutamiques ou aspartiques étant compris entre 0 < i ≤ 0,5 ;
   - lorsque plusieurs radicaux hydrophobes sont portés par un co-polyaminoacide alors ils sont identiques ou différents ;
   - le degré de polymérisation DP en unités glutamiques ou aspartiques est compris entre 5 et 250 ;
   - les fonctions acides libres étant sous forme de sel de cation alkalin choisi dans le groupe constitué par Na⁺ et K⁺ ;
   caractérisée en ce que la composition ne comprend pas une insuline basale dont le point isoélectrique pI est compris entre 5,8 et 8,5.

Dans un mode de réalisation, Hy comprend plus de 30 atomes de carbone.

Dans un mode de réalisation, la composition est caractérisée en ce que le pH est compris entre 6,6 et 7,8.

Dans un mode de réalisation, la composition est caractérisée en ce que le pH est compris entre 7,0 et 7,8.

Dans un mode de réalisation, la composition est caractérisée en ce que le pH est compris entre 6,8 et 7,4.

Dans les formules les * indiquent les sites de rattachements des différents éléments représentés.

Dans les formules, les * indiquent les sites de rattachement des radicaux hydrophobes au co-polyaminoacide. Les radicaux Hy sont rattachés au co-polyaminoacide via des fonctions amides.

Dans les formules VII et VIIa, les * indiquent, les sites de rattachement de GpR :
- au co-polyaminoacide et
- à GpA si a=1 ou à GPC si a=0.

Dans les formules III et III', les * indiquent, de gauche à droite respectivement, les sites de rattachement de GpA :
- à GpR si r=1 ou au co-polyaminoacide si r=0 et
- à GpC.

Dans la formule IV, le * indique le site de rattachement de GpC :
- à GpA si a=1, GpR si r=1 et a=0 ou,
- au co-polyaminoacide si r=0 et a=0.

Tous les rattachements entre les différents groupes GpR, GpA, GpL, GpG et GpC sont des fonctions amides.

Les radicaux Hy, GpR, GpA, GpL, GpG et GpC, et D sont chacun indépendamment identiques ou différents d'une unité monomérique à l'autre.

Les compositions sous forme d'une solution aqueuse injectable selon l'invention sont des solutions limpides.

On entend par « solution limpide », des compositions qui satisfont aux critères décrits dans les pharmacopées américaines et européenne concernant les solutions injectables. Dans la pharmacopée américaine, les solutions sont définies dans la partie <1151> faisant référence à l'injection (<1>) (faisant référence à <788> selon USP 35 et précisé dans <788> selon USP 35 et dans <787>, <788> et <790> USP 38 (à partir du 1^{er} aout 2014), selon USP 38). Dans la pharmacopée européenne, les solutions injectables doivent remplir les critères donnés dans les sections 2.9.19 et 2.9.20.

Dans un mode de réalisation, la composition est caractérisée en ce que lesdits radicaux hydrophobes sont choisis parmi les radicaux hydrophobes de formule I dans laquelle si p est égal à 1 (p=1) et si x est inférieur ou égal à 14 (x ≤ 14) alors r=0 ou r=1.

Dans un mode de réalisation, la composition est caractérisée en ce que lesdits radicaux hydrophobes sont choisis parmi les radicaux hydrophobes de formule I dans laquelle si p est égal à 1 (p=1) et si x est compris entre 15 et 16 (15 ≤ x ≤ 16), alors r=1.

Dans un mode de réalisation, la composition est caractérisée en ce que lesdits radicaux hydrophobes sont choisis parmi les radicaux hydrophobes de formule I dans laquelle si p est égal à 1 (p=1) et si x est supérieur à 17 (17 ≤ x) alors r=1 et R est un radical éther ou polyéther.

Dans un mode de réalisation, la composition est caractérisée en ce que lesdits radicaux hydrophobes sont choisis parmi les radicaux hydrophobes de formule I dans laquelle si p est égal à 1 (p=1) alors x est compris entre 17 et 25 (17 ≤ x ≤ 25).

Dans un mode de réalisation, la composition est caractérisée en ce que lesdits radicaux hydrophobes sont choisis parmi les radicaux hydrophobes de formule I dans laquelle p=1, représentée par la formule V suivante : 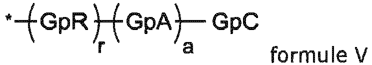 GpR, GpA, GpC, r et a ont les définitions données précédemment.

Dans un mode de réalisation, la composition est caractérisée en ce que le radical hydrophobe est un radical de formule V dans laquelle : r est égal à 1 (r=1) et a est égal à 0 (a=0).

Dans un mode de réalisation, la composition est caractérisée en ce que le radical hydrophobe est un radical de formule V dans laquelle r est égal à 1 (r = 1) et a est égal à 1 (a=1).

Dans un mode de réalisation, la composition est caractérisée en ce que le radical hydrophobe est un radical de formule V dans laquelle GpR est un radical de formule II.

Dans un mode de réalisation, la composition est caractérisée en ce que le radical hydrophobe est un radical de formule V dans laquelle GpR est un radical de formule II dans laquelle R est un radical alkyle linéaire divalent comprenant de 2 à 12 atomes de carbone.

Dans un mode de réalisation, la composition est caractérisée en ce que le radical hydrophobe est un radical de formule V dans laquelle GpR est un radical de formule II dans laquelle R est un radical alkyle divalent comprenant de 2 à 6 atomes de carbone.

Dans un mode de réalisation, la composition est caractérisée en ce que le radical hydrophobe est un radical de formule V dans laquelle GpR est un radical de formule II dans laquelle R est un radical alkyle linéaire divalent comprenant de 2 à 6 atomes de carbone.

Dans un mode de réalisation, la composition est caractérisée en ce que le radical hydrophobe est un radical de formule V dans laquelle GpR est un radical de formule II dans laquelle R est un radical alkyle divalent comprenant de 2 à 4 atomes de carbone.

Dans un mode de réalisation, la composition est caractérisée en ce que le radical hydrophobe est un radical de formule V dans laquelle GpR est un radical de formule II dans laquelle R est un radical alkyle linéaire divalent comprenant de 2 à 4 atomes de carbone.

Dans un mode de réalisation, la composition est caractérisée en ce que le radical hydrophobe est un radical de formule V dans laquelle GpR est un radical de formule II dans laquelle R est un radical alkyle divalent comprenant 2 atomes de carbone.

Dans un mode de réalisation, la composition est caractérisée en ce que le radical hydrophobe est un radical de formule V dans laquelle GpR est un radical de formule II'.

Dans un mode de réalisation, la composition est caractérisée en ce que le radical hydrophobe est un radical de formule V dans laquelle GpR est un radical de formule II' dans laquelle R est un radical alkyle linéaire divalent comprenant de 1 à 11 atomes de carbone.

Dans un mode de réalisation, la composition est caractérisée en ce que le radical hydrophobe est un radical de formule V dans laquelle GpR est un radical de formule II' dans laquelle R est un radical alkyle divalent comprenant de 1 à 6 atomes de carbone.

Dans un mode de réalisation, la composition est caractérisée en ce que le radical hydrophobe est un radical de formule V dans laquelle GpR est un radical de formule II ou II', dans laquelle R est un radical alkyle divalent, comprenant de 2 à 5 atomes de carbone et portant une ou plusieurs fonctions amide (-CONH₂).

Dans un mode de réalisation, la composition est caractérisée en ce que le radical hydrophobe est un radical de formule V dans laquelle GpR est un radical de formule II' ou II, dans laquelle R est un radical alkyle linéaire divalent, comprenant de 2 à 5 atomes de carbone et portant une ou plusieurs fonctions amide (-CONH₂).

Dans un mode de réalisation, la composition est caractérisée en ce que le radical hydrophobe est un radical de formule V dans laquelle GpR est un radical de formule II ou II' dans laquelle R est un radical choisi dans le groupe constitué par les radicaux représentés par les formules ci-dessous :

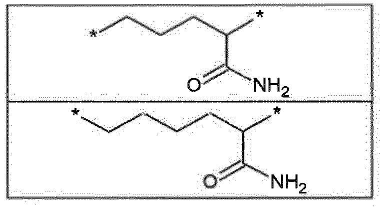

Dans un mode de réalisation, la composition est caractérisée en ce que le radical R est lié au co-polyaminoacide via une fonction amide portée par le carbone en position delta ou epsilon (ou en position 4 ou 5) par rapport à la fonction amide (-CONH₂).

Dans un mode de réalisation, la composition est caractérisée en ce que le radical hydrophobe est un radical de formule V dans laquelle GpR est un radical de formule II ou II', dans laquelle R est un radical linéaire éther ou polyéther non substitué comprenant de 4 à 14 atomes de carbone et de 1 à 5 atomes d'oxygène.

Dans un mode de réalisation, la composition est caractérisée en ce que le radical hydrophobe est un radical de formule V dans laquelle GpR est un radical de formule II ou II', dans laquelle R est un radical éther.

Dans un mode de réalisation, la composition est caractérisée en ce que le radical hydrophobe est un radical de formule V dans laquelle GpR est un radical de formule II ou II', dans laquelle R est un radical éther comprenant de 4 à 6 atomes de carbone.

Dans un mode de réalisation, la composition est caractérisée en ce que le radical hydrophobe est un radical de formule V dans laquelle GpR est un radical de formule II ou II' dans laquelle R est un radical éther représenté par la formule

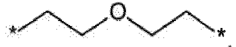

Dans un mode de réalisation, la composition est caractérisée en ce que le radical hydrophobe est un radical de formule V dans laquelle GpR est un radical de formule II ou II', dans laquelle R est un radical polyéther.

Dans un mode de réalisation, la composition est caractérisée en ce que le radical hydrophobe est un radical de formule V dans laquelle GpR est un radical de formule II ou II', dans laquelle R est un radical linéaire polyéther comprenant de 6 à 10 atomes de carbone et de 2 à 3 atomes d'oxygène.

Dans un mode de réalisation, la composition est caractérisée en ce que le radical hydrophobe de formule V dans laquelle GpR est un radical de formule II ou II', dans laquelle R est un radical polyéther choisi dans le groupe constitué par les radicaux représentés par les formules ci-dessous :

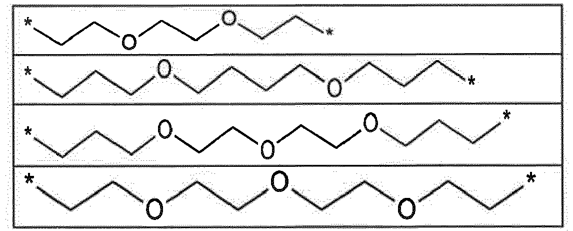

Dans un mode de réalisation, la composition est caractérisée en ce que le radical hydrophobe de formule V dans laquelle GpR est un radical de formule II dans laquelle R est un radical polyéther choisi dans le groupe constitué par les radicaux représentés par les formules ci-dessous :

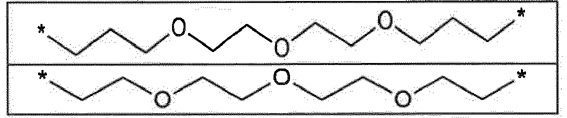

Dans un mode de réalisation, la composition est caractérisée en ce que le radical hydrophobe est un radical de formule V dans laquelle a est égal à 0 (a=0) et r est égal à 0 (r=0).

Dans un mode de réalisation, la composition est caractérisée en ce que le radical hydrophobe est un radical de formule V dans laquelle a est égal à 1 (a=1) et le radical GpA est un radical de formule III' dans laquelle A est choisi dans le groupe constitué des radicaux représentés par les formules ci-dessous :

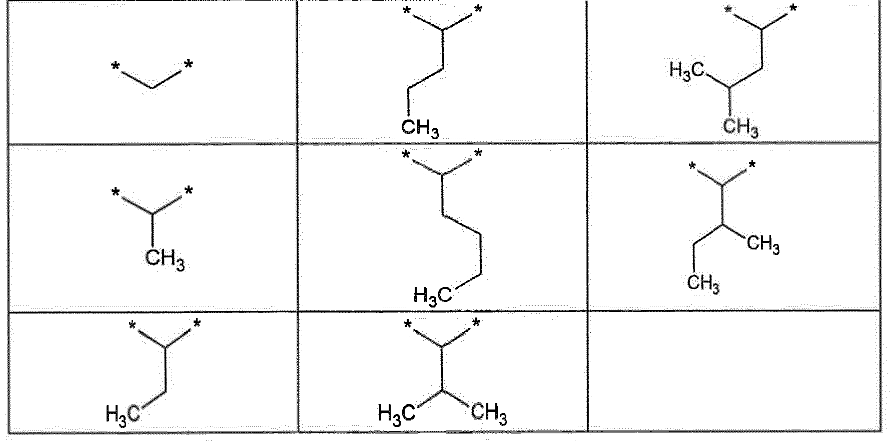

Dans un mode de réalisation, la composition est caractérisée en ce que le radical hydrophobe est un radical de formule V dans laquelle le radical GpC de formule IV est choisi dans le groupe constitué des radicaux de formules IVa, IVb ou IVc ci-après représentées :

| | |
|---|---|
| 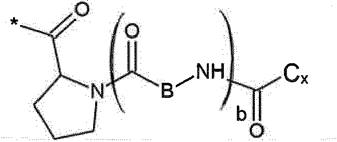 | Formule IVa |
| 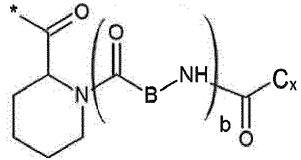 | Formule IVb |
| 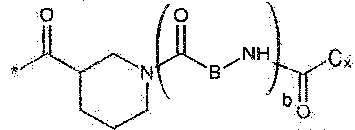 | Formule IVc |

Dans un mode de réalisation, la composition est caractérisée en ce que le radical hydrophobe est un radical de formule V dans laquelle le radical GpC est de formule IVa.

Dans un mode de réalisation, la composition est caractérisée en ce que le radical hydrophobe est un radical de formule V dans laquelle le radical GpC de formule IV est choisi dans le groupe constitué des radicaux de formules IVa, IVb ou IVc dans lesquels b est égal à 0, répondant respectivement aux formules IVd, IVe, et IVf ci-après représentées :

| | |
|---|---|
| 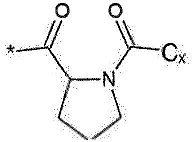 | Formule IVd |
| 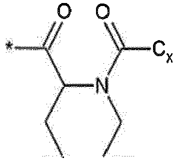 | Formule IVe |
| 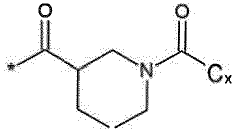 | Formule IVf |

Dans un mode de réalisation, la composition est caractérisée en ce que le radical hydrophobe est un radical de formule V dans laquelle le radical GpC répond à la formule IV ou IVa dans lesquelles b=0, et répond à la formule IVd.

Dans un mode de réalisation, la composition est caractérisée en ce que le radical hydrophobe est un radical de formule V dans laquelle le radical GpC de formule IV dans laquelle b=1 est choisi dans le groupe constitué des radicaux dans lesquels B est un résidu d'acide aminé choisi dans le groupe constitué par les radicaux représentés par les formules ci-dessous :

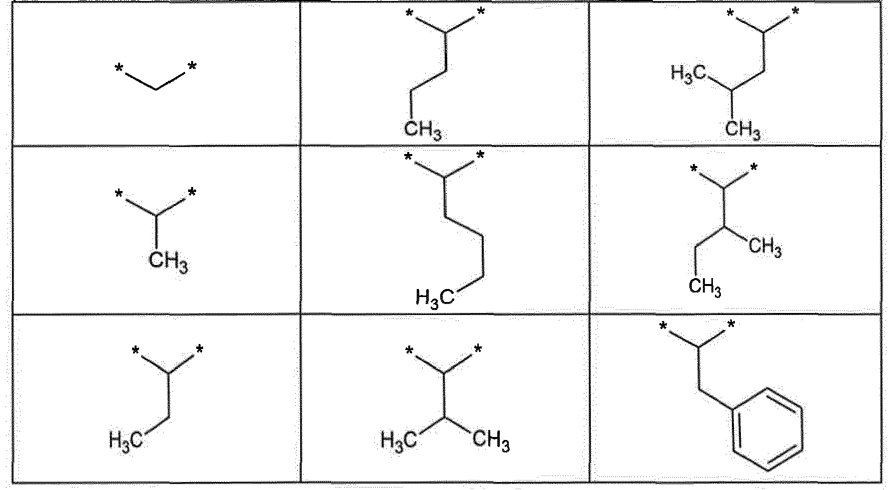

Dans un mode de réalisation, la composition est caractérisée en ce que le radical hydrophobe est un radical de formule V dans laquelle le radical GpC de formule IV ou IVa dans lesquelles b=1, est choisi dans le groupe constitué des radicaux dans lesquels B est un résidu d'acide aminé choisi dans le groupe constitué par les radicaux représentés par les formules ci-dessous :

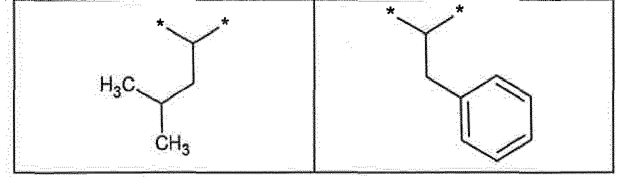

Dans un mode de réalisation, la composition est caractérisée en ce que le radical hydrophobe est un radical de formule V dans laquelle le radical GpC de formule IV est choisi dans le groupe constitué des radicaux dans lesquels Cx est choisi dans le groupe constitué par les radicaux alkyles linéaires.

Dans un mode de réalisation, la composition est caractérisée en ce que le radical hydrophobe est un radical de formule V dans laquelle le radical GpC de formule IV est choisi dans le groupe constitué des radicaux dans lesquels Cx est choisi dans le groupe constitué par les radicaux alkyles ramifiés.

Dans un mode de réalisation, la composition est caractérisée en ce que le radical hydrophobe est un radical de formule V dans laquelle le radical GpC de formule IV est choisi dans le groupe constitué des radicaux dans lesquels Cx est choisi dans le groupe constitué par les radicaux alkyles comprenant entre 11 et 14 atomes de carbone.

Dans un mode de réalisation, la composition est caractérisée en ce que le radical hydrophobe est un radical de formule V dans laquelle le radical GpC de formule IV est choisi dans le groupe constitué des radicaux dans lesquels Cx est choisi dans le groupe constitué par les radicaux représentés par les formules ci-dessous :

| | |
|---|---|
| 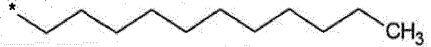 | x=11 |
| 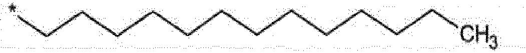 | x=13 |

Dans un mode de réalisation, la composition est caractérisée en ce que le radical hydrophobe est un radical de formule V dans laquelle le radical GpC de formule IV est choisi dans le groupe constitué des radicaux dans lesquels Cx est choisi dans le groupe constitué par les radicaux alkyles comprenant entre 15 et 16 atomes de carbone.

Dans un mode de réalisation, la composition est caractérisée en ce que le radical hydrophobe est un radical de formule V dans laquelle le radical GpC de formule IV est choisi dans le groupe constitué des radicaux dans lesquels Cx est choisi dans le groupe constitué par les radicaux représentés par les formules ci-dessous :

| | |
|---|---|
| 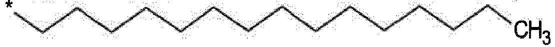 | x=15 |

Dans un mode de réalisation, la composition est caractérisée en ce que le radical hydrophobe est un radical de formule V dans laquelle le radical GpC de formule IV est choisi dans le groupe constitué des radicaux dans lesquels Cx est choisi dans le groupe constitué par les radicaux représentés par les formules ci-dessous :

| | |
|---|---|
| 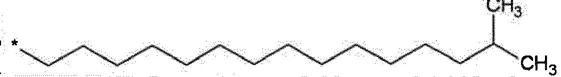 | x=16 |

Dans un mode de réalisation, la composition est caractérisée en ce que le radical hydrophobe est un radical de formule V dans laquelle le radical GpC de formule IV est choisi dans le groupe constitué des radicaux dans lesquels Cx est choisi dans le groupe constitué par les radicaux alkyles comprenant entre 17 et 25 atomes de carbone.

Dans un mode de réalisation, la composition est caractérisée en ce que le radical hydrophobe est un radical de formule V dans laquelle le radical GpC de formule IV est choisi dans le groupe constitué des radicaux dans lesquels Cx est choisi dans le groupe constitué par les radicaux alkyles comprenant entre 17 et 18 atomes de carbone.

Dans un mode de réalisation, la composition est caractérisée en ce que le radical hydrophobe est un radical de formule V dans laquelle le radical GpC de formule IV est choisi dans le groupe constitué des radicaux dans lesquels Cx est choisi dans le groupe constitué par les radicaux alkyles représentés par les formules ci-dessous :

| | |
|---|---|
| 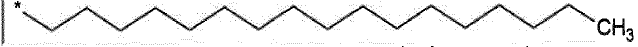 | x=17 |

Dans un mode de réalisation, la composition est caractérisée en ce que le radical hydrophobe est un radical de formule V dans laquelle le radical GpC de formule IV est choisi dans le groupe constitué des radicaux dans lesquels Cx est choisi dans le groupe constitué par les radicaux alkyles comprenant entre 18 et 25 atomes de carbone.

Dans un mode de réalisation, la composition est caractérisée en ce que le radical hydrophobe est un radical de formule V dans laquelle le radical GpC de formule IV est choisi dans le groupe constitué des radicaux dans lesquels Cx est choisi dans le groupe constitué par les radicaux alkyles représentés par les formules ci-dessous :

| | |
|---|---|
| 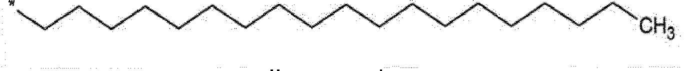 | x=19 |
| 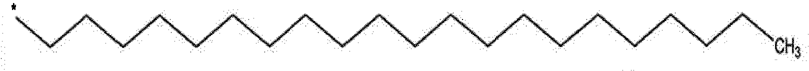 | x=21 |

Dans un mode de réalisation, la composition est caractérisée en ce que lesdits radicaux hydrophobes sont choisis parmi les radicaux hydrophobes de formule I dans laquelle a=1 et p=2, représentée par la formule VI suivante : 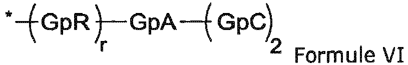 dans laquelle
GpR, GpA, GpC et r ont les définitions données précédemment.

Dans un mode de réalisation, la composition est caractérisée en ce que le radical hydrophobe est un radical de formule VI dans laquelle GpR est un radical de formule II.

Dans un mode de réalisation, la composition est caractérisée en ce que le radical hydrophobe est un radical de formule VI dans laquelle GpR est un radical de formule II dans lequel R est un radical alkyle linéaire divalent comprenant de 2 à 12 atomes de carbone.

Dans un mode de réalisation, la composition est caractérisée en ce que le radical hydrophobe est un radical de formule VI dans laquelle GpR est un radical de formule II dans laquelle R est un radical alkyle divalent comprenant de 2 à 6 atomes de carbone.

Dans un mode de réalisation, la composition est caractérisée en ce que le radical hydrophobe est un radical de formule VI dans laquelle GpR est un radical de formule II dans laquelle R est un radical alkyle linéaire divalent comprenant de 2 à 6 atomes de carbone.

Dans un mode de réalisation, la composition est caractérisée en ce que le radical hydrophobe est un radical de formule VI dans laquelle GpR est un radical de formule II dans laquelle R est un radical alkyle comprenant de 2 à 4 atomes de carbone.

Dans un mode de réalisation, la composition est caractérisée en ce que le radical hydrophobe est un radical de formule VI dans laquelle GpR est un radical de formule II dans laquelle R est un radical alkyle linéaire divalent comprenant de 2 à 4 atomes de carbone.

Dans un mode de réalisation, la composition est caractérisée en ce que le radical hydrophobe est un radical de formule VI dans laquelle GpR est un radical de formule II dans laquelle R est un radical alkyle linéaire divalent comprenant 2 atomes de carbone.

Dans un mode de réalisation, la composition est caractérisée en ce que le radical hydrophobe est un radical de formule VI dans laquelle GpR est un radical de formule II'.

Dans un mode de réalisation, la composition est caractérisée en ce que le radical hydrophobe est un radical de formule VI dans laquelle GpR est un radical de formule II' dans laquelle R est un radical alkyle linéaire divalent comprenant de 1 à 11 atomes de carbone.

Dans un mode de réalisation, la composition est caractérisée en ce que le radical hydrophobe est un radical de formule VI dans laquelle GpR est un radical de formule II' dans laquelle R est un radical alkyle divalent comprenant de 1 à 6 atomes de carbone.

Dans un mode de réalisation, la composition est caractérisée en ce que le radical hydrophobe est un radical de formule VI dans laquelle GpR est un radical de formule II ou II', dans laquelle R est un radical alkyle divalent, comprenant de 2 à 5 atomes de carbone, et portant une ou plusieurs fonctions amide (-CONH₂).

Dans un mode de réalisation, la composition est caractérisée en ce que le radical hydrophobe est un radical de formule VI dans laquelle GpR est un radical de formule II ou II', dans laquelle R est un radical alkyle linéaire divalent, comprenant de 2 à 5 atomes de carbone et portant une ou plusieurs fonctions amide (-CONH₂).

Dans un mode de réalisation, la composition est caractérisée en ce que le radical hydrophobe est un radical de formule VI dans laquelle GpR est un radical de formule II ou II' dans laquelle R est un radical choisi dans le groupe constitué par les radicaux représentés par les formules ci-dessous :

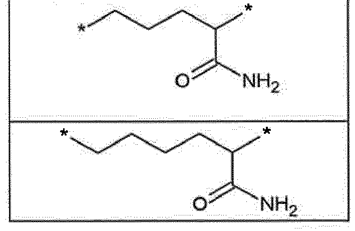

Dans un mode de réalisation, la composition est caractérisée en ce que le radical hydrophobe est un radical de formule VI dans laquelle la fonction amine du radical GpR engagée dans la formation de la fonction amide qui lie ledit radical GpR au co-polyaminoacide est portée par un carbone en position delta ou epsilon (ou en position 4 ou 5) par rapport à la fonction amide (-CONH₂).

Dans un mode de réalisation, la composition est caractérisée en ce que le radical hydrophobe est un radical de formule VI dans laquelle GpR est un radical de formule II ou II', dans laquelle R est un radical linéaire éther ou polyéther non substitué comprenant de 4 à 14 atomes de carbone et de 1 à 5 atomes d'oxygène.

Dans un mode de réalisation, la composition est caractérisée en ce que le radical hydrophobe est un radical de formule VI dans laquelle GpR est un radical de formule II ou II' dans laquelle R est un radical éther.

Dans un mode de réalisation, la composition est caractérisée en ce que le radical éther R est un radical comprenant de 4 à 6 atomes de carbone.

Dans un mode de réalisation, la composition est caractérisée en ce que le radical éther est

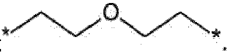

Dans un mode de réalisation, la composition est caractérisée en ce que le radical hydrophobe est un radical de formule VI dans laquelle GpR est un radical de formule II ou II', dans laquelle R est un radical polyéther.

Dans un mode de réalisation, la composition est caractérisée en ce que le radical hydrophobe est un radical de formule VI dans laquelle GpR est un radical de formule II ou II', dans laquelle R est un radical linéaire polyéther comprenant de 6 à 10 atomes de carbone et de 2 à 3 atomes d'oxygène.

Dans un mode de réalisation, la composition est caractérisée en ce que le radical hydrophobe est un radical de formule VI dans laquelle GpR est un radical de formule II ou II' dans laquelle R est un radical linéaire polyéther choisi dans le groupe constitué par les radicaux représentés par les formules ci-dessous :

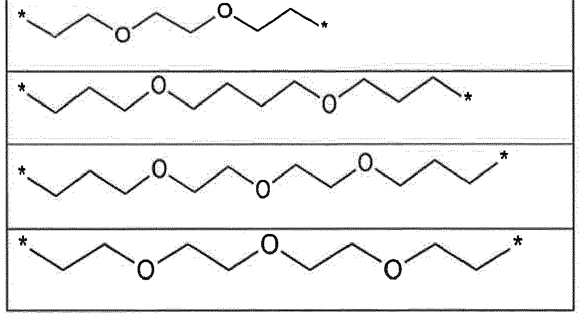

Dans un mode de réalisation, la composition est caractérisée en ce que le radical hydrophobe est un radical de formule VI dans laquelle le radical GpA de formule III est choisi dans le groupe constitué des radicaux de formules IIIa et IIIb ci-après représentées :

| | |
|---|---|
| 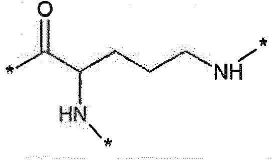 | Formule IIIa |
| 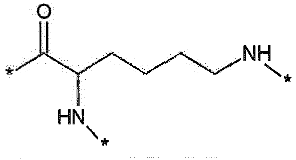 | Formule IIIb |

Dans un mode de réalisation, la composition est caractérisée en ce que le radical hydrophobe est un radical de formule VI dans laquelle le radical GpA de formule III est un radical de formule IIIb ci-après représentée :

| | |
|---|---|
| 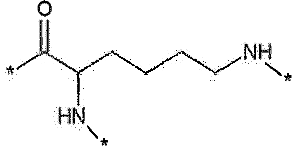 | Formule IIIb |

Dans un mode de réalisation, la composition est caractérisée en ce que le radical hydrophobe est un radical de formule VI dans laquelle le radical GpC de formule IV est choisi dans le groupe constitué des radicaux de formules IVa, IVb et IVc ci-après représentées :

| | |
|---|---|
| 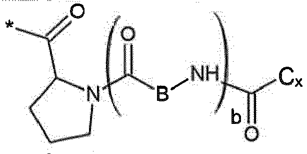 | Formule IVa |
| 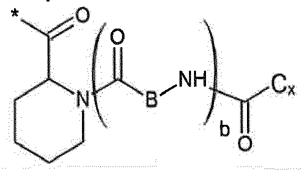 | Formule IVb |
| 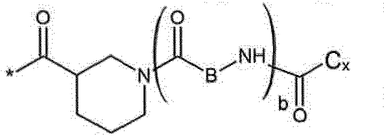 | Formule IVc |

Dans un mode de réalisation, la composition est caractérisée en ce que le radical hydrophobe est un radical de formule VI dans laquelle le radical GpC est de formule IVa.

Dans un mode de réalisation, la composition est caractérisée en ce que le radical hydrophobe est un radical de formule VI dans laquelle le radical GpC de formule IV est choisi dans le groupe constitué des radicaux de formules IVa, IVb ou IVc dans lesquels b est égal à 0 (b=0), répondant respectivement aux formules IVd, IVe, et IVf ci-après représentées :

| | |
|---|---|
| 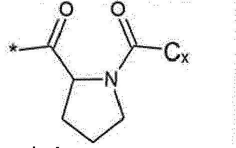 | Formule IVd |
| 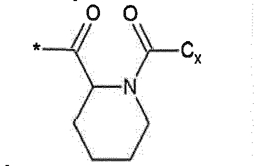 | Formule IVe |
| 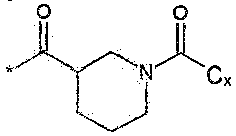 | Formule IVf |

Dans un mode de réalisation, la composition est caractérisée en ce que le radical hydrophobe est un radical de formule VI dans laquelle le radical GpC répond à la formule IV ou IVa dans lesquelles b=0, et répond à la formule IVd.

Dans un mode de réalisation, la composition est caractérisée en ce que le radical hydrophobe est un radical de formule VI dans laquelle le radical GpC de formule IV est choisi dans le groupe constitué des radicaux dans lesquels Cx est choisi dans le groupe constitué par les radicaux alkyles linéaires comprenant entre 9 et 15 atomes de carbone.

Dans un mode de réalisation, la composition est caractérisée en ce que le radical hydrophobe est un radical de formule VI dans laquelle le radical GpC de formule IV est choisi dans le groupe constitué des radicaux dans lesquels Cx est choisi dans le groupe constitué par les radicaux alkyles ramifiés comprenant entre 9 et 15 atomes de carbone.

Dans un mode de réalisation, la composition est caractérisée en ce que le radical hydrophobe est un radical de formule VI dans laquelle le radical GpC de formule IV est choisi dans le groupe constitué des radicaux dans lesquels Cx est choisi dans le groupe constitué par les radicaux alkyles comprenant 9 ou 10 atomes de carbone.

Dans un mode de réalisation, la composition est caractérisée en ce que le radical hydrophobe est un radical de formule VI dans laquelle le radical GpC de formule IV est choisi dans le groupe constitué des radicaux dans lesquels Cx est choisi dans le groupe constitué par les radicaux alkyles comprenant entre 11 et 15 atomes de carbone.

Dans un mode de réalisation, la composition est caractérisée en ce que le radical hydrophobe est un radical de formule VI dans laquelle le radical GpC de formule IV est choisi dans le groupe constitué des radicaux dans lesquels Cx est choisi dans le groupe constitué par les radicaux alkyles comprenant entre 11 et 13 atomes de carbone.

Dans un mode de réalisation, la composition est caractérisée en ce que le radical hydrophobe est un radical de formule VI dans laquelle le radical GpC de formule IV est choisi dans le groupe constitué des radicaux dans lesquels Cx est choisi dans le groupe constitué par les radicaux représentés par les formules ci-dessous :

| | |
|---|---|
| 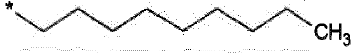 | x = 9 |
| 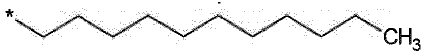 | x = 11 |
| 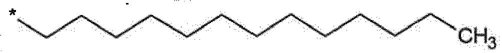 | x = 13 |

Dans un mode de réalisation, la composition est caractérisée en ce que le radical hydrophobe est un radical de formule VI dans laquelle le radical GpC de formule IV est choisi dans le groupe constitué des radicaux dans lesquels Cx est choisi dans le groupe constitué par les radicaux alkyles comprenant 14 ou 15 atomes de carbone.

Dans un mode de réalisation, la composition est caractérisée en ce que le radical hydrophobe est un radical de formule VI dans laquelle le radical GpC de formule IV est choisi dans le groupe constitué des radicaux dans lesquels Cx est choisi dans le groupe constitué par les radicaux représentés par les formules ci-dessous :

| | |
|---|---|
| 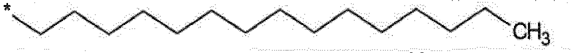 | x=15 |

Dans un mode de réalisation, la composition est caractérisée en ce que le co-polyaminoacide porteur de charges carboxylates et de radicaux hydrophobes est choisi parmi les co-polyaminoacides de formule VII suivante : 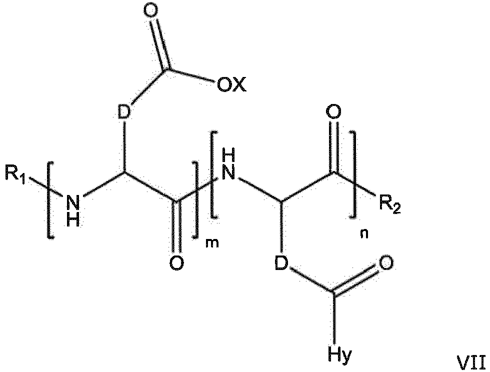 dans laquelle,
- D représente, indépendamment, soit un groupe -CH₂- (unité aspartique) soit un groupe -CH₂-CH₂- (unité glutamique) ;
- Hy est un radical hydrophobe choisi parmi les radicaux hydrophobes de formules I, V ou VI, dans lesquelles r=1 et GpR est un radical de formule II ;
- R₁ est un radical hydrophobe choisi parmi les radicaux hydrophobes de formules I, V ou VI dans lesquelles r=0 ou r=1 et GpR est un radical de formule II', ou un radical choisi dans le groupe constitué par un H, un groupe acyle linéaire en C2 à C10, un groupe acyle ramifié en C3 à C10, un benzyle, une unité « acide aminé » terminale et un pyroglutamate ;
- R₂ est un radical hydrophobe choisi parmi les radicaux hydrophobes de formules I, V ou VI dans lesquelles r=1 et GpR est un radical de formule II, ou un radical -NR'R", R' et R" identiques ou différents étant choisis dans le groupe constitué par H, les alkyles linéaires ou ramifiés ou cycliques en C2 à C10, le benzyle et lesdits R' et R" alkyles pouvant former ensemble un ou des cycles carbonés saturés, insaturés et/ou aromatiques et/ou pouvant comporter des hétéroatomes, choisis dans le groupe constitué par O, N et 5 ;
- X représente un H ou une entité cationique choisie dans le groupe comprenant les cations métalliques ;
- n + m représente le degré de polymérisation DP du co-polyaminoacide, c'est-à-dire le nombre moyen d'unités monomériques par chaîne de co-polyaminoacide et 5 ≤ n + m ≤ 250.

Le co-polyaminoacide porteur de charges carboxylates et d'au moins un radical hydrophobe de formule I peut également être appelé « co-polyaminoacide » dans la présente description.

On appelle « co-polyaminoacide statistique » un co-polyaminoacide porteur de charges carboxylates et d'au moins un radical hydrophobe, un co-polyaminoacide de formule VIIa.

Dans un mode de réalisation, la composition est caractérisée en ce que le co-polyaminoacide porteur de charges carboxylates et de radicaux hydrophobes est choisi parmi les co-polyaminoacides de formules VII, dans laquelle R₁=R'₁ et R₂=R'₂, de formule VIIa suivante : 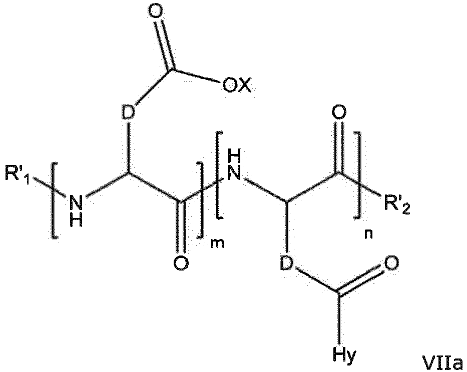 dans laquelle,
- m, n, X, D et Hy ont les définitions données précédemment ;
- R'₁ est un radical choisi dans le groupe constitué par un H, un groupe acyle linéaire en C2 à C10, un groupe acyle ramifié en C3 à C10, un benzyle, une unité « acide aminé » terminale et un pyroglutamate ;
- R'₂ est un radical -NR'R", R' et R" identiques ou différents étant choisis dans le groupe constitué par H, les alkyles linéaires ou ramifiés ou cycliques en C2 à C10, le benzyle et lesdits R' et R" alkyles pouvant former ensemble un ou des cycles carbonés saturés, insaturés et/ou aromatiques et/ou pouvant comporter des hétéroatomes, choisis dans le groupe constitué par O, N et S.

On appelle « co-polyaminoacide défini » un co-polyaminoacide porteur de charges carboxylates et d'au moins un radical hydrophobe, un co-polyaminoacide de formule VIIb.

Dans un mode de réalisation, la composition est caractérisée en ce que le co-polyaminoacide porteur de charges carboxylates et de radicaux hydrophobes est choisi parmi les co-polyaminoacides de formule VII dans laquelle n=0 de formule VIIb suivante : 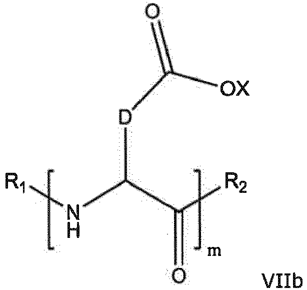 dans laquelle m, X, D, R₁ et R₂ ont les définitions données précédemment et au moins R₁ ou R₂ est un radical hydrophobe de formule I, V ou VI.

Dans un mode de réalisation, la composition est caractérisée en ce que le co-polyaminoacide porteur de charges carboxylates et de radicaux hydrophobes est choisi parmi les co-polyaminoacides de formule VII dans laquelle n=0 de formule VIIb et R₁ ou R₂ est un radical hydrophobe de formule I, V ou VI.

Dans un mode de réalisation, la composition est caractérisée en ce que le co-polyaminoacide porteur de charges carboxylates et de radicaux hydrophobes est choisi parmi les co-polyaminoacides de formule VIIb dans laquelle R₁ est un radical hydrophobe de formule I, V ou VI dans lesquelles r=0 ou r=1 et GpR est de formule II'.

Dans un mode de réalisation, la composition est caractérisée en ce que le co-polyaminoacide porteur de charges carboxylates et de radicaux hydrophobes est choisi parmi les co-polyaminoacides de formules VIIb dans laquelle R₂ est un radical hydrophobe de formule I, V ou VI dans lesquelles r=1 et GpR est de formule II.

Dans un mode de réalisation, la composition est caractérisée en ce que R₁ est un radical choisi dans le groupe constitué par un groupe acyle linéaire en C₂ à C₁₀, un groupe acyle ramifié en C₃ à C₁₀, un benzyle, une unité « acide aminé » terminale et un pyroglutamate.

Dans un mode de réalisation, la composition est caractérisée en ce que R₁ est un radical choisi dans le groupe constitué par un groupe acyle linéaire en C₂ à C₁₀ ou un groupe acyle ramifié en C₃ à C₁₀.

Dans un mode de réalisation, la composition est caractérisée en ce que le co-polyaminoacide porteur de charges carboxylates et de radicaux hydrophobes est choisi parmi les co-polyaminoacides de formules VII, VIIa ou VIIb dans lesquels le groupe D est un groupe -CH₂- (unité aspartique).

Dans un mode de réalisation, la composition est caractérisée en ce que le co-polyaminoacide porteur de charges carboxylates et de radicaux hydrophobes est choisi parmi les co-polyaminoacides de formules VII, VIIa ou VIIb dans lesquels le groupe D est un groupe -CH₂-CH₂- (unité glutamique).

Dans un mode de réalisation, la composition est caractérisée en ce que le ratio i entre le nombre de radicaux hydrophobes et le nombre d'unités glutamiques ou aspartiques est compris entre 0,007 et 0,3.

Dans un mode de réalisation, la composition est caractérisée en ce que le ratio i entre le nombre de radicaux hydrophobes et le nombre d'unités glutamiques ou aspartiques est compris entre 0,01 et 0,3.

Dans un mode de réalisation, la composition est caractérisée en ce que le ratio i entre le nombre de radicaux hydrophobes et le nombre d'unités glutamiques ou aspartiques est compris entre 0,02 et 0,2.

Dans un mode de réalisation, la composition est caractérisée en ce que le radical hydrophobe répond à la formule VI et le ratio i entre le nombre de radicaux hydrophobes et le nombre d'unités glutamiques ou aspartiques est compris entre 0,007 et 0,15.

Dans un mode de réalisation, la composition est caractérisée en ce que le radical hydrophobe répond à la formule VI et le ratio i entre le nombre de radicaux hydrophobes et le nombre d'unités glutamiques ou aspartiques est compris entre 0,01 et 0,1.

Dans un mode de réalisation, la composition est caractérisée en ce que le radical hydrophobe répond à la formule VI et le ratio i entre le nombre de radicaux hydrophobes et le nombre d'unités glutamiques ou aspartiques est compris entre 0,02 et 0,08.

Dans un mode de réalisation, la composition est caractérisée en ce que le radical hydrophobe répond à la formule VI dans laquelle le radical Cx comprend entre 9 et 10 atomes de carbone et le ratio i entre le nombre de radicaux hydrophobes et le nombre d'unités glutamiques ou aspartiques est compris entre 0,03 et 0,15.

Dans un mode de réalisation, la composition est caractérisée en ce que le radical hydrophobe répond à la formule VI dans laquelle le radical Cx comprend entre 11 et 12 atomes de carbone et le ratio i entre le nombre de radicaux hydrophobes et le nombre d'unités glutamiques ou aspartiques est compris entre 0,015 et 0,1.

Dans un mode de réalisation, la composition est caractérisée en ce que le radical hydrophobe répond à la formule VI dans laquelle le radical Cx comprend entre 11 et 12 atomes de carbone et le ratio i entre le nombre de radicaux hydrophobes et le nombre d'unités glutamiques ou aspartiques est compris entre 0,02 et 0,08.

Dans un mode de réalisation, la composition est caractérisée en ce que le radical hydrophobe répond à la formule VI dans laquelle le radical Cx comprend entre 13 et 15 atomes de carbone et le ratio i entre le nombre de radicaux hydrophobes et le nombre d'unités glutamiques ou aspartiques est compris entre 0,01 et 0,1.

Dans un mode de réalisation, la composition est caractérisée en ce que le radical hydrophobe répond à la formule VI dans laquelle le radical Cx comprend entre 13 et 15 atomes de carbone et le ratio i entre le nombre de radicaux hydrophobes et le nombre d'unités glutamiques ou aspartiques est compris entre 0,01 et 0,06.

Dans un mode de réalisation, la composition est caractérisée en ce que le radical hydrophobe répond à la formule V et le ratio i entre le nombre de radicaux hydrophobes et le nombre d'unités glutamiques ou aspartiques est compris entre 0,007 et 0,3.

Dans un mode de réalisation, la composition est caractérisée en ce que le radical hydrophobe répond à la formule V et le ratio i entre le nombre de radicaux hydrophobes et le nombre d'unités glutamiques ou aspartiques est compris entre 0,01 et 0,3.

Dans un mode de réalisation, la composition est caractérisée en ce que le radical hydrophobe répond à la formule V et le ratio i entre le nombre de radicaux hydrophobes et le nombre d'unités glutamiques ou aspartiques est compris entre 0,015 et 0,2.

Dans un mode de réalisation, la composition est caractérisée en ce que le radical hydrophobe répond à la formule V dans laquelle le radical Cx comprend entre 11 et 14 atomes de carbone et le ratio i entre le nombre de radicaux hydrophobes et le nombre d'unités glutamiques ou aspartiques est compris entre 0,1 et 0,2.

Dans un mode de réalisation, la composition est caractérisée en ce que le radical hydrophobe répond à la formule V dans laquelle le radical Cx comprend entre 15 et 16 atomes de carbone et le ratio i entre le nombre de radicaux hydrophobes et le nombre d'unités glutamiques ou aspartiques est compris entre 0,04 et 0,15.

Dans un mode de réalisation, la composition est caractérisée en ce que le radical hydrophobe répond à la formule V dans laquelle le radical Cx comprend entre 17 et 18 atomes de carbone et le ratio i entre le nombre de radicaux hydrophobes et le nombre d'unités glutamiques ou aspartiques est compris entre 0,02 et 0,06.

Dans un mode de réalisation, la composition est caractérisée en ce que le radical hydrophobe répond à la formule V dans laquelle le radical Cx comprend entre 19 et 25 atomes de carbone et le ratio i entre le nombre de radicaux hydrophobes et le nombre d'unités glutamiques ou aspartiques est compris entre 0,01 et 0,06.

Dans un mode de réalisation, la composition est caractérisée en ce que le radical hydrophobe répond à la formule V dans laquelle le radical Cx comprend entre 19 et 25 atomes de carbone et le ratio i entre le nombre de radicaux hydrophobes et le nombre d'unités glutamiques ou aspartiques est compris entre 0,01 et 0,05.

Dans un mode de réalisation, la composition selon l'invention est caractérisée en ce que n + m est compris entre 10 et 250.

Dans un mode de réalisation, la composition selon l'invention est caractérisée en ce que n + m est compris entre 10 et 200.

Dans un mode de réalisation, la composition selon l'invention est caractérisée en ce que n + m est compris entre 15 et 150.

Dans un mode de réalisation, la composition selon l'invention est caractérisée en ce que n + m est compris entre 15 et 100.

Dans un mode de réalisation, la composition selon l'invention est caractérisée en ce que n + m est compris entre 15 et 80.

Dans un mode de réalisation, la composition selon l'invention est caractérisée en ce que n + m est compris entre 15 et 65.

Dans un mode de réalisation, la composition selon l'invention est caractérisée en ce que n + m est compris entre 20 et 60.

Dans un mode de réalisation, la composition selon l'invention est caractérisée en ce que n + m est compris entre 20 et 50.

Dans un mode de réalisation, la composition selon l'invention est caractérisée en ce que n + m est compris entre 20 et 40.

L'invention concerne également lesdits co-polyaminoacides porteurs de charges carboxylates et de radicaux hydrophobes de formule I et les précurseurs desdits radicaux hydrophobes.

Les co-polyaminoacides porteurs de charges carboxylates et de radicaux hydrophobes de formule X sont solubles dans l'eau distillée à un pH compris entre 6 et 8, à une température de 25 °C et à une concentration inférieure à 60 mg/ml.

Dans un mode de réalisation, l'invention concerne aussi les précurseurs desdits radicaux hydrophobes de formule X.

On entend par « soluble », susceptible de permettre de préparer une solution limpide et dépourvue de particules à une concentration inférieure à 100 mg/ml dans de l'eau distillée à 25°C.

On entend par « solution », une composition liquide dépourvue de particules visibles, en utilisant la procédure conforme aux pharmacopées européenne 8.0, au point 2.9.20, et américaine.

On entend par « composition stable physiquement », des compositions qui après une certaine durée de stockage à une certaine température satisfont aux critères de l'inspection visuelle décrite dans la pharmacopée européenne, américaine et internationale, c'est-à-dire des compositions qui sont limpides et qui ne contiennent pas de particules visibles, mais également incolores.

On entend par « composition stable chimiquement », des compositions qui, après stockage un certain temps et à une certaine température, présentent une recouvrance minimum des principes actifs et sont conformes aux cahiers des charges applicables aux produits pharmaceutiques.

On entend par « solution aqueuse injectable » des solutions à base d'eau qui répondent aux conditions des pharmacopées européenne et américaine, et qui sont suffisamment liquides pour être injectées.

On entend par « co-polyaminoacide étant constitué d'unités glutamiques ou aspartiques », des enchaînements linéaires non cycliques d'unités acide glutamique ou acide aspartique liées entre elles par des liaisons peptidiques, lesdits enchaînements présentant une partie C terminale, correspondant à l'acide carboxylique d'une extrémité, et une partie N-terminale, correspondant à l'amine de l'autre extrémité de l'enchainement.

On entend par « radical alkyl », une chaîne carbonée, linéaire ou ramifiée, qui ne comprend pas d'hétéroatome.

Le co-polyaminoacide est un co-polyaminoacide statistique ou bloc.

Le co-polyaminoacide est un co-polyaminoacide statistique dans l'enchaînement des unités glutamiques et/ou aspartiques.

Dans les formules les * indiquent les sites de rattachements des différents éléments représentés.

Dans un mode de réalisation, la composition selon l'invention est caractérisée en ce que le co-polyaminoacide est issu d'un polyaminoacide obtenu par polymérisation.

Dans un mode de réalisation, la composition selon l'invention est caractérisée en ce que le co-polyaminoacide est issu d'un polyaminoacide obtenu par polymérisation par ouverture de cycle d'un dérivé de N-carboxyanhydride d'acide glutamique ou d'un dérivé de N-carboxyanhydride d'acide aspartique.

Dans un mode de réalisation, la composition selon l'invention est caractérisée en ce que le co-polyaminoacide est issu d'un polyaminoacide obtenu par polymérisation d'un dérivé de N-carboxyanhydride d'acide glutamique ou d'un dérivé de N-carboxyanhydride d'acide aspartique comme décrit dans l'article Deming, T.J., Adv. Polym. Sci. 2006, 202, 1-18.

Dans un mode de réalisation, la composition selon l'invention est caractérisée en ce que le co-polyaminoacide est issu d'un polyaminoacide obtenu par polymérisation d'un dérivé de N-carboxyanhydride d'acide glutamique.

Dans un mode de réalisation, la composition selon l'invention est caractérisée en ce que le co-polyaminoacide est issu d'un polyaminoacide obtenu par polymérisation d'un dérivé de N-carboxyanhydride d'acide glutamique choisi dans le groupe constitué par le N-carboxyanhydride poly-glutamate de méthyle (GluOMe-NCA), le N-carboxyanhydride poly-glutamate de benzyle (GluOBzl-NCA) et le N-carboxyanhydride poly glutamate de t-butyle (GluOtBu-NCA).

Dans un mode de réalisation, le dérivé de N-carboxyanhydride d'acide glutamique est le N-carboxyanhydride poly-L-glutamate de méthyle (L-GluOMe-NCA).

Dans un mode de réalisation, le dérivé de N-carboxyanhydride d'acide glutamique est le N-carboxyanhydride poly-L-glutamate de benzyle (L-GluOBzl-NCA).

Dans un mode de réalisation, la composition selon l'invention est caractérisée en ce que le co-polyaminoacide est issu d'un polyaminoacide obtenu par polymérisation d'un dérivé de N-carboxyanhydride d'acide glutamique ou d'un dérivé de N-carboxyanhydride d'acide aspartique en utilisant comme initiateur un complexe organométallique d'un métal de transition comme décrit dans la publication Deming, T.J., Nature 1997, 390, 386-389.

Dans un mode de réalisation, la composition selon l'invention est caractérisée en ce que le co-polyaminoacide est issu d'un polyaminoacide obtenu par polymérisation d'un dérivé de N-carboxyanhydride d'acide glutamique ou d'un dérivé de N-carboxyanhydride d'acide aspartique en utilisant comme initiateur l'ammoniaque ou une amine primaire comme décrit dans le brevet FR 2,801,226 et les références citées par ce brevet.

Dans un mode de réalisation, la composition selon l'invention est caractérisée en ce que le co-polyaminoacide est issu d'un polyaminoacide obtenu par polymérisation d'un dérivé de N-carboxyanhydride d'acide glutamique ou d'un dérivé de N-carboxyanhydride d'acide aspartique en utilisant comme initiateur l'hexaméthyldisilazane comme décrit dans la publication Lu H. ; et al., J. Am. Chem. Soc. 2007, 129, 14114-14115 ou une amine silylée comme décrit dans la publication Lu H. ; et al., J. Am. Chem. Soc. 2008, 130, 12562-12563.

Dans un mode de réalisation, la composition selon l'invention est caractérisée en ce que le procédé de synthèse du polyaminoacide obtenu par polymérisation d'un dérivé de N-carboxyanhydride d'acide glutamique ou d'un dérivé de N-carboxyanhydride d'acide aspartique dont est issu le co-polyaminoacide comprend une étape d'hydrolyse de fonctions ester.

Dans un mode de réalisation, cette étape d'hydrolyse de fonctions ester peut consister en une hydrolyse en milieu acide ou une hydrolyse en milieu basique ou être effectuée par hydrogénation.

Dans un mode de réalisation, cette étape d'hydrolyse de groupements ester est une hydrolyse en milieu acide.

Dans un mode de réalisation, cette étape d'hydrolyse de groupements ester est effectuée par hydrogénation.

Dans un mode de réalisation, la composition selon l'invention est caractérisée en ce que le co-polyaminoacide est issu d'un polyaminoacide obtenu par dépolymérisation d'un polyaminoacide de plus haut poids moléculaire.

Dans un mode de réalisation, la composition selon l'invention est caractérisée en ce que le co-polyaminoacide est issu d'un polyaminoacide obtenu par dépolymérisation enzymatique d'un polyaminoacide de plus haut poids moléculaire.

Dans un mode de réalisation, la composition selon l'invention est caractérisée en ce que le co-polyaminoacide est issu d'un polyaminoacide obtenu par dépolymérisation chimique d'un polyaminoacide de plus haut poids moléculaire.

Dans un mode de réalisation, la composition selon l'invention est caractérisée en ce que le co-polyaminoacide est issu d'un polyaminoacide obtenu par dépolymérisation enzymatique et chimique d'un polyaminoacide de plus haut poids moléculaire.

Dans un mode de réalisation, la composition selon l'invention est caractérisée en ce que le co-polyaminoacide est issu d'un polyaminoacide obtenu par dépolymérisation d'un polyaminoacide de plus haut poids moléculaire choisi dans le groupe constitué par le polyglutamate de sodium et le polyaspartate de sodium.

Dans un mode de réalisation, la composition selon l'invention est caractérisée en ce que le co-polyaminoacide est issu d'un polyaminoacide obtenu par dépolymérisation d'un polyglutamate de sodium de plus haut poids moléculaire.

Dans un mode de réalisation, la composition selon l'invention est caractérisée en ce que le co-polyaminoacide est issu d'un polyaminoacide obtenu par dépolymérisation d'un polyaspartate de sodium de plus haut poids moléculaire.

Dans un mode de réalisation, la composition selon l'invention est caractérisée en ce que le co-polyaminoacide est obtenu par greffage d'un groupe hydrophobe sur un poly-L-glutamique acide ou poly-L-aspartique acide en utilisant les procédés de formation de liaison amide bien connus de l'homme de l'art.

Dans un mode de réalisation, la composition selon l'invention est caractérisée en ce que le co-polyaminoacide est obtenu par greffage d'un groupe hydrophobe sur un poly-L-glutamique acide ou poly-L-aspartique acide en utilisant les procédés de formation de liaison amide utilisés pour la synthèse peptidique.

Dans un mode de réalisation, la composition selon l'invention est caractérisée en ce que le co-polyaminoacide est obtenu par greffage d'un groupe hydrophobe sur un poly-L-glutamique acide ou poly-L-aspartique acide comme décrit dans le brevet FR 2,840,614.

L'amyline, ou « islet amyloid polypeptide » (IAPP), est une hormone peptidique à 37 résidus. Elle est co-sécrétée avec de l'insuline à partir des cellules béta pancréatiques dans le rapport d'environ 100/1. L'amyline joue un rôle dans la régulation glycémique en stoppant la sécrétion du glucagon endogène et en ralentissant la vidange gastrique et en favorisant la satiété, réduisant ainsi les excursions glycémiques postprandiales dans les niveaux de glucose sanguin.

L'IAPP est traité à partir d'une séquence codante de 89 résidus. Le pro-islet polypeptide amyloïde (proIAPP, proamyline, protéine proislet) est produit dans les cellules bêta pancréatiques (cellules béta) sous la forme d'un pro-peptide de 67 acides aminés, 7404 Dalton, et subit des modifications post-traductionnelles comprenant le clivage de protéase pour produire de l'amyline.

Dans la présente demande, l'amyline telle que mentionnée fait référence aux composés décrits dans les brevets US 5,124,314 et US 5,234,906.

On entend par « analogue », lorsqu'il est utilisé par référence à un peptide ou une protéine, un peptide ou une protéine, dans lequel un ou plusieurs résidus d'acides aminés constitutifs de la séquence primaire ont été substitués par d'autres résidus d'acides aminés et / ou dans lequel un ou plusieurs résidus d'acides aminés constitutifs ont été supprimés et / ou dans lequel un ou plusieurs résidus d'acides aminés constitutifs ont été ajoutés. Le pourcentage d'homologie admis pour la présente définition d'un analogue est de 50 %. Dans le cas de l'amyline, un analogue peut être par exemple dérivé de la séquence d'acide aminé primaire de l'amyline en substituant un ou plusieurs acides aminés naturels ou non naturels ou peptidomimétiques.

On entend par « dérivé », lorsqu'il est utilisé par référence à un peptide ou une protéine, un peptide ou une protéine ou un analogue chimiquement modifié par un substituant qui n'est pas présent dans le peptide ou la protéine ou l'analogue de référence, c'est-à-dire un peptide ou une protéine qui a été modifié par création de liaisons covalentes, pour introduire des substituants de type non amino-acide.

Un agoniste au récepteur de l'amyline fait référence à un composé qui imite une ou plusieurs caractéristiques de l'activité de l'amyline.

Des dérivés d'amyline sont décrits dans l'article Yan et al., PNAS, vol. 103, no 7, p 2046-2051, 2006.

Dans un mode de réalisation, le substituant est choisi dans le groupe constitué des chaînes grasses.

Des analogues d'amyline sont décrits dans les brevets US 5,686,411, US 6,114,304 ou bien encore US 6,410,511.

Dans un mode de réalisation, la composition est caractérisée en ce que l'analogue d'amyline est le pramlintide (Symlin^{®}) commercialisé par la société ASTRAZENECA AB.

Dans un mode de réalisation, les ratios molaires co-polyaminoacide / amyline, agoniste au récepteur de l'amyline ou analogue d'amyline sont compris entre 1,5 et 75.

Dans un mode de réalisation, les ratios molaires co-polyaminoacide / amyline, agoniste au récepteur de l'amyline ou analogue d'amyline sont compris entre 1,8 et 50.

Dans un mode de réalisation, les ratios molaires co-polyaminoacide / amyline, agoniste au récepteur de l'amyline ou analogue d'amyline sont compris entre 2 et 35.

Dans un mode de réalisation, les ratios molaires co-polyaminoacide / amyline, agoniste au récepteur de l'amyline ou analogue d'amyline sont compris entre 2,5 et 30.

Dans un mode de réalisation, les ratios molaires co-polyaminoacide / amyline, agoniste au récepteur de l'amyline ou analogue d'amyline sont compris entre 3 et 30.

Dans un mode de réalisation, les ratios molaires co-polyaminoacide / amyline, agoniste au récepteur de l'amyline ou analogue d'amyline sont compris entre 3,5 et 30.

Dans un mode de réalisation, les ratios molaires co-polyaminoacide / amyline, agoniste au récepteur de l'amyline ou analogue d'amyline sont compris entre 4 et 30.

Dans un mode de réalisation, les ratios molaires co-polyaminoacide / amyline, agoniste au récepteur de l'amyline ou analogue d'amyline sont compris entre 5 et 30.

Dans un mode de réalisation, les ratios molaires co-polyaminoacide / amyline, agoniste au récepteur de l'amyline ou analogue d'amyline sont compris entre 7 et 30.

Dans un mode de réalisation, les ratios molaires co-polyaminoacide / amyline, agoniste au récepteur de l'amyline ou analogue d'amyline sont compris entre 9 et 30.

Dans un mode de réalisation, les ratios molaires co-polyaminoacide / amyline sont compris entre 3 et 75.

Dans un mode de réalisation, les ratios molaires co-polyaminoacide / amyline sont compris entre 7 et 50.

Dans un mode de réalisation, les ratios molaires co-polyaminoacide / amyline sont compris entre 10 et 30.

Dans un mode de réalisation, les ratios molaires co-polyaminoacide / amyline sont compris entre 15 et 30.

Dans un mode de réalisation, les ratios molaires co-polyaminoacide / pramlintide sont compris entre 1,5 et 75.

Dans un mode de réalisation, les ratios molaires co-polyaminoacide / pramlintide sont compris entre 2 et 50.

Dans un mode de réalisation, les ratios molaires co-polyaminoacide / pramlintide sont compris entre 3 et 30.

Dans un mode de réalisation, les ratios molaires co-polyaminoacide / pramlintide sont compris entre 4 et 30.

Dans un mode de réalisation, les ratios molaires co-polyaminoacide / pramlintide sont compris entre 5 et 30.

Dans un mode de réalisation, les ratios molaires co-polyaminoacide / pramlintide sont compris entre 8 et 30.

Dans un mode de réalisation, les ratios molaires co-polyaminoacide / pramlintide sont compris entre 10 et 30.

Dans un mode de réalisation, les ratios molaires radical hydrophobe Hy / amyline, agoniste au récepteur de l'amyline ou analogue d'amyline sont compris entre 1,5 et 150.

Dans un mode de réalisation, les ratios molaires radical hydrophobe Hy / amyline, agoniste au récepteur de l'amyline ou analogue d'amyline sont compris entre 1,8 et 100.

Dans un mode de réalisation, les ratios molaires radical hydrophobe Hy / amyline, agoniste au récepteur de l'amyline ou analogue d'amyline sont compris entre 2 et 70.

Dans un mode de réalisation, les ratios molaires radical hydrophobe Hy / amyline, agoniste au récepteur de l'amyline ou analogue d'amyline sont compris entre 2,5 et 60.

Dans un mode de réalisation, les ratios molaires radical hydrophobe Hy / amyline, agoniste au récepteur de l'amyline ou analogue d'amyline sont compris entre 3 et 60.

Dans un mode de réalisation, les ratios molaires radical hydrophobe Hy / amyline, agoniste au récepteur de l'amyline ou analogue d'amyline sont compris entre 3,5 et 60.

Dans un mode de réalisation, les ratios molaires radical hydrophobe Hy / amyline, agoniste au récepteur de l'amyline ou analogue d'amyline sont compris entre 4 et 60.

Dans un mode de réalisation, les ratios molaires radical hydrophobe Hy / amyline, agoniste au récepteur de l'amyline ou analogue d'amyline sont compris entre 5 et 60.

Dans un mode de réalisation, les ratios molaires radical hydrophobe Hy / amyline, agoniste au récepteur de l'amyline ou analogue d'amyline sont compris entre 7 et 60.

Dans un mode de réalisation, les ratios molaires radical hydrophobe Hy / amyline, agoniste au récepteur de l'amyline ou analogue d'amyline sont compris entre 9 et 60.

Dans un mode de réalisation, les ratios molaires radical hydrophobe Hy / amyline sont compris entre 5 et 60.

Dans un mode de réalisation, les ratios molaires radical hydrophobe Hy / amyline sont compris entre 10 et 60.

Dans un mode de réalisation, les ratios molaires radical hydrophobe Hy / amyline sont compris entre 15 et 60.

Dans un mode de réalisation, les ratios molaires radical hydrophobe Hy / pramlintide sont compris entre 1,5 et 60.

Dans un mode de réalisation, les ratios molaires radical hydrophobe Hy / pramlintide sont compris entre 2 et 60.

Dans un mode de réalisation, les ratios molaires radical hydrophobe Hy / pramlintide sont compris entre 3 et 60.

Dans un mode de réalisation, les ratios molaires radical hydrophobe Hy / pramlintide sont compris entre 4 et 60.

Dans un mode de réalisation, les ratios molaires radical hydrophobe Hy / pramlintide sont compris entre 5 et 60.

Dans un mode de réalisation, les ratios molaires radical hydrophobe Hy / pramlintide sont compris entre 8 et 60.

Dans un mode de réalisation, les ratios molaires radical hydrophobe Hy / pramlintide sont compris entre 10 et 60.

Dans un mode de réalisation, les ratios massiques co-polyaminoacide / amyline, agoniste au récepteur de l'amyline ou analogue d'amyline sont compris entre 1,0 et 70.

Dans un mode de réalisation, les ratios massiques co-polyaminoacide / amyline, agoniste au récepteur de l'amyline ou analogue d'amyline sont compris entre 1,2 et 45.

Dans un mode de réalisation, les ratios massiques co-polyaminoacide / amyline, agoniste au récepteur de l'amyline ou analogue d'amyline sont compris entre 1,3 et 30.

Dans un mode de réalisation, les ratios massiques co-polyaminoacide / amyline, agoniste au récepteur de l'amyline ou analogue d'amyline sont compris entre 1,7 et 27.

Dans un mode de réalisation, les ratios massiques co-polyaminoacide / amyline, agoniste au récepteur de l'amyline ou analogue d'amyline sont compris entre 2,0 et 27.

Dans un mode de réalisation, les ratios massiques co-polyaminoacide / amyline, agoniste au récepteur de l'amyline ou analogue d'amyline sont compris entre 2,3 et 27.

Dans un mode de réalisation, les ratios massiques co-polyaminoacide / amyline, agoniste au récepteur de l'amyline ou analogue d'amyline sont compris entre 2,7 et 27.

Dans un mode de réalisation, les ratios massiques co-polyaminoacide / amyline, agoniste au récepteur de l'amyline ou analogue d'amyline sont compris entre 3,3 et 27.

Dans un mode de réalisation, les ratios massiques co-polyaminoacide / amyline, agoniste au récepteur de l'amyline ou analogue d'amyline sont compris entre 4,7 et 27.

Dans un mode de réalisation, les ratios massiques co-polyaminoacide / amyline, agoniste au récepteur de l'amyline ou analogue d'amyline sont compris entre 6,0 et 27.

Dans un mode de réalisation, les ratios massiques co-polyaminoacide / amyline sont compris entre 2,0 et 67.

Dans un mode de réalisation, les ratios massiques co-polyaminoacide / amyline sont compris entre 4,7 et 27.

Dans un mode de réalisation, les ratios massiques co-polyaminoacide / amyline sont compris entre 6,7 et 27.

Dans un mode de réalisation, les ratios massiques co-polyaminoacide / amyline sont compris entre 10 et 27.

Dans un mode de réalisation, les ratios massiques co-polyaminoacide / pramlintide sont compris entre 1,0 et 67.

Dans un mode de réalisation, les ratios massiques co-polyaminoacide / pramlintide sont compris entre 1,3 et 45.

Dans un mode de réalisation, les ratios massiques co-polyaminoacide / pramlintide sont compris entre 2,7 et 27.

Dans un mode de réalisation, les ratios massiques co-polyaminoacide / pramlintide sont compris entre 3,3 et 27.

Dans un mode de réalisation, les ratios massiques co-polyaminoacide / pramlintide sont compris entre 5,3 et 27.

Dans un mode de réalisation, les ratios massiques co-polyaminoacide / pramlintide sont compris entre 6,7 et 27.

Dans un mode de réalisation, la composition est caractérisée en ce qu'elle comprend en outre de l'insuline.

Dans un mode de réalisation, la composition est caractérisée en ce que l'insuline est une insuline prandiale. Les insulines prandiales sont solubles à pH 7.

On entend par insuline prandiale une insuline dite rapide ou « regular ».

Les insulines prandiales dites rapides sont des insulines qui doivent répondre aux besoins provoqués par l'ingestion des protéines et des glucides durant un repas, elles doivent agir en moins de 30 minutes.

Dans un mode de réalisation, l'insuline prandiale dite « regular » est de l'insuline humaine.

Dans un mode de réalisation, l'insuline prandiale est une insuline humaine recombinante telle que décrite dans la pharmacopée européenne et la pharmacopée américaine.

L'insuline humaine est par exemple commercialisée sous les marques Humulin^{®} (ELI LILLY) et Novolin^{®} (NOVO NORDISK).

Les insulines prandiales dites rapides (fast acting) sont des insulines qui sont obtenues par recombinaison et dont la séquence primaire a été modifiée pour diminuer leur temps d'action.

Dans un mode de réalisation, les insulines prandiales dites rapides (fast acting) sont choisies dans le groupe comprenant l'insuline lispro (Humalog^{®}), l'insuline glulisine (Apidra^{®}) et l'insuline aspart (NovoLog^{®}).

Dans un mode de réalisation, l'insuline prandiale est l'insuline lispro.

Dans un mode de réalisation, l'insuline prandiale est l'insuline glulisine.

Dans un mode de réalisation, l'insuline prandiale est l'insuline aspart.

Les unités recommandées par les pharmacopées pour les insulines sont présentées dans le tableau 51 ci-après avec leurs correspondances en mg :

**Tableau 51 : Unités recommandées par les pharmacopées pour les insulines**

| **Insuline** | **Pharmacopée EP 8.0 (2014)** | **Pharmacopée US** - **USP38 (2015)** |
|---|---|---|
| Aspart | 1U = 0,0350 mg d'insuline aspart | 1 USP = 0,0350 mg insulin aspart |
| Lispro | 1U = 0.0347 mg d'insuline lispro | 1 USP = 0,0347 mg d'insuline lispro |
| Humaine | 1UI = 0,0347 mg d'insuline humaine | 1 USP = 0,0347 mg d'insuline humaine |

Dans le cas de l'insuline glulisine, 100U = 3,49 mg d'insuline glulisine (d'après "Annex 1 - Summary of product characteristics" relative à Adipra^{®}).

Néanmoins dans la suite du texte U est systématiquement utilisé indifféremment pour les quantités et les concentrations en toutes les insulines. Les valeurs respectives correspondant en mg sont celles données ci-dessus pour des valeurs exprimées en U, UI ou USP.

Dans un mode de réalisation, elle concerne une formulation pharmaceutique caractérisée en ce que la concentration en insuline est comprise entre 240 et 3000 µM (40 à 500 U/mL).

Dans un mode de réalisation, elle concerne une formulation pharmaceutique caractérisée en ce que la concentration en insuline est comprise entre 600 et 3000 µM (100 à 500 U/mL).

Dans un mode de réalisation, elle concerne une formulation pharmaceutique caractérisée en ce que la concentration en insuline est comprise entre 600 et 2400 µM (100 à 400 U/mL).

Dans un mode de réalisation, elle concerne une formulation pharmaceutique caractérisée en ce que la concentration en insuline est comprise entre 600 et 1800 µM (100 à 300 U/mL).

Dans un mode de réalisation, elle concerne une formulation pharmaceutique caractérisée en ce que la concentration en insuline est comprise entre 600 et 1200 µM (100 à 200 U/mL).

Dans un mode de réalisation, elle concerne une formulation pharmaceutique caractérisée en ce que la concentration en insuline est 600 µM (100 U/mL).

Dans un mode de réalisation, elle concerne une formulation pharmaceutique caractérisée en ce que la concentration en insuline est 1200 µM (200 U/mL).

Dans un mode de réalisation, elle concerne une formulation pharmaceutique caractérisée en ce que la concentration en insuline est 1800 µM (300 U/mL).

Dans un mode de réalisation, elle concerne une formulation pharmaceutique caractérisée en ce que la concentration en insuline est 2400 µM (400 U/mL).

Dans un mode de réalisation, elle concerne une formulation pharmaceutique caractérisée en ce que la concentration en insuline est 3000 µM (500 U/mL).

Dans un mode de réalisation comprenant de l'insuline prandiale, les ratios molaires co-polyaminoacide / amyline, agoniste au récepteur de l'amyline ou analogue d'amyline sont compris entre 1,5 et 75.

Dans un mode de réalisation comprenant de l'insuline prandiale, les ratios molaires co-polyaminoacide / amyline, agoniste au récepteur de l'amyline ou analogue d'amyline sont compris entre 1,8 et 50.

Dans un mode de réalisation comprenant de l'insuline prandiale, les ratios molaires co-polyaminoacide / amyline, agoniste au récepteur de l'amyline ou analogue d'amyline sont compris entre 2 et 35.

Dans un mode de réalisation comprenant de l'insuline prandiale, les ratios molaires co-polyaminoacide / amyline, agoniste au récepteur de l'amyline ou analogue d'amyline sont compris entre 2,5 et 30.

Dans un mode de réalisation comprenant de l'insuline prandiale, les ratios molaires co-polyaminoacide / amyline, agoniste au récepteur de l'amyline ou analogue d'amyline sont compris entre 3 et 30.

Dans un mode de réalisation comprenant de l'insuline prandiale, les ratios molaires co-polyaminoacide / amyline, agoniste au récepteur de l'amyline ou analogue d'amyline sont compris entre 3,5 et 30.

Dans un mode de réalisation comprenant de l'insuline prandiale, les ratios molaires co-polyaminoacide / amyline, agoniste au récepteur de l'amyline ou analogue d'amyline sont compris entre 4 et 30.

Dans un mode de réalisation comprenant de l'insuline prandiale, les ratios molaires co-polyaminoacide / amyline, agoniste au récepteur de l'amyline ou analogue d'amyline sont compris entre 5 et 30.

Dans un mode de réalisation comprenant de l'insuline prandiale, les ratios molaires co-polyaminoacide / amyline, agoniste au récepteur de l'amyline ou analogue d'amyline sont compris entre 7 et 30.

Dans un mode de réalisation comprenant de l'insuline prandiale, les ratios molaires co-polyaminoacide / amyline, agoniste au récepteur de l'amyline ou analogue d'amyline sont compris entre 9 et 30.

Dans un mode de réalisation comprenant de l'insuline prandiale, les ratios molaires co-polyaminoacide / amyline sont compris entre 5 et 75.

Dans un mode de réalisation comprenant de l'insuline prandiale, les ratios molaires co-polyaminoacide / amyline sont compris entre 10 et 50.

Dans un mode de réalisation comprenant de l'insuline prandiale, les ratios molaires co-polyaminoacide / amyline sont compris entre 15 et 30.

Dans un mode de réalisation comprenant de l'insuline prandiale, les ratios molaires co-polyaminoacide / pramlintide sont compris entre 1,5 et 75.

Dans un mode de réalisation comprenant de l'insuline prandiale, les ratios molaires co-polyaminoacide / pramlintide sont compris entre 2 et 50.

Dans un mode de réalisation comprenant de l'insuline prandiale, les ratios molaires co-polyaminoacide / pramlintide sont compris entre 3 et 30.

Dans un mode de réalisation comprenant de l'insuline prandiale, les ratios molaires co-polyaminoacide / pramlintide sont compris entre 4 et 30.

Dans un mode de réalisation comprenant de l'insuline prandiale, les ratios molaires co-polyaminoacide / pramlintide sont compris entre 5 et 30.

Dans un mode de réalisation comprenant de l'insuline prandiale, les ratios molaires co-polyaminoacide / pramlintide sont compris entre 8 et 30.

Dans un mode de réalisation comprenant de l'insuline prandiale, les ratios molaires co-polyaminoacide / pramlintide sont compris entre 10 et 30.

Dans un mode de réalisation comprenant de l'insuline prandiale, les ratios molaires radical hydrophobe Hy / amyline, agoniste au récepteur de l'amyline ou analogue d'amyline sont compris entre 1,5 et 150.

Dans un mode de réalisation comprenant de l'insuline prandiale, les ratios molaires radical hydrophobe Hy / amyline, agoniste au récepteur de l'amyline ou analogue d'amyline sont compris entre 1,8 et 100.

Dans un mode de réalisation comprenant de l'insuline prandiale, les ratios molaires radical hydrophobe Hy / amyline, agoniste au récepteur de l'amyline ou analogue d'amyline sont compris entre 2 et 70.

Dans un mode de réalisation comprenant de l'insuline prandiale, les ratios molaires radical hydrophobe Hy / amyline, agoniste au récepteur de l'amyline ou analogue d'amyline sont compris entre 2,5 et 60.

Dans un mode de réalisation comprenant de l'insuline prandiale, les ratios molaires radical hydrophobe Hy / amyline, agoniste au récepteur de l'amyline ou analogue d'amyline sont compris entre 3 et 60.

Dans un mode de réalisation comprenant de l'insuline prandiale, les ratios molaires radical hydrophobe Hy / amyline, agoniste au récepteur de l'amyline ou analogue d'amyline sont compris entre 3,5 et 60.

Dans un mode de réalisation comprenant de l'insuline prandiale, les ratios molaires radical hydrophobe Hy / amyline, agoniste au récepteur de l'amyline ou analogue d'amyline sont compris entre 4 et 60.

Dans un mode de réalisation comprenant de l'insuline prandiale, les ratios molaires radical hydrophobe Hy / amyline, agoniste au récepteur de l'amyline ou analogue d'amyline sont compris entre 5 et 60.

Dans un mode de réalisation comprenant de l'insuline prandiale, les ratios molaires radical hydrophobe Hy / amyline, agoniste au récepteur de l'amyline ou analogue d'amyline sont compris entre 7 et 60.

Dans un mode de réalisation comprenant de l'insuline prandiale, les ratios molaires radical hydrophobe Hy / amyline, agoniste au récepteur de l'amyline ou analogue d'amyline sont compris entre 9 et 60.

Dans un mode de réalisation comprenant de l'insuline prandiale, les ratios molaires radical hydrophobe Hy / amyline sont compris entre 5 et 60.

Dans un mode de réalisation comprenant de l'insuline prandiale, les ratios molaires radical hydrophobe Hy / amyline sont compris entre 10 et 60.

Dans un mode de réalisation comprenant de l'insuline prandiale, les ratios molaires radical hydrophobe Hy / amyline sont compris entre 15 et 60.

Dans un mode de réalisation comprenant de l'insuline prandiale, les ratios molaires radical hydrophobe Hy / pramlintide sont compris entre 1,5 et 60.

Dans un mode de réalisation comprenant de l'insuline prandiale, les ratios molaires radical hydrophobe Hy / pramlintide sont compris entre 2 et 60.

Dans un mode de réalisation comprenant de l'insuline prandiale, les ratios molaires radical hydrophobe Hy / pramlintide sont compris entre 3 et 60.

Dans un mode de réalisation comprenant de l'insuline prandiale, les ratios molaires radical hydrophobe Hy / pramlintide sont compris entre 4 et 60.

Dans un mode de réalisation comprenant de l'insuline prandiale, les ratios molaires radical hydrophobe Hy / pramlintide sont compris entre 5 et 60.

Dans un mode de réalisation comprenant de l'insuline prandiale, les ratios molaires radical hydrophobe Hy / pramlintide sont compris entre 8 et 60.

Dans un mode de réalisation comprenant de l'insuline prandiale, les ratios molaires radical hydrophobe Hy / pramlintide sont compris entre 10 et 60.

Dans un mode de réalisation comprenant de l'insuline prandiale, les ratios massiques co-polyaminoacide / amyline, agoniste au récepteur de l'amyline ou analogue d'amyline sont compris entre 1,0 et 70.

Dans un mode de réalisation comprenant de l'insuline prandiale, les ratios massiques co-polyaminoacide / amyline, agoniste au récepteur de l'amyline ou analogue d'amyline sont compris entre 1,2 et 45.

Dans un mode de réalisation comprenant de l'insuline prandiale, les ratios massiques co-polyaminoacide / amyline, agoniste au récepteur de l'amyline ou analogue d'amyline sont compris entre 1,3 et 30.

Dans un mode de réalisation comprenant de l'insuline prandiale, les ratios massiques co-polyaminoacide / amyline, agoniste au récepteur de l'amyline ou analogue d'amyline sont compris entre 1,7 et 27.

Dans un mode de réalisation comprenant de l'insuline prandiale, les ratios massiques co-polyaminoacide / amyline, agoniste au récepteur de l'amyline ou analogue d'amyline sont compris entre 2,0 et 27.

Dans un mode de réalisation comprenant de l'insuline prandiale, les ratios massiques co-polyaminoacide / amyline, agoniste au récepteur de l'amyline ou analogue d'amyline sont compris entre 2,3 et 27.

Dans un mode de réalisation comprenant de l'insuline prandiale, les ratios massiques co-polyaminoacide / amyline, agoniste au récepteur de l'amyline ou analogue d'amyline sont compris entre 2,7 et 27.

Dans un mode de réalisation comprenant de l'insuline prandiale, les ratios massiques co-polyaminoacide / amyline, agoniste au récepteur de l'amyline ou analogue d'amyline sont compris entre 3,3 et 27.

Dans un mode de réalisation comprenant de l'insuline prandiale, les ratios massiques co-polyaminoacide / amyline, agoniste au récepteur de l'amyline ou analogue d'amyline sont compris entre 4,7 et 27.

Dans un mode de réalisation comprenant de l'insuline prandiale, les ratios massiques co-polyaminoacide / amyline, agoniste au récepteur de l'amyline ou analogue d'amyline sont compris entre 6,0 et 27.

Dans un mode de réalisation comprenant de l'insuline prandiale, les ratios massiques co-polyaminoacide / amyline sont compris entre 3,3 et 67.

Dans un mode de réalisation comprenant de l'insuline prandiale, les ratios massiques co-polyaminoacide / amyline sont compris entre 6,6 et 27.

Dans un mode de réalisation comprenant de l'insuline prandiale, les ratios massiques co-polyaminoacide / amyline sont compris entre 10 et 27.

Dans un mode de réalisation comprenant de l'insuline prandiale, les ratios massiques co-polyaminoacide / pramlintide sont compris entre 1,0 et 67.

Dans un mode de réalisation comprenant de l'insuline prandiale, les ratios massiques co-polyaminoacide / pramlintide sont compris entre 1,2 et 45.

Dans un mode de réalisation comprenant de l'insuline prandiale, les ratios massiques co-polyaminoacide / pramlintide sont compris entre 1,3 et 27.

Dans un mode de réalisation comprenant de l'insuline prandiale, les ratios massiques co-polyaminoacide / pramlintide sont compris entre 1,7 et 27.

Dans un mode de réalisation comprenant de l'insuline prandiale, les ratios massiques co-polyaminoacide / pramlintide sont compris entre 2,0 et 27.

Dans un mode de réalisation comprenant de l'insuline prandiale, les ratios massiques co-polyaminoacide / pramlintide sont compris entre 2,3 et 27.

Dans un mode de réalisation comprenant de l'insuline prandiale, les ratios massiques co-polyaminoacide / pramlintide sont compris entre 2,7 et 27.

Dans un mode de réalisation comprenant de l'insuline prandiale, les ratios massiques co-polyaminoacide / pramlintide sont compris entre 3,3 et 27.

Dans un mode de réalisation comprenant de l'insuline prandiale, les ratios massiques co-polyaminoacide / pramlintide sont compris entre 4,7 et 27.

Dans un mode de réalisation comprenant de l'insuline prandiale, les ratios massiques co-polyaminoacide / pramlintide sont compris entre 6,0 et 27.

Par ailleurs, il est particulièrement intéressant de combiner l'amyline, un agoniste au récepteur de l'amyline ou un analogue d'amyline, en combinaison ou non avec une insuline prandiale, avec des GLP-1, des analogues de GLP-1, des agonistes de récepteurs au GLP-1, ceux-ci sont couramment appelés GLP-1 RA. Cela permet notamment de potentialiser l'effet de l'insuline et est recommandé dans certains types de traitement du diabète.

Dans un mode de réalisation, les GLP-1, analogues de GLP-1, ou GLP-1 RA sont dits « rapides ».

On entend par « rapide », des GLP-1, analogues de GLP-1, ou GLP-1 RA, dont la demi-vie apparente d'élimination après injection sous-cutanée chez l'homme est inférieure à 8 h, en particulier inférieure à 5 h, de préférence inférieure à 4 h ou bien encore inférieure à 3 h, comme par exemple l'exenatide et le lixisenatide.

Dans un mode de réalisation, les GLP-1, les analogues de GLP-1, ou les GLP-1 RA sont choisis dans le groupe constitué par l'exenatide ou Byetta^{®}(ASTRAZENECA), le lixisenatide ou Lyxumia^{®} (SANOFI), leurs analogues ou dérivés et leurs sels pharmaceutiquement acceptables.

Dans un mode de réalisation, le GLP-1, analogue de GLP-1, ou GLP-1 RA est l'exenatide ou Byetta^{®}, ses analogues ou dérivés et leurs sels pharmaceutiquement acceptables.

Dans un mode de réalisation, GLP-1, analogue de GLP-1, ou GLP-1 RA est le lixisenatide ou Lyxumia^{®}, ses analogues ou dérivés et leurs sels pharmaceutiquement acceptables.

Dans un mode de réalisation, la concentration en exenatide, ses analogues ou dérivés et leurs sels pharmaceutiquement acceptables est comprise dans un intervalle de 0,01 à 1,0 mg pour 100 U d'insuline.

Dans un mode réalisation, la concentration en exenatide, ses analogues ou dérivés et leurs sels pharmaceutiquement acceptables est de 0,01 à 0,5 mg pour 100 U d'insuline.

Dans un mode réalisation, la concentration en exenatide, ses analogues ou dérivés et leurs sels pharmaceutiquement acceptables est de 0,02 à 0,4 mg pour 100 U d'insuline.

Dans un mode réalisation, la concentration en exenatide, ses analogues ou dérivés et leurs sels pharmaceutiquement acceptables est de 0,03 à 0,3 mg pour 100 U d'insuline.

Dans un mode réalisation, la concentration en exenatide, ses analogues ou dérivés et leurs sels pharmaceutiquement acceptables est de 0,04 à 0,2 mg pour 100 U d'insuline.

Dans un mode réalisation, la concentration en exenatide, ses analogues ou dérivés et leurs sels pharmaceutiquement acceptables est de 0,04 à 0,15 mg pour 100 U d'insuline.

Dans un mode de réalisation, la concentration en lixisenatide, ses analogues ou dérivés et leurs sels pharmaceutiquement acceptables est comprise dans un intervalle de 0,01 à 1 mg pour 100 U d'insuline.

Dans un mode réalisation, la concentration en lixisenatide, ses analogues ou dérivés et leurs sels pharmaceutiquement acceptables est comprise dans un intervalle de 0,01 à 0,5 mg pour 100 U d'insuline.

Dans un mode réalisation, la concentration en lixisenatide, ses analogues ou dérivés et leurs sels pharmaceutiquement acceptables est de 0,02 à 0,4 mg pour 100 U d'insuline.

Dans un mode réalisation, la concentration en lixisenatide, ses analogues ou dérivés et leurs sels pharmaceutiquement acceptables est de 0,03 à 0,3 mg pour 100 U d'insuline.

Dans un mode réalisation, la concentration en lixisenatide, ses analogues ou dérivés et leurs sels pharmaceutiquement acceptables est de 0,04 à 0,2 mg pour 100 U d'insuline.

Dans un mode réalisation, la concentration en lixisenatide, ses analogues ou dérivés et leurs sels pharmaceutiquement acceptables est de de 0,04 à 0,15 mg pour 100 U d'insuline.

Dans un mode de réalisation, les compositions selon l'invention sont réalisées par mélange de solutions d'amyline et de solutions commerciales de GLP-1, d'analogue de GLP-1 ou de d'agoniste de récepteurs de GLP-1 RA en ratios volumiques compris dans un intervalle de 10/90 à 90/10 en présence d'un co-polyaminoacide.

Dans un mode de réalisation, la composition comprend en outre des sels de zinc.

Dans un mode de réalisation, la concentration en sels de zinc est comprise entre 0 et 5000 µM.

Dans un mode de réalisation, la concentration en sels de zinc est comprise entre 0 et 4000 µM.

Dans un mode de réalisation, la concentration en sels de zinc est comprise entre 0 et 3000 µM.

Dans un mode de réalisation, la concentration en sels de zinc est comprise entre 0 et 2000 µM.

Dans un mode de réalisation, la concentration en sels de zinc est comprise entre 0 et 1000 µM.

Dans un mode de réalisation, la concentration en sels de zinc est comprise entre 50 et 600 µM.

Dans un mode de réalisation, la concentration en sels de zinc est comprise entre 100 et 500 µM.

Dans un mode de réalisation, la concentration en sels de zinc est comprise entre 200 et 500 µM.

Dans un mode de réalisation, le sel de zinc est le chlorure de zinc.

Dans un mode de réalisation, les compositions selon l'invention comprennent en outre des sels de zinc à une concentration comprise entre 0 et 500 µM pour 100 U d'insuline.

Dans un mode de réalisation, les compositions selon l'invention comprennent en outre des sels de zinc à une concentration comprise entre 0 et 400 µM pour 100 U d'insuline.

Dans un mode de réalisation, les compositions selon l'invention comprennent en outre des sels de zinc à une concentration comprise entre 0 et 300 µM pour 100 U d'insuline.

Dans un mode de réalisation, les compositions selon l'invention comprennent en outre des sels de zinc à une concentration comprise entre 0 et 200 µM pour 100 U d'insuline.

Dans un mode de réalisation, les compositions selon l'invention comprennent en outre des sels de zinc à une concentration comprise entre 0 et 100 µM pour 100 U d'insuline.

Dans un mode de réalisation, les compositions selon l'invention comprennent en outre des tampons.

Dans un mode de réalisation, les compositions selon l'invention comprennent des tampons à des concentrations comprises entre 0 et 100 mM.

Dans un mode de réalisation, les compositions selon l'invention comprennent des tampons à des concentrations comprises entre 15 et 50 mM.

Dans un mode de réalisation, les compositions selon l'invention comprennent un tampon choisi dans le groupe constitué par un tampon phosphate, le Tris (trishydroxyméthylaminométhane) et le citrate de sodium.

Dans un mode de réalisation, le tampon est le phosphate de sodium.

Dans un mode de réalisation, le tampon est le Tris (trishydroxyméthylaminométhane).

Dans un mode de réalisation, le tampon est le citrate de sodium.

Dans un mode de réalisation, les compositions selon l'invention comprennent en outre des conservateurs.

Dans un mode de réalisation, les conservateurs sont choisis dans le groupe constitué par le m-crésol et le phénol, seuls ou en mélange.

Dans un mode de réalisation, la concentration des conservateurs est comprise entre 10 et 50 mM.

Dans un mode de réalisation, la concentration des conservateurs est comprise entre 10 et 40 mM.

Dans un mode de réalisation, les compositions selon l'invention comprennent en outre un tensioactif.

Dans un mode de réalisation, le tensioactif est choisi dans le groupe constitué par le propylène glycol et le polysorbate.

Les compositions selon l'invention peuvent en outre comprendre des additifs tels que des agents de tonicité.

Dans un mode de réalisation, les agents de tonicité sont choisis dans le groupe constitué par la glycérine, le chlorure de sodium, le mannitol et la glycine.

Les compositions selon l'invention peuvent comprendre en outre tous les excipients conformes aux pharmacopées et compatibles avec les insulines utilisées aux concentrations d'usage.

L'invention concerne également une formulation pharmaceutique selon l'invention, caractérisée en ce qu'elle est obtenue par séchage et/ou lyophilisation.

Dans le cas des libérations locale et systémique, les modes d'administration envisagés sont par voie intraveineuse, sous-cutanée, intradermique ou intramusculaire.

Les voies d'administration transdermique, orale, nasale, vaginale, oculaire, buccale, pulmonaire sont également envisagées.

L'invention concerne également une pompe, implantable ou transportable, comprenant une composition selon l'invention.

L'invention concerne encore l'utilisation d'une composition selon l'invention destinée à être placée dans une pompe, implantable ou transportable.

Dans un mode de réalisation, la pompe délivre la composition selon l'invention au travers de bolus, d'un flux basal ou d'une combinaison de bolus et d'un flux basal.

Dans un mode de réalisation, la pompe délivre la composition selon l'invention au travers d'une combinaison de bolus et d'un flux basal.

Dans un mode de réalisation, la pompe délivrant la composition selon l'invention est choisie dans le groupe des références de pompes suivant : Accu-Check^{®} Combo, Accu-Check^{®} Insight, Accu-Check^{®} Spirit, Animas^{®} 2020, Animas^{®} Vibe, CellNovo, Omnipod^{®}, Minimed^{®} 670G, Minimed^{®} 640G, Minimed^{®} 630G, Minimed^{®} 530G, Minimed^{®} Paradigm^{®} Revel, Medtronic Paradigm^{®} Veo^{™}, Tandem t:slim^{®}, Tandem t:slim X2^{®}, Tandem t:flex^{®}, Mylife YpsoPump^{®}, BetaBionics iLet^{®}, Asante Snap, Valeritas V-Go^{®}, OneTouch^{®}, Cequr PaQ^{®} and Unilife Imperium^{®}.

Dans un mode de réalisation, le système d'injection délivrant la composition selon l'invention est un système d'injection dit « close-loop » ou semi « close-loop ».

Dans un mode de réalisation, le système d'injection délivrant la composition selon l'invention est un système « close-loop », système équipé d'un processeur utilisant un algorithme qui prend en compte la quantité d'insuline présente dans l'organisme du patient en estimant par lui-même cette quantité.

Dans un mode de réalisation, le système d'injection « close loop » comprend un capteur fournissant de manière directe ou indirecte le niveau de glucose sanguin du patient, une pompe à infusion qui délivre le produit et un processeur qui reçoit la mesure du capteur, calcule la dose de produit à délivrer par la pompe basée sur cette mesure et sur un algorithme interne qui prédit l'évolution du taux de glucose sanguin, et envoie la commande à la pompe pour délivrer la dose calculée.

Dans un mode de réalisation, le système d'injection délivrant la composition selon l'invention est un système semi « close-loop », système équipé d'un processeur utilisant un algorithme qui prend en compte la quantité d'insuline présente dans l'organisme du patient grâce à des données extérieures.

Dans un mode de réalisation, ces données extérieures sont fournies par le patient.

Dans un mode de réalisation, ces données extérieures fournies par le patient concernent la quantité de carbohydrate ingérée par le patient et le début et la fin d'une activité physique.

Dans un mode de réalisation, ces données extérieures fournies par le patient concernent la quantité de carbohydrate ingérée par le patient.

Dans un mode de réalisation, ces données extérieures fournies par le patient concernent le début et la fin d'une activité physique.

Dans un mode de réalisation, l'algorithme peut prendre en compte d'autres données externes pouvant être données automatiquement par un capteur.

Dans un mode de réalisation, le processeur présent dans le système d'injection de la composition selon l'invention peut inclure des étapes supplémentaires assurant la sécurité de la dose administrée au patient.

Dans un mode de réalisation, le système d'injection de la composition selon l'invention comprend un capteur fournissant de manière directe ou indirecte le niveau de glucose sanguin du patient.

Dans un mode de réalisation, le capteur est choisi parmi le groupe des capteurs ou capteurs équipant les systèmes d'injection Medtronic Paradigm^{®} Veo^{™}, MiniMed^{®} 640G with SmartGuard^{®}, Roche Dexcom^{®} G4 PLATINUM CGM, Roche Dexcom G5^{™} Mobile CGM, Abbott Diabètes Care FreeStyle Libre Flash glucose monitoring system, Abbott Diabètes Care FreeStyle Navigator II CGM system.

Dans un mode de réalisation, le système d'injection de la composition selon l'invention comprend une pompe à deux compartiments avec un compartiment contenant la composition selon l'invention et un compartiment contenant une autre drogue.

Dans un mode de réalisation, le système d'injection de la composition selon l'invention comprend une pompe à deux compartiments avec un compartiment contenant la composition selon l'invention et un compartiment contenant du glucagon.

Dans un mode de réalisation, la composition selon l'invention peut être utilisée dans un système de pancréas artificiel à plusieurs hormones.

Dans un mode de réalisation, le système de pancréas artificiel à plusieurs hormones utilisant la composition selon l'invention est un système « close-loop » ou semi « close-loop ».

Dans un mode de réalisation, le système de pancréas artificiel comprend la composition selon l'invention et du glucagon comme autre hormone.

Dans un mode de réalisation, le système de pancréas artificiel comprend une pompe à deux compartiments, un compartiment contenant la composition selon l'invention et un compartiment contenant du glucagon.

Dans un mode de réalisation, le système de pancréas artificiel comportant une pompe à deux compartiments est équipé d'un processeur utilisant un algorithme calculant deux doses, une de la composition selon l'invention et une de glucagon, les doses calculées par l'algorithme étant envoyées à la pompe et étant délivrées au patient.

Dans un mode de réalisation, le système de pancréas artificiel comprend deux pompes, une pompe délivrant la composition selon l'invention et une pompe délivrant du glucagon.

Dans un mode de réalisation, le système de pancréas artificiel comportant deux pompes comprend un processeur utilisant un algorithme calculant deux doses, une de la composition selon l'invention et une de glucagon, les doses calculées étant envoyées à la pompe contenant la composition selon l'invention et à la deuxième pompe contenant le glucagon respectivement, les doses calculées par l'algorithme étant délivrées au patient.

Dans un mode de réalisation, le système d'injection de la composition selon l'invention est un stylo intelligent.

Dans un mode de réalisation, le système d'injection de la composition selon l'invention est un stylo intelligent capable de déterminer la dose à injecter.

Dans un mode de réalisation, le stylo intelligent capable de déterminer la dose à injecter est équipé d'un capteur fournissant de manière directe ou indirecte le niveau de glucose sanguin du patient.

Dans un mode de réalisation, le stylo intelligent capable de déterminer la dose à injecter est équipé d'un capteur et d'un processeur qui calcule la dose de produit à délivrer en utilisant la mesure du capteur et un algorithme qui prédit l'évolution du taux de glucose sanguin, et envoie la commande au stylo pour enregistrer la dose calculée.

Dans un mode de réalisation, la dose calculée par le processeur du stylo intelligent est la dose administrée par le patient.

Dans un mode de réalisation, le stylo intelligent est équipé d'un processeur utilisant un algorithme prenant en compte la quantité d'insuline dans l'organisme du patient en estimant par lui-même cette quantité ou en recevant cette donnée de manière extérieure.

Dans un mode réalisation, le stylo intelligent est équipé d'un processeur utilisant un algorithme prenant en compte d'autres données externes pouvant être données automatiquement par un capteur ou par le patient tel que la quantité de carbohydrate ingérée par le patient, ou le début et la fin d'une activité physique.

Dans un mode de réalisation, le système d'injection de la composition selon l'invention comprend une pompe, deux pompes ou un stylo intelligent équipés d'un processeur utilisant un algorithme.

Dans un mode de réalisation, l'algorithme utilisé par le processeur est choisi parmi les algorithmes de type « PID » (Proportional Integral Derivative) dont un exemple est décrit dans l'article (Ruiz, J., et al., Journal od diabètes science and technology, 1123-1130,2012), les algorithmes de type « Fuzzy logic » dont un exemple est décrit dans l'article (Atlas, E., et al, Diabètes care, 1072-1076, 2010), les algorithmes de type « MPC » (Mode) Prédictive Control) dont un exemple est décrit dans l'article (Hovorka, R., et al, Physiol. Meas., 905-920, 2004) et les algorithmes de type « PD » (Proportional Dérivative) dont un exemple est décrit dans l'article (Jacobs, P., et al, IEEE Trans Biomed Eng, 2569-2581, 2014).

L'invention concerne également des formulations unidoses à pH compris entre 6,0 et 8,0 comprenant de l'amyline, un agoniste au récepteur de l'amyline ou un analogue d'amyline et un co-polyaminoacide selon l'invention.

L'invention concerne également des formulations unidoses à pH compris entre 6,0 et 8,0 comprenant de l'amyline, un agoniste au récepteur de l'amyline ou analogue d'amyline, un co-polyaminoacide selon l'invention et un GLP-1, un analogue de GLP-1 ou un GLP-1 RA, tel que définie précédemment.

L'invention concerne également des formulations unidoses à pH compris entre 6,6 et 7,8 comprenant de l'amyline, un agoniste au récepteur de l'amyline ou un analogue d'amyline et un co-polyaminoacide selon l'invention.

L'invention concerne également des formulations unidoses à pH compris entre 6,6 et 7,8 comprenant de l'amyline, un agoniste au récepteur de l'amyline ou un analogue d'amyline, un co-polyaminoacide selon l'invention et une insuline prandiale, telle que définie précédemment.

L'invention concerne également des formulations unidoses à pH compris entre 6,6 et 7,6 comprenant de l'amyline, un agoniste au récepteur de l'amyline ou un analogue d'amyline et un co-polyaminoacide selon l'invention.

L'invention concerne également des formulations unidoses à pH compris entre 6,6 et 7,6 comprenant de l'amyline, un agoniste au récepteur de l'amyline ou un analogue d'amyline, un co-polyaminoacide selon l'invention et une insuline prandiale, telle que définie précédemment.

Dans un mode de réalisation, les formulations unidoses comprennent en outre un co-polyaminoacide tel que défini précédemment.

Dans un mode de réalisation, les formulations sont sous forme d'une solution injectable.

La préparation d'une composition selon l'invention présente l'avantage de pouvoir être réalisée par simple mélange d'une solution aqueuse de l'amyline, d'un agoniste au récepteur de l'amyline ou d'un analogue d'amyline, et d'un co-polyaminoacide porteur de charges carboxylates et d'au moins un radical hydrophobe selon l'invention, en solution aqueuse ou sous forme lyophilisée. Si nécessaire, le pH de la préparation est ajusté à pH compris entre 6 et 8.

La préparation d'une composition selon l'invention présente l'avantage de pouvoir être réalisée par simple mélange d'une solution aqueuse de l'amyline, d'un agoniste au récepteur de l'amyline ou d'un analogue d'amyline, d'insuline prandiale, et d'un co-polyaminoacide porteur de charges carboxylates et d'au moins un radical hydrophobe selon l'invention, en solution aqueuse ou sous forme lyophilisée. Si nécessaire, le pH de la préparation est ajusté à pH compris entre 6 et 8.

Dans un mode de réalisation, le mélange d'insuline prandiale et de co-polyaminoacide est concentré par ultrafiltration.

Si nécessaire, la composition du mélange est ajustée en excipients tels que glycérine, m-crésol, chlorure de zinc, et polysorbate (Tween^{®}) par ajout de solutions concentrées de ces excipients au sein du mélange. Si nécessaire, le pH de la préparation est ajusté à pH compris entre 6 et 8.

Dans un mode de réalisation, les compositions sont caractérisées en ce que lesdites compositions présentent une stabilité mesurée par ThT supérieure à celle d'une composition de référence comprenant de l'amyline, un agoniste au récepteur de l'amyline ou un analogue d'amyline mais ne comprenant pas de co-polyaminoacide porteur de charges carboxylates et de radicaux hydrophobes Hy.

Dans un mode de réalisation, les compositions sont caractérisées en ce que lesdites compositions présentent une stabilité mesurée par ThT supérieure à celle d'une composition de référence comprenant de l'amyline, un agoniste au récepteur de l'amyline ou un analogue d'amyline en combinaison avec une insuline mais ne comprenant pas de co-polyaminoacide porteur de charges carboxylates et de radicaux hydrophobes Hy.

Dans un mode de réalisation, les compositions sont caractérisées en ce que lesdites compositions présentent une stabilité mesurée par ThT supérieure à celle d'une composition de référence comprenant de l'amyline, un agoniste au récepteur de l'amyline ou un analogue d'amyline en combinaison avec un GLP-1, un analogue de GLP-1 ou un agoniste de récepteur de GLP-1, mais ne comprenant pas de co-polyaminoacide porteur de charges carboxylates et de radicaux hydrophobes Hy.

Dans un mode de réalisation, les compositions sont caractérisées en ce que lesdites compositions présentent une stabilité mesurée par ThT supérieure à celle d'une composition de référence comprenant de l'amyline, un agoniste au récepteur de l'amyline ou un analogue d'amyline en combinaison avec une insuline et un GLP-1, un analogue de GLP-1 ou un agoniste de récepteur de GLP-1, mais ne comprenant pas de co-polyaminoacide porteur de charges carboxylates et de radicaux hydrophobes Hy.

L'invention concerne également une utilisation d'un co-polyaminoacide porteur de charges carboxylates et de radicaux hydrophobes Hy pour stabiliser une composition comprenant de l'amyline, un agoniste au récepteur de l'amyline ou un analogue d'amyline.

L'invention concerne également une utilisation d'un co-polyaminoacide porteur de charges carboxylates et de radicaux hydrophobes Hy pour stabiliser une composition comprenant de l'amyline, un agoniste au récepteur de l'amyline ou un analogue d'amyline et une insuline prandiale, et éventuellement un GLP-1, un analogue de GLP-1 ou un agoniste de récepteur de GLP-1.

L'invention concerne une méthode de stabilisation de composition comprenant de l'amyline, un agoniste au récepteur de l'amyline ou un analogue d'amyline ou une méthode de stabilisation de composition comprenant de l'amyline, un agoniste au récepteur de l'amyline ou un analogue d'amyline et une insuline prandiale, et éventuellement un GLP-1, un analogue de GLP-1 ou un agoniste de récepteur de GLP-1.

### Description des Figures :

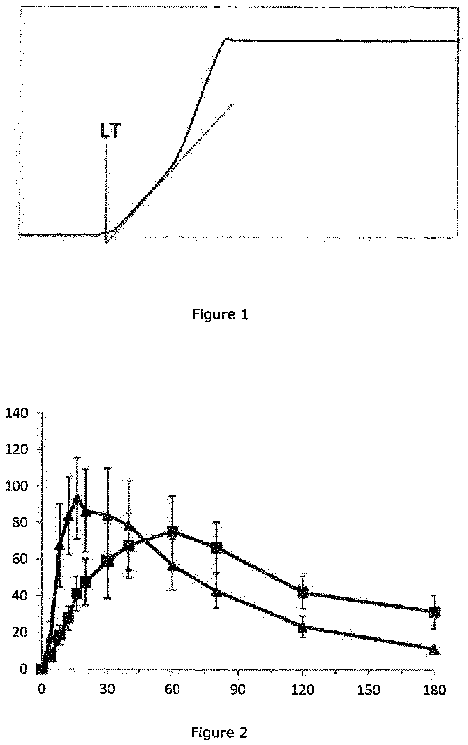

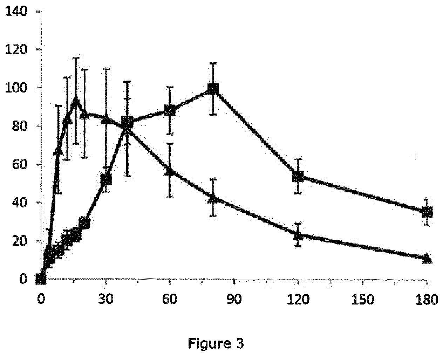

**Figure 1** :
   Sur cette figure est représentée de façon graphique la détermination du temps de latence (LT) par suivi de la fluorescence de la Thioflavine T, sur une courbe ayant en ordonnées la valeur de la fluorescence (en u.a. unités arbitraires) et en abscisses le temps en minutes.
**Figure 2** :
   Sont présentés à la Figure 2 les résultats de pharmacocinétique du pramlintide obtenus avec les compositions décrites dans les exemples CA1/CA2 et CA3. L'analyse de ces profils indique que la composition de l'exemple CA3 comprenant le co-polyaminoacide BB15, 100 UI/mL d'insuline et 0,6 mg/mL de pramlintide (courbe tracée avec les carrés correspondant à l'exemple CA3) permet d'obtenir une absorption du pramlintide plus lente que celle de la composition de l'exemple en double injection comprenant uniquement du pramlintide et de l'insuline (courbe tracée avec les triangles correspondant à l'exemple double injection CA1/CA2).
**Figure 3** :
   Sont présentés à la Figure 3 les résultats de pharmacocinétique du pramlintide obtenus avec les compositions décrites dans les exemples CA1/CA2 et CA4. L'analyse de ces profils indique que la composition de l'exemple CA4 comprenant le co-polyaminoacide AB24, 100 UI/mL d'insuline et 0,6 µg/mL de pramlintide (courbe tracée avec les carrés correspondant à l'exemple CA4) permet d'obtenir une absorption du pramlintide plus lente que celle de la composition de l'exemple en double injection comprenant uniquement du pramlintide et de l'insuline (courbe tracée avec les triangles correspondant à l'exemple double injection CA1/CA2).

Les exemples suivants illustrent, de manière non-limitative, l'invention.

### Partie A

### AA : Synthèse des molécules hydrophobes dans lesquelles p=1

Les radicaux hydrophobes sont représentés dans le tableau suivant par la molécule hydrophobe correspondante avant greffage sur le co-polyaminoacide.

**Tableau 1A : liste et structures des molécules hydrophobes synthétisées selon l'invention.**

| **N°** | **STRUCTURE DE LA MOLECULE HYDROPHOBE AVANT GREFFAGE SUR LE CO-POLYAMINOACIDE** |
|---|---|
| **AA1** | 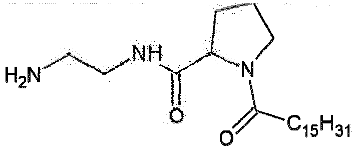 |
| **AA2** | 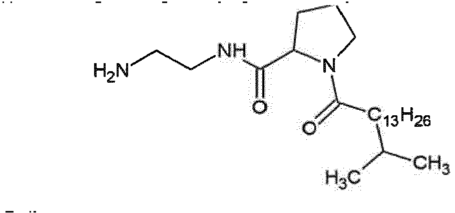 |
| **AA3** | 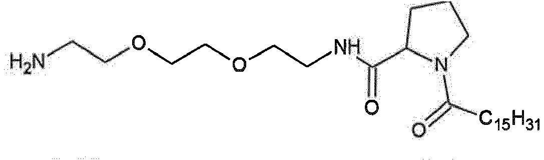 |
| **AA4** | 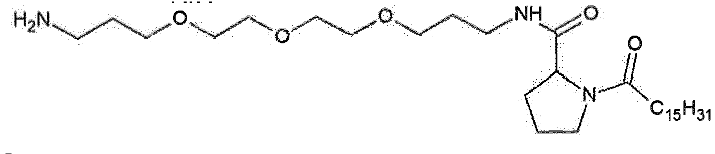 |
| **AA5** | 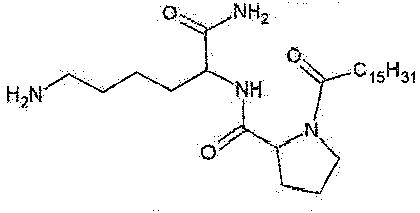 |
| **AA6** | 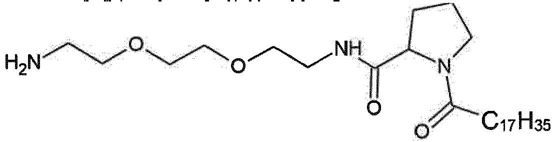 |
| **AA7** | 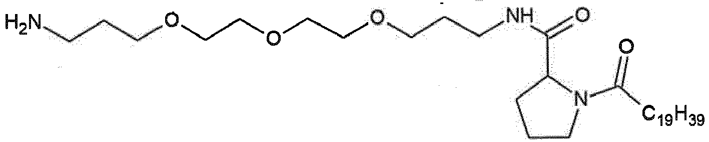 |
| AA8 | 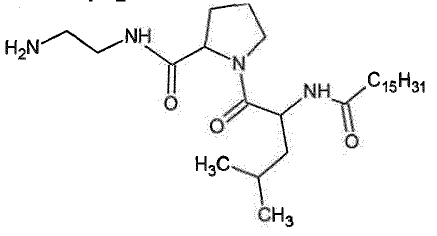 |
| AA9 | 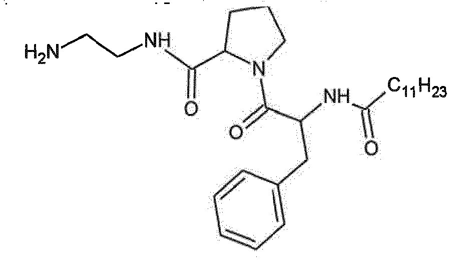 |
| AA10 | 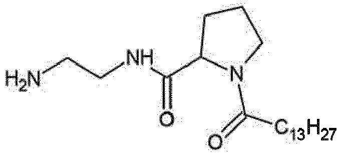 |
| AA11 | 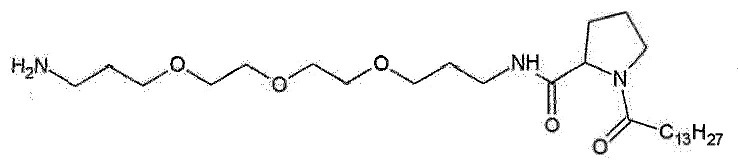 |
| AA12 | 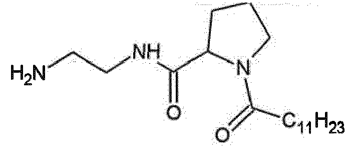 |
| AA13 | 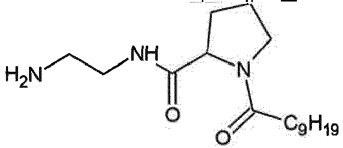 |
| AA14 | 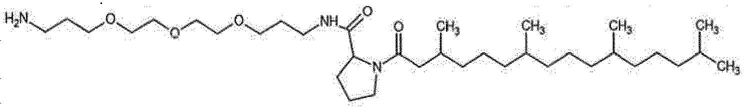 |

### Exemple AA1 : molécule AA1

### Molécule A1 : Produit obtenu par la réaction entre le chlorure de palmitoyle et la L-proline.

À une solution de L-proline (10,6 g, 92,1 mmol) dans de la soude aqueuse 1 N (230 mL, 230 mmol) est ajoutée goutte à goutte pendant 90 min une solution de chlorure de palmitoyle (23,0 g, 83,7 mmol) dans l'acétone (167 mL). Après 14 h d'agitation à température ambiante, le mélange hétérogène est refroidi à 0 °C, puis filtré sur fritté pour donner un solide blanc qui est lavé avec de l'eau (2 x 100 mL) puis du diisopropyléther (100 mL). Le solide est séché sous pression réduite. Le solide est alors dissous à reflux dans 200 mL d'eau puis 8 mL d'une solution d'acide chlorhydrique à 37 % sont ajoutés pour obtenir un pH=1. Le milieu réactionnel opalescent est alors refroidi à 0 °C. Le précipité obtenu est filtré sur fritté puis lavé avec de l'eau (5 x 50 mL) jusqu'à obtenir des filtrats de pH physiologique compris entre 6,0 et 8,0 pour être ensuite séché dans une étuve à 50 °Csous vide pendant une nuit. Le produit est purifié par recristallisation dans le diisopropyléther. Un solide blanc est obtenu.
Rendement : 22,7 g (77 %).
RMN ¹H (CDCl₃, ppm) : 0,88 (3H) ; 1,19-1,45 (24H) ; 1,58-1,74 (2H) ; 1,88-2,14 (3H) ; 2,15-2,54 (3H) ; 3,47 (1H) ; 3,58 (1H) ; 4,41 (0,1H) ; 4,61 (0,9H) ; 6,60-8,60 (1H).

### Molécule A2 : Produit obtenu par réaction entre la molécule A1 et la Boc-éthylènediamine.

À une solution de molécule A1 (75,1 g, 212,4 mmol) dans 1500 mL de chloroforme sont ajoutés successivement et à température ambiante de la *N,N-*diisopropyléthylamine (DIPEA) (68,8 g, 532,3 mmol), du 1-hydroxybenzotriazole (HOBt) (37,1 g, 274,6 mmol) puis du *N*-(3-diméthylaminopropyl)-*N'*-éthylcarbodiimide (EDC) (53,1 g, 277,0 mmol). Après 15 min d'agitation à température ambiante, une solution de Boc-éthylènediamine (BocEDA) (37,6 g, 234,7 mmol) dans 35 mL de chloroforme est additionnée. Après 18 h d'agitation à température ambiante, une solution de HCl 0,1 N (2,1 L), puis une solution saturée de NaCl (1 L) sont ajoutées. Les phases sont séparées puis la phase organique est lavée successivement avec une solution de HCl 0,1 N / NaCl saturée (2,1 L/1 L), une solution de NaCl saturée (2 L), une solution de NaHCO₃ saturée (2 L), puis une solution de NaCl saturée (2 L). La phase organique est séchée sur sulfate de sodium anhydre, filtrée puis concentrée sous pression réduite. Le solide obtenu est purifié par triturations dans le diisopropyléther (3 x 400 mL), pour donner un solide après séchage sous vide à 40 °C.
Rendement : 90,4 g (86 %).
RMN ¹H (CDCl₃, ppm) : 0,88 (3H) ; 1,20-1,37 (24H) ; 1,44 (9H) ; 1,54-1,70 (2H) ; 1,79-1,92 (1H) ; 1,92-2,04 (1H) ; 2,03-2,17 (1H) ; 2,17-2,44 (3H) ; 3,14-3,36 (4H) ; 3,43 (1H) ; 3,56 (1H) ; 4,29 (0,1 H) ; 4,51 (0,9 H) ; 4,82 (0,1H) ; 5,02 (0,9H) ; 6,84 (0,1H) ; 7,22 (0,9H).

### Molécule AA1

À une solution de molécule A2 (20,1 g, 40,5 mmol) dans 330 mL de dichlorométhane est ajoutée goutte à goutte et à 0 °Cune solution d'acide chlorhydrique 4 N dans le dioxane (100 mL, 400 mmol). Après 3 h 30 d'agitation à température ambiante, la solution est concentrée sous pression réduite. Le résidu est purifié par chromatographie flash (méthanol, dichlorométhane) pour donner un solide blanc de molécule AA1 sous forme de sel de chlorhydrate.
Rendement : 16,3 g (93 %).
RMN ¹H (CDCl₃, ppm) : 0,88 (3H) ; 1,07-1,40 (24H) ; 1,49-1,63 (2H) ; 1,77-2,18 (4H) ; 2,18-2,45 (2H) ; 3,14-3,32 (2H) ; 3,42-3,63 (2H) ; 3,63-3,84 (2H) ; 4,37 (0,1H) ; 4,48 (0,9H) ; 6,81-8,81 (4H).
LC/MS (ESI) : 396,5 ; (calculé ([M+H]⁺) : 396,4).

### Exemple AA2 : molécule AA2

### Molécule A3 ; 15-méthylhexadécan-1-ol.

Dans un tricol sous argon est introduit du magnésium (9,46 g, 389 mmol) en copeaux. Le magnésium est recouvert de THF (40 mL) anhydre et quelques gouttes de 1-bromo-3-méthylbutane sont ajoutées à température ambiante pour initier la réaction. Après l'observation d'un exotherme et un léger trouble du milieu, le reste du 1-bromo-3-méthylbutane (53,87 g, 357 mmol) est ajouté au goutte à goutte en 90 min alors que la température du milieu reste stable entre 50 et 60 °C. Le milieu réactionnel est ensuite chauffé à 70 °Cpendant 2 h.

Dans un tricol sous argon, à une solution de CuCl (482 mg, 4,86 mmol) dissous dans la NMP (62 mL) à 0 °C est ajoutée au goutte à goutte une solution de 12-bromo-1-dodécanol (43 g, 162,1 mmol) dans le THF (60 mL). À cette solution est ensuite ajoutée au goutte à goutte la solution de l'organomagnésien chaude préparée extemporanément tout en maintenant la température du milieu en dessous de 20 °C. Le mélange est ensuite agité à température ambiante pendant 16 h. Le milieu est refroidi à 0 °Cet la réaction est stoppée par addition d'une solution aqueuse d'HCl 1 N jusqu'à pH 1 et le milieu est extrait à l'acétate d'éthyle. Après lavage de la phase organique avec une solution saturée en NaCl et séchage sur Na₂SO₄, la solution est filtrée et concentrée sous vide pour donner une huile. Après purification par DCVC sur gel de silice (cyclohexane, acétate d'éthyle), une huile qui cristallise à température ambiante est obtenue.
Rendement: 32,8 g (74 %).
RMN ¹H (CDCl₃, ppm) : 0,87 (6H) ; 1,14 (2H) ; 1,20-1,35 (22H) ; 1,50-1,55 (3H) ; 3,64 (2H).

### Molécule A4 : acide 15-méthylhexadécanoïque.

À une solution de molécule A3 (20,65 g, 80,5 mmol) et bromure de tétrabutylammonium (14,02 g, 42,5 mmol) dans un mélange d'acide acétique/dichloréthane/eau (124/400/320 mL) à température ambiante est ajouté par petites portions du permanganate de potassium (38,2 g, 241,5 mmol). Après agitation à reflux pendant 5 h et retour à température ambiante, le milieu est acidifié à pH 1 par ajout progressif de HCl 5 N. Na₂SO₃ (44,6 g, 354,3 mmol) est ensuite ajouté progressivement jusqu'à décoloration du milieu. La phase aqueuse est extraite au dichlorométhane et les phases organiques combinées sont séchées sur Na₂SO₄, filtrées et concentrées sous vide. Après purification par chromatographie sur gel de silice (cyclohexane, acétate d'éthyle, acide acétique), un solide blanc est obtenu.
Rendement : 19,1 g (quantitatif).
RMN ¹H (CDCl₃, ppm) : 0,87 (6H) ; 1,14 (2H) ; 1,22-1,38 (20H) ; 1,51 (1H) ; 1,63 (2H) ; 2,35 (2H).

### Molécule A5 : Produit obtenu par réaction entre la molécule A4 et la L-proline.

À une solution de molécule A4 (10 g, 37 mmol) dans le THF (360 mL) à 0 °C sont ajoutés successivement du dicyclohexyle carbodiimide (DCC) (8,01 g, 38,8 mmol) et du *N*-hydroxysuccinimide (NHS) (4,47 g, 38,8 mmol). Après 17 h d'agitation à température ambiante, le milieu est refroidi à 0 °Cpendant 20 min, filtré sur fritté. De la L-proline (4 g, 37,7 mmol), de la triéthylamine (34 mL) et de l'eau (30 mL) sont ajoutées au filtrat. Après 20 h d'agitation à température ambiante, le milieu est traité avec une solution aqueuse d'HCl 1 N jusqu'à pH 1. La phase aqueuse est extraite avec du dichlorométhane (2 x 125 mL). Les phases organiques combinées sont lavées avec une solution aqueuse de HCl 1 N (2 x 100 mL), de l'eau (100 mL), puis une solution aqueuse saturée en NaCl (100 mL). Après séchage sur Na₂SO₄, la phase organique est filtrée, concentrée sous vide et le résidu est purifié par chromatographie sur gel de silice (cyclohexane, acétate d'éthyle, acide acétique)
Rendement : 9,2 g (72 %).
RMN ¹H (CDCl₃, ppm) : 0,86 (6H) ; 1,14 (2H) ; 1,22-1,38 (20H) ; 1,50 (1H) ; 1,67 (2H) ; 1,95-2,10 (3H) ; 2,34 (2H) ; 2,49 (1H) ; 3,47 (1H) ; 3,56 (1H) ; 4,61 (1H). LC/MS (ESI) : 368,3 ; (calculé ([M+H]⁺) : 368,6).

### Molécule A6 : Produit obtenu par réaction entre la molécule A5 et la Boc-éthylènediamine.

À une solution de molécule A5 (9,22 g, 25,08 mmol) dans un mélange THF/DMF (200/50 mL) sont ajoutés de la triéthylamine (TEA) (5,23 mL) et du 2-(1H-benzotriazol-1-yl)-1,1,3,3-tétraméthyluronium tétrafluoroborate (TBTU) à température ambiante. Après 10 min d'agitation, de la Boc-éthylènediamine (4,42 g, 27,6 mmol) est ajoutée. Après agitation à température ambiante pendant 17 h, le mélange est dilué avec de l'eau (300 mL) à 0 °C et agité à froid pendant 20 min. Le précipité formé est filtré sur fritté et le filtrat est extrait à l'acétate d'éthyle. Les phases organiques combinées sont lavées avec une solution saturée de NaHCO₃, séchées sur Na₂SO₄, filtrées, concentrées sous vide et le résidu est purifié par chromatographie flash (acétate d'éthyle, méthanol).
Rendement : 6,9 g (54 %).
RMN ¹H (CDCl₃, ppm) : 0,86 (6H) ; 1,15 (2H) ; 1,22-1,38 (20H) ; 1,43 (9H) ; 1,50 (1H) ; 1,64 (4H) ; 1,85 (1H) ; 1,95 (1H) ; 2,10 (1H) ; 2,31 (2H) ; 3,20-3,35 (3H) ; 3,45 (1H) ; 3,56 (1H) ; 4,51 (1H) ; 5,05 (1H) ; 7,24 (1H).
LC/MS (ESI) : 510,6 ; (calculé ([M+H]⁺) : 510,8).

### Molécule AA2

À une solution de la molécule A6 (5,3 g, 10,40 mmol) dans le dichlorométhane (50 mL) à 0 °C est ajoutée une solution de HCl 4 N dans le dioxane (13 mL). Après 5 h d'agitation à 0 °C, le milieu est concentré sous vide, repris dans de l'eau et lyophilisé pour donner un solide blanc de molécule AA2 sous forme de sel de chlorhydrate.
Rendement : 4,6 g (99%).
RMN ¹H (D₂O, ppm) : 0,91 (6H) ; 1,22 (2H) ; 1,22-1,50 (20H) ; 1,63 (3H) ; 1,98 (1H) ; 2,10 (2H) ; 2,26 (1H) ; 2,39 (1H) ; 2,43 (1H) ; 3,22 (2H) ; 3,45-3,60 (3H) ; 3,78 (1H) ; 4,42 (1H).
LC/MS (ESI) : 410,4 ; (calculé ([M+H]⁺) : 410,7).

### Exemple AA3 : molécule AA3

### Molécule A7 : Produit obtenu par la réaction entre la molécule A1 et la Boc-tri(éthylèneglycol)diamine.

Par un procédé similaire à celui utilisé pour la préparation de la molécule A2 appliqué à la molécule A1 (4,0 g, 11,3 mmol) et à la Boc-tri(éthylèneglycol)diamine (3,1 g, 12,4 mmol), une huile incolore est obtenue après purification par chromatographie flash (méthanol, toluène).
Rendement : 5,5 g (84 %).
RMN ¹H (CDCl₃, ppm) : 0,88 (3H) ; 1,09-1,39 (24H) ; 1,44 (9H) ; 1,64 (2H) ; 1,79-2,01 (2H) ; 2,06-2,43 (4H) ; 3,23-3,68 (14H) ; 4,33 (0,2H) ; 4,56 (0,8H) ; 5,25 (1H) ; 6,49 (0,2H) ; 7,13-7,50 (0,8H).

### Molécule AA3

Par un procédé similaire à celui utilisé pour la préparation de la molécule AA1 appliqué à la molécule A7 (5,5 g, 9,4 mmol), un solide blanc de molécule AA3 sous forme de sel de chlorhydrate est obtenu après purification par chromatographie flash (méthanol, dichlorométhane).
Rendement : 4,3 g (92 %).
RMN ¹H (DMSO-d₆, ppm) : 0,85 (3H) ; 1,08-1,40 (24H) ; 1,40-1,52 (2H) ; 1,71-2,02 (4H) ; 2,02-2,31 (2H) ; 2,90-2,98 (2H) ; 3,15-3,47 (5H) ; 3,50-3,66 (7H) ; 4,24 (0,6H) ; 4,32 (0,4H) ; 7,83 (0,6H) ; 7,95 (3H) ; 8,17 (0,4H).
LC/MS (ESI) : 484,6 ; (calculé ([M+H]⁺ ) : 484,4).

### Exemple AA4 : molécule AA4

### Molécule A8 : Produit obtenu par la réaction entre la molécule A1 et la Boc-1-amino-4,7,10-trioxa-13-tridécane amine.

Par un procédé similaire à celui utilisé pour la préparation de la molécule A2 appliqué à la molécule A1 (4,5 g, 12,7 mmol) et à la Boc-1-amino-4,7,10-trioxa-13-tridécane amine (4,5 g, 14,0 mmol), une huile jaune est obtenue après purification par chromatographie flash (méthanol, dichlorométhane).
Rendement : 7,7 g (92 %).
RMN ¹H (CDCl₃, ppm) : 0,88 (3H) ; 1,22-1,37 (24H) ; 1,44 (9H) ; 1,59-1,67 (2H) ; 1,67-2,00 (6H) ; 2,06-2,45 (4H) ; 3,18-3,76 (18H) ; 4,28 (0,2H) ; 4,52 (0,8H) ; 4,69-5,04 (1H) ; 6,77 (0,2H) ; 7,20 (0,8H).

### Molécule AA4

Par un procédé similaire à celui utilisé pour la préparation de la molécule AA1 appliqué à la molécule A8 (7,7 g, 11,8 mmol), une huile jaune est obtenue après purification par chromatographie flash (méthanol, dichlorométhane). Une co-évaporation avec du diisopropyléther permet d'obtenir la molécule AA4 sous forme de sel de chlorhydrate sous forme d'un solide blanc qui est séché sous vide à 50 °C.
Rendement : 5,4 g (76 %).
RMN ¹H (CDCl₃, ppm) : 0,88 (3H) ; 1,08-1,40 (24H) ; 1,49-1,65 (2H) ; 1,76-2,39 (10H) ; 3,07-3,28 (3H) ; 3,34-3,80 (15H) ; 4,34 (0,05H) ; 4,64 (0,95H) ; 7,35 (0,05H) ; 7,66-8,58 (3,95H).
LC/MS (ESI) : 556,7 ; (calculé ([M+H]⁺) : 556,5).

### Exemple AA5 : molécule AA5

### Molécule A9 : Produit obtenu par réaction entre la molécule A1 et l'ester méthylique de la N-Boc-L-lysine.

Par un procédé similaire à celui utilisé pour la préparation de la molécule A2 appliqué à la molécule A1 (4 g, 11,3 mmol) et à l'ester méthylique de la *N*-Boc-L-lysine (3,2 g, 12,4 mmol), une huile incolore est obtenue après purification par chromatographie flash (méthanol, dichlorométhane).
Rendement : 4,9 g (73 %).
RMN ¹H (CDCl₃, ppm) : 0,88 (3H) ; 0,99-1,54 (37H) ; 1,54-1,75 (3H) ; 1,75-2,04 (3H) ; 2,04-2,41 (4H) ; 2,94-3,19 (2H) ; 3,19-3,81 (5H) ; 4,28-4,64 (2H) ; 4,94 (1H) ; 6,45 (0,1H) ; 7,36 (0,9H).
LC/MS (ESI) : 596,7 ; (calculé ([M+H]⁺) : 596,5).

### Molécule A10 : Produit obtenu par traitement de la molécule A9 avec de l'ammoniaque.

À une suspension de molécule A9 (4,9 g, 8,2 mmol) dans 10 mL de méthanol sont ajoutés 320 mL d'une solution d'ammoniaque 7 N dans le méthanol. Après 19 h d'agitation à température ambiante en atmosphère fermée, 100 mL supplémentaires de solution d'ammoniaque sont ajoutés. Après 24 h d'agitation à température ambiante en atmosphère fermée, le milieu réactionnel est concentré sous pression réduite. Le résidu est purifié par trituration dans le diisopropyléther à reflux (100 mL), pour donner un solide blanc qui est séché sous vide à 50 °C.
Rendement : 4,1 g (85 %).
RMN ¹H (CDCl₃, ppm) : 0,88 (3H) ; 1,06-1,57 (37H) ; 1,57-1,79 (3H) ; 1,88-2,41 (7H) ; 3,09 (2H) ; 3,49 (1H) ; 3,62 (1H) ; 4,34 (1H) ; 4,51 (1H) ; 4,69-4,81 (1H) ; 5,43 (0,95H) ; 5,57 (0,05H) ; 6,25 (0,05H) ; 6,52 (0,95H) ; 6,83 (0,05H) ; 7,11 (0,95H).

### Molécule AA5

Par un procédé similaire à celui utilisé pour la préparation de la molécule AA1 appliqué à la molécule A10 (388 mg, 0,67 mmol), un solide blanc de molécule AA5 sous forme de sel de chlorhydrate est obtenu après purification par trituration dans le diisopropyléther.
Rendement : 292 mg (85 %).
RMN ¹H (DMSO-d6, ppm) : 0,85 (3H) ; 1,06-2,34 (38H) ; 2,61-2,81 (2H) ; 3,29-3,68 (2H) ; 4,05-4,17 (1,7H) ; 4,42 (0,3H) ; 7,00 (1H) ; 7,16 (0,7H) ; 7,43 (0,3H) ; 7,73-8,04 (3,7H) ; 8,16 (0,3H).
LC/MS (ESI) : 481,6 ; (calculé ([M+H]⁺) : 481,4).

### Exemple AA6 : molécule AA6

### Molécule A11 : Produit obtenu par la réaction entre le chlorure de stéaroyle et la L-proline.

Par un procédé similaire à celui utilisé pour la préparation de la molécule A1 appliqué à la L-proline (5,0 g, 43,4 mmol) et au chlorure de stéaroyle (12,0 g, 39,6 mmol), un solide blanc est obtenu après purification par chromatographie flash (méthanol, dichlorométhane).
Rendement : 5,37 g (36 %).
RMN ¹H (CDCl₃, ppm) : 0,88 (3H) ; 1,26-1,37 (28H) ; 1,64-1,70 (2H) ; 1,88-2,10 (3H) ; 2,36 (2H) ; 2,54-2,58 (1H) ; 3,46 (1H) ; 3,56 (1H) ; 4,62 (1H).
LC/MS (ESI) : 382,6 ; (calculé ([M+H]⁺) : 382,3).

### Molécule A12 : Produit obtenu par réaction entre la molécule A11 et la Boc-tri(éthylèneglycol)diamine.

Par un procédé similaire à celui utilisé pour la préparation de la molécule A6 appliqué à la molécule A11 (33,81 g, 88,6 mmol) et à la Boc-tri(éthylèneglycol)diamine (26,4 g, 106,3 mmol) dans le THF en utilisant la DIPEA au lieu de la TEA, un solide blanc est obtenu après purification par chromatographie flash (acétate d'éthyle, méthanol).
Rendement : 43,3 g (80 %).
RMN ¹H (CDCl₃, ppm) : 0,87 (3H) ; 1,24 (30H) ; 1,43 (9H) ; 1,61 (2H) ; 1,82 (1H) ; 1,96 (1H) ; 2,25-2,45 (2H) ; 3,25-3,65 (14H) ; 4,30 (0,15H) ; 4,53 (0,85H) ; 5,25 (1H) ; 6,43 (0,15H) ; 7,25 (0,85H).
LC/MS (ESI) : 612,6 ; (calculé ([M+H]⁺) : 612,9).

### Molécule AA6

Par un procédé similaire à celui utilisé pour la préparation de la molécule AA2 appliqué à la molécule A12 (43 g, 70,3 mmol), le résidu obtenu après concentration sous vide est trituré dans l'acétonitrile. La suspension est filtrée et le solide lavé avec de l'acétonitrile puis de l'acétone. Après séchage sous vide, un solide blanc de molécule AA6 sous forme de sel de chlorhydrate est obtenu.
Rendement : 31,2 g (81 %).
RMN ¹H (DMSO-d₆, ppm) : 0,85 (3H) ; 1,23 (28H) ; 1,45 (2H) ; 1,70-2,05 (4H) ; 2,13 (1H) ; 2,24 (1H) ; 2,95 (2H) ; 3,10-3,25 (2H) ; 3,30-3,65 (10H) ; 4,20-4,45 (1H) ; 7,85-8,25 (4H).
LC/MS (ESI) : 512,4 ; (calculé ([M+H]⁺) : 512,8).

### Exemple AA7 : molécule AA7

### Molécule A13 ; Produit obtenu par réaction entre l'acide arachidique et la L-proline.

Par un procédé similaire à celui utilisé pour la préparation de la molécule A5 appliqué à l'acide arachidique (15,51 g, 49,63 mmol) et à la L-proline (6 g, 52,11 mmol) en utilisant la DIPEA à la place de la TEA, un solide blanc est obtenu après purification par colonne chromatographique sur gel de silice (cyclohexane, acétate d'éthyle, acide acétique).
Rendement : 12,9 g (63 %).
RMN ¹H (CDCl₃, ppm) : 0,88 (3H) ; 1,28 (34H) ; 1,66 (2H) ; 1,95-2,15 (2H) ; 2,34 (2H) ; 2,45 (1H) ; 3,47 (1H) ; 3,56 (1H) ; 4,60 (1H).
LC/MS (ESI): 410,4 ; (calculé ([M+H]⁺) : 410,6).

### Molécule A14 : Produit obtenu par la réaction entre la molécule A13 et la Boc-1-amino-4,7,10-trioxa-13-tridécane.

Par un procédé similaire à celui utilisé pour la préparation de la molécule A12 appliqué à la molécule A13 (10,96 g, 26,75 mmol) et à la Boc-1-amino-4,7,10-trioxa-13-tridécane (10,29 g, 32,11 mmol), un solide est obtenu après purification par colonne chromatographique sur gel de silice (cyclohexane, acétate d'éthyle, méthanol).
Rendement : 14,2 g (75 %).
RMN ¹H (CDCl₃, ppm) : 0,88 (3H) ; 1,24 (32H) ; 1,43 (9H) ; 1,61 (2H) ; 1,80 (1H) ; 1,96 (1H) ; 2,10-2,45 (4H) ; 3,20-3,75 (18H) ; 4,30 (0,20H) ; 4,55 (0,80H) ; 5,03 (1H) ; 6,75 (0,20H) ; 7,20 (0,80H).
LC/MS (ESI): 712,8 ; (calculé ([M+H]⁺) : 713,1).

### Molécule AA7

Après un procédé similaire à celui utilisé pour la préparation de la molécule AA2 appliqué à la molécule A14 (14,25 g, 20,01 mmol), le résidu obtenu après concentration sous vide du milieu réactionnel est dissous dans le méthanol et évaporé sous pression réduite, l'opération étant répétée 4 fois pour donner un solide blanc de molécule AA7 sous forme de sel de chlorhydrate.
Rendement : 12,7 g (98 %).
RMN ¹H (DMSO-d₆, ppm) : 0,85 (3H) ; 1,23 (32H) ; 1,45 (2H) ; 1,64 (2H) ; 1,70-2,05 (6H) ; 2,10-2,30 (2H) ; 2,82 (2H) ; 3,08 (2H) ; 3,30-3,60 (15H) ; 4,15-4,30 (1H) ; 7,73-8,13 (4H).
LC/MS (ESI): 612,7 ; (calculé ([M+H]⁺) : 612,9).

### Exemple AA8 : molécule AA8

### Molécule A15 : Produit obtenu par la réaction entre la L-leucine et le chlorure de palmitoyle.

Par un procédé similaire à celui utilisé pour la préparation de la molécule A1 appliqué à de la L-leucine (15,0 g, 114,4 mmol) et au chlorure de palmitoyle (34,5 g, 125 mmol), un solide blanc est obtenu par trituration dans le diisopropyléther.
Rendement : 13,0 g (31 %).
RMN ¹H (CDCl₃, ppm) : 0,88 (3H) ; 0,96 (6H) ; 1,16-1,35 (24H) ; 1,55-1,77 (5H) ; 2,23 (2H) ; 4,55-4,60 (1H) ; 5,88 (1H).

Molécule A16 : Produit obtenu par la réaction entre la molécule A15 et l'ester méthylique de la L-proline.

Par un procédé similaire à celui utilisé pour la préparation de la molécule A2 appliqué à la molécule A15 (6,00 g, 16,2 mmol) et à l'ester méthylique de la L-proline (3,23 g, 19,5 mmol), une huile légèrement jaune est obtenue après purification par chromatographie flash (méthanol, dichlorométhane).
Rendement : 5,8 g (74 %).
RMN ¹H (CDCl₃, ppm) : 0,83-1,00 (9H) ; 1,18-1,32 (24H) ; 1,40-1,73 (5H) ; 1,84-2,33 (6H) ; 3,47-3,89 (2H) ; 3,70 (1,14H) ; 3,71 (1,21H) ; 3,74 (0,53H) ; 3,76 (0,12H) ; 4,40-4,56 (1H) ; 4,63-4,67 (0,04H) ; 4,84 (0,38) ; 4,90 (0,40) ; 5,06 (0,18) ; 5,99 (0,18H) ; 6,08-6,21 (0,82).
LC/MS (ESI) : 481,6 ; (calculé ([M+H]⁺) : 481,4).

### Molécule A17 : Produit obtenu par la saponification de l'ester méthylique de la molécule A16.

À une solution de molécule A16 (5,8 g, 12,06 mmol) dans 30 mL de méthanol est ajoutée de la soude 1 N (13,5 mL, 13,5 mmol). Après 20 h d'agitation à température ambiante, la solution est diluée avec de l'eau puis acidifiée par 20 mL d'acide chlorhydrique 1 N à 0 °C. Le précipité est filtré puis rincé avec de l'eau (50 mL) avant d'être solubilisé dans 50 mL de dichlorométhane. La phase organique est séchée sur Na₂SO₄, filtrée puis concentrée sous pression réduite pour donner une huile incolore.
Rendement : 4,5 g (80 %).
RMN ¹H (CDCl₃, ppm) : 0,85-0,99 (9H) ; 1,14-1,41 (24H) ; 1,43-1,72 (5H) ; 1,87-2,47 (7H) ; 3,48-3,55 (0,6H) ; 3,56-3,62 (0,4H) ; 3,83-3,90 (0,4H) ; 3,90-3,96 (0,6H) ; 4,52-4,56 (0,6H) ; 4,56-4,59 (0,4H) ; 4,80-4,86 (0,4H) ; 4,86-4,91 (0,6H) ; 6,05 (0,4H) ; 6,11 (0,6H).
LC/MS (ESI) : 467,6 ; (calculé ([M+H]⁺) : 467,4).

### Molécule A18 : Produit obtenu par la réaction entre la Boc-éthylènediamine et la molécule A17.

Par un procédé similaire à celui utilisé pour la préparation de la molécule A2 appliqué à la molécule A17 (4,5 g, 9,64 mmol) et à la Boc-éthylènediamine (1,70 g, 10,61 mmol), une huile incolore est obtenue après purification par chromatographie flash (méthanol, dichlorométhane).
Rendement : 2,0 g (34 %).
RMN ¹H (CDCl₃, ppm) : 0,83-0,99 (9H) ; 1,19-1,32 (24H) ; 1,44 (9H) ; 1,48-2,37 (14H) ; 3,09-3,99 (4H) ; 4,28-5,01 (2H) ; 5,64-6,04 (1H) ; 6,87-7,06 (1H).
LC/MS (ESI) : 609,7 ; (calculé ([M+H]⁺) : 609,5).

### Molécule AA8

Par un procédé similaire à celui utilisé pour la préparation de la molécule AA1 appliqué à la molécule A18 (2 g, 3,28 mmol), un solide de molécule AA8 sous forme de sel de chlorhydrate est obtenu après purification par chromatographie flash (méthanol, dichlorométhane).
Rendement : 1,5 g (90 %).
RMN ¹H (CDCl₃, ppm) : 0,83-1,00 (9H) ; 1,18-1,32 (24H) ; 1,37-1,77 (5H) ; 1,93-2,41 (6H) ; 3,07-3,97 (6H) ; 4,44-4,77 (2H) ; 7,66-8,21 (2H).
LC/MS (ESI) : 509,6 ; (calculé ([M+H]⁺) : 509,4).

### Exemple AA9 : molécule AA9

### Molécule A19 : Produit obtenu par la réaction entre l'acide laurique et la L-phénylalanine.

Par un procédé similaire à celui utilisé pour la préparation de la molécule A5 appliqué à l'acide laurique (8,10 g, 40,45 mmol) et à la L-phénylalanine (7 g, 42,38 mmol), un solide blanc est obtenu.
Rendement : 12,7 g (98 %).
RMN ¹H (DMSO-d₆, ppm) : 0,86 (3H) ; 1,10-1,30 (16H) ; 1,36 (2H) ; 2,02 (2H) ; 2,82 (1H) ; 3,05 (1H) ; 4,42 (1H) ; 7,15-7,30 (5H) ; 8,05 (1H) ; 12,61 (1H).
LC/MS (ESI) : 348,2 ; (calculé ([M+H]⁺) : 348,5).

### Molécule A20 : Produit obtenu par la réaction entre la molécule A19 et le sel de chlorhydrate de l'ester méthylique de la L-proline.

Par un procédé similaire à celui utilisé pour la préparation de la molécule A6 appliqué à la molécule A19 (9,98 g, 28,72 mmol) et au sel chlorhydrate de l'ester méthylique de la L-proline (5,23 g, 31,59 mmol), une huile incolore est obtenue après purification par colonne chromatographique sur gel de silice (cyclohexane, acétate d'éthyle).
Rendement : 5,75 g (44 %).
RMN ¹H (CDCl₃, ppm) : 0,88 (3H) ; 1,10-1,30 (16H) ; 1,50-1,75 (3H) ; 1,80-2,02 (3H) ; 2,17 (2H) ; 2,65 (0,5H) ; 2,95 (1H) ; 3,05-3,20 (1,5H) ; 3,50-3,65 (1H) ; 3,75 (3H) ; 4,29 (0,5H) ; 4,46 (0,5H) ; 4,70 (0,1H) ; 4,95 (0,9H) ; 6,20-6,30 (1H) ; 7,15-7,30 (5H).
LC/MS (ESI) : 459,2 ; (calculé ([M+H]⁺) : 459,6).

### Molécule A21 : Produit obtenu par saponification de la molécule A20.

À une solution de molécule A20 (5,75 g, 12,54 mmol) dans un mélange THF/méthanol/eau (40/40/40 mL) à 0 °Cest ajoutée de l'hydroxyde de lithium (LiOH) (600,49 mg, 25,07 mmol) puis le mélange est agité à température ambiante pendant 20 h. Après évaporation des solvants organiques sous vide, la phase aqueuse est diluée dans l'eau, acidifiée avec une solution aqueuse de HCl 1 N jusqu'à pH 1. Le produit est alors extrait à l'acétate d'éthyle. Les phases organiques combinées sont lavées avec une solution aqueuse saturée de NaCl, séchées sur Na₂SO₄, filtrées et concentrées sous pression réduite pour donner une huile incolore.
Rendement : 5,7 g (quantitatif).
RMN ¹H (CDCl₃, ppm) : 0,88 (3H) ; 1,10-1,30 (16H) ; 1,50-1,80 (3H) ; 1,67-2,02 (2H) ; 2,20 (2H) ; 2,25 (0,4H) ; 2,60 (0,6H) ; 2,85-3,10 (2,6H) ; 3,55-3,65 (1,4H) ; 4,35 (0,6H) ; 4,55 (0,4H) ; 4,94 (1H) ; 6,28 (0,4H) ; 6,38 (0,6H) ; 7,20-7,30 (5H).
LC/MS (ESI) : 445,2 ; (calculé ([M+H]⁺) : 445,6).

### Molécule A22 : Produit obtenu par réaction entre la Boc-éthylènediamine et la molécule A21.

Par un procédé similaire à celui utilisé pour la préparation de la molécule A6 appliqué à la molécule A21 (5,67 g, 12,75 mmol) et à la Boc-éthylènediamine (2,25 g, 14,03 mmol), une huile incolore est obtenue après purification par colonne chromatographique sur gel de silice (dichlorométhane, méthanol).
Rendement : 5,7 g (76%).
RMN ¹H (CDCl₃, ppm) : 0,88 (3H) ; 1,25 (16H) ; 1,43 (9H) ; 1,58 (2,6H) ; 1,75-1,95 (1,4H) ; 2,15-2,30 (3H) ; 2,64 (0,5H) ; 2,95-3,10 (2,5H) ; 3,20-3,40 (4H) ; 3,45 (0,5H) ; 3,55 (0,2H) ; 3,66 (1H) ; 4,44 (1H) ; 4,50 (0,2H) ; 4,60 (0,6H) ; 4,99 (0,7H) ; 5,54 (0,5H) ; 5,95 (0,2H) ; 6,17 (1H) ; 6,60 (0,5H) ; 7,07 (0,5H) ; 7,20-7,40 (5H).
LC/MS (ESI) : 587,4 ; (calculé ([M+H]⁺) : 587,8).

### Molécule AA9

Après un procédé similaire à celui utilisé pour la préparation de la molécule AA2 appliqué à la molécule A22 (5,66 g, 9,65 mmol), le résidu obtenu après concentration sous vide du milieu réactionnel est dissous dans le méthanol et évaporé sous pression réduite, l'opération étant répétée 4 fois pour donner une mousse blanche de molécule AA9 sous forme de sel de chlorhydrate.
Rendement : 4,9 g (97 %).
RMN ¹H (DMSO-d₆, 120 °C, ppm) : 0,89 (3H) ; 1,26 (16H) ; 1,43 (2H) ; 1,68 (0,6H) ; 1,75-2,00 (3H) ; 2,05-2,25 (2,4H) ; 2,82-3,05 (5H) ; 3,38 (2H) ; 3,50-3,70 (1,4H) ; 4,25 (0,6H) ; 4,63 (0,4H) ; 4,77 (0,6H) ; 7,25-7,50 (5H) ;7,55-8,20 (4H).
LC/MS (ESI) : 487,4 ; (calculé ([M+H]⁺) : 487,7).

### Exemple AA10 : molécule AA10

### Molécule A23 : Produit obtenu par la réaction entre la molécule B7 et la Boc-éthylènediamine.

À une solution de molécule B7 (190,00 g, 583,73 mmol) à 0 °C dans le DCM (2,9 L) sont ajoutés successivement du HOBt (8,94 g, 58,37 mmol) puis de la Boc-éthylènediamine (112,20 g, 700,00 mmol) en solution dans le DCM (150 mL). De l'EDC (123,10 g, 642,00 mmol) est ajouté puis le mélange est agité 17 h entre 0 °C et température ambiante. Le mélange réactionnel est ensuite lavé avec une solution aqueuse saturée en NaHCOs (2x1,5 L), une solution aqueuse de HCl 1 N (2x1,5 L) puis une solution aqueuse saturée en NaCl (1,5 L), séché sur Na₂SO₄, filtré et concentré sous pression réduite. Un solide blanc est obtenu après recristallisation dans l'acétonitrile.
Rendement : 256,50 g (93 %).
RMN ¹H (CDCl₃, ppm) : 0,88 (3H) ; 1,16-1,38 (20H) ; 1,44 (9H) ; 1,56-1,71 (2H) ; 1,78-2,45 (6H) ; 3,11-3,72 (6H) ; 4,30 (0,1H); 4,51 (0,9H) ; 4,87 (0,1H); 5,04 (0,9H) ; 6,87 (0,1H) ; 7,23 (0,9H).
LC/MS (ESI) : 468,0 ; (calculé ([M+H]⁺) : 468,4).

### Molécule AA10

Après un procédé similaire à celui utilisé pour la préparation de la molécule AA1 appliqué à la molécule A23 (256,50 g, 548,43 mmol), un solide blanc de molécule AA10 sous forme de sel de chlorhydrate est obtenu par trituration dans du pentane (1,6 L) et séchage sous pression réduite à 40 °C.
Rendement : 220,00 g (99 %).
RMN ¹H (MeOD-d4, ppm) : 0,90 (3H) ; 1,21-1,43 (20H) ; 1,54-1,66 (2H) ; 1,85-2,28 (4H) ; 2,39 (2H) ; 3,00-3,17 (2H) ; 3,30-3,40 (1H) ; 3,43-3,71 (3H) ; 4,29 (0,94H) ; 4,48 (0,06H).
LC/MS (ESI) : 368,2 ; (calculé ([M+H]⁺) : 368,3).

### Exemple AA11 : molécule AA11

### Molécule A24 : Produit obtenu par la réaction entre la molécule B7 et le Boc-1-amino-4,7,10-trioxa-13-tridécane amine.

Par un procédé similaire à celui utilisé pour la préparation de la molécule A23 appliqué à la molécule B7 (24,00 g, 73,73 mmol) et au Boc-1-amino-4,7,10-trioxa-13-tridécane amine (28,35 g, 88,48 mmol), une huile orange de molécule A24 est obtenue.
Rendement : 44,50 g (96 %).
RMN ¹H (CDCl₃, ppm) : 0,87 (3H) ; 1,08-1,56 (20H) ; 1,43 (9H) ; 1,58-1,67 (2H) ; 1,70-2,00 (6H) ; 2,04-2,41 (4H) ; 3,16-3,77 (18H) ; 4,26-4,29 (0,2H) ; 4,50-4,54 (0,8H) ; 4,68-5,10 (1H) ; 6,74 (0,2H) ; 7,19 (0,8H).
LC/MS (ESI) : 628,4 ; (calculé ([M+H]⁺) : 628,5).

### Molécule AA11

Après un procédé similaire à celui utilisé pour la préparation de la molécule AA1 appliqué à la molécule A24 (43,40 g, 69,12 mmol), un solide blanc de molécule AA11 sous forme de sel de chlorhydrate est obtenu après trituration 3 fois dans le diéthyléther, solubilisation du résidu dans l'eau et lyophilisation.
Rendement : 38,70 g (98 %).
RMN ¹H (DMSO, ppm) : 0,85 (3H) ; 1,07-1,38 (20H) ; 1,41-1,52 (2H) ; 1,55-1,66 (2H) ; 1,70-2,02 (6H) ; 2,08-2,30 (2H) ; 2,78-2,87 (2H) ; 3,00-3,16 (2H) ; 3,29-3,66 (14H) ; 4,16-4,22 (0,65 H) ; 4,25-4,30 (0,35H) ; 7,74 (0,65H) ; 7,86 (3H) ; 8,10 (0,35H). LC/MS (ESI) : 528,4 ; (calculé ([M+H]⁺) : 528,4).

### Exemple AA12 : molécule AA12

### Molécule A25 : Produit obtenu par la réaction entre la molécule B4 et la Boc-éthylènediamine.

Par un procédé similaire à celui utilisé pour la préparation de la molécule A23 appliqué à la molécule B4 (12,00 g, 40,35 mmol) et à la Boc-éthylènediamine (7,76 g, 48,42 mmol), une huile incolore est obtenue et utilisée sans autre purification.
Rendement : 17,40 g (94 %).
RMN ¹H (CDCl₃, ppm) : 0,86 (3H) ; 1,11-1,68 (18H) ; 1,41 (9H) ; 1,80-2,38 (6H) ; 3,06-3,35 (4H) ; 3,37-3,49 (1H) ; 3,51-3,73 (1H) ; 4,26-4,31 (0,1H) ; 4,45-4,52 (0,9H) ; 4,91-5,19 (1H) ; 6,97 (0,1H) ; 7,23 (0,9H).
LC/MS (ESI) : 440,4 (calculé ([M+H]⁺) : 440,3).

### Molécule AA12

Après un procédé similaire à celui utilisé pour la préparation de la molécule AA1 appliqué à la molécule A25 (8,85 g, 20,13 mmol), un solide blanc de molécule AA12 est obtenu après lavage basique, concentration sous pression réduite puis recristallisation dans l'acétonitrile.
Rendement : 6,53 g (96 %).
RMN ¹H (DMSO, ppm) : 0,85 (3H) ; 1,07-1,56 (20H) ; 1,68-2,03 (4H) ; 2,09-2,29 (2H) ; 2,50-2,58 (2H) ; 2,96-3,11 (2H) ; 3,21-3,59 (2H) ; 4,17-4,21 (0,65H) ; 4,25-4,29 (0,35H) ; 7,68 (0,65H) ; 8,00 (0,35H).
LC/MS (ESI) : 340,3 ; (calculé ([M+H]⁺) : 340,3).

### Exemple AA13 : molécule AA13

### Molécule A26 : Produit obtenu par couplage entre la molécule B1 et la Boc-éthylènediamine.

Par un procédé similaire à celui utilisé pour la préparation de la molécule A23 appliqué à la molécule B1 (30,00 g, 111,36 mmol) et à la Boc-éthylènediamine (21,41 g, 133,64 mmol), un solide blanc est obtenu après recristallisation dans l'acétonitrile.
Rendement : 34,90 g (76 %).
RMN ¹H (CDCl₃, ppm) : 0,88 (3H) ; 1,10-1,70 (14H) ; 1,43 (9H) ; 1,80-1,91 (1H) ; 1,92-2,01 (1H) ; 2,04-2,42 (4H) ; 3,13-3,70 (6H) ; 4,27-4,31 (0,15H) ; 4,47-4,53 (0,85H) ; 4,83 (0,15H) ; 5,02 (0,85H) ; 6,85 (0,15H) ; 7,21 (0,85H).
LC/MS (ESI) : 412,2 ; (calculé ([M+H]⁺) : 412,3).

### Molécule AA13

Après un procédé similaire à celui utilisé pour la préparation de la molécule AA1 appliqué à la molécule A26 (34,90 g, 84,79 mmol), un solide blanc de molécule AA13 sous forme de sel de chlorhydrate est obtenu après solubilisation dans un mélange DCM/acétonitrile et concentration sous pression réduite.
Rendement : 29,50 g (99 %).
RMN ¹H (DMSO, ppm) : 0,85 (3H) ; 1,07-1,61 (14H) ; 1,70-2,06 (4H) ; 2,10-2,35 (2H) ; 2,76-2,87 (2H) ; 3,24-3,47 (3,25H) ; 3,56-3,64 (0,75H) ; 4,13-4,19 (0,75H) ; 4,31-4,36 (0,25H) ; 8,05-8,36 (3,75H) ; 8,50 (0,25H).
LC/MS (ESI) : 312,2 ; (calculé ([M+H]⁺) : 312,3).

### Exemple AA14 : molécule AA14

### Molécule A27 : Produit obtenu par hydrogénation du phytol.

À une solution de phytol (30,00 g, 101,20 mmol) dans le THF (450 mL) sous argon est ajouté de l'oxyde de platine (PtO₂, 1,15 g, 6,61 mmol) et le milieu est placé sous 1 bar de dihydrogène puis agité pendant 4 h à température ambiante. Après filtration sur célite en rinçant au THF, une huile noire de molécule A27 est obtenue par concentration sous pression réduite.
Rendement: 29,00 g (96 %).
RMN ¹H (CDCl₃, ppm) : 0,84 (6H) ; 0,86 (6H) ; 0,89 (3H) ; 1,00-1,46 (22H) ; 1,46-1,68 (3H) ; 3,61-3,73 (2H).

### Molécule A28 : Produit obtenu par oxydation de la molécule A27.

À une solution de molécule A27 (29,0 g, 97,13 mmol) dans un mélange dichloroéthane/eau (485 mL/388 mL) sont ajoutés successivement du bromure de tétrabutylammonium (16,90 g, 52,45 mmol), de l'acide acétique (150 mL, 2,62 mol) puis du KMnO₄ (46,05 g, 291,40 mmol) par petites fractions en maintenant la température entre 16 et 19 °C. Le milieu réactionnel est ensuite agité pendant 4 h 30 au reflux, refroidi à 10 °C puis acidifié jusqu'à pH 1 avec une solution de HCl 6 N (20 mL). Du Na₂SO₃ (53,90 g) est ajouté progressivement en maintenant la température à 10 °C et le milieu est agité jusqu'à décoloration complète. De l'eau (200 mL) est ajoutée, les phases sont séparées et la phase aqueuse est extraite au DCM (2 x 400 mL). Les phases organiques combinées sont lavées par une solution aqueuse de HCl à 10 % (20 mL), de l'eau (2 x 200 mL), une solution aqueuse saturée en NaCl (200 mL), séchées sur Na₂SO₄, filtrées et concentrées sous pression réduite. Une huile jaune de molécule A28 est obtenue après purification par chromatographie flash (éluant : cyclohexane, AcOEt).
Rendement : 28,70 g (94 %).
RMN ¹H (CDCl₃, ppm) : 0,84 (6H) ; 0,86 (6H) ; 0,97 (3H) ; 1,00-1,41 (20H) ; 1,52 (1H) ; 1,96 (1H) ; 2,14 (1H) ; 2,35 (1H) ; 11,31 (1H).
LC/MS (ESI) : 311,1 (calculé ([M-H]-) : 311,3).

### Molécule A29 : Produit obtenu par couplage entre la molécule A28 et la L-prolinate de méthyle.

Par un procédé similaire à celui utilisé pour la préparation de la molécule A2 appliqué à la molécule A28 (18,00 g, 57,59 mmol) et au chlorhydrate de L-prolinate de méthyle (14,31 g, 86,39 mmol), une huile jaune de molécule A29 est obtenue après lavage de la phase organique par une solution aqueuse saturée en NaHCOa (2 x 150 mL), une solution aqueuse de HCl à 10 % (2 x 150 mL), une solution aqueuse saturée en NaCl (2 x 150 mL), puis séchage sur Na₂SO₄, filtration et concentration sous pression réduite.
Rendement : 23,20 g (95 %).
RMN ¹H (DMSO-d₆, ppm) : 0,78-0,89 (15H) ; 0,97-1,43 (20H) ; 1,43-1,56 (1H) ; 1,70-1,96 (4H) ; 1,96-2,32 (3H) ; 3,33-3,56 (2H) ; 3,59 (0,6H) ; 3,67 (2,4H) ; 4,27 (0,8H) ; 4,57 (0,2H).
LC/MS (ESI) : 424,4 (calculé ([M+H]⁺) : 424,4).

### Molécule A30 : Produit obtenu par la saponification de la molécule A29.

Par un procédé similaire à celui utilisé pour la préparation de la molécule A21 appliqué à la molécule A29 (21,05 g, 49,68 mmol), une huile jaune de molécule A30 est obtenue.
Rendement : 20,40 g (99 %).
RMN ¹H (DMSO-d₆, ppm) : 0,77-0,91 (15H) ; 0,97-1,43 (20H) ; 1,43-1,56 (1H) ; 1,67-1,96 (4H) ; 1,96-2,29 (3H) ; 3,26-3,56 (2H) ; 4,20 (0,8H) ; 4,41 (0,2H).
LC/MS (ESI) : 410,3 (calculé ([M+H]⁺): 410,4).

### Molécule A31 : Produit obtenu par le couplage entre la molécule A30 et le Boc-1-amino-4.7.10-trioxa-13-tridécane amine.

Par un procédé similaire à celui utilisé pour la préparation de la molécule A23 appliqué à la molécule A30 (8,95 g, 21,85 mmol) et au Boc-1-amino-4,7,10-trioxa-13-tridécane amine (8,40 g, 26,21 mmol), une huile incolore de molécule A31 est obtenue après purification par chromatographie flash (éluant : DCM, AcOEt, méthanol).
Rendement : 10,08 g (65 %).
RMN ¹H (DMSO-d₆, ppm) : 0,78-0,89 (15H) ; 0,97-1,43 (29H) ; 1,43-1,55 (1H) ; 1,55-1,66 (4H) ; 1,71-2,30 (7H) ; 2,95 (2H) ; 3,00-3,19 (2H) ; 3,34-3,58 (14H) ; 4,17-4,29 (1H) ; 6,30-6,79 (1H) ; 7,67 (0,65H) ; 8,00 (0,35H).
LC/MS (ESI) : 712,6 (calculé ([M+H]⁺) : 712,6).

### Molécule AA14

Après un procédé similaire à celui utilisé pour la préparation de la molécule AA1 appliqué à la molécule A31 (10,08 g, 14,16 mmol), le résidu obtenu après concentration sous pression réduite est solubilisé dans le DCM (200 mL), la phase organique est lavée par une solution aqueuse de NaOH 2 N (2 x 100 mL), séchée sur Na₂SO₄, filtrée et concentrée sous pression réduite. Une huile incolore de molécule AA14 sous forme d'amine neutre est obtenue.
Rendement : 8,23 g (95 %).
RMN ¹H (DMSO-d₆, ppm) : 0,78-0,89 (15H) ; 0,97-1,43 (20H) ; 1,43-1,69 (6H) ; 1,69-2,30 (8H) ; 2,56 (2H) ; 2,99-3,19 (2H) ; 3,31-3,58 (14H) ; 4,15-4,29 (1H) ; 7,70 (0,65H) ; 8,04 (0,35H).
LC/MS (ESI) : 612,5 (calculé ([M+H]⁺): 612,5).

### AB : Synthèse des co-polyaminoacides modifiés par des molécules hydrophobes dans lesquelles p=1

### Co-polyaminoacides statistiques de formule VII ou VIIa

**Tableau 1B : Liste des co-polyaminoacides de formules VII ou VIIa synthétisés selon l'invention**

| **n°** | **co-polyaminoacides porteur de charges carboxylates et de radicaux hydrophobes** |
|---|---|
| AB1 | 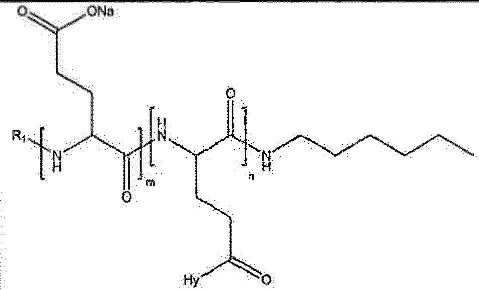 i = 0,05, DP (m + n) = 23 |
| | 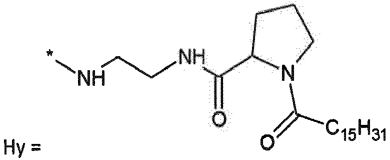 R₁ = H ou pyroglutamate |
| AB2 | 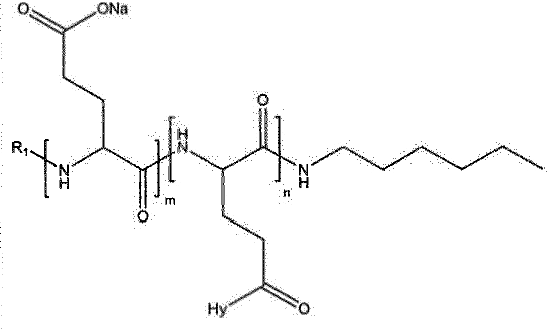 i = 0,05, DP (m + n) = 35 |
| | 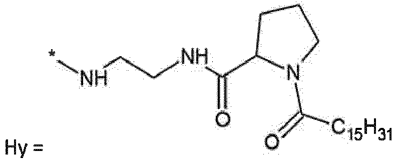 R₁ = H ou pyroglutamate |
| AB3 | 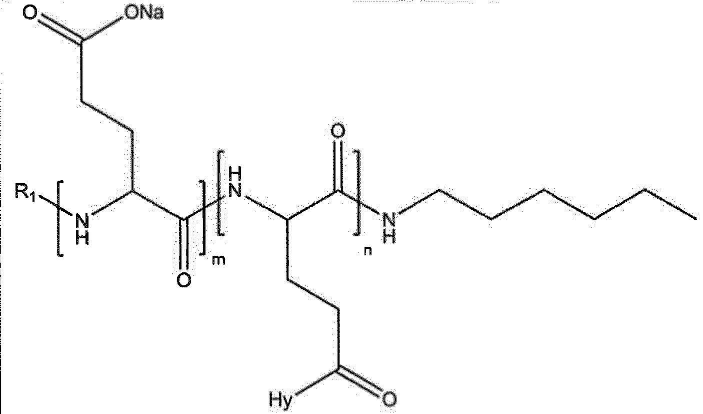 i = 0,10, DP (m + n) = 35 |
| | 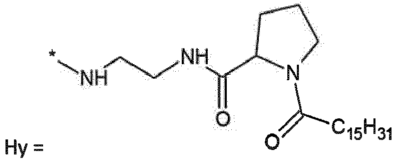 R₁ = H ou pyroglutamate |
| AB4 | 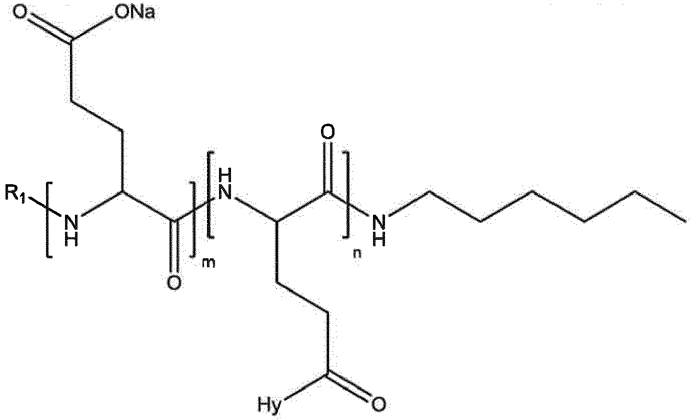 i = 0,052, DP (m + n) = 35 |
| | 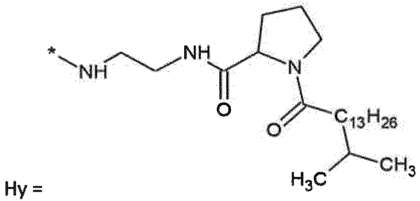 R₁ = H ou pyroglutamate |
| AB5 | 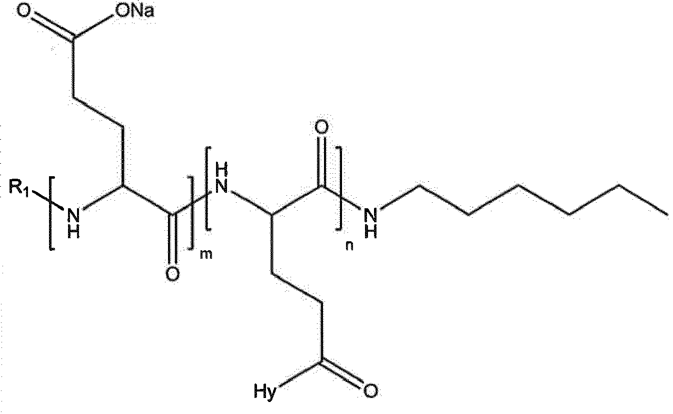 i = 0,05, DP (m + n) = 23 |
| | 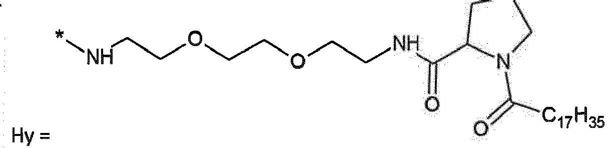 R₁ = H ou pyroglutamate |
| **AB6** | 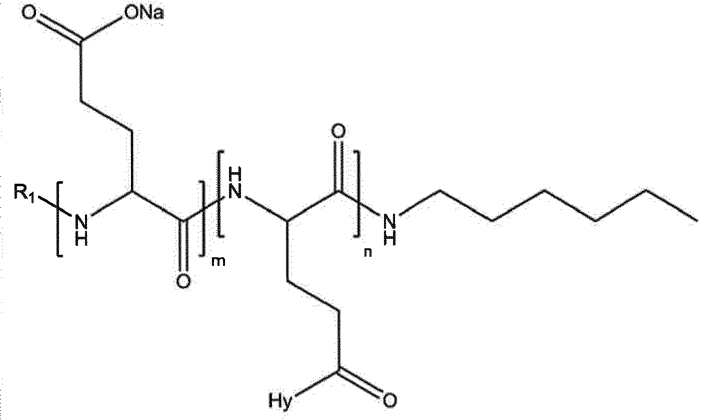 i = 0,025, DP (m + n) = 20 |
| | 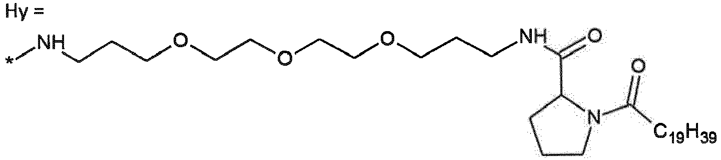 R₁ = H ou pyroglutamate |
| AB7 | 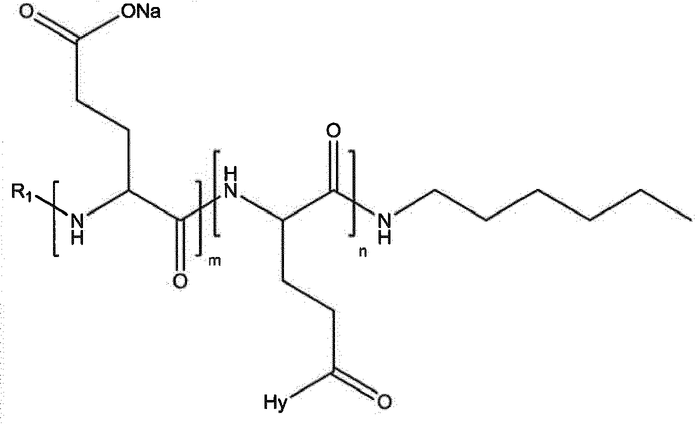 i = 0,03, DP (m + n) = 21 |
| | 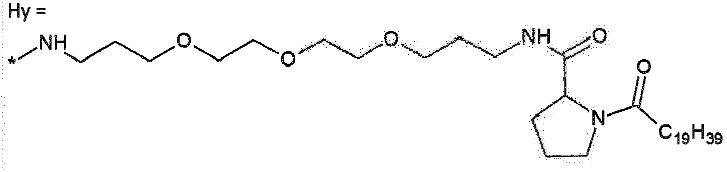 R1=CH₃-CO-, H ou pyroglutamate |
| AB8 | 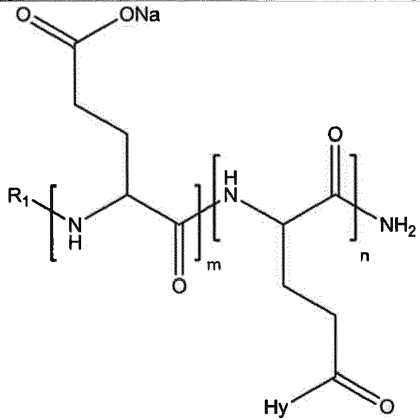 i = 0,03, DP (m + n) = 24 |
| | 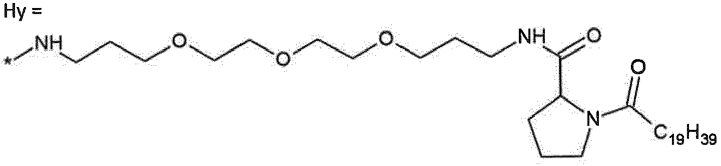 R₁ = H ou pyroglutamate |
| AB9 | 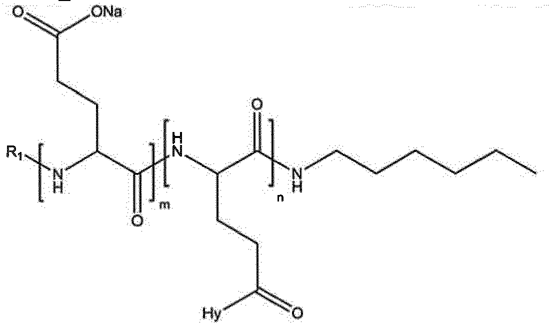 i = 0,12, DP (m + n) = 30 |
| | 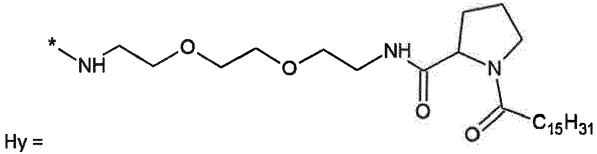 R₁ = H ou pyroglutamate |
| AB10 | 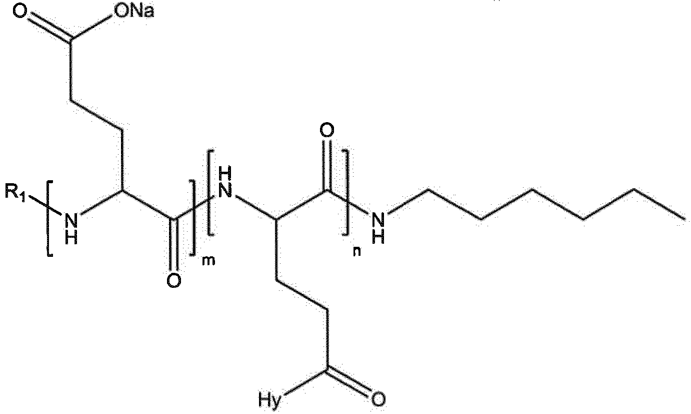 i = 0,08, DP (m + n) = 25 |
| | 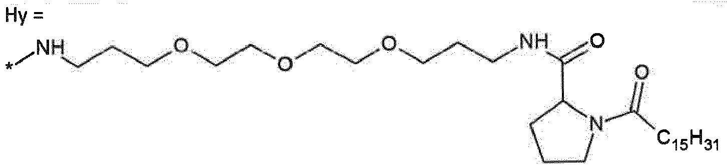 R₁ = CH₃-CO- ou H ou pyroglutamate |
| AB11 | 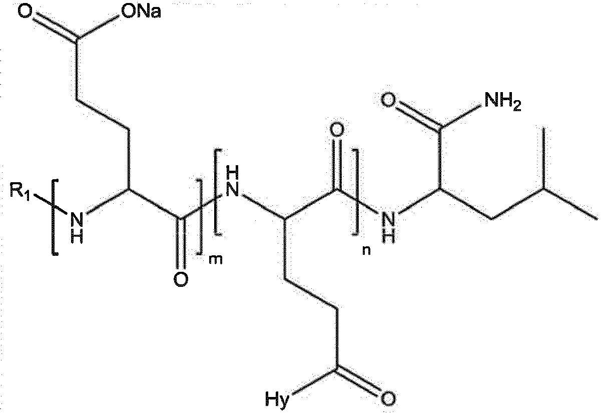 i = 0,05, DP (m + n) = 23 |
| | 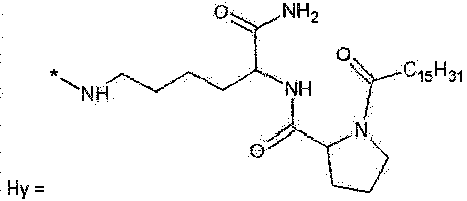 R₁ = H ou pyroglutamate |
| AB12 | 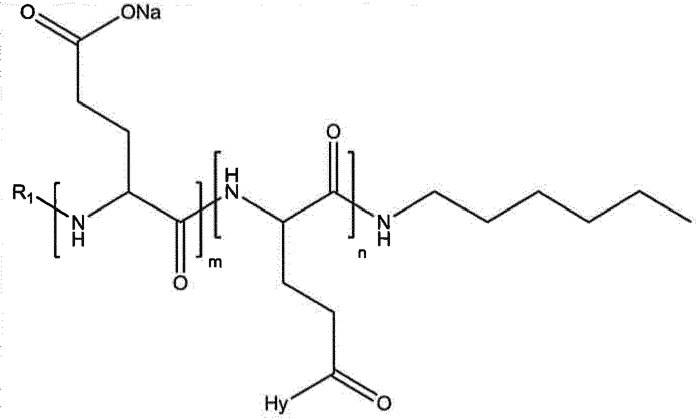 i = 0,04, DP (m + n) = 26 |
| | 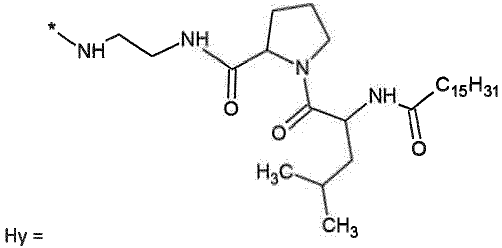 R₁ = H ou pyroglutamate |
| AB13 | 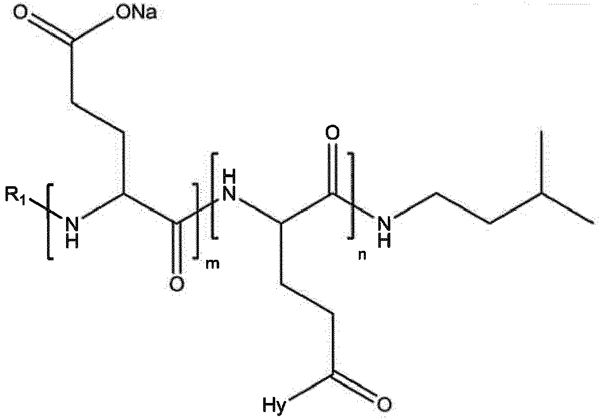 i = 0,12, DP (m+n) = 35 |
| | 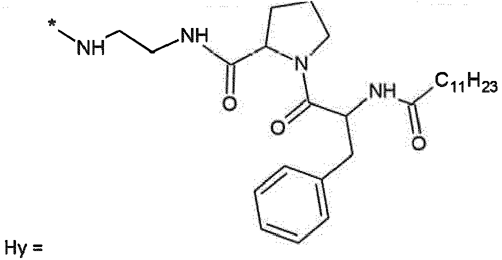 R₁ = H ou pyroglutamate |
| AB21 | 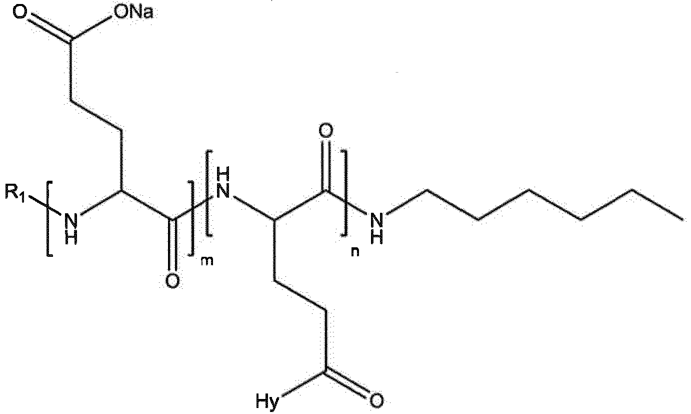 i = 0,056, DP (m + n) = 22 |
| | 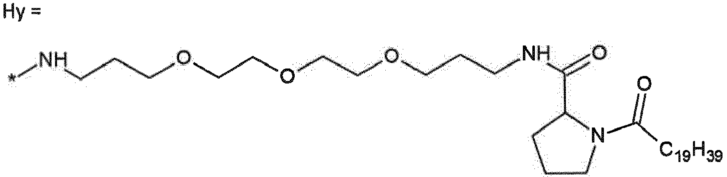 R₁ = H ou pyroglutamate |
| AB22 | 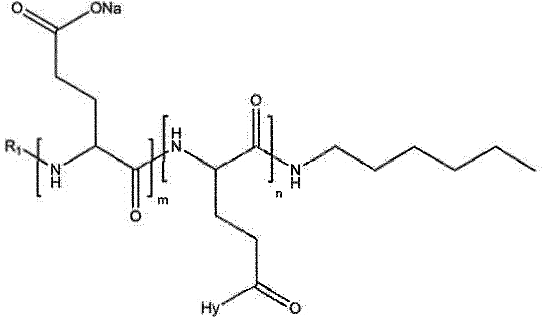 i = 0,16, DP (m + n) = 38 |
| | 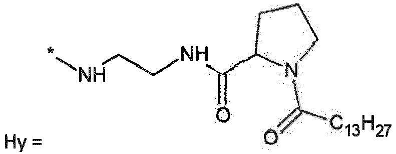 R₁ = CH₃CO ou pyroglutamate |
| AB23 | 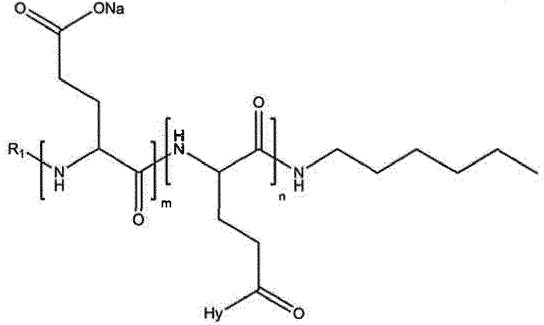 i = 0,10, DP (m + n) = 60 |
| | 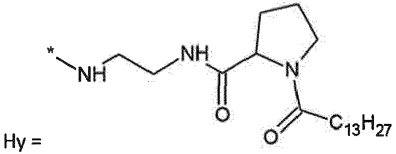 R₁ = H ou pyroglutamate |
| AB24 | 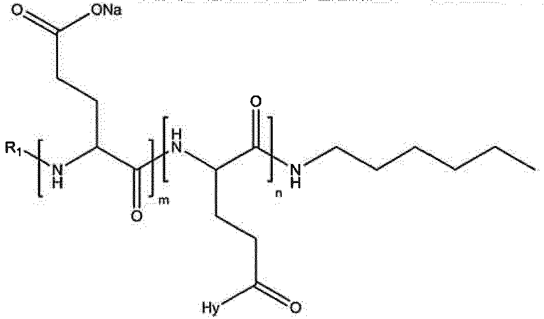 i = 0,15, DP (m + n) = 39 |
| | 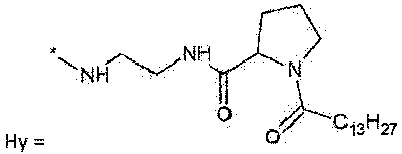 R₁ = H ou pyroglutamate |
| AB25 | 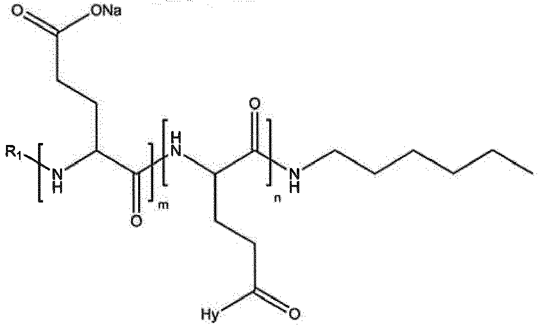 i = 0,20, DP (m + n) = 39 |
| | 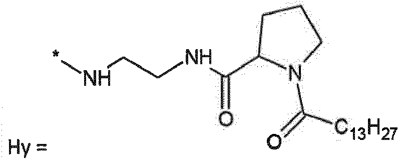 R₁ = H ou pyroglutamate |
| AB26 | 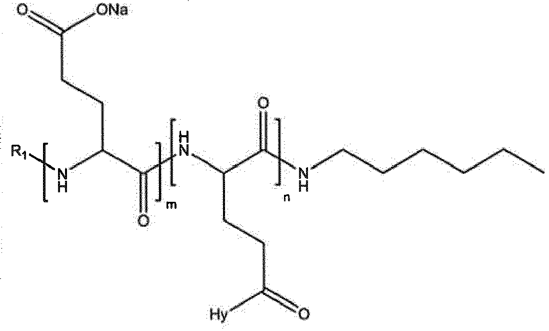 i = 0,20, DP (m + n) = 22 |
| | 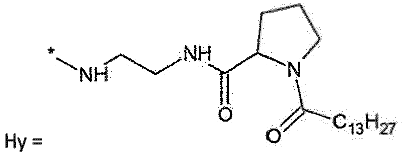 R₁ = H ou pyroglutamate |
| AB27 | 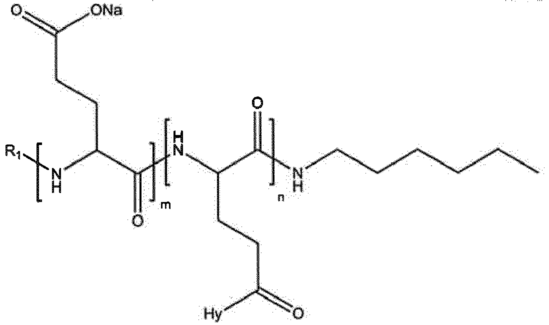 i = 0,15, DP (m + n) = 39 |
| | 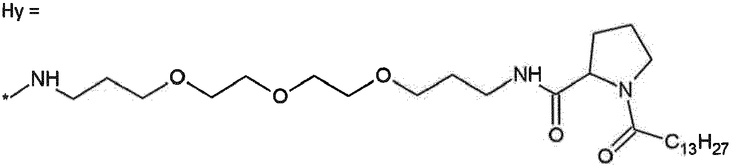 R₁ = H ou pyroglutamate |
| AB28 | 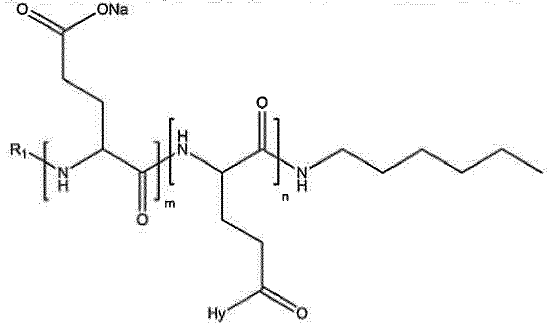 i = 0,15, DP (m + n) = 39 |
| | 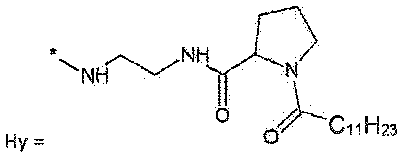 R₁ = H ou pyroglutamate |
| A629 | 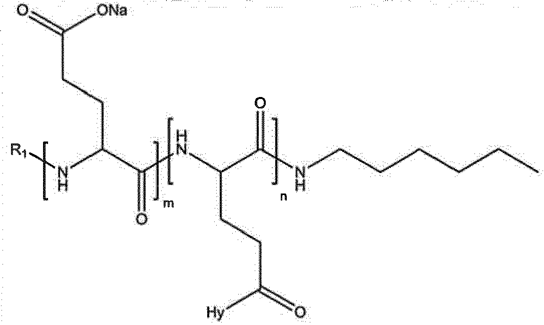 i = 0,15, DP (m + n) = 40 |
| | 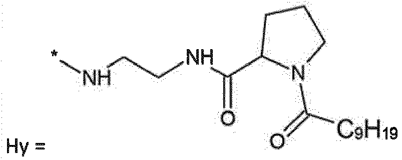 R₁ = pyroglutamate |
| AB30 | 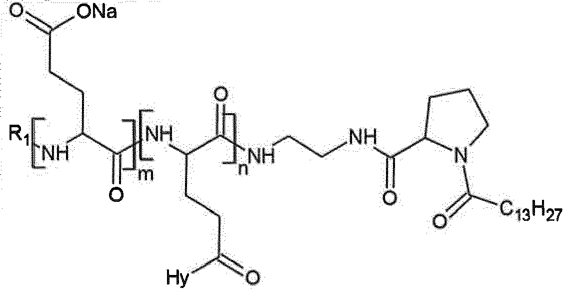 i = 0,125, DP (m + n) = 40 |
| | 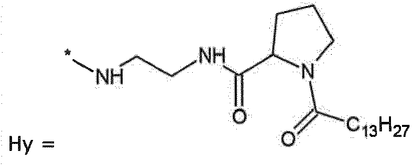 R₁ = pyroglutamate |
| AB31 | 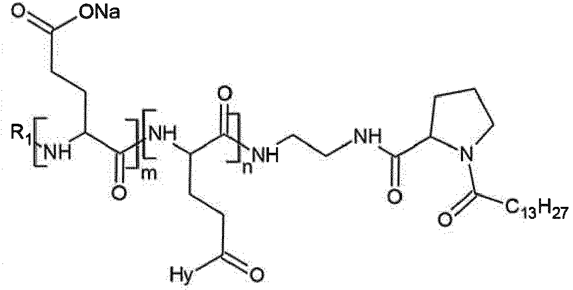 i = 0,175, DP (m + n) = 40 |
| | 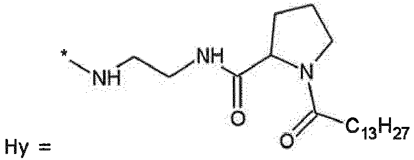 R₁ = pyroglutamate |
| AB32 | 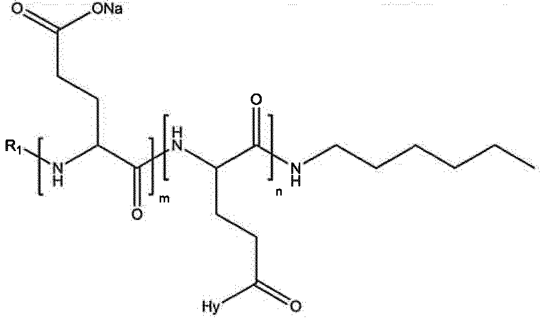 i = 0,109 DP (m + n) = 40 |
| | 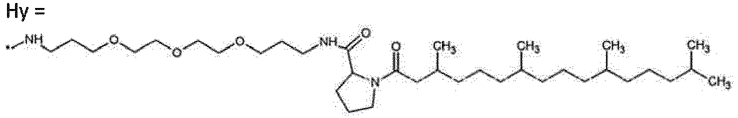 R₁ = pyroglutamate |

### Co-polyaminoacides définis de formule VII ou VIIb

**Tableau 1C : liste des co-polyaminoacides de formule VII ou VIIb synthétisés selon l'invention.**

| **n° exemple** | **co-polyaminoacides porteur de charges carboxylates et de radicaux hydrophobes** |
|---|---|
| AB14 | 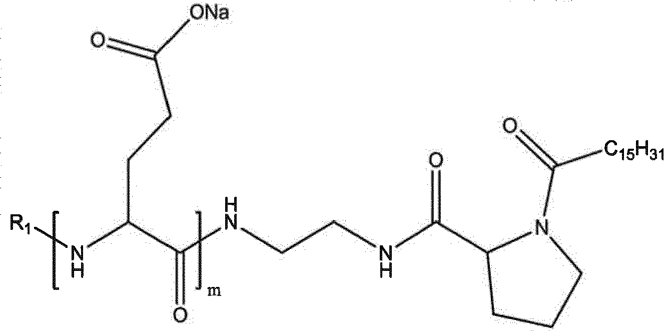 i = 0,04, DP (m) = 25 |
| | R₁ = H ou pyroglutamate |
| AB15 | 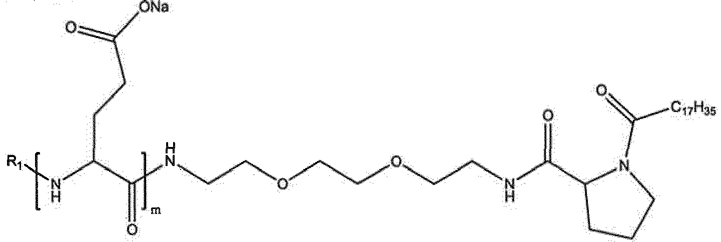 i = 0,033, DP (m) = 30 |
| | R₁ = H ou pyroglutamate |
| AB16 | 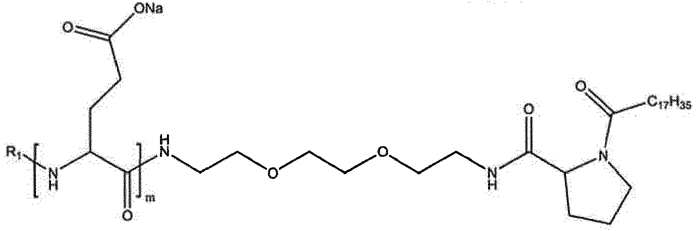 i = 0,021, DP (m) = 48 |
| | R₁ = H ou pyroglutamate |
| AB17 | 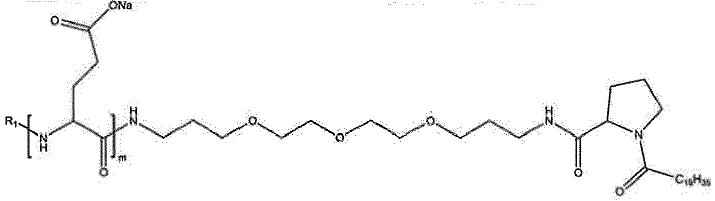 i = 0,038, DP (m) = 26 |
| | R₁ = H ou pyroglutamate |
| AB18 | 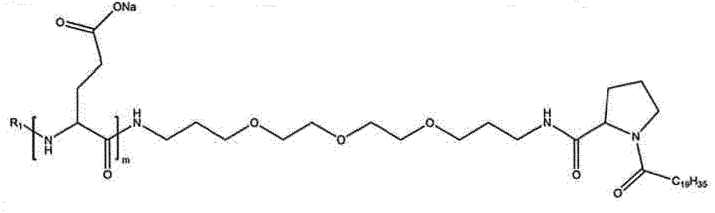 i = 0,045, DP (m) = 22 |
| | R₁ = H ou pyroglutamate |
| AB19 | 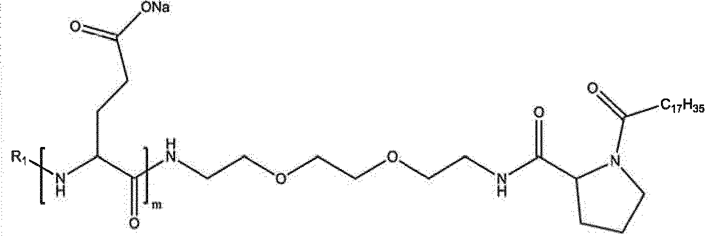 i = 0,015, DP (m) = 65 |
| | R₁ = H ou pyroglutamate |
| AB20 | 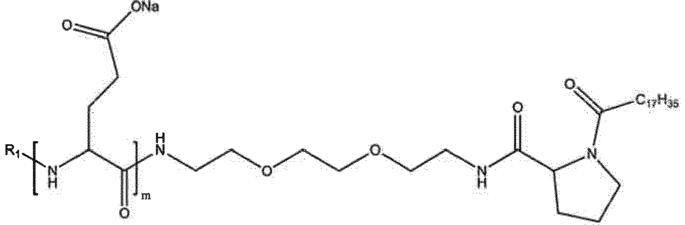 i = 0,017, DP (m) = 60 |
| | R1 = CH₃-CO-, H ou pyroglutamate |

### Co-polyaminoacides de formule VII ou VIIa

### Exemple AB1 : Co-polyaminoacide AB1 - poly-L-glutamate de sodium modifié par la molécule AA1 et ayant une masse molaire moyenne en nombre (Mn) de 2900 g/mol.

Co-polyaminoacide AB1-1 : acide poly-L-glutamique de masse molaire moyenne en nombre (Mn) relative 3861 g/mol issu de la polymérisation du γ-benzyl-L-glutamate N-carboxyanhydride initiée par l'hexylamine.

Dans un ballon préalablement séché à l'étuve est placé sous vide du γ-benzyl-L-glutamate N-carboxyanhydride (89,9 g, 341 mmol) pendant 30 min, puis du DMF anhydre (200 mL) est introduit. Le mélange est alors agité sous argon jusqu'à complète dissolution, refroidi à 4 °C, puis de l'hexylamine (2,05 mL 15,5 mmol) est introduite rapidement. Le mélange est agité entre 4 °C et température ambiante pendant 2 jours. Le milieu réactionnel est ensuite chauffé à 65 °Cpendant 2 h, refroidi à température ambiante puis coulé goutte à goutte dans du diisopropyléther (3 L) sous agitation. Le précipité blanc est récupéré par filtration, lavé avec du diisopropyléther (2 x 200 mL) puis séché sous vide à 30 °C pour donner un acide poly(gamma-benzyl-L-glutamique) (PBLG).

À une solution de PBLG (74,8 g) dans l'acide trifluoroacétique (TFA, 340 mL) à 4 °C est ajoutée goutte à goutte une solution d'acide bromhydrique (HBr) à 33 % dans l'acide acétique (240 mL, 1,37 mol). Le mélange est agité à température ambiante pendant 2 h, puis coulé goutte à goutte sur un mélange 1:1 (v/v) de diisopropyléther et d'eau sous agitation (4 L). Après 2 h d'agitation, le mélange hétérogène est laissé au repos pendant une nuit. Le précipité blanc est récupéré par filtration, lavé avec un mélange 1:1 (v/v) de diisopropyléther et d'eau (340 mL) puis avec de l'eau (340 mL).

Le solide obtenu est alors solubilisé dans de l'eau (1,5 L) en ajustant le pH à 7 par ajout d'une solution aqueuse de soude 10 N puis une solution aqueuse de soude 1 N. Après solubilisation, la concentration théorique est ajustée à 20 g/L théorique par addition d'eau pour obtenir un volume final de 2,1 L.

La solution est filtrée sur filtre 0,45 µm puis purifiée par ultrafiltration contre une solution de NaCl 0,9 %, puis de l'eau jusqu'à ce que la conductimétrie du perméat soit inférieure à 50 µS/cm. La solution de co-polyaminoacide est ensuite concentrée jusqu'à obtenir un volume final de 1,8 L.

La solution aqueuse est alors acidifiée par ajout de solution d'acide chlorhydrique 37 % jusqu'à atteindre un pH de 2. Après 4 h d'agitation, le précipité obtenu est filtré, lavé avec de l'eau (2 x 340 mL) puis séché sous vide à 30 °C pour donner un acide poly-L-glutamique de masse molaire moyenne en nombre (Mn) 3861 g/mol par rapport à un standard de polyoxyéthylène (PEG).

### Co-polyaminoacide AB1

Le co-polyaminoacide AB1-1 (10,0 g) est solubilisé dans le DMF (700 mL) à 30 °C puis refroidi à 0 °C. La molécule AA1 sous forme de sel de chlorhydrate (1,64 g, 3,8mmol) est mise en suspension dans du DMF (23 mL) et de la triéthylamine (0,39 g, 3,8 mmol) est ensuite ajoutée et le mélange est légèrement chauffé sous agitation jusqu'à complète dissolution. À la solution de co-polyaminoacide à 0 °C, de la *N-*méthylmorpholine (NMM, 7,6 g, 75 mmol) dans le DMF (14 mL) et du chloroformate d'éthyle (ECF, 8,2 g, 75 mmol) sont ajoutés. Après 10 min à 0 °C, la solution contenant la molécule AA1 est ajoutée et le milieu maintenu à 30 °Cdurant 2 h. Le milieu réactionnel est coulé goutte-à-goutte sur 5,5 L d'eau contenant du NaCl à 15 % massique et du HCl (pH 2), puis laissé reposer une nuit. Le précipité est collecté par filtration et séché sous vide pendant environ 30 min. Le solide blanc obtenu est repris dans de l'eau (500 mL) et le pH est ajusté à 7 par ajout lent d'une solution aqueuse de NaOH 1 N. Après filtration sur filtre 0,45 µm, la solution limpide obtenue est purifiée par ultrafiltration contre une solution de NaCl 0,9 % puis de l'eau, jusqu'à ce que la conductimétrie du perméat soit inférieure à 50 µS/cm. Après déchargement, la solution est filtrée sur filtre 0,2 µm et stockée à 2-8 °C.
Extrait sec : 24,9 mg/g.
Un degré de polymérisation moyen (DP) de 23 est estimé par RMN ¹H dans D₂O en comparant l'intégration des signaux provenant de l'hydrophobe greffé à celle des signaux provenant de la chaîne principale.
D'après la RMN ¹H : i = 0,05.

La masse molaire moyenne calculée du co-polyaminoacide AB1 est calculée sur la base des masses molaires des radicaux R₁ et R₂, des résidus aspartate et/ou glutamate (y compris une liaison amide), du radical hydrophobe, du DS et du DP.

La masse molaire moyenne calculée du co-polyaminoacide AB1 est de 3945 g/mol. HPLC-SEC aqueuse (calibrant PEG) : Mn = 2900 g/mol.

### Exemple AB2 : Co-polyaminoacide AB2 - poly-L-glutamate de sodium modifié par la molécule AA1 et ayant une masse molaire moyenne en nombre (Mn) de 3700 g/mol.

Par un procédé similaire à celui utilisé pour la préparation du co-polyaminoacideAB1 appliqué au sel de chlorhydrate de la molécule AA1 (1,64 g, 3,8 mmol) et à un acide poly-L-glutamique de Mn relative 5200 g/mol (10,0 g) obtenu par un procédé similaire à celui utilisé pour la préparation du co-polyaminoacide AB1-1, un poly-L-glutamate de sodium modifié par la molécule AA1 est obtenu.
Extrait sec : 14,1 mg/g.
DP (estimé d'après la RMN ¹H) : 35.
D'après la RMN ¹H : i = 0,05.
La masse molaire moyenne calculée du co-polyaminoacide AB2 est de 5972 g/mol. HPLC-SEC aqueuse (calibrant PEG) : Mn = 3700 g/mol.

### Exemple AB3 : Co-polyaminoacide AB3 - poly-L-glutamate de sodium modifié par la molécule AA1 et ayant une masse molaire moyenne en nombre (Mn) de 4900 g/mol.

Par un procédé similaire à celui utilisé pour la préparation du co-polyaminoacide AB1 appliqué au sel de chlorhydrate de la molécule AA1 (3,30 g, 7,6 mmol) et à un acide poly-L-glutamique de masse moyenne en nombre (Mn) relative 5200 g/mol (10,0 g) obtenu par un procédé similaire à celui utilisé pour la préparation du co-polyaminoacide AB1-1, un poly-L-glutamate de sodium modifié par la molécule AA1 est obtenu.
Extrait sec : 23,4 mg/g.
DP (estimé d'après la RMN ¹H) : 35.
La masse molaire moyenne calculée du co-polyaminoacide AB3 est de 6594 g/mol. D'après la RMN ¹H : i = 0,10.
HPLC-SEC aqueuse (calibrant PEG) : Mn = 4900 g/mol.

### Exemple AB4 : Co-polyaminoacide AB4 - poly-L-glutamate de sodium modifié par la molécule AA2 et ayant une masse molaire moyenne en nombre (Mn) de 1800 g/mol.

Par un procédé similaire à celui utilisé pour la préparation du co-polyaminoacide AB1 appliqué au sel de chlorhydrate de la molécule AA2 (1,09 g, 2,4 mmol) et à un acide poly-L-glutamique de masse moyenne Mn = 5600 g/mol (6,3 g) obtenu par un procédé similaire à celui utilisé pour la préparation du co-polyaminoacide AB1-1 mais avec une étape de déprotection des esters benzyliques utilisant l'iodure de triméthylsilane selon le protocole décrit dans la publication Subramanian G., et al., J. Am. Chem. Soc. 2000, 122, 26-34, un poly-L-glutamate de sodium modifié par la molécule AA2 est obtenu.
Extrait sec : 21,5 mg/g.
DP (estimé d'après la RMN ¹H) : 35.
D'après la RMN ¹H : i = 0,052.
La masse molaire moyenne calculée du co-polyaminoacide AB4 est de 6022 g/mol. HPLC-SEC aqueuse (calibrant PEG) : Mn = 1800 g/mol.

### Exemple ABS : Co-polyaminoacide AB5 - poly-L-glutamate de sodium modifié par la molécule AA6 et ayant une masse molaire moyenne en nombre (Mn) de 2600 g/mol.

Par un procédé similaire à celui utilisé pour la préparation du co-polyaminoacide AB1 appliqué au sel de chlorhydrate de la molécule AA6 (2,06 g, 3,8 mmol) et à un acide poly-L-glutamique (9,8 g) obtenu par un procédé similaire à celui utilisé pour la préparation du co-polyaminoacide AB1-1, un poly-L-glutamate de sodium modifié par la molécule AA6 est obtenu.
Extrait sec : 20,9 mg/g.
DP (estimé d'après la RMN 1H) : 23.
D'après la RMN 1H : i = 0,05.
La masse molaire moyenne calculée du co-polyaminoacide AB5 est de 4079 g/mol. HPLC-SEC aqueuse (calibrant PEG) : Mn = 2600 g/mol.

### Exemple AB6 : Co-polyaminoacide AB6 - poly-L-glutamate de sodium modifié par la molécule AA7 et ayant une masse molaire moyenne en nombre (Mn) de 4000 g/mol.

Un acide poly-L-glutamique de masse moyenne Mn = 3500 g/mol et de degré de polymérisation 22 (10,0 g) obtenu par un procédé similaire à celui utilisé pour la préparation du co-polyaminoacide AB1-1 est solubilisé dans le DMF (420 mL) à 30-40 °C puis maintenu à cette température. En parallèle, le sel de chlorhydrate de la molécule AA7 (1,47 g, 2,3 mmol) est mis en suspension dans du DMF (12 mL) et de la triéthylamine (0,23 g, 2,3 mmol) est ajoutée, puis le mélange est légèrement chauffé sous agitation jusqu'à complète dissolution. À la solution de co-polyaminoacide dans le DMF, de la NMM (7,6 g, 75 mmol), la solution de AA7 puis de la N-oxyde de 2-hydroxypyridine (HOPO, 0,84 g, 7,5 mmol) sont ajoutées successivement. Le milieu réactionnel est alors refroidi à 0 °C, puis du EDC (1,44 g, 7,5 mmol) est ajouté et le milieu est remonté à température ambiante durant 2 h. Le milieu réactionnel est filtré sur filtre tissé 0,2 mm et coulé au goutte-à-goutte sur 3,5 L d'eau contenant du NaCl à 15 % massique et du HCl (pH 2) sous agitation. À la fin de l'ajout, le pH est réajusté à 2 avec une solution de HCl 37 %, et la suspension est laissée reposer une nuit. Le précipité est collecté par filtration, puis rincé par 100 mL d'eau. Le solide blanc obtenu est solubilisé dans 500 mL d'eau par ajout lent d'une solution aqueuse de NaOH 1 N jusqu'à pH 7 sous agitation, puis la solution est filtrée sur filtre 0,45 µm. La solution limpide obtenue est purifiée par ultrafiltration contre une solution de NaCl 0,9 % puis de l'eau, jusqu'à ce que la conductimétrie du perméat soit inférieure à 50 µS/cm. La solution est filtrée sur filtre 0,2 µm et stockée à 2-8 °C.
Extrait sec : 21,6 mg/g.
DP (estimé d'après la RMN 1H) : 20.
D'après la RMN 1H : i = 0,025.
La masse molaire moyenne calculée du co-polyaminoacide AB6 est de 3369 g/mol. HPLC-SEC aqueuse (calibrant PEG) : Mn = 4000 g/mol.

### Exemple AB7 : Co-polyaminoacide AB7 - poly-L-glutamate de sodium cappé à une de ses extrémités par un groupement acétyle et modifié par la molécule AA7 et ayant une masse molaire moyenne en nombre (Mn) de 3300 g/mol.

Co-polyamingacide AB7-1 : acide poly-L-glutamique de masse molaire moyenne en nombre (Mn) relative 3600 g/mol et de DP 21 issu de la polymérisation du γ-benzyl-L-glutamate N-carboxyanhydride initiée par l'hexylamine et cappé à l'une de ses extrémités par un groupement acétyle.

Dans un ballon préalablement séché à l'étuve est placé sous vide du γ-benzyl-L-glutamate N-carboxyanhydride (Glu(OBn)-NCA, 100,0 g, 380 mmol) pendant 30 min puis du DMF anhydre (225 mL) est introduit. Le mélange est alors agité sous argon jusqu'à complète dissolution, refroidi à 4 °C, puis de l'hexylamine (1,78 g, 17 mmol) est introduite rapidement. Le mélange est agité entre 4 °C et température ambiante pendant 2 jours puis précipité dans du diisopropyléther (3,4 L). Le précipité est récupéré par filtration, lavé deux fois avec du diisopropyléther (225 mL) puis séché pour donner un solide blanc qui est dissous dans 450 mL de THF. À cette solution sont ajoutés successivement de la DIPEA, (31 mL, 176 mmol) puis de l'anhydride acétique (17 mL, 176 mmol). Après une nuit d'agitation à température ambiante, la solution est versée lentement dans du diisopropyléther (3 L) sous agitation. Après 1 h d'agitation, le précipité est filtré, lavé deux fois avec du diisopropyléther (250 mL) puis séché sous vide à 30 °C pour donner un acide poly(gamma-benzyl-L-glutamique) cappé à une de ses extrémités par un groupement acétyle.

À une solution du co-polyaminoacide ci-dessus (72 g) dans l'acide trifluoroacétique (TFA, 335 mL) à 4 °C est ajoutée goutte à goutte une solution d'acide bromhydrique (HBr) à 33 % dans l'acide acétique (235 mL). Le mélange est agité à température ambiante pendant 3 h 30, puis coulé goutte à goutte sur un mélange 1:1 (v/v) de diisopropyléther et d'eau sous agitation (4 L). Après 2 h d'agitation, le mélange hétérogène est laissé au repos pendant une nuit. Le précipité blanc est récupéré par filtration, lavé avec un mélange 1:1 (v/v) de diisopropyléther et d'eau (340 mL) puis avec de l'eau (340 mL).

Le solide obtenu est alors solubilisé dans de l'eau (1,5 L) en ajustant le pH à 7 par ajout d'une solution aqueuse de soude 10 N puis une solution aqueuse de soude 1 N. Après solubilisation, la solution est diluée par addition d'eau pour obtenir un volume final de 2,1 L. La solution est filtrée sur filtre 0,45 µm puis purifiée par ultrafiltration contre une solution de NaCl 0,9 %, puis de l'eau jusqu'à ce que la conductimétrie du perméat soit inférieure à 50 µS/cm. La solution de co-polyaminoacide est ensuite concentrée jusqu'à obtenir un volume final de 1,8 L.

La solution aqueuse est alors acidifiée par ajout de solution d'acide chlorhydrique 37 % jusqu'à atteindre un pH de 2. Après 4 h d'agitation, le précipité obtenu est filtré, lavé avec de l'eau (330 mL) puis séché sous vide à 30 °C pour donner un acide poly-L-glutamique de masse molaire moyenne en nombre (Mn) 3600 g/mol par rapport à un standard de polyoxyéthylène (PEG), et de degré moyen de polymérisation 21.

### Co-polyaminoacide AB7 :

Par un procédé similaire à celui utilisé pour la préparation du co-polyaminoacide AB6 appliqué au sel de chlorhydrate de la molécule AA7 (1,43 g, 2,2 mmol) et au co-polyaminoacide AB7-1 (10,0 g), un acide poly-L-glutamate de sodium modifié par la molécule AA7 est obtenu.
Extrait sec : 24,3 mg/g.
DP (estimé d'après la RMN 1H) : 21.
D'après la RMN 1H : i = 0,03.
La masse molaire moyenne calculée du co-polyaminoacide AB7 est de 3677 g/mol. HPLC-SEC aqueuse (calibrant PEG) : Mn = 3300 g/mol.

### Exemple AB8 : Co-polyaminoacide AB8 - poly-L-glutamate de sodium modifié par la molécule AA7 et ayant une masse molaire moyenne en nombre (Mn) de 3600 g/mol.

Co-polyaminoacide AB8-1_: acide poly-L-glutamique de masse molaire moyenne en nombre (Mn) 3800 g/mol et de degré de polymérisation 24 issu de la polymérisation du γ-méthyl-L-glutamate N-carboxyanhydride initiée par l'ammoniac.

Par un procédé similaire à celui décrit dans la demande de brevet FR-A-2 801 226 appliqué au γ-méthyl-L-acide glutamique N-carboxyanhydride (25,0 g, 133,6 mmol) et à une solution d'ammoniaque 0,5 N dans le dioxane (12,1 mL, 6,05 mmol), un acide poly-L-glutamique est obtenu.

### Co-polyaminoacide AB8 :

Par un procédé similaire à celui utilisé pour la préparation du co-polyaminoacide AB6 appliqué au sel de chlorhydrate de la molécule AA7 (2,1 g, 3,24 mmol) et au co-polyaminoacide AB8-1 (14,3 g), un poly-L-glutamate de sodium modifié par la molécule AA7 est obtenu.
Extrait sec : 25,2 mg/g.
DP (estimé d'après la RMN 1H) : 24.
D'après la RMN ¹H : i = 0,03.
La masse molaire moyenne calculée du co-polyaminoacide AB8 est de 4099 g/mol. HPLC-SEC aqueuse (calibrant PEG) : Mn = 3600 g/mol.

### Exemple AB9 : Co-polyaminoacide AB9 - poly-L-glutamate de sodium modifié par la molécule AA3 et ayant une masse molaire moyenne en nombre (Mn) de 3200 g/mol.

Par un procédé similaire à celui utilisé pour la préparation du co-polyaminoacide AB1 appliqué au sel de chlorhydrate de la molécule AA3 et à un acide poly-L-glutamique obtenu par un procédé similaire à celui utilisé pour la préparation du co-polyaminoacide AB1-1, un poly-L-glutamate de sodium modifié par la molécule AA3 est obtenu.
Extrait sec : 14,7 mg/g.
DP (estimé d'après la RMN ¹H) : 30.
D'après la RMN ¹H : i = 0,12.
La masse molaire moyenne calculée du co-polyaminoacide AB9 est de 6192 g/mol. HPLC-SEC aqueuse (calibrant PEG) : Mn = 3200 g/mol.

### Exemple AB10 : Co-polyaminoacide AB10 - poly-L-glutamate de sodium modifié par la molécule AA4 et ayant une masse molaire moyenne en nombre (Mn) de 2600 g/mol.

Par un procédé similaire à celui utilisé pour la préparation du co-polyaminoacide AB7 appliqué au sel de chlorhydrate de la molécule AA4 et à un acide poly-L-glutamique obtenu par un procédé similaire à celui utilisé pour la préparation du co-polyaminoacide AB1-1, un poly-L-glutamate de sodium modifié par la molécule AA4 est obtenu.
Extrait sec : 18,3 mg/g.
DP (estimé d'après la RMN ¹H) : 25.
D'après la RMN ¹H : i = 0,08.
La masse molaire moyenne calculée du co-polyaminoacide AB10 est de 4870 g/mol. HPLC-SEC aqueuse (calibrant PEG) : Mn = 2600 g/mol.

### Exemple AB11 : Co-polyaminoacide AB11 - poly-L-glutamate de sodium modifié par la molécule AA5 et ayant une masse molaire moyenne en nombre (Mn) de 2700 g/mol.

Par un procédé similaire à celui utilisé pour la préparation du co-polyaminoacide AB6 appliqué au sel de chlorhydrate de la molécule AA5 et à un acide poly-L-glutamique obtenu par un procédé similaire à celui utilisé pour la préparation du co-polyaminoacide AB1-1, un poly-L-glutamate de sodium modifié par la molécule AA5 est obtenu.
Extrait sec : 20,2 mg/g.
DP (estimé d'après la RMN ¹H) : 23.
D'après la RMN ¹H : i = 0,05.
La masse molaire moyenne calculée du co-polyaminoacide AB11 est de 4072 g/mol. HPLC-SEC aqueuse (calibrant PEG) : Mn = 2700 g/mol.

### Exemple AB12 : Co-polyaminoacide AB12 - poly-L-glutamate de sodium modifié par la molécule AA8 et ayant une masse molaire moyenne en nombre (Mn) de 3000 g/mol.

Par un procédé similaire à celui utilisé pour la préparation du co-polyaminoacide AB1 appliqué au sel de chlorhydrate de la molécule AA8 et à un acide poly-L-glutamique obtenu par un procédé similaire à celui utilisé pour la préparation du co-polyaminoacide AB1-1, un poly-L-glutamate de sodium modifié par la molécule AA8 est obtenu.
Extrait sec : 19,5 mg/g.
DP (estimé d'après la RMN ¹H) : 26.
D'après la RMN ¹H : i = 0,04.
La masse molaire moyenne calculée du co-polyaminoacide AB12 est de 4477 g/mol. HPLC-SEC aqueuse (calibrant PEG) : Mn = 3000 g/mol.

### Exemple AB13 : Co-polyaminoacide AB13 - poly-L-glutamate de sodium modifié par la molécule AA9 et ayant une masse molaire moyenne en nombre (Mn) de 3300 g/mol.

Par un procédé similaire à celui utilisé pour la préparation du co-polyaminoacide AB6 appliqué au sel de chlorhydrate de la molécule AA9 et à un acide poly-L-glutamique obtenu par un procédé similaire à celui utilisé pour la préparation du co-polyaminoacide AB1-1 en utilisant l'isoamylamine comme initiateur en place de l'hexylamine, un poly-L-glutamate de sodium modifié par la molécule AA9 est obtenu.
Extrait sec : 22,3 mg/g.
DP (estimé d'après la RMN ¹H) : 35.
D'après la RMN ¹H : i = 0,12.
La masse molaire moyenne calculée du co-polyaminoacide AB13 est de 7226 g/mol. HPLC-SEC aqueuse (calibrant PEG) : Mn = 3300 g/mol.

### Exemple AB21 : Co-polyaminoacide AB21 - poly-L-glutamate de sodium modifié par la molécule AA7 et ayant une masse molaire moyenne en nombre (Mn) de 3400 g/mol.

Par un procédé similaire à celui utilisé pour la préparation du co-polyaminoacide AB6 appliqué au sel de chlorhydrate de la molécule AA7 (2,44 g, 2,4 mmol) et à un acide poly-L-glutamique (10 g) obtenu par un procédé similaire à celui utilisé pour la préparation du co-polyaminoacide AB1-1, un poly-L-glutamate de sodium modifié par la molécule AA7 est obtenu.
Extrait sec : 22,7 mg/g.
DP (estimé d'après la RMN ¹H) : 22.
D'après la RMN ¹H : i = 0,056.
La masse molaire moyenne calculée du co-polyaminoacide AB21 est de 4090 g/mol. HPLC-SEC aqueuse (calibrant PEG) : Mn = 3400 g/mol.

### Exemple AB22 : Co-polyaminoacide AB22 - poly-L-glutamate de sodium cappé à une de ses extrémités par un groupement acétyle et modifié par la molécule AA10 et ayant une masse molaire moyenne en nombre (Mn) de 4000 g/mol.

Le sel de chlorhydrate de la molécule AA10 (4,56 g, 11,29 mmol) est mis en solution dans du chloroforme (60 mL) et de la triéthylamine (1,14 g, 11,29 mmol) est ajoutée. À une solution de co-polyaminoacide (10,0 g, 75,3 mmol) obtenu selon un procédé similaire à celui utilisé pour la préparation du co-polyaminoacide B7-1 dans le DMF (420 mL), de la NMM (7,6 g, 75,26 mmol), puis de la HOPO (2,51 g, 22,58 mmol) sont ajoutées successivement. Le milieu réactionnel est alors refroidi à 0 °C, puis du EDC (4,33 g, 22,58 mmol) est ajouté, le milieu est agité 1 h à 0 °C puis la solution de molécule AA10 est ajoutée. Le mélange réactionnel est agité durant 2 h entre 0 °C et température ambiante. Le milieu réactionnel est filtré sur filtre tissé 0,2 mm et coulé au goutte-à-goutte sur 3,95 L d'eau contenant du NaCl à 15 % massique et du HCl (pH 2) sous agitation. À la fin de l'ajout, le pH est réajusté à 2 avec une solution de HCl 37 %, et la suspension est laissée reposer une nuit. Le précipité est collecté par filtration, puis solubilisé dans 780 mL d'eau par ajout lent d'une solution aqueuse de NaOH 1 N jusqu'à pH 7 sous agitation. Après filtration sur filtre 0,45 µm, la solution est diluée par addition d'eau puis de l'acétone est ajoutée pour obtenir une solution contenant 30 % massique d'acétone. Cette solution est filtrée sur filtre de charbon actif puis l'acétone est distillée (40 °C, 100 mbar). Après filtration sur filtre 0,45 µm, le produit est purifié par ultrafiltration contre une solution aqueuse de NaCl à 0,9 %, une solution de tampon carbonate (150 mM), une solution aqueuse de NaCl à 0,9 %, une solution de tampon phosphate (150 mM), une solution aqueuse de NaCl à 0,9 %, puis de l'eau jusqu'à ce que la conductimétrie du perméat soit inférieure à 50 µS/cm. La solution est ensuite concentrée, filtrée sur filtre 0,2 µm et stockée à 2-8 °C.
Extrait sec : 19,7 mg/g.
DP (estimé d'après la RMN ¹H) : 38.
D'après la RMN ¹H : i = 0,16.
La masse molaire moyenne calculée du co-polyaminoacide AB22 est de 7877 g/mol. HPLC-SEC organique (calibrant PEG) : Mn = 4000 g/mol.

### Exemple AB23 : Co-polyaminoacide AB23 - poly-L-glutamate de sodium et modifié par la molécule AA10 et ayant une masse molaire moyenne en nombre (Mn) de 7600 g/mol.

Co-polyaminoacide AB23-1 : acide poly-L-glutamique issu de la polymérisation du γ-benzyl-L-glutamate N-carboxyanhydride initiée par l'hexylamine et cappé à l'une de ses extrémités par un groupement pyroglutamate.

Un acide poly-L-glutamique (20,0 g) obtenu par un procédé similaire à celui utilisé pour la préparation du co-polyaminoacide AB1-1 est solubilisé dans le DMF à 80 °C puis maintenu à cette température. Après 24 h, le milieu réactionnel est coulé dans une solution de NaCl à 15 % et à pH 2. Après 4 h, le solide blanc est collecté par filtration, rincé à l'eau, puis séché sous vide à 30 °C.

### Co-polyaminoamide AB23

Par un procédé similaire à celui utilisé pour la préparation du co-polyaminoacide AB22 appliqué au sel de chlorhydrate de la molécule AA10 (2,742 g, 6,79 mmol) et au co-polyaminoacide AB23-1 (9,0 g), un acide poly-L-glutamate de sodium modifié par la molécule AA10 est obtenu.
Extrait sec : 21,9 mg/g.
DP (estimé d'après la RMN ¹H) : 60.
D'après la RMN ¹H : i = 0,1.
La masse molaire moyenne calculée du co-polyaminoacide AB23 est de 11034 g/mol. HPLC-SEC organique (calibrant PEG) : Mn = 7600 g/mol.

### Exemple AB24 : Co-polyaminoacide AB24 - poly-L-glutamate de sodium et modifié par la molécule AA10 et ayant une masse molaire moyenne en nombre (Mn) de 4300 g/mol.

Par un procédé similaire à celui utilisé pour la préparation du co-polyaminoacide AB23 appliqué au sel de chlorhydrate de la molécule AA10 et à un acide poly-L-glutamique obtenu par un procédé similaire à celui utilisé pour la préparation du co-polyaminoacide AB23-1, un poly-L-glutamate de sodium modifié par la molécule AA10 est obtenu.
Extrait sec : 22,9 mg/g.
DP (estimé d'après la RMN ¹H) : 39.
D'après la RMN ¹H : i = 0,15.
La masse molaire moyenne calculée du co-polyaminoacide AB24 est de 7870 g/mol. HPLC-SEC organique (calibrant PEG) : Mn = 4300 g/mol.

### Exemple AB25 : Co-polyaminoacide AB25 - poly-L-glutamate de sodium et modifié par la molécule AA10 et ayant une masse molaire moyenne en nombre (Mn) de 4200 g/mol.

Par un procédé similaire à celui utilisé pour la préparation du co-polyaminoacide AB23 appliqué au sel de chlorhydrate de la molécule AA10 et à un acide poly-L-glutamique de obtenu par un procédé similaire à celui utilisé pour la préparation du co-polyaminoacide AB23-1, un poly-L-glutamate de sodium modifié par la molécule AA10 est obtenu.
Extrait sec : 25,9 mg/g.
DP (estimé d'après la RMN ¹H) : 39.
D'après la RMN ¹H : i = 0,2.
La masse molaire moyenne calculée du co-polyaminoacide AB25 est de 8509 g/mol. HPLC-SEC organique (calibrant PEG) : Mn = 4200 g/mol.

### Exemple AB26 : Co-polyaminoacide AB26 - poly-L-glutamate de sodium et modifié par la molécule AA10 et ayant une masse molaire moyenne en nombre (Mn) de 2700 g/mol.

Par un procédé similaire à celui utilisé pour la préparation du co-polyaminoacide AB23 appliqué au sel de chlorhydrate de la molécule AA10 et à un acide poly-L-glutamique obtenu par un procédé similaire à celui utilisé pour la préparation du co-polyaminoacide AB23-1, un poly-L-glutamate de sodium modifié par la molécule AA10 est obtenu.
Extrait sec : 23,9 mg/g.
DP (estimé d'après la RMN ¹H) : 22.
D'après la RMN ¹H : i = 0,21.
La masse molaire moyenne calculée du co-polyaminoacide AB26 est de 4899 g/mol. HPLC-SEC organique (calibrant PEG) : Mn = 2700 g/mol.

### Exemple AB27 : Co-polyaminoacide AB27 - poly-L-glutamate de sodium et modifié par la molécule AA11 et ayant une masse molaire moyenne en nombre (Mn) de 4500 g/mol.

Par un procédé similaire à celui utilisé pour la préparation du co-polyaminoacide AB23 appliqué au sel de chlorhydrate de la molécule AA11 et à un acide poly-L-glutamique obtenu par un procédé similaire à celui utilisé pour la préparation du co-polyaminoacide AB23-1, un poly-L-glutamate de sodium modifié par la molécule AA11 est obtenu.
Extrait sec : 26,8 mg/g.
DP (estimé d'après la RMN ¹H) : 39.
D'après la RMN ¹H : i = 0,15.
La masse molaire moyenne calculée du co-polyaminoacide AB27 est de 8808 g/mol. HPLC-SEC organique (calibrant PEG) : Mn = 4500 g/mol.

### Exemple AB28 : Co-polyaminoacide AB28 - poly-L-glutamate de sodium et modifié par la molécule AA12 et ayant une masse molaire moyenne en nombre (Mn) de 4000 g/mol.

Par un procédé similaire à celui utilisé pour la préparation du co-polyaminoacide AB23 appliqué au sel de chlorhydrate de la molécule AA12 et à un acide poly-L-glutamique obtenu par un procédé similaire à celui utilisé pour la préparation du co-polyaminoacide AB23-1, un poly-L-glutamate de sodium modifié par la molécule AA12 est obtenu.
Extrait sec : 22,9 mg/g.
DP (estimé d'après la RMN ¹H) : 39.
D'après la RMN ¹H : i = 0,15.
La masse molaire moyenne calculée du co-polyaminoacide AB28 est de 7706 g/mol. HPLC-SEC organique (calibrant PEG) : Mn = 4000 g/mol.

### Exemple AB29 : Co-polyaminoacide AB29 - poly-L-glutamate de sodium et modifié par la molécule AA13 et ayant une masse molaire moyenne en nombre (Mn) de 4000 g/mol.

Co-polyaminoacide B29-1 : acide poly-L-glutamique issu de la polymérisation du γ-benzyl-L-glutamate *N*-carboxyanhydride initiée par l'hexylamine. Dans un réacteur à double enveloppe, du γ-benzyl-L-glutamate *N*-carboxyanhydride (500 g, 1,90 mol) est solubilisé dans du DMF anhydre (1100 mL). Le mélange est alors agité jusqu'à complète dissolution, refroidi à 0 °C, puis de l'hexylamine (6,27 mL, 47,5 mmol) est introduite rapidement. Le mélange est agité à 0 °Cpendant 5 h, entre 0 °C et 20 °C pendant 7 h, puis à 20 °C pendant 7 h. Le milieu réactionnel est ensuite chauffé à 65 °C pendant 2 h, refroidi à 55 °C et du méthanol (3300 mL) est introduit en 1 h 30. Le mélange réactionnel est alors refroidi à 0 °C et laissé sous agitation pendant 18 h. Le précipité blanc est récupéré par filtration, lavé au diisopropyléther (2 x 800 mL) puis séché sous pression réduite à 30 °C pour donner un acide poly(gamma-benzyl-L-glutamique) (PBLG).

À une solution de PBLG (180 g) dans du *N,N*-diméthylacétamide (DMAc, 450 mL) est ajouté du Pd/Al₂O₃ (36 g) sous atmosphère d'argon. Le mélange est placé sous atmosphère d'hydrogène (10 bar) et agité à 60 °C pendant 24 h. Après refroidissement à température ambiante et filtration du catalyseur sur fritté P4 puis sur membrane Omnipore 0,2 µm PTFE hydrophile, une solution d'eau à pH 2 (2700 mL) est coulée goutte à goutte sur la solution de DMAc, sur une période de 45 min et sous agitation. Après 18 h sous agitation, le précipité blanc est récupéré par filtration, lavé avec de l'eau puis séché sous pression réduite à 30 °C.

### Co-polyaminoacide AB29

Par un procédé similaire à celui utilisé pour la préparation du co-polyaminoacide AB23 appliqué au sel de chlorhydrate de la molécule AA13 et au co-polyaminoacide AB29-1, un poly-L-glutamate de sodium modifié par la molécule AA13 est obtenu.
Extrait sec : 16,1 mg/g.
DP (estimé d'après la RMN ¹H) : 40.
D'après la RMN ¹H : i = 0,15.
La masse molaire moyenne calculée du co-polyaminoacide AB29 est de 7734 g/mol. HPLC-SEC organique (calibrant PEG) : Mn = 4000 g/mol.

### Exemple AB30 : Co-polyaminoacide AB30 - poly-L-glutamate de sodium et modifié par la molécule AA10 et ayant une masse molaire moyenne en nombre (Mn) de 4300 g/mol.

Par un procédé similaire à celui utilisé pour la préparation du co-polyaminoacide AB29 appliqué au sel de chlorhydrate de la molécule AA10 et à un acide poly-L-glutamique obtenu par un procédé similaire à celui utilisé pour la préparation du co-polyaminoacide AB29-1 en utilisant la molécule AA10 comme initiateur en place de l'hexylamine, un poly-L-glutamate de sodium modifié par la molécule AA10 est obtenu.
Extrait sec : 29,2 mg/g.
DP (estimé d'après la RMN ¹H) : 40.
D'après la RMN ¹H : i = 0,125.
La masse molaire moyenne calculée du co-polyaminoacide AB30 est de 7682 g/mol. HPLC-SEC organique (calibrant PEG) : Mn = 4300 g/mol.

### Exemple AB31 : Co-polyaminoacide AB30 - poly-L-glutamate de sodium et modifié par la molécule AA10 et ayant une masse molaire moyenne en nombre (Mn) de 6300 g/mol.

Par un procédé similaire à celui utilisé pour la préparation du co-polyaminoacide AB29 appliqué au sel de chlorhydrate de la molécule AA10 et à un acide poly-L-glutamique obtenu par un procédé similaire à celui utilisé pour la préparation du co-polyaminoacide AB29-1 en utilisant la molécule AA10 comme initiateur au lieu de l'hexylamine, un poly-L-glutamate de sodium modifié par la molécule AA10 est obtenu.
Extrait sec : 23,1 mg/g.
DP (estimé d'après la RMN ¹H) : 40.
D'après la RMN ¹H : i = 0,175.
La masse molaire moyenne calculée du co-polyaminoacide AB31 est de 8337 g/mol. HPLC-SEC organique (calibrant PEG) : Mn = 6300 g/mol.

### Exemple AB32 : Co-polyaminoacide AB32 - poly-L-glutamate de sodium et modifié par la molécule AA14 et ayant une masse molaire moyenne en nombre (Mn) de 4700 g/mol.

Par un procédé similaire à celui utilisé pour la préparation du co-polyaminoacide AB29 appliqué à la molécule AA14 et à l'acide poly-L-glutamique AB29-1, un poly-L-glutamate de sodium modifié par la molécule AA14 est obtenu.
Extrait sec : 13,5 mg/g.
DP (estimé d'après la RMN ¹H) : 40.
D'après la RMN ¹H : i = 0,109.
La masse molaire moyenne calculée du co-polyaminoacide AB32 est de 8599 g/mol. HPLC-SEC organique (calibrant PEG) : Mn = 4700 g/mol.

### Co-polyaminoacides définis de formule VII ou VIIb

### Exemple AB14 : Co-polyaminoacide AB14 - poly-L-glutamate de sodium modifié à une de ses extrémités par la molécule AA1 et ayant une masse molaire moyenne en nombre (Mn) de 3400 g/mol.

Dans un contenant adapté sont introduits successivement le sel de chlorhydrate de la molécule AA1 (2,03 g, 4,70 mmol), du chloroforme (5 mL), du tamis moléculaire 4 Å (1,3 g), ainsi que de la résine échangeuse d'ion Amberlite IRN 150 (1,3 g). Après 1 h d'agitation sur rouleaux, le milieu est filtré et la résine est rincée avec du chloroforme. Le mélange est évaporé puis co-évaporé avec du toluène. Le résidu est solubilisé dans du DMF anhydre (30 mL) pour être utilisé directement dans la réaction de polymérisation.

Dans un ballon préalablement séché à l'étuve, du γ-benzyl-L-glutamate N-carboxyanhydride (25,59 g, 97,2 mmol) est placé sous vide pendant 30 min puis du DMF anhydre (140 mL) est introduit. Le mélange est agité sous argon jusqu'à solubilisation complète, refroidi à 4 °C, puis la solution de molécule AA1 préparée comme décrit précédemment est introduite rapidement. Le mélange est agité entre 4 °C et température ambiante pendant 2 jours, puis chauffé à 65 °C pendant 2 h. Le mélange réactionnel est alors refroidi à température ambiante puis versé goutte à goutte dans du diisopropyléther (1,7 L) sous agitation. Le précipité blanc est récupéré par filtration, lavé deux fois avec du diisopropyléther (140 mL) puis séché sous vide à 30 °C pour obtenir un solide blanc. Le solide est dilué dans du TFA (160 mL), et une solution d'acide bromhydrique (HBr) à 33 % dans de l'acide acétique (62 mL, 354 mmol) est alors ajoutée goutte à goutte et à 0 °C. La solution est agitée pendant 2 h à température ambiante puis est coulée goutte à goutte sur un mélange 1:1 (v/v) de diisopropyléther / eau et sous agitation (1,9 L). Après 2 h d'agitation, le mélange hétérogène est laissé au repos pendant une nuit. Le précipité blanc est récupéré par filtration, lavé successivement avec un mélange 1:1 (v/v) de diisopropyléther et d'eau (280 mL) puis avec de l'eau (140 mL). Le solide obtenu est solubilisé dans de l'eau (530 mL) en ajustant le pH à 7 par ajout d'une solution aqueuse de soude 10 N puis une solution aqueuse de soude 1 N. Après solubilisation, la concentration théorique est ajustée à 20 g/L théorique par addition d'eau pour obtenir un volume final de 800 mL. Le mélange est filtré sur filtre 0,45 µm puis est purifié par ultrafiltration contre une solution de NaCl 0,9 % puis de l'eau jusqu'à ce que la conductimétrie du perméat soit inférieure à 50 µS/cm. La solution de co-polyaminoacide est ensuite concentrée à environ 30 g/L théorique et le pH est ajusté à 7,0. La solution aqueuse est filtrée sur 0,2 µm et conservée à 4 °C.
Extrait sec : 24,1 mg/g.
DP (estimé par RMN ¹H) = 25 donc i = 0,04.
La masse molaire moyenne calculée du co-polyaminoacide AB14 est de 3378 g/mol. HPLC-SEC aqueuse (calibrant PEG) : Mn = 3400 g/mol.

### Exemple AB15 : Co-polyaminoacide AB15 - poly-L-glutamate de sodium modifié à une de ses extrémités par la molécule AA6 et ayant une masse molaire moyenne en nombre (Mn) 4100 g/mol.

Par un procédé similaire à celui utilisé pour la préparation du co-polyaminoacide AB14 appliqué au sel de chlorhydrate de la molécule AA6 (2,16 g, 3,94 mmol) et à 25,58 g (97,2 mmol) de γ-benzyl-L-glutamate *N*-carboxyanhydride, un poly-L-glutamate de sodium modifié à une de ses extrémités par la molécule AA6 est obtenu.
Extrait sec : 45,5 mg/g.
DP (estimé par RMN ¹H) = 30 donc i = 0,033.
La masse molaire moyenne calculée du co-polyaminoacide AB15 est de 5005 g/mol. HPLC-SEC aqueuse (calibrant PEG) : Mn = 4100 g/mol.

### Exemple AB16 : Co-polyaminoacide AB16 - poly-L-glutamate de sodium modifié à une de ses extrémités par la molécule AA6 et ayant une masse molaire moyenne en nombre (Mn) de 6500 g/mol.

Par un procédé similaire à celui utilisé pour la préparation du co-polyaminoacide AB14 appliqué au sel de chlorhydrate de la molécule AA6 (2,39 g, 4,36 mmol) et à 50,0 g (189,9 mmol) de γ-benzyl-L-glutamate *N*-carboxyanhydride, un poly-L-glutamate de sodium modifié à une de ses extrémités par la molécule AA6 est obtenu.
Extrait sec : 28,5 mg/g.
DP (estimé par RMN ¹H) = 48 donc i = 0,021.
La masse molaire moyenne calculée du co-polyaminoacide AB16 est de 7725 g/mol. HPLC-SEC aqueuse (calibrant PEG) : Mn = 6500 g/mol.

### Exemple AB17 : Co-polyaminoacide AB17 - poly-L-glutamate de sodium modifié à une de ses extrémités par la molécule AA7 et ayant une masse molaire moyenne en nombre (Mn) de 3500 g/mol.

Par un procédé similaire à celui utilisé pour la préparation du co-polyaminoacide AB14 appliqué au sel de chlorhydrate de la molécule AA7 (2,80 g, 4,32 mmol) et à 25,0 g (94,9 mmol) de γ-benzyl-L-glutamate *N*-carboxyanhydride, un poly-L-glutamate de sodium modifié à une de ses extrémités par la molécule AA7 est obtenu.
Extrait sec : 25,2 mg/g.
DP (estimé par RMN ¹H) = 26 donc i = 0,038.
La masse molaire moyenne calculée du co-polyaminoacide AB17 est de 4500 g/mol. HPLC-SEC aqueuse (calibrant PEG) : Mn = 3500 g/mol.

### Exemple AB18 : Co-polyaminoacide AB18 - poly-L-glutamate de sodium modifié à une de ses extrémités par la molécule AA7 et ayant une masse molaire moyenne en nombre (Mn) de 3700 g/mol.

Un poly-L-glutamate de sodium modifié à une de ses extrémités par la molécule AA7 est obtenu par polymérisation du γ-méthyl *N*-carboxyanhydride d'acide glutamique (25,0 g, 133,6 mmol) en utilisant le sel chlorhydrate de la molécule AA7 (2,80 g, 4,32 mmol) comme initiateur et en effectuant une déprotection des esters méthyliques par utilisation d'une solution d'acide chlorhydrique à 37 % selon le procédé décrit dans la demande de brevet FR-A-2 801 226.
Extrait sec : 44,3 mg/g.
DP (estimé par RMN ¹H) = 22 donc i = 0,045.
La masse molaire moyenne calculée du co-polyaminoacide AB18 est de 3896 g/mol. HPLC-SEC aqueuse (calibrant PEG) : Mn = 3700 g/mol.

### Exemple AB19 : Co-polyaminoacide AB19 - poly-L-glutamate de sodium modifié à une de ses extrémités par la molécule AA6 et ayant une masse molaire moyenne en nombre (Mn) de 10500 g/mol.

Par un procédé similaire à celui utilisé pour la préparation du co-polyaminoacide AB14 appliqué au sel de chlorhydrate de la molécule AA6 (1,64 g, 2,99 mmol) et au γ-benzyl-L-glutamate *N*-carboxyanhydride (49,3 g, 187 mmol), un poly-L-glutamate de sodium modifié à une de ses extrémités par la molécule AA6 est obtenu.
Extrait sec : 23,4 mg/g.
DP (estimé par RMN ¹H) = 65 donc i = 0,015.
La masse molaire moyenne calculée du co-polyaminoacide AB19 est de 10293 g/mol. HPLC-SEC aqueuse (calibrant PEG) : Mn = 10500 g/mol.

### Exemple AB20 : Co-polyaminoacide AB20 - poly-L-glutamate de sodium cappé à une de ses extrémités par un groupement acétyle et modifié à une de ses extrémités par la molécule AA6 et ayant une masse molaire moyenne en nombre (Mn) de 10400 g/mol.

Dans un contenant adapté sont introduits successivement le sel de chlorhydrate de la molécule AA6 (0,545 g, 1,00 mmol), du chloroforme (10 mL), du tamis moléculaire 4 Å (3 g), ainsi que de la résine échangeuse d'ion Amberlite IRN 150 (3 g). Après 1 h d'agitation sur rouleaux, le milieu est filtré et la résine est rincée avec du chloroforme. Le mélange est évaporé puis co-évaporé avec du toluène. Le résidu est solubilisé dans du DMF anhydre (10 mL) pour être utilisé directement dans la réaction de polymérisation.

Dans un ballon préalablement séché à l'étuve, du γ-benzyl-L-glutamate *N-*carboxyanhydride (17,0 g, 64,6 mmol) est placé sous vide pendant 30 min puis du DMF anhydre (30 mL) est introduit. Le mélange est agité sous argon jusqu'à solubilisation complète, refroidi à 4 °C, puis la solution de molécule AA6 préparée comme décrit précédemment est introduite rapidement. Le mélange est agité entre 4 °C et température ambiante pendant 2 jours, puis précipité dans du diisopropyléther (0,6 L). Le précipité est récupéré par filtration, lavé deux fois avec du diisopropyléther (40 mL) puis séché pour donner un solide blanc qui est dissous dans 80 mL de THF. À cette solution sont ajoutés successivement de la DIPEA, (1,7 mL, 9,8 mmol) puis de l'anhydride acétique (0,9 mL, 9,5 mmol). Après une nuit d'agitation à température ambiante, la solution est versée lentement dans du diisopropyléther (480 mL) sur une durée de 30 min et sous agitation. Après 1 h d'agitation, le précipité est filtré, lavé deux fois avec du diisopropyléther (80 mL) puis séché sous vide à 30 °C pour donner un acide poly(gamma-benzyl-L-glutamique) cappé à une de ses extrémités par un groupement acétyle et modifié à l'autre de ses extrémités par la molécule AA6 sous la forme d'un solide blanc.

Le solide est dilué dans du TFA (65 mL), et une solution d'acide bromhydrique (HBr) à 33 % dans de l'acide acétique (45 mL, 257,0 mmol) est alors ajoutée goutte à goutte et à 4 °C. La solution est agitée pendant 2 h à température ambiante puis est coulée goutte à goutte sur un mélange 1:1 (v/v) de diisopropyléther / eau et sous agitation (780 mL). Après 2 h d'agitation, le mélange hétérogène est laissé au repos pendant une nuit. Le précipité blanc est récupéré par filtration, lavé successivement avec un mélange 1:1 (v/v) de diisopropyléther et d'eau (70 mL) puis avec de l'eau (70 mL). Le solide obtenu est solubilisé dans de l'eau (300 mL) en ajustant le pH à 7 par ajout d'une solution aqueuse de soude 10 N puis une solution aqueuse de soude 1 N. Après solubilisation, la concentration théorique est ajustée à 20 g/L théorique par addition d'eau pour obtenir un volume final de 440 mL. Le mélange est filtré sur filtre 0,45 µm puis est purifié par ultrafiltration contre une solution de NaCl 0,9 % puis de l'eau jusqu'à ce que la conductimétrie du perméat soit inférieure à 50 µS/cm. La solution de co-polyaminoacide est ensuite concentrée à environ 30 g/L théorique et le pH est ajusté à 7,0. La solution aqueuse est filtrée sur 0,2 µm et conservée à 4 °C.
Extrait sec : 21,5 mg/g.
DP (estimé par RMN ¹H) = 60 donc i = 0,017.
La masse molaire moyenne calculée du co-polyaminoacide AB20 est de 9619 g/mol. HPLC-SEC aqueuse (calibrant PEG) : Mn = 10400 g/mol.

### BA : synthèse des molécules hydrophobes dans lesquelles p = 2

Les radicaux sont représentés dans le tableau suivant par la molécule hydrophobe correspondante avant greffage sur le co-polyaminoacide.

**Tableau 1d : liste des molécules hydrophobes synthétisées selon l'invention dans lesquelles p=2.**

| **N°** | **Structure de la molécule hydrophobe avant greffage sur le co-polyaminoacide** |
|---|---|
| BA1 | 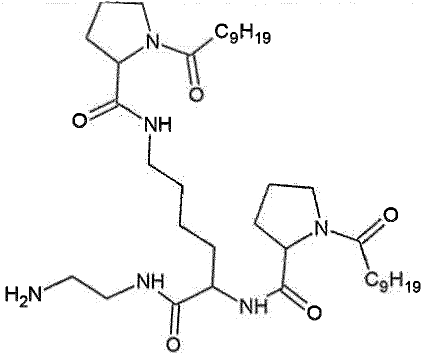 |
| BA2 | 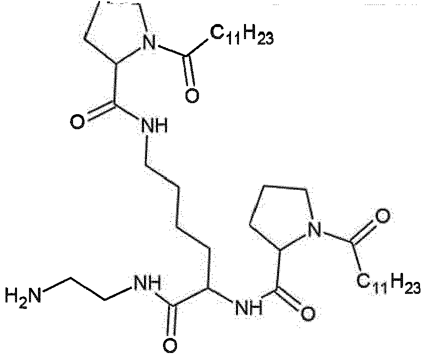 |
| BA3 | 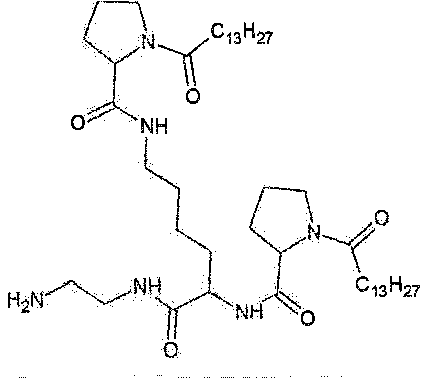 |
| BA4 | 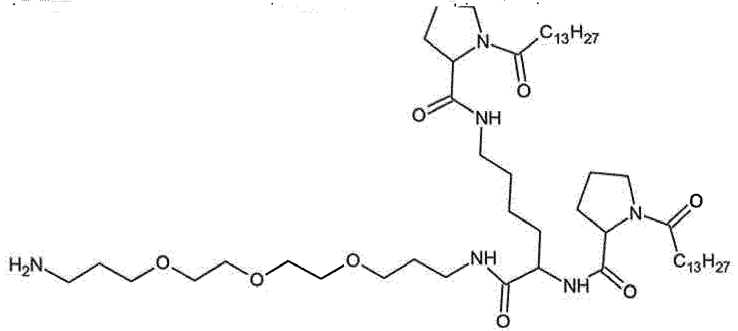 |
| BA5 | 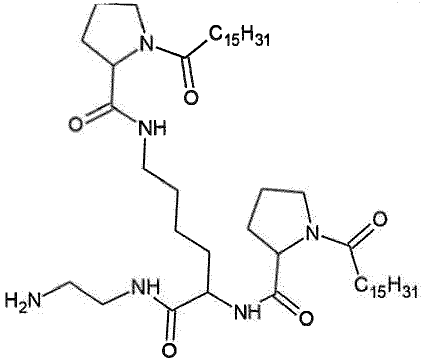 |
| BA6 | 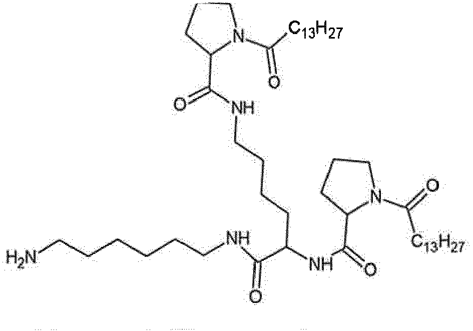 |
| BA7 | 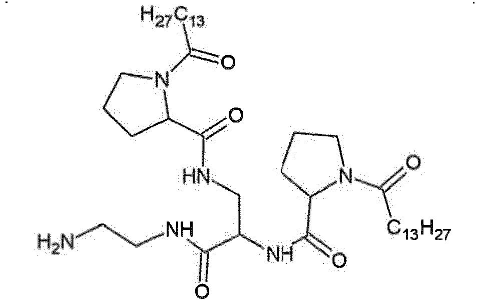 |

### Exemple BA1 : molécule BA1

### Molécule B1 : Produit obtenu par la réaction entre l'acide décanoïque et la L-proline.

À une solution d'acide décanoïque (14,28 g, 82,91 mmol) dans le THF (520 mL) à 0 °C sont ajoutés successivement du dicyclohexyle carbodiimide (DCC) (16,29 g, 78,96 mmol) et du /V-hydroxysuccinimide (NHS) (9,09 g, 78,96 mmol). Après 60 h d'agitation à température ambiante, le milieu est refroidi à 0 °C pendant 20 min, filtré sur fritté. De la L-proline (10 g, 86,86 mmol), de la diisopropyléthylamine (DIPEA) (68,8 mL) et de l'eau (60 mL) sont ajoutés au filtrat. Après 24 h d'agitation à température ambiante, le milieu est dilué avec de l'eau (300 mL). La phase aqueuse est lavée avec de l'acétate d'éthyle (2 x 250 mL), acidifiée jusqu'à pH ~1 avec une solution aqueuse d'HCl 1 N puis extraite avec du dichlorométhane (3 x 150 mL). Les phases organiques combinées sont séchées sur Na₂SO₄, filtrées, concentrées sous vide et le résidu est purifié par chromatographie sur gel de silice (cyclohexane, acétate d'éthyle).
Rendement : 14,6 g (69 %).
RMN ¹H (CDCl₃, ppm) : 0,87 (3H) ; 1,26 (12H) ; 1,65 (2H) ; 2,02 (3H) ; 2,34 (2H) ; 2,41 (1H) ; 3,48 (1H) ; 3,56 (1H) ; 4,58 (1H).
LC/MS (ESI) : 270,2; (calculé ([M+H]⁺) : 270,4).

### Molécule B2 : Produit obtenu par la réaction entre la molécule B1 et la L-lysine.

Par un procédé similaire à celui utilisé pour la préparation de la molécule B1 appliqué à la molécule B1 (14,57 g, 54,07 mmol) et à la L-lysine (4,15 g, 28,39 mmol), une huile jaune est obtenue.
Rendement : 16,4 g (93 %).
RMN ¹H (CDCl₃, ppm) : 0,88 (6H) ; 1,26 (24H) ; 1,35-1,65 (8H) ; 1,85-2,35 (12H) ; 2,53 (0,2H) ; 2,90 (0,8H) ; 3,45-3,75 (5H) ; 4,50-4,70 (3H) ; 7,82 (1H).
LC/MS (ESI) : 649,6; (calculé ([M+H]⁺) : 649,9).

### Molécule B3 : Produit obtenu par réaction entre la molécule B2 et la Boc-éthylènediamine.

À une solution de molécule B2 (16,4 g, 25,27 mmol) dans le THF (170 mL) sont ajoutés de la DIPEA (8,80 mL) et du 2-(1-benzotriazol-1-yl)-1,1,3,3-tétraméthyluronium tétrafluoroborate (TBTU, 8,52 g, 26,54 mmol) à température ambiante. Après 30 min d'agitation, de la Boc-éthylènediamine (4,45 g, 27,8 mmol) est ajoutée. Après agitation à température ambiante pendant 2 h, le solvant est évaporé sous pression réduite et le résidu est dilué avec de l'acétate d'éthyle (400 mL). La phase organique est lavée avec de l'eau (250 mL), une solution aqueuse saturée de NaHCO₃ (250 mL), une solution aqueuse de 1 N HCl (250 mL), une solution aqueuse saturée en NaCl (250 mL) et est séchée sur Na₂SO₄. Après filtration et concentration sous vide, le résidu obtenu est purifié par chromatographie sur gel de silice (acétate d'éthyle, méthanol) pour donner une huile incolore.
Rendement : 12,8 g (64 %).
RMN ¹H (CDCl₃, ppm) : 0,87 (6H) ; 1,25-1,60 (42H) ; 1,80-2,05 (4H) ; 2,15-2,45 (9H) ; 3,10-3,75 (10H) ; 4,30 (1H) ; 4,50 (2H) ; 5,50 (0,6H) ; 5,89 (0,2H) ; 6,15 (0,2H) ; 7,03 (1H) ; 7,47 (1H).
LC/MS (ESI) : 791,8 ; (calculé ([M+H]⁺) : 792,1).

### Molécule BA1

À une solution de la molécule B3 (12,78 g, 16,15 mmol) dans le dichlorométhane (110 mL) à 5 °C est ajoutée une solution de HCl 4 N dans le dioxane (20,2 mL). Après 20 h d'agitation à 5 °C, le milieu est concentré sous vide. Le résidu obtenu est dissous dans le méthanol et évaporé sous vide, cette opération étant répétée 4 fois pour donner un solide blanc de molécule BA1 sous forme de sel de chlorhydrate.
Rendement : 11,4 g (97 %).
RMN ¹H (DMSO-de, ppm) : 0,85 (6H) ; 1,25-1,50 (33H) ; 1,57 (1H) ; 1,70-2,40 (12H) ; 2,82 (2H) ; 3,00 (2H) ; 3,25-3,70 (6H) ; 4,05-4,50 (3H) ; 7,75-8,45 (6H).
LC/MS (ESI) : 691,6 ; (calculé ([M+H]⁺) : 692,0).

### Exemple BA2 : molécule BA2

### Molécule B4 : Produit obtenu par la réaction entre l'acide laurique et la L-proline.

Par un procédé similaire à celui utilisé pour la préparation de la molécule B1 appliqué à l'acide laurique (31,83 g, 157,9 mmol) et à la L-proline (20 g, 173,7 mmol), une huile jaune est obtenue.
Rendement : 34,3 g (73 %).
RMN ¹H (CDCl3, ppm) : 0,87 (3H) ; 1,26 (16H) ; 1,70 (2H) ; 1,90 2,10 (3H) ; 2,35 (2H) ; 2,49 (1H) ; 3,48 (1H) ; 3,56 (1H) ; 4,60 (1H).
LC/MS (ESI) : 298,2 ; (calculé ([M+H]⁺) : 298,4).

### Molécule B5 : Produit obtenu par la réaction entre la molécule B4 et la L-lysine.

Par un procédé similaire à celui utilisé pour la préparation de la molécule B1 appliqué à la molécule B4 (33,72 g, 113,36 mmol) et à la L-lysine (8,70 g, 59,51 mmol), un solide blanc est obtenu.
Rendement : 26,2 g (66 %).
RMN ¹H (CDCl₃, ppm) : 0,88 (6H) ; 1,26 (32H) ; 1,35-1,65 (8H) ; 1,85-2,35 (15H) ; 2,87 (1H) ; 3,40-3,75 (5H) ; 4,50-4,75 (3H) ; 7,87 (1H).
LC/MS (ESI) : 705,6 ; (calculé ([M+H]⁺) : 706,0).

### Molécule B6 : produit obtenu par réaction entre la Boc-éthylènediamine et la molécule B5.

Par un procédé similaire à celui utilisé pour la préparation de la molécule B3 appliqué à la molécule B5 (25,74 g, 36,51 mmol) et à la Boc-éthylènediamine (6,43 g, 40,16 mmol), une huile incolore est obtenue.
Rendement : 30,9 g (quantitatif).
RMN ¹H (CDCl₃, ppm) : 0,88 (6H) ; 1,35-1,65 (50H) ; 1,85-2,35 (13H) ; 3,05-3,75 (10H) ; 4,25-4,65 (3H) ; 5,50 (0,4H) ; 5,88 (0,2H) ; 6,16 (0,2H) ; 7,08 (1H) ; 7,26 (1H) ; 7,49 (0,2H).
LC/MS (ESI) : 847,8 ; (calculé ([M+H]⁺) : 848,2).

### Molécule BA2

Après un procédé similaire à celui utilisé pour la préparation de la molécule BA1 appliqué à la molécule B6 (30,9 g, 36,47 mmol), le résidu obtenu après concentration sous vide est dissous dans le méthanol et évaporé sous vide, cette opération étant répétée 4 fois pour donner un solide blanc de molécule BA2 sous forme de sel de chlorhydrate après séchage sous pression réduite.
Rendement : 27,65 g (97 %).
RMN ¹H (DMSO-ds, ppm) : 0,85 (6H) ; 1,10-2,40 (54H) ; 2,75-3,15 (4H) ; 3,25-3,60 (6H) ; 4,05-4,50 (3H) ; 7,50-8,50 (6H).
LC/MS (ESI) : 747,6 ; (calculé ([M+H]⁺) : 748,1).

### Exemple BA3 : molécule BA3

### Molécule B7 : Produit obtenu par la réaction entre l'acide myristique et la L-proline.

Par un procédé similaire à celui utilisé pour la préparation de la molécule B1 appliqué à l'acide myristique (18,93 g, 82,91 mmol) et à la L-proline (10 g, 86,86 mmol), une huile jaunâtre est obtenue.
Rendement : 20 g (78 %).
RMN ¹H (CDCl₃, ppm) : 0,88 (3H) ; 1,28 (20H) ; 1,70 (2H) ; 1,90-2,10 (3H) ; 2,36 (2H) ; 2,51 (1H) ; 3,47 (1H) ; 3,56 (1H) ; 4,61 (1H).
LC/MS (ESI) : 326,2; (calculé ([M+H]⁺) : 326,6).

### Molécule B8 : Produit obtenu par la réaction entre la molécule B7 et la L-lysine.

Par un procédé similaire à celui utilisé pour la préparation de la molécule B1 appliqué à la molécule B7 (20,02 g, 61,5 mmol) et à la L-lysine (4,72 g, 32,29 mmol), un solide blanc est obtenu.
Rendement : 12,3 g (53 %).
RMN ¹H (DMSO-de, ppm) : 0,85 (6H) ; 1,26 (40H) ; 1,35-1,50 (6H) ; 1,50-2,10 (10H) ; 2,10-2,25 (4H) ; 3,01 (2H) ; 3,31-3,55 (4H) ; 4,10-4,40 (3H) ; 7,68 (0,6H) ; 7,97 (1H) ; 8,27 (0,4H) ; 12,50 (1H).
LC/MS (ESI) : 761,8 ; (calculé ([M+H]⁺) : 762,1).

### Molécule B9 : Produit obtenu par la réaction entre la Boc-éthylènediamine et la molécule B8.

Par un procédé similaire à celui utilisé pour la préparation de la molécule B3 appliqué à la molécule B8 (12 g, 15,77 mmol) et à la Boc-éthylènediamine (3,03 g, 18,92 mmol), une huile incolore est obtenue après purification par colonne chromatographique sur gel de silice (acétate d'éthyle, méthanol).
Rendement : 12,5 g (88 %).
RMN ¹H (DMSO-de, ppm) : 0,85 (6H) ; 1,20-1,55 (55H) ; 1,50-2,25 (14H) ; 2,95-3,10 (6H) ; 3,31-3,55 (4H) ; 4,10-4,40 (3H) ; 6,74 (1H) ; 7,60-8,25 (3H).
LC/MS (ESI) : 904,1 ; (calculé ([M+H]⁺) : 904,3).

### Molécule BA3

Après un procédé similaire à celui utilisé pour la préparation de la molécule BA1 appliqué à la molécule B9 (12,5 g, 13,84 mmol), le résidu obtenu après concentration sous vide est dissous dans le méthanol et évaporé sous vide, cette opération étant répétée 4 fois pour donner un solide blanc de molécule BA3 sous forme de sel de chlorhydrate après séchage sous pression réduite.
Rendement : 9,2 g (79 %).
RMN ¹H (DMSO-de, ppm) : 0,85 (6H) ; 1,10-1,65 (48H) ; 1,70-2,35 (12H) ; 2,85 (2H) ; 3,01 (2H) ; 3,25-3,65 (6H) ; 4,10-4,50 (3H) ; 7,70-8,40 (6H).
LC/MS (ESI) : 803,9 ; (calculé ([M+H]⁺) : 804,2).

### Exemple BA4 : molécule BA4

### Molécule B10: Produit obtenu par la réaction entre la molécule B8 et le Boc-1-amino-4,7,10-trioxa-13-tridécane amine.

Par un procédé similaire à celui utilisé pour la préparation de la molécule B3 appliqué à la molécule B8 (29,80 g, 39,15 mmol) et au Boc-1-amino-4,7,10-trioxa-13-tridécane amine (15,05 g, 46,96 mmol), une huile épaisse incolore est obtenue.
Rendement : 25,3 g (61 %).
RMN ¹H (DMSO-de, ppm) : 0,85 (6H) ; 1,25-2,35 (75H) ; 2,85-3,20 (6H) ; 3,25-3,65 (16H) ; 4,10-4,45 (3H) ; 6,38 (0,1H) ; 6,72 (0,9H) ; 7,50-8,25 (3H).
LC/MS (ESI) : 1064,2 ; (calculé ([M+H]⁺) : 1064,5).

### Molécule BA4

Après un procédé similaire à celui utilisé pour la préparation de la molécule BA1 appliqué à la molécule B10 (25,3 g, 23,8 mmol), le résidu obtenu après concentration sous vide est dissous dans le méthanol et évaporé sous vide, cette opération étant répétée 4 fois pour donner un solide blanc de molécule BA4 sous forme de sel de chlorhydrate après séchage sous pression réduite.
Rendement : 20,02 g (84 %).
RMN ¹H (DMSO-de, ppm) : 0,85 (6H) ; 1,15-2,35 (66H) ; 2,80-3,20 (6H) ; 3,30-3,65 (16H) ; 4,10-4,45 (3H) ; 7,55-8,60 (6H).
LC/MS (ESI) : 964,9 ; (calculé ([M+H]⁺) : 964,6).

### Exemple BAS : molécule BAS

### Molécule B11 : Produit obtenu par réaction entre la molécule A1 et la L-Lysine

Par un procédé similaire à celui utilisé pour la préparation de la molécule B1 appliqué à la molécule A1 (19,10 g, 54,02 mmol) et à la L-lysine (4,15 g, 28,36 mmol), un résidu huileux est obtenu après concentration du milieu réactionnel sous pression réduite. Ce résidu est dilué dans de l'eau (150 mL), lavé à l'acétate d'éthyle (2 x 75 mL) puis la phase aqueuse est acidifiée jusqu'à pH 1 par addition lente de HCl 6 N. Le produit est extrait 3 fois au dichlorométhane, la phase organique est séchée sur Na₂SO₄ puis filtrée et concentrée sous pression réduite pour donner 11,2 g de résidu huileux jaune. Parallèlement, la phase organique d'acétate d'éthyle précédente est lavée avec une solution aqueuse de HCl 2 N (2 x 75 mL), une solution aqueuse saturée en NaCl (75 mL), séchée sur Na₂SO₄,filtrée et concentrée pour donner 10,2 g de résidu huileux jaune. Un solide blanc est obtenu après recristallisation de chacun de ces résidus dans l'acétone.
Rendement : 11,83 g (54 %).
RMN ¹H (CDCl₃, ppm) : 0,87 (6H) ; 1,06-2,44 (70H) ; 2,78-2,96 (1H) ; 3,35-3,75 (5H) ; 4,28-4,43 (0,1H) ; 4,43-4,52 (0,2H) ; 4,52-4,61 (1,8H) ; 4,61-4,75 (0,9H) ; 7,74-8,02 (2H).
LC/MS (ESI) : 818,0 ; (calculé ([M+H]⁺) : 818,7).

### Molécule B12 : Produit obtenu par couplage entre la molécule B11 et la Boc-éthylènediamine.

Par un procédé similaire à celui utilisé pour la préparation de la molécule B3 appliqué à la molécule B11 (18,00 g, 22,02 mmol) en solution dans le THF et à la Boc-éthylènediamine (4,23 g, 26,43 mmol), un solide blanc est obtenu après recristallisation deux fois dans l'acétonitrile.
Rendement : 17,5 g (83 %).
RMN ¹H (DMSO-de, ppm) : 0,85 (6H) ; 1,15-2,29 (79H) ; 2,92-3,12 (6H) ; 3,30-3,59 (4H) ; 4,06-4,13 (0,65H) ; 4,16-4,29 (2H) ; 4,38-4,42 (0,35H) ; 6,71-6,76 (1H) ; 7,60-7,69 (1,3H) ; 7,76-7,81 (0,65H) ; 7,93-7,97 (0,35H) ; 8,00-8,04 (0,35H) ; 8,10-8,17 (0,35H).
LC/MS (ESI) : 960,4 ; (calculé ([M+H]⁺) : 960,8).

### Molécule BA5

Par un procédé similaire à celui utilisé pour la préparation de la molécule BA1 appliqué à la molécule B12 (24,4 g, 25,43 mmol), le résidu obtenu après concentration sous vide est solubilisé dans du dichlorométhane (150 mL), la phase organique est lavée 2 fois avec une solution aqueuse de soude 2 N (90 mL). De l'acétonitrile (120 mL) est ajouté et le dichlorométhane est éliminé par concentration sous pression réduite. Le milieu est ensuite laissé au repos pendant 72 h et un solide blanc est obtenu après filtration et rinçage à l'acétonitrile puis séchage sous pression réduite. Cette opération est répétée 4 fois.
Rendement : 14,28 g (65 %).
RMN ¹H (DMSO-de, ppm) : 0,85 (6H) ; 1,06-2,32 (70H) ; 2,53-2,63 (2H) ; 2,89-3,61 (10H) ; 4,04-4,43 (3H) ; 7,55-7,62 (0,65H) ; 7,65-7,72 (0,65H) ; 7,80 (0,65H) ; 7,91 (0,35H) ; 8,03 (0,35H) ; 8,14-8,23 (0,35H).
LC/MS (ESI) : 860,0 ; (calculé ([M+H]⁺) : 860,8).

### Exemple BA6 : molécule BA6

### Molécule B13 : Produit obtenu par la réaction entre le N-(tert-butoxycarbonyl)-1,6-diaminohexane et la molécule B8.

Par un procédé similaire à celui utilisé pour la préparation de la molécule B3 appliqué à la molécule B8 (10 g, 13,14 mmol) et au *N*-(*tert*-butoxycarbonyl)-1,6-diaminohexane (3,41 g, 15,77 mmol) dans le dichlorométhane, un solide blanc est obtenu après recristallisation dans l'acétonitrile.
Rendement : 10,7 g (85 %).
RMN ¹H (CDCl₃, ppm) : 0,88 (6H) ; 1,17-2,40 (79H) ; 3,00-3,71 (10H) ; 4,26-4,58 (3H) ; 4,67 (1H) ; 6,74 (1H) ; 7,34-7,49 (2H).
LC/MS (ESI) : 959,9 ; (calculé ([M+H]⁺) : 959,8).

### Molécule BA6

Après un procédé similaire à celui utilisé pour la préparation de la molécule BA1 appliqué à la molécule B13 (10,5 g, 10,94 mmol), une solution aqueuse de NaOH 2 N est ajoutée goutte à goutte au milieu réactionnel refroidi à 0 °C. La phase aqueuse est extraite au dichlorométhane puis la phase organique est lavée 3 fois avec une solution aqueuse de NaCl 5 %. Après séchage sur Na₂SO₄, la phase organique est filtrée, concentrée sous vide et le résidu est recristallisé dans l'acétonitrile.
Rendement : 5,4 g (58 %).
RMN ¹H (CDCl₃, ppm) : 0,88 (6H) ; 1,19-2,40 (72H) ; 2,67 (2H) ; 3,03-3,70 (8H) ; 4,26-4,57 (3H) ; 6,71 (1H) ; 7,39-7,49 (2H).
LC/MS (ESI) : 859,8 ; (calculé ([M+H]⁺) : 859,7).

### Exemple BA7 : molécule BA7

### Molécule B14 : Produit obtenu par couplage entre la molécule B7 et l'acide 2,3-diaminopropionique.

Par un procédé similaire à celui utilisé pour la préparation de la molécule B1 appliqué à la molécule B7 (80,00 g, 245,78 mmol) et au dichlorhydrate de l'acide 2,3-diaminopropionique (22,84 g, 129,04 mmol), un solide blanc est obtenu après recristallisation dans l'acétonitrile.
Rendement : 69 g (78 %).
RMN ¹H (DMSO-de, ppm) : 0,86 (6H) ; 1,08-1,38 (40H) ; 1,40-1,55 (4H) ; 1,68-2,30 (12H) ; 3,16-3,66 (6H) ; 4,20-4,39 (3H) ; 7,67-8,31 (2H) ; 12,70 (1H).
LC/MS (ESI) : 719,4 ; 741,5 ; (calculé ([M+H]⁺) : 719,6 ; ([M+Na]⁺) : 741,6).

### Molécule B15 : produit obtenu par couplage entre la molécule B14 et la Boc-éthylènediamine.

Par un procédé similaire à celui utilisé pour la préparation de la molécule B3 appliqué à la molécule B14 (32,00 g, 44,50 mmol) en solution dans le dichlorométhane et à la Boc-éthylènediamine (8,56 g, 53,40 mmol), une huile incolore est obtenue après purification par chromatographie sur gel de silice (acétate d'éthyle, méthanol).
Rendement : 24,5 g (64 %).
RMN ¹H (DMSO-de, ppm) : 0,85 (6H) ; 1,16-2,42 (65H) ; 2,89-3,14 (4H) ; 3,17-3,66 (6H) ; 4,11-4,43 (3H) ; 6,77 (1H) ; 7,38-8,23 (3H).
LC/MS (ESI) : 861,7 ; (calculé ([M+H]⁺) : 861,7).

### Molécule BA7

Après un procédé similaire à celui utilisé pour la préparation de la molécule BA1 appliqué à la molécule B15 (24,50 g, 28,45 mmol), le milieu réactionnel est concentré sous pression réduite, le résidu est solubilisé dans le dichlorométhane, la phase organique est lavée par une solution aqueuse de NaOH 2 N), séchée sur Na₂SO₄, filtrée et concentrée sous pression réduite. Un solide blanc est obtenu après recristallisation dans l'acétonitrile.
Rendement : 19,7 g (91 %).
RMN ¹H (DMSO-d₆, ppm) : 0,85 (6H) ; 1,10-2,40 (58H) ; 2,51-2,62 (2H) ; 2,90-3,16 (2H) ; 3,16-3,67 (6H) ; 4,04-4,47 (3H) ; 7,33-8,27 (3H).
LC/MS (ESI) : 761,5 ; (calculé ([M+H]⁺) : 761,6).

### BB : Synthèse des copolyaminoacides modifiés par des molécules hydrophobes dans lesquelles p = 2

### Co-polyaminoacides de formule VII ou VIIa

**Tableau 1e : liste des copolyaminoacides de formule VII ou VIIa selon l'invention.**

| **n°** | **co-polyaminoacides porteurs de charges carboxylates et de radicaux hydrophobes** |
|---|---|
| BB1 | 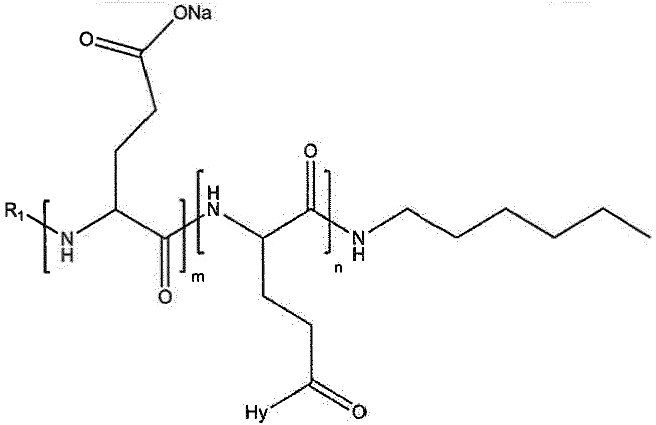 i = 0,05, DP (m + n) = 23 |
| | 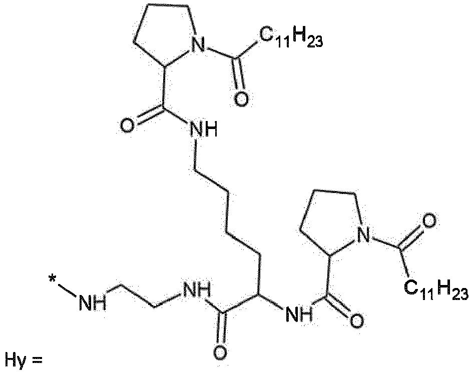 R₁ = H ou pyroglutamate |
| BB2 | 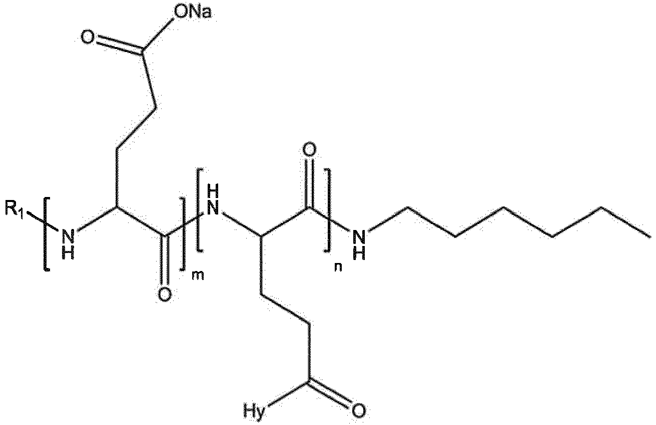 i = 0,047, DP (m + n) = 21 |
| | 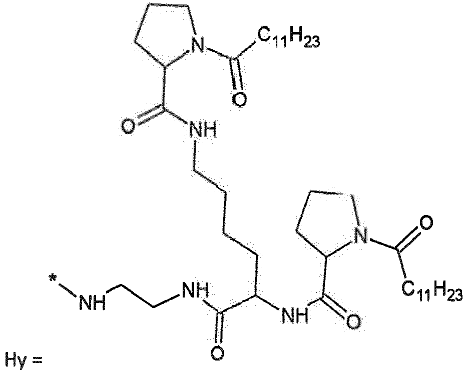 R₁ = H ou pyroglutamate |
| BB3 | 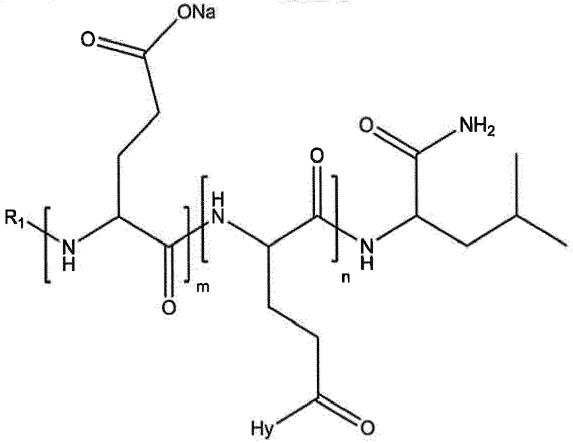 i = 0,049, DP (m + n) = 34 |
| | 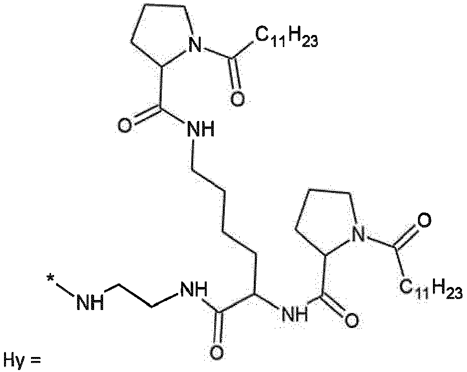 R₁ = H ou pyroglutamate |
| BB4 | 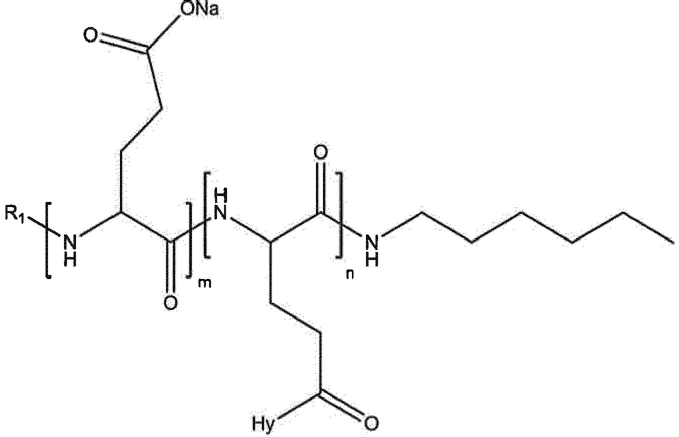 i = 0,04, DP (m + n) = 65 |
| | 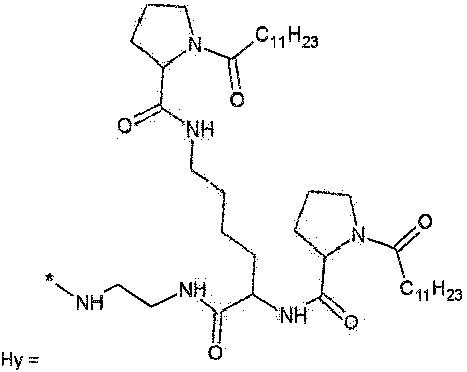 R₁ = H ou pyroglutamate |
| BB5 | 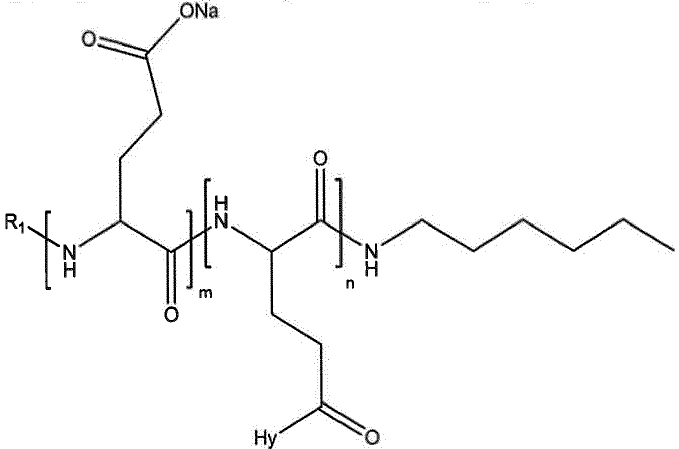 i = 0,042, DP (m + n) = 23 |
| | 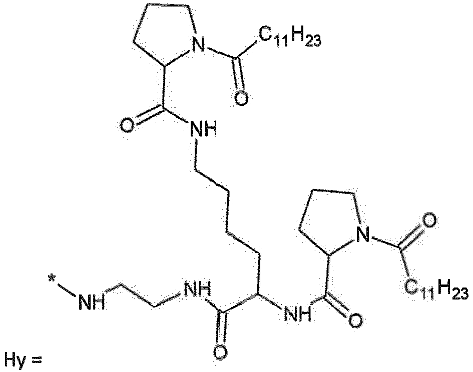 R1= CH₃-CO-, H ou pyroglutamate |
| BB6 | 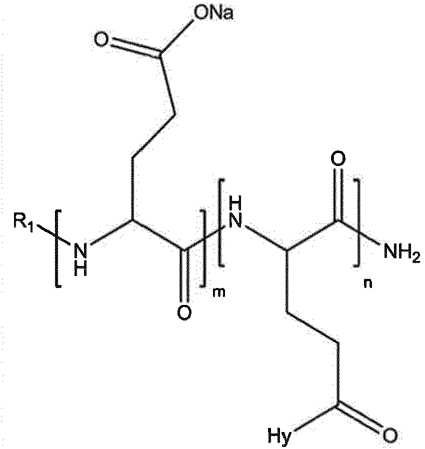 i = 0,04, DP (m + n) = 24 |
| | 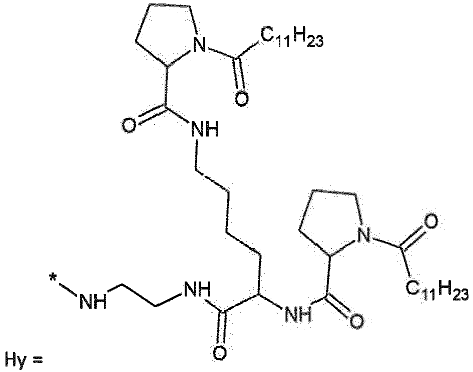 R1= CH₃-CO-, H ou pyroglutamate |
| BB7 | 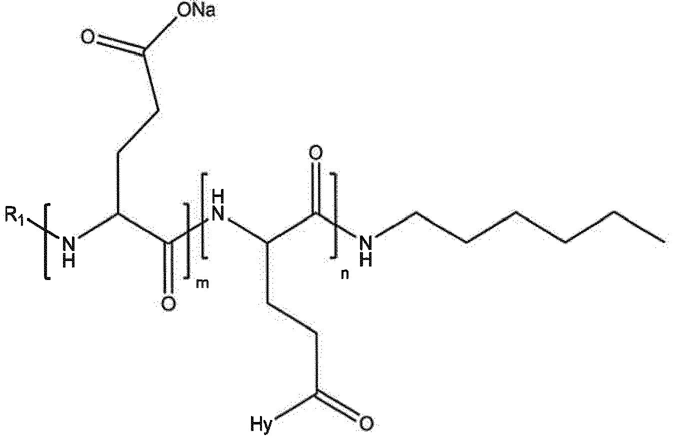 i = 0,042, DP (m + n) = 22 |
| | 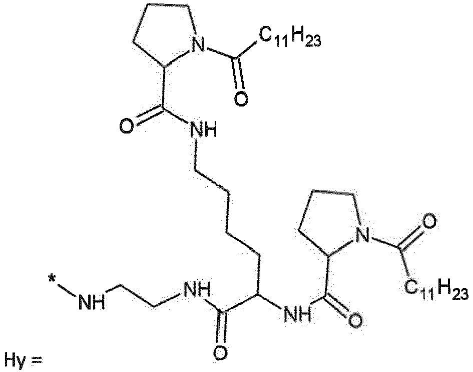 R₁ = H ou pyroglutamate |
| BB8 | 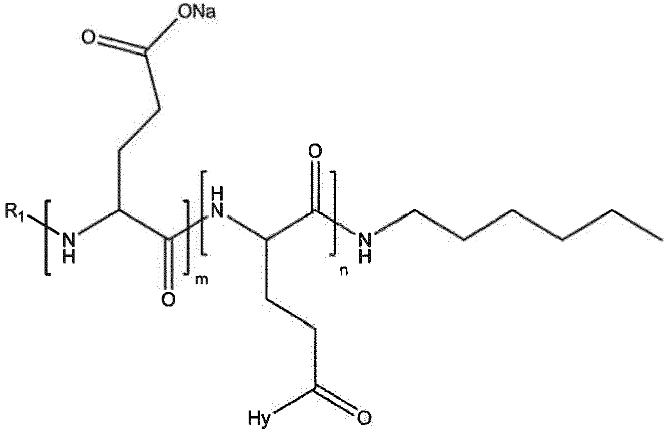 i = 0,026, DP (m + n) = 21 |
| | 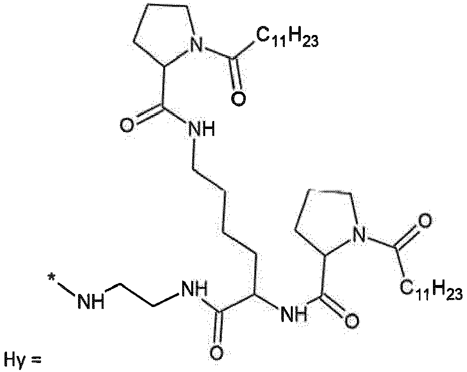 R₁= H ou pyroglutamate |
| BB9 | 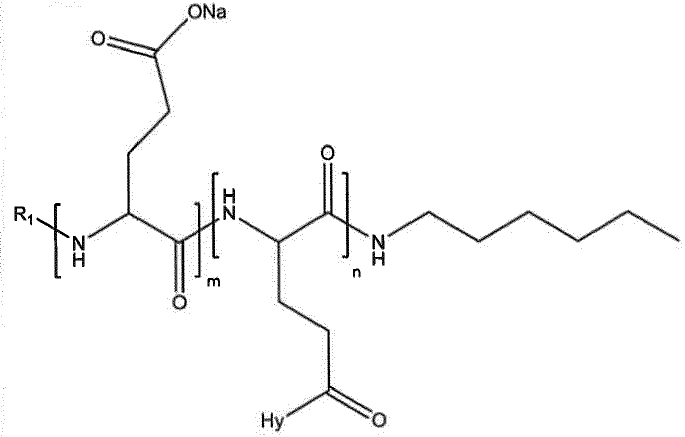 i = 0,05, DP (m + n) = 26 |
| | 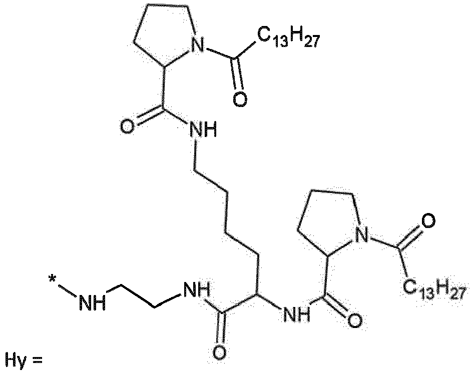 R₁ = H ou pyroglutamate |
| BB10 | 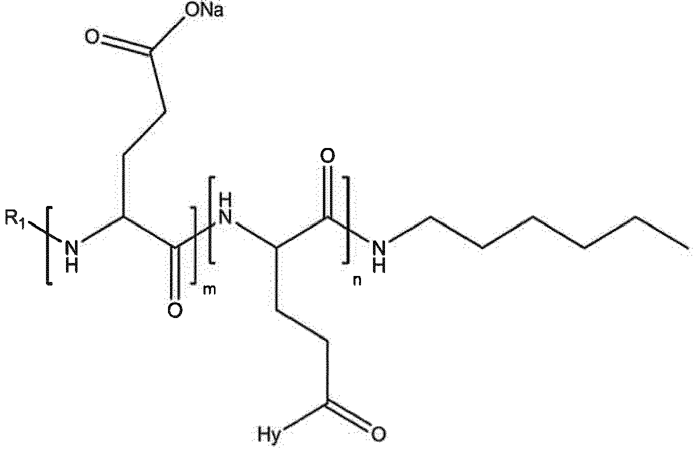 i = 0,029, DP (m + n) = 22 |
| | 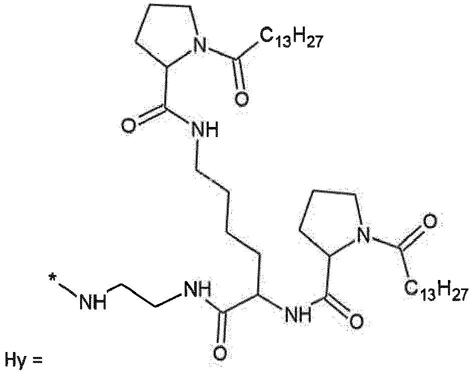 R₁ = H ou pyroglutamate |
| BB11 | 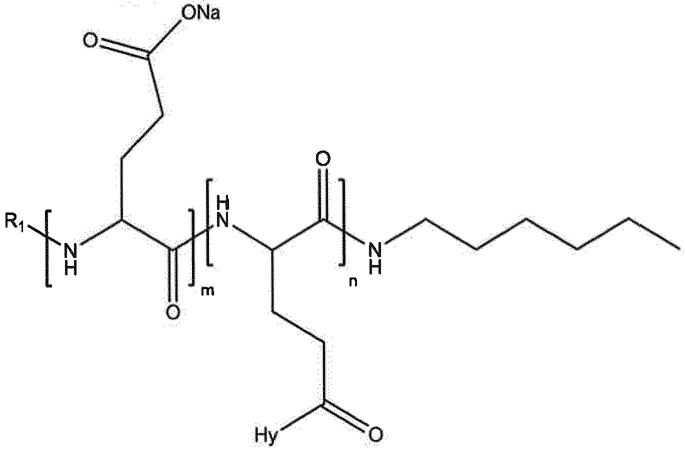 i = 0,032, DP (m + n) = 22 |
| | 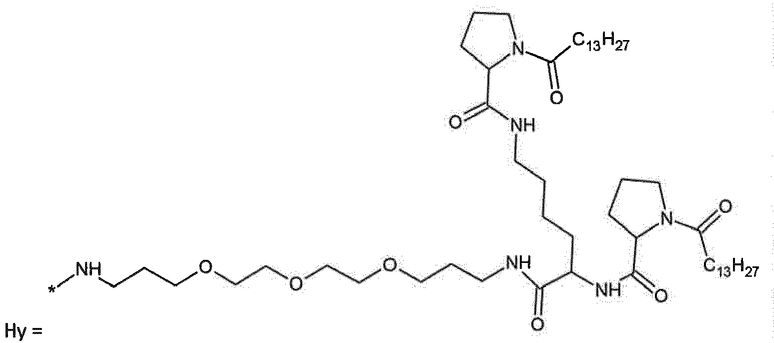 R1= CH₃-CO-, H ou pyroglutamate |
| BB12 | 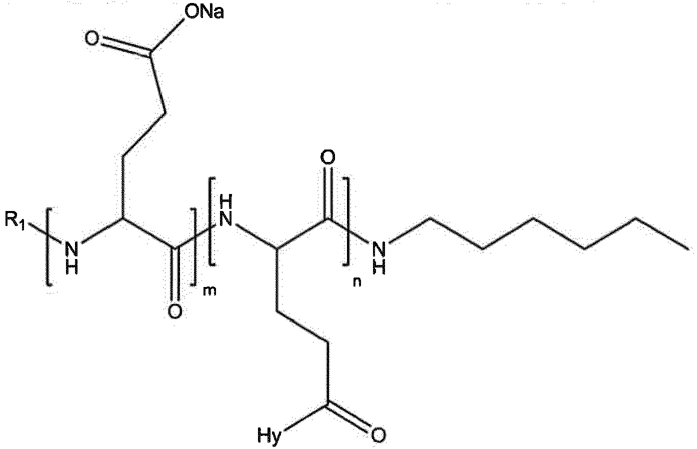 i = 0,03, DP (m + n) = 23 |
| | 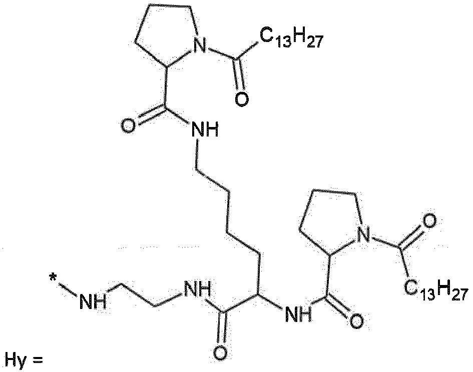 R1= CH₃-CO-, H ou pyroglutamate |
| BB13 | 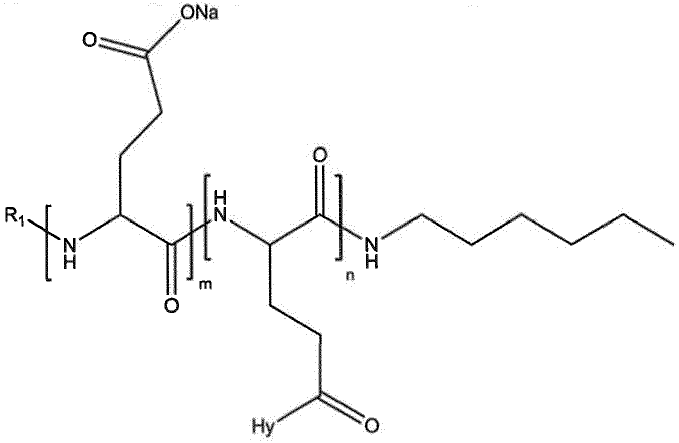 i = 0,08, DP = 25 |
| | 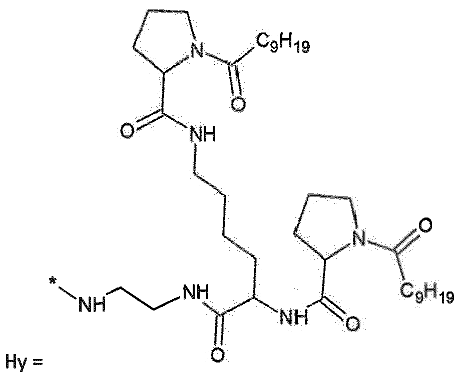 |
| | R₁ = H ou pyroglutamate |

### Co-polyaminoacides de formule VII ou VIIb

**Tableau 1f : liste des co-polyaminoacides de formule VII ou VIIb synthétisés selon l'invention.**

| **n°** | **co-polyaminoacides porteur de charges carboxylates et de radicaux hydrophobes** |
|---|---|
| **BB14** | 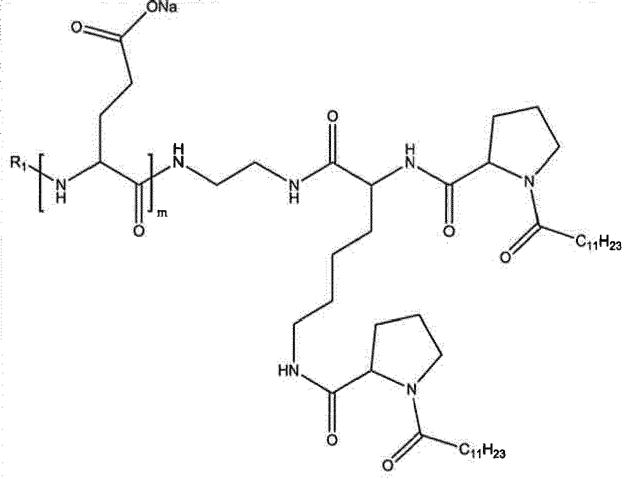 i = 0,034, DP (m) = 29 |
| | R₁ = H ou pyroglutamate |
| **BB15** | 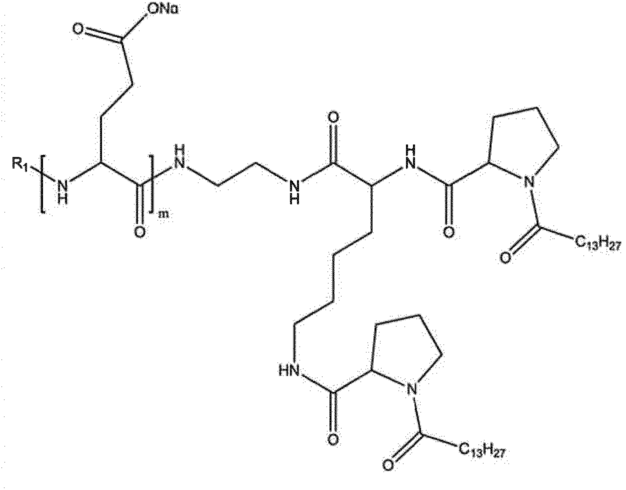 i = 0,045, DP (m) = 22 |
| | R₁ = H ou pyroglutamate |
| **BB16** | 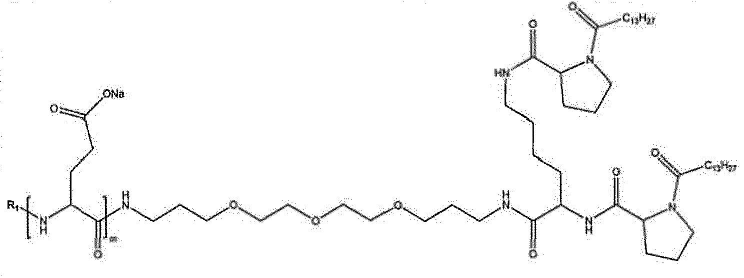 i = 0,043, DP (m) = 23 |
| | R₁ = H ou pyroglutamate |
| **BB17** | 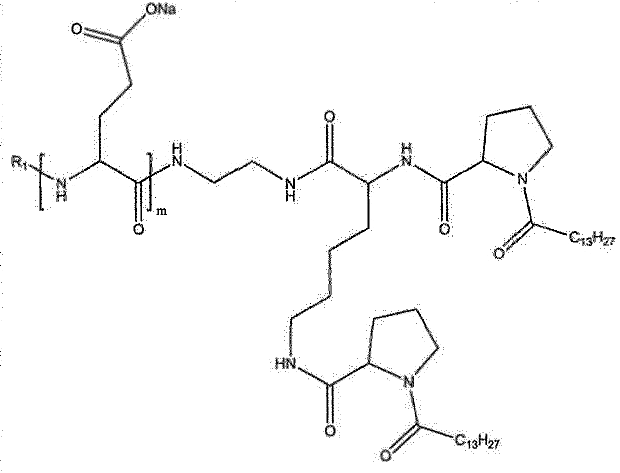 i = 0,015, DP (m) = 65 |
| | R₁ = H ou pyroglutamate |
| **BB18** | 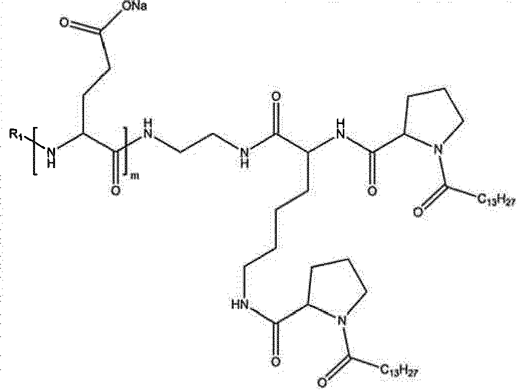 i = 0,025, DP (m) = 40 |
| | R1 = H ou pyroglutamate |
| **BB19** | 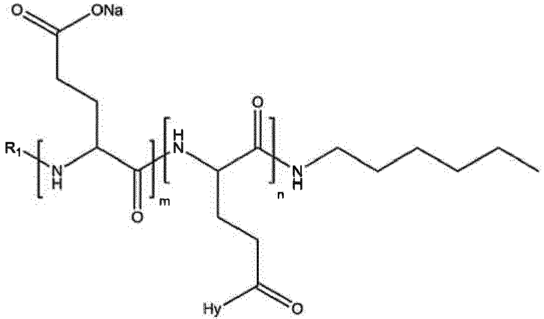 i = 0,045, DP (m + n) = 60 |
| | 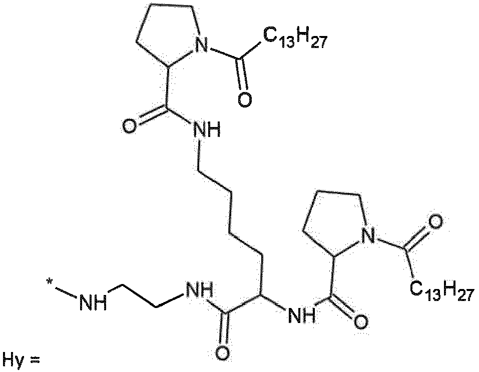 R₁ = pyroglutamate |
| **BB20** | 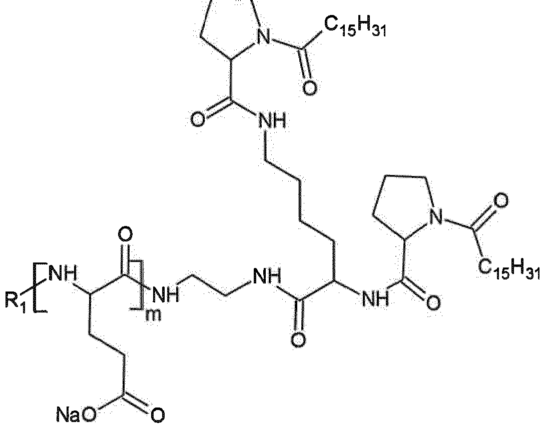 i = 0,043, DP (m) = 23 |
| | R1 = H ou pyroglutamate |
| **BB21** | 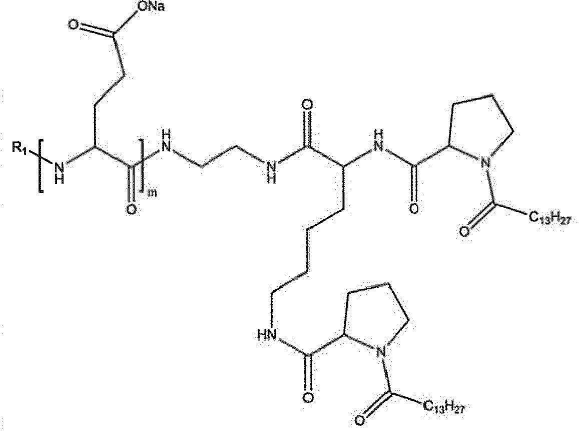 i = 0,11, DP (m) = 9 |
| | R1 = H ou pyroglutamate |
| **BB22** | 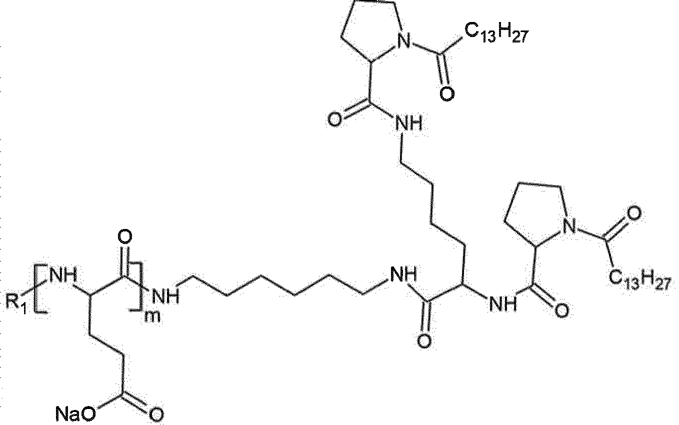 i = 0,04, DP (m) = 25 |
| | R1 = H ou pyroglutamate |
| **BB23** | 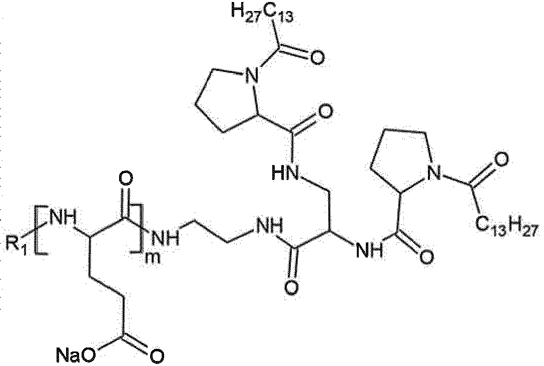 i = 0,048, DP (m) = 21 |
| | R1 = H ou pyroglutamate |
| **BB24** | 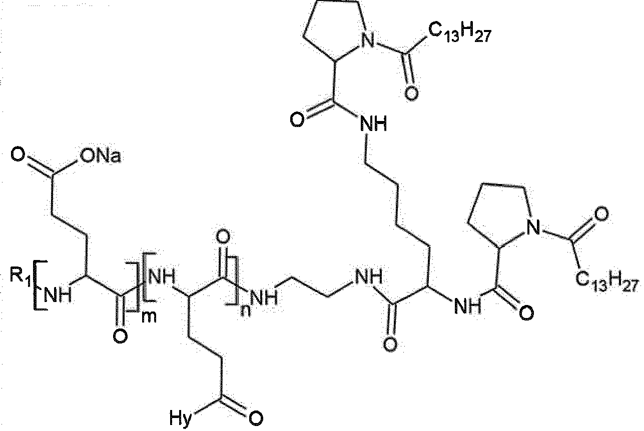 i = 0,089, DP (m) = 22 |
| | 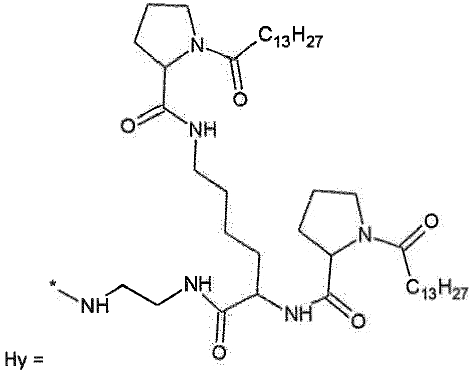 R1= H ou pyroglutamate |
| **BB25** | 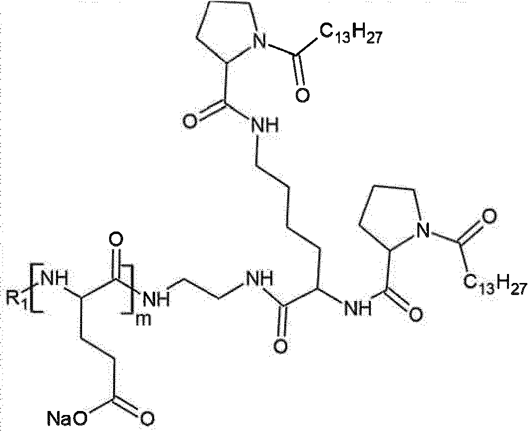 i = 0,09, DP (m) = 22 |
| | 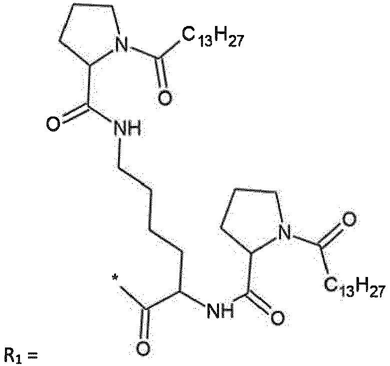 |

### Exemple BB1 : Co-polyaminoacide BB1 - poly-L-glutamate de sodium modifié par la molécule BA2 et ayant une masse molaire moyenne en nombre (Mn) de 2400 g/mol.

Co-polyaminoacideBB1-1 : acide poly-L-glutamique de masse molaire moyenne en nombre (Mn) relative 3860 g/mol issu de la polymérisation du γ-benzyl-L-glutamate *N*-carboxyanhydride initiée par l'hexylamine.

Dans un ballon préalablement séché à l'étuve est placé sous vide du γ-benzyl-L-glutamate *N*-carboxyanhydride (90,0 g, 342 mmol) pendant 30 min, puis du DMF anhydre (465 mL) est introduit. Le mélange est alors agité sous argon jusqu'à complète dissolution, refroidi à 4 °C, puis de l'hexylamine (1,8 mL 14 mmol) est introduite rapidement. Le mélange est agité entre 4 °C et température ambiante pendant 2 jours. Le milieu réactionnel est ensuite chauffé à 65 °C pendant 4 h, refroidi à température ambiante puis coulé goutte à goutte dans du diisopropyléther froid (6 L) sous agitation. Le précipité blanc est récupéré par filtration, lavé avec du diisopropyléther (500 mL puis 250 mL) puis séché sous vide à 30 °C pour donner un acide poly(γ-benzyl-L-glutamique) (PBLG).

À une solution de PBLG (42,1 g) dans l'acide trifluoroacétique (TFA, 325 mL) à 4 °C est ajoutée goutte à goutte une solution d'acide bromhydrique (HBr) à 33 % dans l'acide acétique (135 mL, 0,77 mol). Le mélange est agité à température ambiante pendant 2 h, puis coulé goutte à goutte sur un mélange 1:1 (v/v) de diisopropyléther et d'eau sous agitation (1,6 L). Après 1 h 30 d'agitation, le mélange hétérogène est laissé au repos pendant une nuit. Le précipité blanc est récupéré par filtration, lavé avec un mélange 1:1 (v/v) de diisopropyléther et d'eau (200 mL).

Le solide obtenu est alors solubilisé dans de l'eau (1 L) en ajustant le pH à 7 par ajout d'une solution aqueuse de soude 10 N puis une solution aqueuse de soude 1 N. Après solubilisation, la concentration théorique est ajustée à 25 g/L théorique par addition d'eau pour obtenir un volume final de 1,5 L.

La solution est filtrée sur filtre 0,45 µm puis purifiée par ultrafiltration contre une solution de NaCl 0,9 %, puis de l'eau jusqu'à ce que la conductimétrie du perméat soit inférieure à 50 µS/cm.

La solution aqueuse est alors acidifiée par ajout de solution d'acide chlorhydrique 37 % jusqu'à atteindre un pH de 2. Après 4 h d'agitation, le précipité obtenu est filtré puis séché sous vide à 30 °C pour donner un acide poly-L-glutamique de masse molaire moyenne en nombre (Mn) 3860 g/mol par rapport à un standard de polyoxyéthylène (PEG).

### Co-polyaminoacide BB1

Le co-polyaminoacide BB1-1 (10,0 g) est solubilisé dans le DMF (700 mL) à 30-40 °C puis refroidi à 0 °C. Le sel de chlorhydrate de la molécule BA2 (2,95 g, 3,8 mmol) est mis en suspension dans du DMF (45 mL) et de la triéthylamine (0,39 g, 3,8 mmol) est ensuite ajoutée à cette suspension puis le mélange est légèrement chauffé sous agitation jusqu'à complète dissolution. À la solution de co-polyaminoacide à 0 °C, de la N-méthylmorpholine (NMM, 7,6 g, 75 mmol) dans le DMF (14 mL) et du chloroformate d'éthyle (ECF, 8,1 g, 75 mmol) sont ajoutés. Après 10 min à 0 °C, la solution de molécule BA2 est ajoutée et le milieu maintenu à 30 °C durant 1 h. Le milieu réactionnel est coulé goutte-à-goutte sur 6 L d'eau contenant du NaCl à 15 % massique et du HCl (pH 2), puis laissé reposer une nuit. Le précipité est collecté par filtration, lavé par la solution de chlorure de sodium à pH 2 (1 L) et séché sous vide pendant environ 1 h. Le solide blanc obtenu est repris dans de l'eau (600 mL) et le pH est ajusté à 7 par ajout lent d'une solution aqueuse de NaOH 1 N. Le volume est ajusté à 700 mL par ajout d'eau. Après filtration sur filtre 0,45 µm, la solution limpide obtenue est purifiée par ultrafiltration contre une solution de NaCl 0,9 % puis de l'eau, jusqu'à ce que la conductimétrie du perméat soit inférieure à 50 µS/cm. Après déchargement, la solution est filtrée sur filtre 0,2 µm et stockée à 2-8 °C.
Extrait sec : 19,7 mg/g.
DP (estimé d'après la RMN ¹H) : 23.
D'après la RMN ¹H : i = 0,05.
La masse molaire moyenne calculée du co-polyaminoacide BB1 est de 4350 g/mol.
HPLC-SEC aqueuse (calibrant PEG) : Mn = 2400 g/mol.

### Exemple BB2 : co-polyaminoacide BB2 - poly-L-glutamate de sodium modifié par la molécule BA2 et ayant une mase molaire moyenne en nombre (Mn) de 4900 g/mol.

Un acide poly-L-glutamique de masse molaire moyenne en nombre (Mn) 4100 g/mol (5,0 g) obtenu par un procédé similaire à celui utilisé pour la préparation du co-polyaminoacide BB1-1 est solubilisé dans le DMF (205 mL) à 30-40 °C puis maintenu à cette température. En parallèle, le sel de chlorhydrate de la molécule BA2 (1,44 g, 1,84 mmol) est mis en suspension dans du DMF (10 mL) et de la triéthylamine (0,19 g, 1,84 mmol) est ajoutée, puis le mélange est légèrement chauffé sous agitation jusqu'à complète dissolution. À la solution de co-polyaminoacide dans le DMF, de la NMM (3,7 g, 36,7 mmol), la solution de molécule BA2 puis de la *N*-oxyde de 2-hydroxypyridine (HOPO, 0,31 g, 2,76 mmol) sont ajoutées successivement. Le milieu réactionnel est alors refroidi à 0 °C, puis du EDC (0,53 g, 2,76 mmol) est ajouté et le milieu est remonté à température ambiante durant 3 h. Le milieu réactionnel est coulé au goutte-à-goutte sur 1,55 L d'eau contenant du NaCl à 15 % massique et du HCl (pH 2) sous agitation. A la fin de l'ajout, le pH est réajusté à 2 avec une solution de HCl 1 N, et la suspension est laissée reposer une nuit. Le précipité est collecté par filtration, puis rincé par 100 mL d'eau. Le solide blanc obtenu est solubilisé dans 200 mL d'eau par ajout lent d'une solution aqueuse de NaOH 1 N jusqu'à pH 7 sous agitation, puis la solution est filtrée sur filtre 0,45 µm. La solution limpide obtenue est purifiée par ultrafiltration contre une solution de NaCl 0,9 % puis de l'eau, jusqu'à ce que la conductimétrie du perméat soit inférieure à 50 µS/cm. La solution obtenue est filtrée sur filtre 0,2 µm et stockée à 2-8 °C.
Extrait sec : 16,3 mg/g.
DP (estimé d'après la RMN ¹H) : 21.
D'après la RMN ¹H : i = 0,047.
La masse molaire moyenne calculée du co-polyaminoacide BB2 est de 3932 g/mol.
HPLC-SEC aqueuse (calibrant PEG) : Mn = 4900 g/mol.

### Exemple BB3 : Co-polyaminoacide BB3 - poly-L-glutamate de sodium modifié par la molécule BA2 et ayant une masse molaire moyenne en nombre (Mn) de 6400 g/mol.

Co-polyaminoacide BB3-1 : acide poly-L-glutamique de masse molaire moyenne en nombre (Mn) 17500 g/mol issu de la polymérisation du γ-méthyl-L-glutamate *N*-carboxyanhydride initiée par la L-leucinamide.

Un acide poly-L-glutamique de masse molaire moyenne en nombre (Mn) 17500 g/mol relative à un standard polymétacrylate de méthyle (PMMA) est obtenu par polymérisation du γ-méthyl *N*-carboxyanhydride d'acide glutamique en utilisant la L-leucinamide comme initiateur et en effectuant une déprotection des esters méthyliques par utilisation d'une solution d'acide chlorhydrique à 37 % selon le procédé décrit dans la demande de brevet FR-A-2 801 226.

Par un procédé similaire à celui utilisé pour la préparation du co-polyaminoacide BB2 appliqué au sel chlorhydrate de la molécule BA2 (3,23 g, 4,1 mmol) et au co-polyaminoacie BB3-1 (11 g), un poly-L-glutamate de sodium modifié par la molécule BA2 est obtenu.
Extrait sec : 27,5 mg/g.
DP (estimé d'après la RMN ¹H) : 34.
D'après la RMN ¹H : li = 0,049.
La masse molaire moyenne calculée du co-polyaminoacide BB3 est de 6405 g/mol.
HPLC-SEC aqueuse (calibrant PEG) : Mn = 6400 g/mol.

### Exemple BB4 : co-polyaminoacide BB4 - poly-L-glutamate de sodium modifié par la molécule BA2 et ayant une masse molaire moyenne en nombre (Mn) de 10500 g/mol.

Par un procédé similaire à celui utilisé pour la préparation du co-polyaminoacide BB2 appliqué au sel de chlorhydrate de la molécule BA2 (5 g, 6,35 mmol) et à un acide poly-L-glutamique de masse molaire moyenne en nombre Mn=10800 g/mol (21,7 g) obtenu par un procédé similaire à celui utilisé pour la préparation du co-polyaminoacide BB1-1, un poly-L-glutamate de sodium modifié par la molécule BA2 est obtenu.
Extrait sec : 28,2 mg/g.
DP (estimé d'après la RMN ¹H) : 65.
D'après la RMN ¹H : i = 0,04.
La masse molaire moyenne calculée du co-polyaminoacide BB4 est de 11721 g/mol.
HPLC-SEC aqueuse (calibrant PEG) : Mn = 10500 g/mol.

### Exemple BB5 : Co-polyaminoacide BB5 - poly-L-glutamate de sodium cappé à une de ses extrémités par un groupement acétyle et modifié par la molécule BA2 et ayant une masse molaire moyenne en nombre (Mn) de 3600 g/mol.

Co-polyaminoacide BB5-1 : acide poly-L-glutamlque de Mn 3700 g/mol Issu de la polymérisation du γ-benzyl-L-glutamate *N*-carboxyanhydride initiée par l'hexylamine et cappé à une de ses extrémités par un groupement acétyle.

Dans un ballon préalablement séché à l'étuve est placé sous vide du γ-benzyl-L-glutamate *N*-carboxyanhydride (100,0 g, 380 mmol) pendant 30 min puis du DMF anhydre (250 mL) est introduit. Le mélange est alors agité sous argon jusqu'à complète dissolution, refroidi à 4 °C, puis de l'hexylamine (2,3 mL, 17 mmol) est introduite rapidement. Le mélange est agité entre 4 °C et température ambiante pendant 2 jours puis précipité dans du diisopropyléther (3,4 L). Le précipité est récupéré par filtration, lavé deux fois avec du diisopropyléther (225 mL) puis séché pour donner un solide blanc qui est dissout dans 450 mL de THF. À cette solution sont ajoutés successivement de la *N*,*N*-diisopropyléthylamine (DIPEA, 31 mL, 176 mmol) puis de l'anhydride acétique (17 mL, 176 mmol). Après une nuit d'agitation à température ambiante, la solution est versée lentement dans du diisopropyléther (3 L) sur une durée de 30 min et sous agitation. Après 1 h d'agitation, le précipité est filtré, lavé deux fois avec du diisopropyléther (200 mL) puis séché sous vide à 30 °C pour donner un acide poly(γ-benzyl-L-glutamique) cappé à une de ses extrémités par un groupement acétyle.

À une solution du co-polyaminoacide cappé (72 g) dans l'acide trifluoroacétique (TFA, 335 mL) à 4 °C est ajoutée goutte à goutte une solution d'acide bromhydrique (HBr) à 33 % dans l'acide acétique (235 mL, 1,34 mol). Le mélange est agité à température ambiante pendant 3 h 30, puis coulé goutte à goutte sur un mélange 1:1 (v/v) de diisopropyléther et d'eau sous agitation (4 L). Après 2 h d'agitation, le mélange hétérogène est laissé au repos pendant une nuit. Le précipité blanc est récupéré par filtration, lavé avec un mélange 1:1 (v/v) de diisopropyléther et d'eau (340 mL) puis avec de l'eau (340 mL). Le solide obtenu est alors solubilisé dans de l'eau (1,5 L) en ajustant le pH à 7 par ajout d'une solution aqueuse de soude 10 N puis une solution aqueuse de soude 1 N. Après solubilisation, la concentration théorique est ajustée à 20 g/L théorique par addition d'eau pour obtenir un volume final de 2,1 L. La solution est filtrée sur filtre 0,45 µm puis purifiée par ultrafiltration contre une solution de NaCl 0,9 %, puis de l'eau jusqu'à ce que la conductimétrie du perméat soit inférieure à 50 µS/cm. La solution de co-polyaminoacide est ensuite concentrée jusqu'à obtenir un volume final de 1,8 L. La solution aqueuse est alors acidifiée par ajout de solution d'acide chlorhydrique 37 % jusqu'à atteindre un pH de 2. Après 4 h d'agitation, le précipité obtenu est filtré, lavé avec de l'eau (330 mL) puis séché sous vide à 30 °C pour donner un acide poly-L-glutamique de masse molaire moyenne en nombre (Mn) 3700 g/mol par rapport à un standard de polyoxyéthylène (PEG).

### Co-polyaminoacide BB5

Par un procédé similaire à celui utilisé pour la préparation du co-polyaminoacide BB2 appliqué au sel chlorhydrate de la molécule BA2 (6,92 g, 8,8 mmol) et au co-polyaminoacide BB5-1 (30,0 g), un poly-L-glutamate de sodium cappé à une de ses extrémités par un groupement acétyle et modifié par la molécule BA2 est obtenu.
Extrait sec : 29,4 mg/g.
DP (estimé d'après la RMN ¹H) : 23.
D'après la RMN ¹H : i = 0,042.
La masse molaire moyenne calculée du co-polyaminoacide BB5 est de 4302 g/mol.
HPLC-SEC aqueuse (calibrant PEG) : Mn = 3600 g/mol.

### Exemple BB6 : Co-polyaminoacide BB6 - poly-L-glutamate de sodium cappé à une de ses extrémités par un groupement acétyle et modifié par la molécule BA2 et ayant une masse molaire moyenne en nombre (Mn) de 4100 g/mol.

Par un procédé similaire à celui utilisé pour la préparation du co-polyaminoacide BB2 appliqué au sel chlorhydrate de la molécule BA2 (5,8 g, 7,4 mmol) et à un acide poly-L-glutamique de masse molaire moyenne en nombre Mn = 3800 g/mol (25 g) obtenu par un procédé similaire à celui utilisé pour la préparation du co-polyaminoacide BB5-1 en utilisant l'ammoniac à la place de l'hexylamine, un poly-L-glutamate de sodium cappé à une de ses extrémités par un groupement acétyle et modifié par la molécule BA2 est obtenu.
Extrait sec : 27,6 mg/g.
DP (estimé d'après la RMN ¹H) : 24.
D'après la RMN ¹H : i = 0,04.
La masse molaire moyenne calculée du co-polyaminoacide BB6 est de 4387 g/mol.
HPLC-SEC aqueuse (calibrant PEG) : Mn = 4100 g/mol.

### Exemple BB7 : Co-polyaminoacide BB7 - poly-L-glutamate de sodium modifié par la molécule BA2 et ayant une masse molaire moyenne en nombre (Mn) de 4200 g/mol.

Par un procédé similaire à celui utilisé pour la préparation du co-polyaminoacide BB2 appliqué au sel chlorhydrate de la molécule BA2 (7,07 g, 9,0 mmol) et à un acide poly-L-glutamique de masse molaire moyenne en nombre Mn = 3600 g/mol (30,0 g) obtenu par un procédé similaire à celui utilisé pour la préparation du co-polyaminoacide BB1-1, un poly-L-glutamate de sodium modifié par la molécule BA2 est obtenu.
Extrait sec : 28,3 mg/g.
DP (estimé d'après la RMN ¹H) : 22.
D'après la RMN ¹H : i = 0,042.
La masse molaire moyenne calculée du co-polyaminoacide BB7 est de 4039 g/mol.
HPLC-SEC aqueuse (calibrant PEG) : Mn = 4200 g/mol.

### Exemple BB8 : co-polyaminoacide BB8 - poly-L-glutamate de sodium modifié par la molécule BA2 et ayant une masse molaire moyenne en nombre (Mn) de 5200 g/mol.

Par un procédé similaire à celui utilisé pour la préparation du co-polyaminoacide BB2 appliqué au sel chlorhydrate de la molécule BA2 (0,85 g, 1,1 mmol) et à un acide poly-L-glutamique de masse molaire moyenne en nombre Mn = 4100 g/mol (5,0 g) obtenu par un procédé similaire à celui utilisé pour la préparation du co-polyaminoacide BB1-1, un poly-L-glutamate de sodium modifié par la molécule BA2 est obtenu.
Extrait sec : 28,6 mg/g.
DP (estimé d'après la RMN ¹H) : 21.
D'après la RMN ¹H : i = 0,026.
La masse molaire moyenne calculée du co-polyaminoacide BB8 est de 3620 g/mol.
HPLC-SEC aqueuse (calibrant PEG) : Mn = 5200 g/mol.

### Exemple BB9 : co-polyaminoacide BB9 - poly-L-glutamate de sodium modifié par la molécule BA3 et ayant une masse molaire moyenne en nombre (Mn) de 4700 g/mol.

Par un procédé similaire à celui utilisé pour la préparation du co-polyaminoacide BB2 appliqué au sel chlorhydrate de la molécule BA3 (3,05 g, 3,6 mmol) et à un acide poly-L-glutamique de masse molaire moyenne en nombre Mn = 4100 g/mol (10,0 g) obtenu par un procédé similaire à celui utilisé pour la préparation du co-polyaminoacide BB1-1, un poly-L-glutamate de sodium modifié par la molécule BA3 est obtenu.
Extrait sec : 28,6 mg/g.
DP (estimé d'après la RMN ¹H) : 26.
D'après la RMN ¹H : i = 0,05.
La masse molaire moyenne calculée du co-polyaminoacide BB9 est de 4982 g/mol.
HPLC-SEC aqueuse (calibrant PEG) : Mn = 4700 g/mol.

### Exemple BB10 : co-polyaminoacide BB10 - poly-L-glutamate de sodium modifié par la molécule BA3 et ayant une masse molaire moyenne en nombre (Mn) de 4200 g/mol.

Par un procédé similaire à celui utilisé pour la préparation du co-polyaminoacide BB2 appliqué au sel chlorhydrate de la molécule BA3 (1,90 g, 2,3 mmol) et à un acide poly-L-glutamique de masse molaire moyenne en nombre Mn = 3500 g/mol (10,0 g) obtenu par un procédé similaire à celui utilisé pour la préparation du co-polyaminoacide BB1-1, un poly-L-glutamate de sodium modifié par la molécule BA3 est obtenu.
Extrait sec : 25,9 mg/g.
DP (estimé d'après la RMN ¹H) : 22.
D'après la RMN ¹H : i = 0,029.
La masse molaire moyenne calculée du co-polyaminoacide BB10 est de 3872 g/mol.
HPLC-SEC aqueuse (calibrant PEG) : Mn = 4200 g/mol.

### Exemple BB11 : co-polyaminoacide BB11 - poly-L-glutamate de sodium cappé à une de ses extrémités par un groupement acétyle et modifié par la molécule BA4 et ayant une masse molaire moyenne en nombre (Mn) de 3900 g/mol.

Par un procédé similaire à celui utilisé pour la préparation du co-polyaminoacide BB2 appliqué au sel chlorhydrate de la molécule BA4 (2,21 g, 2,2 mmol) et à un acide poly-L-glutamique de masse molaire moyenne en nombre Mn = 3700 g/mol (10 g) obtenu par un procédé similaire à celui utilisé pour la préparation du co-polyaminoacide BB5-1, un poly-L-glutamate de sodium cappé à une de ses extrémités par un groupement acétyle et modifié par la molécule BA4 est obtenu.
Extrait sec : 28,1 mg/g.
DP (estimé d'après la RMN ¹H) : 22.
D'après la RMN ¹H : i = 0,032.
La masse molaire moyenne calculée du co-polyaminoacide BB11 est de 4118 g/mol.
HPLC-SEC aqueuse (calibrant PEG) : Mn = 3900 g/mol.

### Exemple BB12 : co-polyaminoacide BB12 - poly-L-glutamate de sodium cappé à une de ses extrémités par un groupement acétyle et modifié par la molécule BA3 et ayant une masse molaire moyenne en nombre (Mn) de 3900 g/mol.

Par un procédé similaire à celui utilisé pour la préparation du co-polyaminoacide BB2 appliqué au sel chlorhydrate de la molécule BA3 (1,9 g, 2,3 mmol) et à un acide poly-L-glutamique de masse moyenne en nombre Mn = 3600 g/mol (10 g) obtenu par un procédé similaire à celui utilisé pour la préparation du co-polyaminoacide BB5-1, un poly-L-glutamate de sodium cappé à une de ses extrémités par un groupement acétyle et modifié par la molécule BA3 est obtenu.
Extrait sec : 26,7 mg/g.
DP (estimé d'après la RMN ¹H) : 23.
D'après la RMN ¹H : i = 0,03.
La masse molaire moyenne calculée du co-polyaminoacide BB12 est de 4145 g/mol.
HPLC-SEC aqueuse (calibrant PEG) : Mn = 3900 g/mol.

### Exemple BB13 : co-polyaminoacide BB13 - poly-L-glutamate de sodium modifié par la molécule BA1 et ayant une masse molaire moyenne en nombre (Mn) de 2800 g/mol.

Par un procédé similaire à celui utilisé pour la préparation du co-polyaminoacide BB1 appliqué au sel de chlorhydrate de la molécule BA1 (3,65 g, 5 mmol) et à un acide poly-L-glutamique de masse molaire moyenne en nombre Mn = 3600 g/mol (10 g) obtenu par un procédé similaire à celui utilisé pour la préparation du co-polyaminoacide BB1-1, un poly-L-glutamate de sodium modifié par la molécule BA1 est obtenu.
Extrait sec : 25,6 mg/g.
DP (estimé d'après la RMN ¹H) : 25.
D'après la RMN ¹H : i = 0,08.
La masse molaire moyenne calculée du co-polyaminoacide BB13 est de 5253 g/mol.
HPLC-SEC aqueuse (calibrant PEG) : Mn = 2800 g/mol.

### Exemple BB14 : co-polyaminoacide BB14 - poly-L-glutamate de sodium modifié à une de ses extrémités par la molécule BA2 et ayant une masse molaire moyenne en nombre (Mn) de 4020 g/mol.

Dans un contenant adapté sont introduits successivement le sel de chlorhydrate de la molécule BA2 (2,12 g, 2,70 mmol), du chloroforme (40 mL), du tamis moléculaire 4 Å (1,5 g), ainsi que de la résine échangeuse d'ion Amberlite IRN 150 (1,5 g). Après 1 h d'agitation sur rouleaux, le milieu est filtré et la résine est rincée avec du chloroforme. Le mélange est évaporé puis co-évaporé avec du toluène. Le résidu est solubilisé dans du DMF anhydre (20 mL) pour être utilisé directement dans la réaction de polymérisation.

Dans un ballon préalablement séché à l'étuve, du γ-benzyl-L-glutamate *N-*carboxyanhydride (18 g, 68,42 mmol) est placé sous vide pendant 30 min puis du DMF anhydre (100 mL) est introduit. Le mélange est agité sous argon jusqu'à solubilisation complète, refroidi à 4 °C, puis la solution de molécule BA2 préparée comme décrit précédemment est introduite rapidement. Le mélange est agité entre 4 °Cet température ambiante pendant 2 jours, puis chauffé à 65 °C pendant 2 h. Le mélange réactionnel est alors refroidi à température ambiante puis versé goutte à goutte dans du diisopropyléther (1,2 L) sous agitation. Le précipité blanc est récupéré par filtration, lavé deux fois avec du diisopropyléther (100 mL) puis séché sous vide à 30 °C pour obtenir un solide blanc. Le solide est dilué dans du TFA (105 mL), et une solution d'acide bromhydrique (HBr) à 33 % dans de l'acide acétique (38 mL, 220 mmol) est alors ajoutée goutte à goutte et à 0 °C. La solution est agitée pendant 2 h à température ambiante puis est coulée goutte à goutte sur un mélange 1:1 (v/v) de diisopropyléther/eau et sous agitation (600 mL). Après 2 h d'agitation, le mélange hétérogène est laissé au repos pendant une nuit. Le précipité blanc est récupéré par filtration, lavé successivement avec un mélange 1:1 (v/v) de diisopropyléther et d'eau (200 mL) puis avec de l'eau (100 mL). Le solide obtenu est solubilisé dans de l'eau (450 mL) en ajustant le pH à 7 par ajout d'une solution aqueuse de soude 10 N puis une solution aqueuse de soude 1 N. Le mélange est filtré sur filtre 0,45 µm puis est purifié par ultrafiltration contre une solution de NaCl 0,9 % puis de l'eau jusqu'à ce que la conductimétrie du perméat soit inférieure à 50 µS/cm. La solution de co-polyaminoacide est ensuite concentrée à environ 30 g/L théorique et le pH est ajusté à 7,0. La solution aqueuse est filtrée sur 0,2 µm et conservée à 4 °C.
Extrait sec : 22,3 mg/g.
DP (estimé par RMN ¹H) = 29 donc i = 0,034.
La masse molaire moyenne calculée du co-polyaminoacide BB14 est de 5089 g/mol.
HPLC-SEC aqueuse (calibrant PEG) : Mn = 4020 g/mol.

### Exemple BB15 : co-polyaminoacide BB15 - poly-L-glutamate de sodium modifié à une de ses extrémités par la molécule BA3 et ayant une masse molaire moyenne en nombre (Mn) de 3389 g/mol.

Par un procédé similaire à celui utilisé pour la préparation du co-polyaminoacide BB14 appliqué au sel chlorhydrate de la molécule BA3 (3,62 g, 4,32 mmol) et à 25,0 g (94,97 mmol) de γ-benzyl-L-glutamate *N*-carboxyanhydride, un poly-L-glutamate de sodium modifié à une de ses extrémités par la molécule BA3 est obtenu.
Extrait sec : 30,4 mg/g.
DP (estimé par RMN ¹H) = 24 donc i = 0,042.
La masse molaire moyenne calculée du co-polyaminoacide BB15 est de 4390 g/mol.
HPLC-SEC aqueuse (calibrant PEG) : Mn = 3389 g/mol.

### Exemple BB16 : co-polyaminoacide BB16 - poly-L-glutamate de sodium modifié à une de ses extrémités par la molécule BA4 et ayant une masse molaire moyenne en nombre (Mn) de 3300 g/mol.

Par un procédé similaire à celui utilisé pour la préparation du co-polyaminoacide BB14 appliqué au sel chlorhydrate de la molécule BA4 (5,70 g, 5,70 mmol) et à 29,99 g (113,9 mmol) de γ-benzyl-L-glutamate *N*-carboxyanhydride, un poly-L-glutamate de sodium modifié à une de ses extrémités par la molécule BA4 est obtenu.
Extrait sec : 32,3 mg/g.
DP (estimé par RMN ¹H) = 23 donc i = 0,043.
La masse molaire moyenne calculée du co-polyaminoacide BB16 est de 4399 g/ g/mol.
HPLC-SEC aqueuse (calibrant PEG) : Mn = 3300 g/ g/mol.

### Exemple BB17 : co-polyaminoacide BB17 - poly-L-glutamate de sodium modifié à une de ses extrémités par la molécule BA3 et ayant une masse molaire moyenne en nombre de 10700 g/mol.

Par un procédé similaire à celui utilisé pour la préparation du co-polyaminoacide BB14 appliqué au sel chlorhydrate de la molécule BA3 (2,51 g, 3 mmol) et à 52,7 g (200 mmol) de γ-benzyl-L-glutamate *N*-carboxyanhydride, un poly-L-glutamate de sodium modifié à une de ses extrémités par la molécule BA3 est obtenu.
Extrait sec : 24,5 mg/g.
DP (estimé par RMN ¹H) = 65 donc i = 0,015.
La masse molaire moyenne calculée du co-polyaminoacide BB17 est de 10585 g/ g/mol.
HPLC-SEC aqueuse (calibrant PEG) : Mn = 10700 g/ g/mol.

### Exemple BB18 : co-polyaminoacide BB18 - poly-L-glutamate de sodium modifié à une de ses extrémités par la molécule BA3 et ayant une masse molaire moyenne en nombre de 6600 g/mol.

Par un procédé similaire à celui utilisé pour la préparation du co-polyaminoacide BB14 appliqué au sel chlorhydrate de la molécule BA3 (2,51 g, 3 mmol) et à 31,6 g (120 mmol) de γ-benzyl-L-glutamate *N*-carboxyanhydride, un poly-L-glutamate de sodium modifié à une de ses extrémités par la molécule BA3 est obtenu.
Extrait sec : 27,3 mg/g.
DP (estimé par RMN ¹H) = 40 donc i = 0,025.
La masse molaire moyenne calculée du co-polyaminoacide BB18 est de 6889 g/ g/mol.
HPLC-SEC aqueuse (calibrant PEG) : Mn = 6600 g/ g/mol.

### Exemple BB19 : Co-polyaminoacide BB19 - poly-L-glutamate de sodium et modifié par la molécule BA3 et ayant une masse molaire moyenne en nombre (Mn) de 7700 g/mol.

Par un procédé similaire à celui utilisé pour la préparation du co-polyaminoacide AB23 appliqué au sel de chlorhydrate de la molécule BA3 et au co-polyaminoacide AB23-1, un poly-L-glutamate de sodium modifié par la molécule BA3 est obtenu.
Extrait sec : 25,3 mg/g.
DP (estimé d'après la RMN ¹H) : 60.
D'après la RMN ¹H : i = 0,045.
La masse molaire moyenne calculée du co-polyaminoacide BB19 est de 11188 g/ g/mol.
HPLC-SEC organique (calibrant PEG) : Mn = 7700 g/ g/mol.

### Exemple BB20 : co-polyaminoacide BB20 - poly-L-glutamate de sodium modifié à une de ses extrémités par la molécule BA5 et ayant une masse molaire moyenne en nombre (Mn) de 2800 g/mol.

Par un procédé similaire à celui utilisé pour la préparation du co-polyaminoacide BB14 appliqué à la molécule BA5 sous forme d'amine libre (1,70 g, 1,98 mmol) et de γ-benzyl-L-glutamate *N*-carboxyanhydride (11,46 g, 43,5 mmol), un poly-L-glutamate de sodium modifié à une de ses extrémités par la molécule BA5 est obtenu.
Extrait sec : 20,7 mg/g.
DP (estimé par RMN ¹H) = 23 donc i = 0,043.
La masse molaire moyenne calculée du co-polyaminoacide BB20 est de 4295 g/ g/mol.
HPLC-SEC aqueuse (calibrant PEG) : Mn = 2800 g/ g/mol.

### Exemple BB21 : co-polyaminoacide BB21 - poly-L-glutamate de sodium modifié à une de ses extrémités par la molécule BA3 et ayant une masse molaire moyenne en nombre (Mn) de 1100 g/mol.

Par un procédé similaire à celui utilisé pour la préparation du co-polyaminoacide BB14 appliqué à la molécule BA3 sous forme d'amine libre (3,814 g, 4,75 mmol) et de γ-benzyl-L-glutamate *N*-carboxyanhydride (10,0 g, 38,0 mmol), un poly-L-glutamate de sodium modifié à une de ses extrémités par la molécule BA3 est obtenu.
Extrait sec : 16,1 mg/g.
DP (estimé par RMN ¹H) = 9 donc i = 0,11.
La masse molaire moyenne calculée du co-polyaminoacide BB21 est de 2123 g/ g/mol.
HPLC-SEC aqueuse (calibrant PEG) : Mn = 1100 g/ g/mol.

### Exemple BB22 : co-polyaminoacide BB22 - poly-L-glutamate de sodium modifié à une de ses extrémités par la molécule BA6 et ayant une masse molaire moyenne en nombre (Mn) de 3300 g/mol.

Par un procédé similaire à celui utilisé pour la préparation du co-polyaminoacide BB14 appliqué à la molécule BA6 sous forme d'amine libre (4,45 g, 5,18 mmol) et à 30,0 g (113,96 mmol) de γ-benzyl-L-glutamate *N*-carboxyanhydride, un poly-L-glutamate de sodium modifié à une de ses extrémités par la molécule BA6 est obtenu.
Extrait sec : 29,0 mg/g.
DP (estimé par RMN ¹H) = 25 donc i = 0,04.
La masse molaire moyenne calculée du co-polyaminoacide BB22 est de 4597 g/mol.
HPLC-SEC aqueuse (calibrant PEG) : Mn = 3300 g/mol.

### Exemple BB23 : co-polyaminoacide BB23 - poly-L-glutamate de sodium modifié à une de ses extrémités par la molécule BA7 et ayant une masse molaire moyenne en nombre (Mn) de 2900 g/mol.

Par un procédé similaire à celui utilisé pour la préparation du co-polyaminoacide BB14 appliqué à la molécule BA7 sous forme d'amine libre (3,05 g, 4,01 mmol) et de γ-benzyl-L-glutamate *N*-carboxyanhydride (22,78 g, 86,5 mmol), un poly-L-glutamate de sodium modifié à une de ses extrémités par la molécule BA7 est obtenu.
Extrait sec : 16,9 mg/g.
DP (estimé par RMN ¹H) = 21 donc i = 0,048.
La masse molaire moyenne calculée du co-polyaminoacide BB23 est de 3894 g/mol.
HPLC-SEC aqueuse (calibrant PEG) : Mn = 2900 g/mol.

### Exemple BB24 : co-polyaminoacide BB27 - poly-L-glutamate de sodium modifié à l'une de ses extrémités par la molécule BA3 et modifié par la molécule BA3 et ayant une masse molaire moyenne en nombre (Mn) de 2300 g/mol.

Co-polyaminoacide BB24-1 : acide poly-L-glutamique modifié à une de ses extrémités par la molécule BA3 et cappé à l'autre extrémité par l'acide pidolique.

Dans un ballon préalablement séché à l'étuve, du γ-benzyl-L-glutamate *N*-carboxyanhydride (122,58 g, 466 mmol) est placé sous vide pendant 30 min puis du DMF anhydre (220 mL) est introduit. Le mélange est agité sous argon jusqu'à solubilisation complète, refroidi à -10 °C, puis une solution de molécule BA3 sous forme d'amine libre (17,08 g, 21,3 mmol) dans le chloroforme (40 mL) est introduite rapidement. Le mélange est agité entre 0 °C et température ambiante pendant 2 jours, puis chauffé à 65 °C pendant 4 h. Le mélange réactionnel est alors refroidi à 25 °C puis est ajouté de l'acide pidolique (13,66 g, 105,8 mmol), du HOBt (2,35 g, 15,3 mmol) et de l'EDC (20,28 g, 105,8 mmol). Après 24 h d'agitation à 25 °C, la solution est concentrée sous vide pour éliminer le chloroforme et 50 % du DMF. Le mélange réactionnel est alors chauffé à 55 °C et 1150 mL de méthanol sont introduit en 1 h. Le mélange réactionnel est alors refroidi à 0 °C. Après 18 h, le précipité blanc est récupéré par filtration, lavé trois fois avec 270 mL de diisopropyl éther puis séché sous vide à 30 °Cpour obtenir un solide blanc. Le solide est dilué dans du TFA (390 mL), et une solution d'acide bromhydrique (HBr) à 33 % dans de l'acide acétique (271 mL, 1547 mmol) est alors ajoutée goutte à goutte et à 0 °C. La solution est agitée pendant 2 h à température ambiante puis est coulée goutte à goutte sur un mélange 1:1 (v/v) de diisopropyléther / eau et sous agitation (970 mL). Après 2 h d'agitation, le mélange hétérogène est laissé au repos pendant une nuit. Le précipité blanc est récupéré par filtration, lavé successivement avec du diisopropyl éther (380 mL) puis deux fois avec de l'eau (380 mL mL). Le solide obtenu est solubilisé dans de l'eau (3,6 L) en ajustant le pH à 7 par ajout d'une solution aqueuse de soude 10 N puis une solution aqueuse de soude 1 N. Le mélange est filtré sur filtre 0,45 µm puis est purifié par ultrafiltration contre une solution de NaCl 0,9 %, une solution de NaOH 0,1 N, une solution de NaCl 0,9 %, une solution de tampon phosphate (150 mM), une solution de NaCl 0,9 % puis de l'eau jusqu'à ce que la conductimétrie du perméat soit inférieure à 50 µS/cm. La solution de co-polyaminoacide est ensuite concentrée à environ 30 g/L théorique, filtrée sur 0,2 µm puis acidifié à pH 2 sous agitation par addition d'une solution de HCl à 37 %. Le précipité est alors récupéré par filtration, lavé deux fois avec de l'eau puis séché sous vide à 30 °Cpour obtenir un solide blanc.

### Co-polyaminoacide BB24

Par un procédé similaire à celui utilisé pour la préparation du co-polyaminoacide BB2 appliqué à la molécule BA3 sous forme d'amine libre (1,206 g, 1,50 mmol) et au co-polyaminoacide BB24-1 (5,5 g, 33,4 mmol), un poly-L-glutamate de sodium modifié à l'une de ses extrémités par la molécule BA3 et modifié par la molécule BA3 est obtenu.
Extrait sec : 19,0 mg/g.
DP (estimé d'après la RMN ¹H) : 22.
D'après la RMN ¹H : i = 0,089.
La masse molaire moyenne calculée du co-polyaminoacide BB24 est de 4826 g/mol.
HPLC-SEC aqueuse (calibrant PEG) : Mn = 2300 g/mol.

### Exemple BB25 : co-polyaminoacide BB25 - poly-L-glutamate de sodium modifié à un de ses extrémités par la molécule BA3 et à l'autre extrémité par la molécule B8 et ayant une masse molaire moyenne en nombre (Mn) de 2000 g/mol.

À une solution de molécule B8 (0,946 g, 1,24 mmol) dans le DMF (8 mL) sont introduits du DCC (0,257 g, 1,24 mmol) et du NHS (0,143 g, 1,24 mmol). Après 16 h d'agitation à température ambiante, la solution est filtrée pour être utilisée directement dans la réaction suivante.

Dans un ballon préalablement séché à l'étuve, du γ-benzyl-L-glutamate N-carboxyanhydride (6,0 g, 22,8 mmol) est placé sous vide pendant 30 min puis du DMF anhydre (14 mL) est introduit. Le mélange est alors agité sous argon jusqu'à complète dissolution, refroidi à 0 °C, puis une solution de molécule BA3 sous forme d'amine libre (0,832 g, 1,04 mmol) dans le chloroforme (2,0 mL) est introduit rapidement. Après 18 h d'agitation à 0 °C, la solution de molécule B8 préparée précédemment est additionnée. La solution est agitée entre 0 °C et température ambiante pendant 22 h puis coulée goutte à goutte dans du diisopropyléther (0,34 L) sous agitation. Le précipité est récupéré par filtration, lavé avec du diisopropyléther (7 fois 15 mL) puis séché sous vide à 0 °Cpour donner un solide blanc. Le solide est dilué dans du TFA (23 mL), puis la solution est refroidie à 4 °C. Une solution de HBr à 33 % dans l'acide acétique (15 mL, 85,7 mmol) est alors ajoutée goutte à goutte. Le mélange est agité à température ambiante pendant 2 h, puis coulé goutte à goutte sur un mélange 1:1 (v/v) de diisopropyléther et d'eau sous agitation (0,28 L). Après 2 h d'agitation, le mélange hétérogène est laissé au repos pendant une nuit. Le précipité blanc est récupéré par filtration, lavé deux fois avec un mélange 1:1 (v/v) de diisopropyléther et d'eau (24 mL) puis deux fois avec de l'eau (24 mL). Le solide obtenu est alors solubilisé dans de l'eau (0,16 L) en ajustant le pH à 12 par ajout d'une solution aqueuse de soude 10 N puis une solution aqueuse de soude 1 N. Après 30 min le pH est ajusté à 7 par ajout lent d'une solution aqueuse de HCl 1 N. La solution est filtrée sur filtre 0,45 µm puis purifiée par ultrafiltration contre une solution de NaCl 0,9 % %, puis de l'eau jusqu'à ce que la conductimétrie du perméat soit inférieure à 50 µS/cm. La solution obtenue est filtrée sur filtre 0,2 µm et stockée à 2-8°C.
Extrait sec : 18,9 mg/g.
DP (estimé d'après la RMN ¹H) : 22.
D'après la RMN ¹H : i₁ = 0,09.
La masse molaire moyenne calculée du co-polyaminoacide BB25 est de 4871 g/mol.
HPLC-SEC aqueuse (calibrant PEG) : Mn = 2000 g/mol.

### C. COMPOSITIONS

### Exemple CV1 : Préparation d'une solution d'amyline humaine à 0,6 mg/mL contenant du m-crésol (29 mM), de la glycérine (174 mM) à pH 7,4.

Une solution d'amyline humaine concentrée à 3 mg/mL est préparée par dissolution d'amyline humaine sous forme de poudre achetée chez AmbioPharm. Cette solution est ajoutée à une solution concentrée d'excipients (m-crésol, glycérine) de manière à obtenir la composition finale visée. Le pH final est ajusté à 7,4 par ajout de NaOH/HCl.

### Exemple CV2 : Préparation d'une solution d'amyline humaine à 0,6 mg/mL contenant du co-polyaminoacide BB15, du m-crésol (29 mM) et de la glycérine (174 mM) à pH 7,4.

Une solution concentrée de co-polyaminoacide BB15 et d'excipients est préparée en ajoutant des solutions concentrées d'excipients (m-crésol, glycérine) à une solution concentrée de co-polyaminoacide BB15.

La solution d'amyline humaine concentrée à 3 mg/mL C1 est ajoutée à cette solution concentrée de co-polyaminoacide BB15 et d'excipients de manière à obtenir les compositions finales CV5 à CV11 (tableau 1g). Le pH final est ajusté à 7,4 par ajout de NaOH/HCl.

**Tableau 1g : Compositions et aspect visuel des solutions d'amyline humaine à pH 7,4 à différentes concentrations en co-polyaminoacide BB15.**

| Solution | Ratio BB15/amyline humaine | Concentration en co-polyaminoacide BB15 | | Aspect visuel de la solution |
|---|---|---|---|---|
| | mol/mol | mg/mL | mM | |
| CV1 | - | - | - | Limpide |
| CV5 | 5 | 3 | 0,73 | Limpide |
| CV6 | 6 | 3,6 | 0,88 | Limpide |
| CV7 | 7 | 4,2 | 1,03 | Limpide |
| CV8 | 8 | 4,8 | 1,17 | Limpide |
| CV9 | 9 | 5,4 | 1,32 | Limpide |
| CV10 | 10 | 6 | 1,47 | Limpide |
| CV11 | 17 | 10,5 | 2,57 | Limpide |

### Exemple CY1: Préparation d'une solution de pramlintide à 0,9 mg/mL contenant du m-crésol (29 mM) et de la glycérine (174 mM) à pH 7,4.

Une solution de pramlintide concentrée à 5 mg/mL est préparée par dissolution de pramlintide sous forme de poudre achetée chez Hyblo. Cette solution est ajoutée à une solution concentrée d'excipients (m-crésol, glycérine) de manière à obtenir la composition finale visée. Le pH final est ajusté à 7,4 par ajout de NaOH/HCl.

### Exemple CW1: Préparation d'une solution de pramlintide à 0,4 mg/mL contenant du phénol (30 mM), de la glycérine (174 mM) et de la glycylglycine (8 mM) à pH 7,4.

Par un procédé similaire à celui utilisé à l'exemple CY1, une solution de pramlintide à 0,4 mg/mL contenant du phénol (30 mM), de la glycérine (174 mM) et de la glycylglycine (8 mM) à pH 7,4 est obtenue.

### Exemple CY0: Préparation d'une solution de pramlintide à 0,9 mg/mL contenant du co-polyaminoacide BB15, du m-crésol (29 mM) et de la glycérine (174 mM) à pH 7,4.

Une solution concentrée de co-polyaminoacide BB15 et d'excipients est préparée en ajoutant des solutions concentrées d'excipients (m-crésol, glycérine) à une solution concentrée de co-polyaminoacide BB15.

Une solution de pramlintide concentrée à 5 mg/mL est ajoutée à cette solution concentrée de co-polyaminoacide BB15 et d'excipients de manière à obtenir les compositions finales CY2 à CY7 (tableau 3). Le pH final est ajusté à 7,4 par ajout de NaOH/HCl.

**Tableau 3 : Compositions et aspect visuel de solutions de pramlintide à pH 7,4 à différentes concentrations en co-polyaminoacide BB15.**

| Solution | Ratio BB15/pramlintide | Concentration en co-polyaminoacide BB15 | | Aspect visuel de la solution |
|---|---|---|---|---|
| | mol/mol | mg/mL | mM | |
| CY1 | - | - | - | Limpide |
| CY2 | 2 | 1,8 | 0,44 | Limpide |
| CY3 | 3 | 2,7 | 0,66 | Limpide |
| CY4 | 4 | 3,6 | 0,88 | Limpide |
| CY5 | 5 | 4,5 | 1,10 | Limpide |
| CY6 | 6 | 5,4 | 1,32 | Limpide |
| CY7 | 10 | 9 | 2,20 | Limpide |

### Exemple CW0: Préparation d'une solution de pramlintide à 0,4 mg/mL contenant du co-polyaminoacide BB15, du phénol (30 mM), de la glycérine (174 mM) et de la glycylglycine (8 mM) à pH 7,4.

Par un protocole similaire à celui utilisé à l'exemple CY0, à partir d'une solution de pramlintide à 0,4 mg/mL CW1, des solutions de pramlintide à 0,4 mg/mL contenant du co-polyaminoacide BB15, du phénol (30 mM), de la glycérine (174 mM) et de la glycylglycine (8 mM) à pH 7,4 CW2 et CW3 sont obtenues.

**Tableau 4 : Compositions et aspect visuel de solutions de pramlintide à 0,4 mg/ml à pH 7,4 à différentes concentrations en co-polyaminoacide BB15.**

| Solution | Concentration en co-polyaminoacide BB15 | | Ratio BB15/pramlintide | Aspect visuel de la solution |
|---|---|---|---|---|
| | mg/mL | mM | mol/mol | |
| CW2 | 2,4 | 0,59 | 6 | Limpide |
| CW3 | 4 | 0,98 | 10 | Limpide |

### Exemple CP0: Préparation d'une solution de pramlintide à 0,9 mg/mL contenant différents co-polyaminoacides de l'invention, du m-crésol (29 mM) et de la glycérine (174 mM) à pH 7,4.

Par un protocole similaire à celui décrit à l'exemple CY0, des solutions de pramlintide à 0,9 mg/mL contenant différents co-polyaminoacides de l'invention, du m-crésol (29 mM) et de la glycérine (174 mM) à pH 7,4 CP2 à CP12 sont obtenues.

**Tableau 8 : Compositions et aspect visuel de solutions de pramlintide à 0,9 mg/ml à pH 7,4 en présence de différents co-polyaminoacides.**

| Solutio n | Co-polya mino acide | Concentration en co-polyaminoacide | | Ratio co-polyaminoacide/pra mlintide | Aspect visuel de la solution |
|---|---|---|---|---|---|
| | | mg/m L | mM | mol/mol | |
| CP2 | BB15 | 5 | 1,22 | 5,4 | Limpide |
| | | 10 | 2,45 | 10,8 | Limpide |
| CP3 | BB14 | 5 | 0,98 | 4,3 | Limpide |
| | | 10 | 1,96 | 8,6 | Limpide |
| CP4 | AB17 | 5 | 1,11 | 4,9 | Limpide |
| | | 10 | 1,22 | 9,8 | Limpide |
| CP5 | AB15 | 5 | 0,99 | 4,4 | Limpide |
| | | 10 | 1,99 | 8,8 | Limpide |
| CP6 | AB14 | 5 | 1,48 | 6,5 | Limpide |
| | | 10 | 2,96 | 13 | Limpide |
| CP10 | BB18 | 5 | 0,72 | 3,2 | Limpide |
| | | 10 | 1,44 | 6,3 | Limpide |
| CP11 | BB9 | 5 | 1 | 4,4 | Limpide |
| | | 10 | 2,01 | 8,8 | Limpide |
| CP12 | BB2 | 5 | 1,27 | 5,6 | Limpide |
| | | 10 | 2,54 | 11,2 | Limpide |

### Exemple CH1: Préparation d'une solution de pramlintide à 0,6 mg/mL contenant du m-crésol (29 mM) et de la glycérine (174 mM) à pH 6,6.

Une solution de pramlintide concentrée à 5 mg/mL est préparée par dissolution de pramlintide sous forme de poudre achetée chez Ambiopharm. Cette solution est ajoutée à une solution concentrée d'excipients (m-crésol, glycérine) de manière à obtenir la composition finale visée. Le pH final est ajusté à 6,6 par ajout de NaOH/HCl.

### Exemple CH0: Préparation d'une solution de pramlintide à 0,6 mg/mL contenant du co-polyaminoacide BB15, du m-crésol (29 mM) et de la glycérine (174 mM) à pH 6,6.

Une solution concentrée de co-polyaminoacide BB15 et d'excipients est préparée en ajoutant des solutions concentrées d'excipients (m-crésol, glycérine) à une solution concentrée de co-polyaminoacide BB15.

Une solution de pramlintide concentrée à 5 mg/mL à pH 4 est ajoutée à cette solution concentrée de co-polyaminoacide BB15 et d'excipients de manière à obtenir les compositions finales CH2 à CH8 (tableau 9). Le pH final est ajusté à 6,6 par ajout de NaOH/HCl.

**Tableau 9 : Compositions et aspect visuel des solutions de pramlintide à pH 6,6 à différentes concentrations en co-polyaminoacide BB15.**

| Solution | Ratio BB15/pramlintide | Concentration en co-polyaminoacide BB15 | | Aspect visuel de la solution |
|---|---|---|---|---|
| | mol/mol | mg/mL | mM | |
| CH1 | - | - | - | Limpide |
| CH2 | 2 | 1,3 | 0,29 | Limpide |
| CH3 | 3 | 2 | 0,45 | Limpide |
| CH4 | 4 | 2,7 | 0,61 | Limpide |
| CH5 | 6 | 4 | 0,90 | Limpide |
| CH6 | 8 | 5,3 | 1,19 | Limpide |
| CH7 | 10 | 6,7 | 1,50 | Limpide |
| CH8 | 15 | 10 | 2,24 | Limpide |

### Exemple CI0: Préparation d'une solution de pramlintide à 0,6 mg/mL contenant différents co-polyaminoacides de l'invention, du m-crésol (29 mM) et de la glycérine (174 mM) à pH 6,6.

Par un protocole similaire à celui décril à l'exemple CH0, des solutions de pramlintide à 0,6 mg/mL contenant différents co-polyaminoacide de l'invention, du m-crésol (29 mM) et de la glycérine (174 mM) à pH 6,6, CI1 à CI14 sont obtenues.

**Tableau 10 : Compositions et aspect visuel des solutions de pramlintide à pH 6,6 en présence de différents co-polyaminoacides.**

| Solution | Co-polyaminoacide | Concentration en co-polyaminoacide | | Ratio co-polyaminoacide/pramlintide | Aspect visuel de la solution |
|---|---|---|---|---|---|
| | | mg/mL | mM | mol/mol | |
| CI1 | BB20 | 1,3 | 0,3 | 2 | Limpide |
| | | 2,6 | 0,6 | 4 | Limpide |
| CI2 | BB21 | 1,3 | 0,6 | 4 | Limpide |
| CI3 | AB22 | 2,4 | 0,3 | 2 | Limpide |
| CI4 | BB24 | 2,9 | 0,6 | 4 | Limpide |
| CI5 | BB25 | 1,5 | 0,3 | 2 | Limpide |
| | | 3 | 0,6 | 4 | Limpide |
| CI6 | AB23 | 3,4 | 0,23 | 2 | Limpide |
| CI7 | AB28 | 2,3 | 0,3 | 2 | Limpide |
| | | 4,7 | 0,6 | 4 | Limpide |
| CI8 | AB24 | 1,2 | 0,15 | 1 | Limpide |
| | | 2,4 | 0,3 | 2 | Limpide |
| CI9 | AB25 | 1,3 | 0,15 | 1 | Limpide |
| | | 2,6 | 0,3 | 2 | Limpide |
| CI10 | AB26 | 0,7 | 0,15 | 1 | Limpide |
| | | 1,5 | 0,3 | 2 | Limpide |
| CI11 | AB27 | 1,3 | 0,15 | 1 | Limpide |
| | | 2,7 | 0,3 | 2 | Limpide |
| CI12 | AB31 | 1,3 | 0,15 | 1 | Limpide |
| | | 2,5 | 0,3 | 2 | Limpide |
| CI13 | AB29 | 8,9 | 1,15 | 7,6 | Limpide |
| CI14 | AB32 | 1,3 | 0,15 | 1 | Limpide |
| | | 2,5 | 0,3 | 2 | Limpide |

### Exemple CT0: Préparation d'une solution de pramlintide à 0,6 mg/mL contenant du co-polyaminoacide AB14, du m-crésol (29 mM), de la glycérine (174 mM) du NaCl et du chlorure de zinc à pH 6,6

Une solution concentrée de co-polyaminoacide AB14 et d'excipients est préparée en ajoutant des solutions concentrées d'excipients (m-crésol, glycérine, NaCl, chlorure de zinc) à une solution concentrée de co-polyaminoacide AB14.

Une solution de pramlintide concentrée à 5 mg/mL à pH 4 est ajoutée à cette solution concentrée de co-polyaminoacide AB14 et d'excipients de manière à obtenir les compositions finales CT1 à CT5 (tableau 11). Le pH final est ajusté à 6,6 par ajout de NaOH/HCl.

**Tableau 11 : Compositions et aspect visuel des solutions de pramlintide à pH 6,6 en présence du co-polyamnioacide AB14 et de différentes teneurs en chlorure de sodium et en chlorure de zinc.**

| Solution | Co-polyaminoacide | Concentration en co-polyaminoacide | | [NaCl] | [ZnCl₂] | Aspect visuel de la solution |
|---|---|---|---|---|---|---|
| | | mg/mL | mM | (mM) | (mM) | |
| CT1 | AB14 | 6,3 | 1,87 | - | 0,75 | Limpide |
| CT2 | AB14 | 6,3 | 1,87 | 50 | - | Limpide |
| CT3 | AB14 | 6,3 | 1,87 | 100 | - | Limpide |
| CT4 | AB14 | 6,3 | 1,87 | 50 | 0,75 | Limpide |
| CT5 | AB14 | 6,3 | 1,87 | 100 | 0,75 | Limpide |

### Exemple CS0: Préparation d'une solution de pramlintide à 0,6 mg/mL contenant différents co-polyaminoacides de l'invention, du m-crésol (29 mM), de la glycérine (174 mM) du NaCl et du chlorure de zinc à pH 6,6

Par un protocole similaire à celui décrit à l'exemple CA4, des solutions de pramlintide à 0,6 mg/mL contenant différents co-polyaminoacides de l'invention, du m-crésol (29 mM) et de la glycérine (174 mM), du chlorure de sodium et du chlorure de zinc à pH 6,6 BS1 à BS11 sont obtenues.

**Tableau 12 : Compositions et aspect visuel des solutions de pramlintide à pH 6,6 en présence de diiférents co-polyaminoacides et de différentes teneurs en chlorure de sodium et en chlorure de zinc**

| Solution | Co-polyaminoacide | Concentration en co-polyaminoacide | | [NaCl] | [ZnCl₂] | Aspect visuel de la solution |
|---|---|---|---|---|---|---|
| | | mg/mL | mM | (mM) | (mM) | |
| CS1 | AB15 | 7,8 | 1,6 | - | - | Limpide |
| CS2 | AB15 | 11,7 | 2,3 | - | - | Limpide |
| CS3 | AB15 | 3,9 | 0,8 | 50 | - | Limpide |
| CS4 | AB15 | 6,3 | 1,3 | 50 | - | Limpide |
| CS5 | AB15 | 7,8 | 1,6 | 50 | - | Limpide |
| CS6 | AB15 | 3,9 | 0,8 | 100 | - | Limpide |
| CS7 | AB16 | 12,4 | 1,5 | - | - | Limpide |
| CS8 | AB16 | 16,7 | 2,1 | - | - | Limpide |
| CS9 | AB16 | 7,4 | 0,9 | 50 | | Limpide |
| CS10 | AB16 | 12,4 | 1,5 | 50 | | Limpide |
| CS11 | AB16 | 7,4 | 0,9 | 50 | 1 | Limpide |

### Exemple CX1 : Préparation d'une solution d'amyline humaine à 0,6 mg/mL et d'insuline humaine à 100 UI/mL contenant du m-crésol (29 mM), de la glycérine (174 mM) et du chlorure de zinc (229 µM) à pH 7,4.

La solution d'amyline humaine concentrée à 3 mg/mL CV1 est ajoutée à une solution concentrée d'excipients (m-crésol, glycérine). Une solution d'insuline humaine à 500 UI/mL est préparée par dissolution d'insuline humaine sous forme de poudre achetée chez Amphastar. Cette solution est ajoutée à la solution concentrée d'amyline humaine et d'excipients de manière à obtenir la composition finale visée. Le pH final est ajusté à 7,4 par ajout de NaOH/HCl.

### Exemple CX2 : Préparation d'une solution d'amyline humaine à 0,6 mg/mL et d'insuline humaine à 100 UI/mL contenant du co-polyaminoacide BB15, du m-crésol (29 mM), de la glycérine (174 mM) et du chlorure de zinc (229 µM) à pH 7,4.

Une solution concentrée de co-polyaminoacide BB15 et d'excipients est préparée en ajoutant des solutions concentrées d'excipients (m-crésol, glycérine, chlorure de zinc) à une solution concentrée de co-polyaminoacide BB15.

Une solution d'amyline humaine concentrée à 3 mg/mL puis une solution d'insuline humaine à 500 UI/mL sont ajoutées à la solution concentrée de co-polyaminoacide BB15 et d'excipients de manière à obtenir la composition finale visée (tableau 13). Le pH final est ajusté à 7,4 par ajout de NaOH/HCl.

Les solutions CX1, CX6, CX10 et CX11 sont préparées selon le protocole ci-dessus.

**Tableau 11 : Compositions et aspect visuel de solutions d'amyline humaine et d'insuline humaine à pH 7,4 à différentes concentrations en co-polyaminoacide BB15.**

| Solution | Ratio BB15/amyline humaine | Concentration en co-polyaminoacide BB15 | | Aspect visuel de la solution |
|---|---|---|---|---|
| | mol/mol | mg/Ml | mM | |
| CX1 | - | - | - | Turbide |
| CX6 | 6 | 3,6 | 0,88 | Limpide |
| CX10 | 10 | 6 | 1,47 | Limpide |
| CX11 | 17 | 10,5 | 2,57 | Limpide |

En présence du co-polyaminoacide BB15, une solution limpide d'amyline humaine (0,6 mg/mL) et d'insuline humaine (100 UI/mL) est obtenue à pH 7,4.

### Exemple CN1 : Préparation d'une solution de pramlintide à 0,4 mg/mL et d'insuline humaine à 100 UI/mL contenant du phénol (30 mM), de la glycérine (174 mM), de la glycylglycine (8 mM) et du chlorure de zinc (229 µM) à pH 7,4.

Une solution de pramlintide concentrée à 5 mg/mL est ajoutée à une solution concentrée d'excipients (m-crésol, glycérine, glycylglycine, chlorure de zinc). Une solution d'insuline humaine à 500 UI/mL est ajoutée à cette solution concentrée de pramlintide et d'excipients de manière à obtenir la composition finale visée. Le pH final est ajusté à 7,4 par ajout de NaOH/HCl.

### Exemple CR1 : Préparation d'une solution de pramlintide à 0,9 mg/mL et d'insuline humaine à 100 UI/mL contenant du m-crésol (29 mM), de la glycérine (174 mM) et du chlorure de zinc (229 µM) à pH 7,4.

Par un procédé similaire à celui utilisé à l'exemple CN1, une solution de pramlintide à 0,9 mg/mL et d'insuline humaine à 100 UI/mL contenant du m-crésol (29 mM), de la glycérine (174 mM) et du chlorure de zinc (229 **µM)** à pH 7,4 est obtenue.

### Exemple CN0: Préparation d'une solution de pramlintide à 0,4 mg/mL et d'insuline humaine à 100 UI/mL contenant du co-polyaminoacide BB15, du phénol (30 mM), de la glycérine (174 mM), de la glycylglycine (8 mM) et du chlorure de zinc (229 µM) à pH 7,4.

Une solution concentrée de co-polyaminoacide BB15 et d'excipients est préparée en ajoutant des solutions concentrées d'excipients (m-crésol, glycérine, glycylglycine, chlorure de zinc) à une solution concentrée de co-polyaminoacide BB15.

Une solution de pramlintide concentrée à 5 mg/mL puis une solution d'insuline humaine à 500 UI/mL sont ajoutées à cette solution concentrée de co-polyaminoacide BB15 et d'excipients de manière à obtenir la composition finale visée (tableau 14). Le pH final est ajusté à 7,4 par ajout de NaOH/HCl.

Les solutions CN2 et CN3 sont préparées selon le protocole ci-dessus.

**Tableau 14 : Compositions et aspect visuel des solutions de pramlintide à 0,4 mg/ml et d'insuline humaine à 100 UI/ml à pH 7,4 à différentes concentrations en co-polyaminoacide BB15.**

| Solution | Concentration en co-polyaminoacide BB15 | | Ratio BB15/pramlintide | Aspect visuel du mélange |
|---|---|---|---|---|
| | mg/mL | mM | mol/mol | |
| CN1 | - | | - | Turbide |
| CN2 | 2,4 | 0,59 | 6 | Limpide |
| CN3 | 4 | 0,98 | 10 | Limpide |

En présence du co-polyaminoacide BB15, une solution limpide de pramlintide (0,4 mg/mL) et d'insuline humaine (100 UI/mL) est obtenue à pH 7,4.

### Exemple CR0 : Préparation d'une solution de pramlintide à 0,9 mg/mL et d'insuline humaine à 100 UI/mL contenant du co-polyaminoacide BB15, du m-crésol (29 mM), de la glycérine (174 mM) et du chlorure de zinc (229 µM) à pH 7,4.

Par un procédé similaire à celui utilisé à l'exemple CN0, une solution de pramlintide à 0,9 mg/mL et d'insuline humaine à 100 UI/mL contenant du co-polyaminoacide BB15, du m-crésol (29 mM), de la glycérine (174 mM) et du chlorure de zinc (229 µM) à pH 7,4 est obtenue.

Les solutions CR2 à CR4 et CU2 à CU8 sont préparées selon le protocole ci-dessus.

**Tableau 15 : Compositions et aspect des solutions de pramlintide à 0,9 mg/ml et d'insuline humaine à 100 UI/ml à pH 7,4 à différentes concentrations en co-polyaminoacide BB15.**

| Solution | Concentration en co-polyaminoacide BB15 | | Ratio BB15/pramlintide mol/mol | Aspect visuel de la solution |
|---|---|---|---|---|
| | mg/mL | mM | | |
| CR1 | - | | - | Turbide |
| CR2 | 2,7 | 0,66 | 3 | Limpide |
| CR3 | 3,6 | 0,88 | 4 | Limpide |
| CR4 | 4,5 | 1,10 | 5 | Limpide |
| CU2 | 0,9 | 0,22 | 1 | Limpide |
| CU3 | 1,8 | 0,44 | 2 | Limpide |
| CU7 | 5,4 | 1,32 | 6 | Limpide |
| CU8 | 9 | 2,20 | 10 | Limpide |

En présence de co-polyaminoacide BB15, une solution limpide de pramlintide (0,9 mg/mL) et d'insuline humaine (100 UI/mL) à pH 7,4 est obtenue.

### Exemple CG0 : Préparation d'une solution de pramlintide à 0,9 mg/mL et d'insuline humaine à 100 UI/mL contenant différents co-polyaminoacides de l'invention, du m-crésol (29 mM), de la glycérine (174 mM) et du chlorure de zinc (229 µM) à pH 7,4.

Par un procédé similaire à l'exemple CN0, une solution de pramlintide à 0,9 mg/mL et d'insuline humaine à 100 UI/mL contenant un co-polyaminoacide de l'invention, du m-crésol (29 mM), de la glycérine (174 mM) et du chlorure de zinc (229 µM) à pH 7,4 est obtenue.

Les solutions CG2 à CG12 sont préparées selon le protocole décrit ci-dessus.

**Tableau 16 : Compositions et aspect visuel de solutions de pramlintide à 0,9 mg/ml et d'insuline humaine à 100 UI/ml à pH 7,4 à différentes concentrations en co-polyaminoacides.**

| Solution | Co-polyaminoacide | Concentration en co-polyaminoacide | | Ratio Co-polyaminoacide/pramlintide | Aspect visuel de la solution |
|---|---|---|---|---|---|
| | | mg/mL | mM | mol/mol | |
| CG2 | BB15 | 5 | 1,22 | 5,4 | Limpide |
| | | 10 | 2,45 | 10,8 | Limpide |
| CG3 | BB14 | 5 | 0,98 | 4,3 | - |
| | | 10 | 1,96 | 8,6 | Limpide |
| CG4 | AB17 | 5 | 1,11 | 4,9 | Limpide |
| | | 10 | 1,22 | 9,8 | Limpide |
| CG5 | AB15 | 5 | 0,99 | 4,4 | - |
| | | 10 | 1,99 | 8,8 | Limpide |
| CG6 | AB14 | 5 | 1,48 | 6,5 | - |
| | | 10 | 2,96 | 13 | Limpide |
| CG10 | BB18 | 5 | 0,72 | 3,2 | Limpide |
| | | 10 | 1,44 | 6,3 | Limpide |
| CG11 | BB9 | 5 | 1 | 4,4 | Limpide |
| | | 10 | 2,01 | 8,8 | Limpide |
| CG12 | BB2 | 5 | 1,27 | 5,6 | Limpide |
| | | 10 | 2,54 | 11,2 | Limpide |

### Exemple CD1 : Préparation d'une solution de pramlintide à 0,9 mg/mL et d'insuline lispro à 100 UI/mL contenant du m-crésol (29 mM), de la glycérine (174 mM) et du chlorure de zinc (300 µM) à pH 7,4.

Une solution de pramlintide concentrée à 5 mg/mL est ajoutée à une solution concentrée d'excipients (m-crésol, glycérine, chlorure de zinc). Une solution d'insuline lispro à 500 UI/mL est ajoutée à cette solution concentrée de pramlintide et d'excipients de manière à obtenir la composition finale visée. Le pH final est ajusté à 7,4 par ajout de NaOH/HCl.

### Exemple CD0 : Préparation d'une solution de pramlintide à 0,9 mg/mL et d'insuline lispro à 100 UI/mL contenant du co-polyaminoacide BB15, du m-crésol (29 mM), de la glycérine (174 mM) et du chlorure de zinc (300 µM) à pH 7,4.

Une solution concentrée de co-polyaminoacide BB15 et d'excipients est préparée en ajoutant des solutions concentrées d'excipients (m-crésol, glycérine, chlorure de zinc) à une solution concentrée de co-polyaminoacide BB15.

Une solution concentrée de pramlintide à 5 mg/mL puis une solution d'insuline lispro à 500 UI/mL sont ajoutées à la solution concentrée de co-polyaminoacide BB15 et d'excipients de manière à obtenir la composition finale visée. Le pH final est ajusté à pH 7,4 par ajout de NaOH/HCl.

Les solutions CD3 à CD9 sont préparées selon le protocole décrit ci-dessus.

**Tableau 17 : Compositions et aspect visuel des solutions de pramlintide à 0,9 mg/ml et d'insuline lispro à 100 UI/ml à pH 7,4 à différentes concentrations en co-polyaminoacide BB15.**

| Solution | Ratio BB15/pramlintide | Concentration en co-polyaminoacide BB15 | | Aspect visuel de la solution |
|---|---|---|---|---|
| | mol/mol | mg/ml | mM | |
| CD1 | - | - | - | Turbide |
| CD3 | 2 | 1,8 | 0,44 | Limpide |
| CD4 | 3 | 2,7 | 0,66 | Limpide |
| CD5 | 4 | 3,6 | 0,88 | Limpide |
| CD6 | 5 | 4,5 | 1.10 | Limpide |
| CD7 | 6 | 5,4 | 1,32 | Limpide |
| CD8 | 10 | 9 | 2,20 | Limpide |
| CD9 | 15 | 13,5 | 3,30 | Limpide |

En présence du co-polyaminoacide BB15, une solution limpide de pramlintide (0,9 mg/mL) et d'insuline lispro (100 UI/mL) à pH 7,4 est obtenue.

### Exemple CK1 : Préparation d'une solution de pramlintide à 0,6 mg/mL et d'insuline humaine à 100 UI/mL contenant du m-crésol (29 mM), de la glycérine (174 mM) et du chlorure de zinc (229 µM) à pH 6,6.

Par un procédé similaire à celui utilisé à l'exemple BR1, une solution de pramlintide à 0,6 mg/mL et d'insuline humaine à 100 UI/mL contenant du m-crésol (29 mM), de la glycérine (174 mM) et du chlorure de zinc (229 µM) à pH 6,6 est obtenue.

### Exemple CK0 : Préparation d'une solution de pramlintide à 0,6 mg/mL et d'insuline humaine à 100 UI/mL contenant du co-polyaminoacide BB15, du m-crésol (29 mM), de la glycérine (174 mM) et du chlorure de zinc (229 µM) à pH 6,6.

Par un procédé similaire à celui utilisé à l'exemple BR0, une solution de pramlintide à 0,6 mg/mL et d'insuline humaine à 100 UI/mL contenant du co-polyaminoacide BB15, du m-crésol (29 mM), de la glycérine (174 mM) et du chlorure de zinc (229 µM) à pH 6,6 est obtenue.

Les solutions CK2 à CK8 sont préparées selon le protocole ci-dessus.

**Tableau 18 : Compositions et aspect visuel des solutions de pramlintide à 0,6 mg/mL et d'insuline humaine à 100 UI/mL à pH 6,6 à différentes concentrations en co-polyaminoacide BB15.**

| Solution | Ratio BB15/pramlintide | Concentration en co-polyaminoacide BB15 | | Aspect visuel de la solution |
|---|---|---|---|---|
| | mol/mol | mg/mL | mM | |
| CK1 | - | - | - | Turbide |
| CK2 | 2 | 1,3 | 0,29 | Limpide |
| CK3 | 3 | 2 | 0,45 | Limpide |
| CK4 | 4 | 2,7 | 0,61 | Limpide |
| CK5 | 6 | 4 | 0,90 | Limpide |
| CK6 | 8 | 5,3 | 1,19 | Limpide |
| CK7 | 10 | 6,7 | 1,50 | Limpide |
| CK8 | 15 | 10 | 2,24 | Limpide |

En présence de co-polyaminoacide BB15, une solution limpide de pramlintide (0,6 mg/mL) et d'insuline humaine (100 UI/mL) à pH 6,6 est obtenue.

### Exemple CF1 : Préparation de compositions contenant des concentrations variables de pramlintide, d'insuline humaine à 100 UI/mL, du m-crésol (29 mM), de la glycérine (174 mM) et du chlorure de zinc (229 µM) à pH 6,6.

Par un procédé similaire à celui utilisé à l'exemple CR1, des solutions contenant différentes concentrations de pramlintide, de l'insuline humaine à 100 UI/mL, du m-crésol (29 mM), de la glycérine (174 mM) et du chlorure de zinc (229 µM) à pH 6,6 sont obtenues.

**Tableau 18a : Compositions et aspect visuel des solutions de pramlintide à différentes concentrations et d'insuline humaine à 100 UI/mL à pH 6,6.**

| Solution | Concentration en pramlintide (mg/mL) | Aspect visuel de la solution |
|---|---|---|
| CF1A | 0,9 | turbide |
| CF1B | 0,8 | turbide |
| CF1C | 0,6 | turbide |
| CF1D | 0,3 | turbide |
| CF1E | 0,2 | turbide |

### Exemple CF0: Préparation de compositions contenant des concentrations variables de pramlintide et d'insuline humaine à 100 UI/mL en présence de co-polyaminoacide AB24, de m-crésol (29 mM), de glycérine (174 mM) et de chlorure de zinc (229 µM) à pH 6,6.

Par un procédé similaire à celui utilisé à l'exemple CR0, des solutions contenant différentes concentrations de pramlintide, de l'insuline humaine à 100 UI/mL, du co-polyaminoacide AB24, du m-crésol (29 mM), de la glycérine (174 mM) et du chlorure de zinc (229 µM) à pH 6,6 sont obtenues.

**Tableau 18b : Compositions et aspect visuel des solutions de pramlintide à différentes concentrations et d'insuline humaine à 100 UI/mL en présence de co-polyaminoacide AB24 à pH 6,6.**

| Solution | Concentration en pramlintide | Concentration en co-polyaminoacide AB24 | | Ratio co-polyaminoacide/ pramlintide | Aspect visuel de la solution |
|---|---|---|---|---|---|
| | (mg/mL) | mg/mL | mM | mol/mol | |
| CF2 | 0,9 | 5,4 | 0,67 | 3 | limpide |
| CF3 | 0,8 | 4,8 | 0,6 | 3 | limpide |
| CF4 | 0,6 | 3,6 | 0,45 | 3 | limpide |
| CF5 | 0,3 | 1,8 | 0,22 | 3 | limpide |
| CF6 | 0,2 | 1 | 0,125 | 2,5 | limpide |

### Exemple CM0: Préparation d'une solution de pramlintide à 0,6 mg/mL et d'insuline humaine à 100 UI/mL contenant différents co-polyaminoacides de l'invention, du m-crésol (29 mM), de la glycérine (174 mM) et du chlorure de zinc (229 µM) à pH 6,6.

Par un procédé similaire à l'exemple CG0, des solutions de pramlintide à 0,6 mg/mL et d'insuline humaine à 100 UI/mL contenant différents co-polyaminoacide de l'invention, du m-crésol (29 mM), de la glycérine (174 mM) et du chlorure de zinc (229 µM) à pH 6,6 sont obtenues.

Les solutions CM1 à CM18 sont préparées selon le protocole décrit ci-dessus.

**Tableau 19 : Compositions et aspect visuel des solutions de pramlintide à 0,6 mg/mL et d'insuline humaine à 100 UI/mL à pH 6,6 en présence de différents co-polyaminoacides.**

| Solution | Co-polyaminoacide | Concentration en co-polyaminoacide | | Ratio co-polyaminoacide/pramlintide | Aspect visuel de la solution |
|---|---|---|---|---|---|
| | | mg/Ml | mM | mol/mol | |
| CM1 | BB20 | 2,6 | 0,61 | 4 | Limpide |
| | | 5,3 | 1,22 | 8 | Limpide |
| CM2 | BB21 | 1,3 | 0,6 | 4 | Limpide |
| CM3 | AB22 | 2,4 | 0,3 | 2 | Limpide |
| CM4 | BB24 | 2,9 | 0,6 | 4 | Limpide |
| CM5 | BB23 | 3 | 0,76 | 5 | Limpide |
| CM6 | BB25 | 1,5 | 0,3 | 2 | Limpide |
| CM7 | BB22 | 2,7 | 0,6 | 4 | Limpide |
| CM8 | AB23 | 7,7 | 0,69 | 4,6 | Limpide |
| CM9 | BB19 | 4,7 | 0,4 | 2,8 | Limpide |
| CM10 | AB28 | 2,3 | 0,3 | 2 | Limpide |
| CM11 | AB24 | 1,2 | 0,15 | 1 | Limpide |
| | | 2,4 | 0,3 | 2 | Limpide |
| | | 3,6 | 0,45 | 3 | |
| CM12 | AB25 | 2,6 | 0,3 | 2 | Limpide |
| CM13 | AB26 | 1,5 | 0,3 | 2 | Limpide |
| | | 2,3 | 0,5 | 3 | Limpide |
| CM14 | AB27 | 1,3 | 0,15 | 1 | Limpide |
| | | 2,7 | 0,3 | 2 | Limpide |
| CM15 | AB30 | 1,2 | 0,15 | 1 | Limpide |
| | | 2,3 | 0,3 | 2 | Limpide |
| CM16 | AB31 | 1,3 | 0,15 | 1 | Limpide |
| | | 2,5 | 0,3 | 2 | Limpide |
| CM17 | AB29 | 5,9 | 0,8 | 5 | Limpide |
| | | 8.9 | 1,15 | 7,6 | Limpide |
| CM18 | AB32 | 2,5 | 0,3 | 2 | Limpide |

### Exemple CQ1 : Préparation d'une solution de pramlintide à 0,6 mg/mL et d'insuline humaine à 100 IU/mL à pH 6,6 contenant du co-polyaminoacide AB14, du m-crésol (29 mM), de la glycérine (174 mM), du chlorure de sodium (100 mM) et du chlorure de zinc (1 mM)

Une solution concentrée de co-polyaminoacide AB14 et d'excipients est préparée en ajoutant des solutions concentrées d'excipients (m-crésol, glycérine, chlorure de sodium, chlorure de zinc) à une solution concentrée de co-polyaminoacide AB14.

Une solution de pramlintide concentrée à 5 mg/mL à pH 4 puis une solution d'insuline humaine à 500 IU/mL sont ajoutées à cette solution concentrée de co-polyaminoacide AB14 et d'excipients de manière à obtenir la composition finale visée. Le pH final est ajusté à 6,6 par ajout de NaOH/HCl.

La solution CQ1 est préparée selon le protocole ci-dessus.

### Exemple CQ0: Préparation d'une solution de pramlintide à 0,6 mg/mL et d'insuline humaine à 100 IU/mL contenant différents co-polyaminoacides de l'invention, du m-crésol (29 mM), de la glycérine (174 mM), et différentes teneurs en chlorure de sodium et en chlorure de zinc

Par un procédé similaire à l'exemple CQ0, des solutions de pramlintide à 0,6 mg/mL et d'insuline humaine à 100 IU/mL contenant différents co-polyaminoacide de l'invention, du m-crésol (29 mM), de la glycérine (174 mM), du chlorure de sodium et du chlorure de zinc à pH 6,6 sont obtenues.

Les solutions CQ2 à CQ12 sont préparées selon le protocole ci-dessus.

**Tableau 20 : Compositions et aspect visuel des solutions de pramlintide à 0,6 mg/mL et d'insuline humaine à 100 IU/mL pH 6,6 en présence de différents co-polyaminoacides et de différentes teneurs en chlorure de sodium et en chlorure de zinc.**

| Solution | Co-polyaminoacide | Concentration en co-polyaminoacide | | [NaCl] | [ZnCl₂] | Aspect visuel de la solution |
|---|---|---|---|---|---|---|
| | | mg/mL | mM | (mM) | (mM) | |
| CQ1 | AB14 | 6,3 | 1,87 | 100 | 1 | Limpide |
| CQ2 | AB15 | 7,8 | 1,6 | - | 0,23 | Limpide |
| CQ3 | AB15 | 11,7 | 2,3 | - | 0,23 | Limpide |
| CQ4 | AB15 | 3,9 | 0,8 | 50 | 0,23 | Limpide |
| CQ5 | AB15 | 6,3 | 1,3 | 50 | 0,23 | Limpide |
| CQ6 | AB15 | 7,8 | 1,6 | 50 | 0,23 | Limpide |
| CQ7 | AB15 | 3,9 | 0,8 | 100 | 0,23 | Limpide |
| CQ8 | AB15 | 6,3 | 1,3 | 100 | 0,23 | Limpide |
| CQ9 | AB15 | 7,8 | 1,6 | 100 | 0,23 | Limpide |
| CQ10 | AB16 | 7,4 | 0,9 | 50 | 0,23 | Limpide |
| CQ11 | AB16 | 12,4 | 1,5 | 50 | 0,23 | Limpide |
| CQ12 | AB16 | 7,4 | 0,9 | 50 | 1 | Limpide |

### Exemple CZ0: Préparation d'une solution de pramlintide à 0,6 mg/mL contenant du DMPG, du m-crésol (29 mM), de la glycérine (174 mM) à pH 6,6.

Une solution concentrée de DMPG et d'excipients est préparée en ajoutant des solutions concentrées d'excipients (m-crésol, glycérine) à une solution concentrée de DMPG.

Une solution de pramlintide concentrée à 10 mg/mL est ajoutée à cette solution concentrée de DMPG et d'excipients de manière à obtenir la composition finale visée. Le pH final est ajusté à 6,6 par ajout de NaOH/HCl.

### Exemple CZ1 : Préparation d'une solution de pramlintide à 0,6 mg/mL contenant du DMPG (4,5 mM), du phénol (30 mM), de la glycérine (174 mM) et de la glycylglycine (8 mM) à pH 7,4.

**Tableau 21 : Compositions et aspect visuel des solutions de pramlintide à 0,6 mg/mL en présence de DMPG.**

| Solution | DMPG (mM) | Concentration pramlintide (mg/mL) | pH | Excipients | Aspect visuel de la solution |
|---|---|---|---|---|---|
| CZ0 | 4,5 | 0,6 | 6,6 | m-crésol 29 mM | Limpide |
| | | | | Glycérine 174 mM | |
| CZ1 | 4,5 | 0,6 | 7,4 | phénol 30 mM | Limpide |
| | | | | Glycylglycine 8mM | |
| | | | | Glycérine 174 mM | |

### Exemple CA1 : Préparation d'une solution de pramlintide à 1 mg/mL contenant du m-crésol (20 mM), du mannitol (43 mg/mL), et un tampon acétate de sodium, à pH 4.

Une solution concentrée de pramlintide à 10 mg/mL est ajoutée à une solution concentrée d'excipients (m-crésol, mannitol, acétate de sodium) de manière à obtenir la composition finale visée. Le pH final est ajusté à 4 par ajout de NaOH/HCl.La solution limpide est filtrée (0,22 µm) et introduite dans des cartouches en verre de 3 mL pour stylo injecteur.

### Exemple CA2 : Mise en cartouches d'une solution commerciale d'insuline humaine à 100 UI/mL (Humulin^{®}) contenant du m-crésol (23 mM), de la glycérine (174 mM) et du chlorure de zinc (230 µM).

Une solution commerciale d'Humulin^{®} en vial de 10 mL est prélevée et introduite dans des cartouches en verre de 3 mL pour stylo injecteur.

### Exemple CA3 : Préparation d'une solution de pramlintide à 0,6 mg/mL et d'insuline humaine à 100 UI/mL contenant du co-polyaminoacide BB15 (3,1 mg/mL), du m-crésol (29 mM), de la glycérine (35 mM), du mannitol (2,6 % % w/v) , du tris (18,75 mM), de l'acétate de sodium (18 mM), et du chlorure de zinc (260 µM) à pH 6,2.

Des vials en verre de 3 mL sont remplis avec 0,5 mL d'une solution contenant 10 mg/mL de co-polyaminoacide BB15 et 60 mM de tris à pH 8,3. La solution est lyophilisée de manière à obtenir des vials de 3 mL contenant 5 mg de co-polyaminoacide BB15 et 30 µmol de tris.

Une solution d'insuline humaine à 500 UI/mL contenant 23 mM de m-cresol, 174 mM de glycérine et 260 µM de chlorure de zinc à pH 7,4 est préparée en ajoutant des solutions concentrées d'excipients (m-crésol, glycérine, chlorure de zinc) à une solution concentrée d'insuline humaine concentrée à 760 UI/mL.

Dans le vial contenant 5 mg de co-polyaminoacide BB15 et 30 µmol de tris sont introduits succesivement :
- 0,96 mL de la solution de Pramlintide à 1 mg/mL à pH 4 décrite à l'exemple CA1 ;
- 0,32 mL d'eau stérile pour injection ;
- 0,32 mL d'insuline humaine concentrée à 500 UI/mL contenant 23 mM de m-cresol, 174 mM de glycérine et 260 µM de chlorure de zinc à pH 7,4.

La solution limpide est filtrée (0,22 µm) et introduite dans des cartouches en verre de 3 mL pour stylo injecteur.

### Exemple CA4 : Mise en cartouches d'une solution de pramlintide à 0,6 mg/mL et d'insuline humaine à 100 UI/mL en présence de co-polyaminoacide AB24 à 2,4 mg/mL, de m-crésol (29 mM), de glycérine (174 mM) et de chlorure de zinc (229 µM) à pH 6,6.

La solution de pramlintide à 0,6 mg/mL et d'insuline humaine à 100 UI/mL en présence de co-polyaminoacide AB24 à 2,4 mg/mL, de m-crésol (29 mM), de glycérine (174 mM) et de chlorure de zinc (229 µM) à pH 6,6 décrite à l'exemple CF4 est filtrée (0,22 µM) est introduite dans des cartouches en verre de 3 mL pour stylo injecteur.

### Exemple CA5 (stabilité en pompe) : Préparation d'une solution de pramlintide à 0,6 mg/mL et d'insuline lispro à 100 UI/mL à pH 6,6 contenant du co-polyaminoacide AB24, du m-crésol (29 mM), de la glycérine (174 mM) et du chlorure de zinc (300 µM).

Une solution concentrée de co-polyaminoacide AB24 et d'excipients est préparée en ajoutant des solutions concentrées d'excipients (m-crésol, glycérine, chlorure de zinc) à une solution concentrée de co-polyaminoacide AB24.

Une solution de pramlintide concentrée à 10 mg/mL puis une solution d'insuline lispro à 200 UI/mL sont ajoutées à cette solution concentrée de co-polyaminoacide AB24 et d'excipients de manière à obtenir la composition finale visée. Le pH final est ajusté à 6,6 par ajout de NaOH/HCI.

### Exemple CA6 : Préparation d'une solution d'insuline humaine à 100 IU/mL contenant du co-polyaminoacide BB15 (10 mg/mL), du m-crésol (29 mM), de la glycérine (174 mM) et du chlorure de zinc (229 µM) à pH 6,6

Une solution concentrée de co-polyaminoacide BB15 et d'excipients est préparée en ajoutant des solutions concentrées d'excipients (m-crésol, glycérine, chlorure de zinc) à une solution concentrée de co-polyaminoacide BB15.

Une solution d'insuline humaine à 800 IU/mL est ajoutée à cette solution concentrée de co-polyaminoacide BB15 et d'excipients de manière à obtenir la composition finale visée. Le pH final est ajusté à 6,6 par ajout de NaOH/HCI. La solution limpide est filtrée (0,22 µM) et introduite dans des cartouches en verre de 3 mL pour stylo injecteur.

### Exemple CA7 : Préparation d'une solution de pramlintide à 0,6 mg/mL et d'insuline humaine à 100 IU/mL contenant du co-polyaminoacide BB15 (10 mg/mL), du m-crésol (29 mM), de la glycérine (174 mM) et du chlorure de zinc (229 µM) à pH 6,6

Une solution concentrée de co-polyaminoacide BB15 et d'excipients est préparée en ajoutant des solutions concentrées d'excipients (m-crésol, glycérine, chlorure de zinc) à une solution concentrée de co-polyaminoacide BB15.

Une solution concentrée de pramlintide à 10 mg/mL puis une solution concentrée d'insuline humaine à 500 IU/mL sont ajoutées à cette solution concentrée de co-polyaminoacide BB15 et d'excipients de manière à obtenir la composition finale visée. Le pH final est ajusté à 6,6 par ajout de NaOH/HCl. La solution limpide est filtrée (0,22 µM) et introduite dans des cartouches en verre de 3 mL

### D. PHYSICO-CHIMIE

### D I : Résultats des observations visuelles au mélange et des mesures de fibrillation par ThT

### Principe

La mauvaise stabilité d'un peptide peut conduire à la formation de fibrilles amyloïdes, définies comme des structures macromoléculaires ordonnées. Celles-ci peuvent éventuellement résulter à la formation de gel au sein de l'échantillon.

L'essai de suivi de la fluorescence de la Thioflavine T (ThT) est utilisé pour analyser la stabilité physique des solutions. La Thioflavine est une petite molécule sonde ayant une signature de fluorescence caractéristique lorsque se lie à des fibrilles de type amyloïdes (Naiki et al. (1989) Anal. BioChem. 177, 244-249 ; LeVine (1999) Methods. Enzymol. 309, 274-284).

Cette méthode permet de suivre la formation de fibrilles pour de faibles concentrations de ThT au sein de solutions non diluées. Ce suivi est réalisé dans des conditions de stabilité accélérées : sous agitation et à 37 °C.

### Conditions expérimentales

Les échantillons ont été préparés juste avant le début de la mesure. La préparation de chaque composition est décrite dans l'exemple associé. La Thioflavine T a été ajoutée dans la composition à partir d'une solution mère concentrée de manière à induire une dilution négligeable de la composition. La concentration de Thioflavine T dans la composition est de 1, 2 ou 40 µM selon le type de composition : 40 µM dans le cas des compositions d'amyline humaine à 0,6 mg/mL, 2 µM dans le cas des compositions de pramlintide à 0,9 mg/mL et 1 µM dans les compositions de pramlintide à 0,4 mg/mL. Cette concentration est rappelée dans la légende relative au tableau de résultats des temps de latence pour chaque type de composition.

Un volume de 150 µL de la composition a été introduit au sein d'un puit d'une plaque 96 puits. Chaque composition a été analysée en trois essais (triplicat) au sein d'une même plaque. La plaque a été scellée par du film transparent afin d'éviter l'évaporation de la composition.

Cette plaque a ensuite été placée dans l'enceinte d'un lecteur de plaques (EnVision 2104 Multilabel, Perkin Elmer). La température est réglée à 37 °C, et une agitation latérale de 960 rpm avec 1 mm d'amplitude est imposée.

Une lecture de l'intensité de fluorescence dans chaque puit est réalisée avec une longueur d'onde d'excitation de 442 nm, et une longueur d'onde d'émission de 482 nm au cours du temps.

Le processus de fibrillation se manifeste par une forte augmentation de la fluorescence après un délai appelé temps de latence.

Pour chaque puit, ce délai a été déterminé graphiquement comme l'intersection entre la ligne de base du signal de fluorescence et la pente de la courbe de fluorescence en fonction du temps déterminée pendant la forte augmentation initiale de fluorescence. La valeur de temps de latence reportée correspond à la moyenne des mesures de temps de latence faites sur trois puits.

Un exemple de détermination graphique est représenté à la Figure 1.

Sur cette figure est représentée de façon graphique la détermination du temps de latence (LT) par suivi de la fluorescence de la Thioflavine T, sur une courbe ayant en ordonnées la valeur de la fluorescence (en u.a. unités arbitraires) et en abscisses le temps en minutes.

### Exemple D1 : Stabilité de solutions d'amyline humaine à 0,6 mg/mL à pH 7,4 en présence de co-polyaminoacide BB15 à différentes concentrations.

**Tableau 22 : Mesure du temps de latence par ThT (40 µM) des solutions CV1 à CV10.**

| Solution | Ratio BB15/amyline humaine | Concentration en co-polyaminoacide BB15 | | Temps de latence (h) |
|---|---|---|---|---|
| | mol/mol | mg/ ml | mM | |
| CV1 | - | - | - | < 0,02 |
| CV5 | 5 | 3 | 0,73 | > 1 |
| CV6 | 6 | 3,6 | 0,88 | > 5 |
| CV7 | 7 | 4,2 | 1,03 | > 30 |
| CV8 | 8 | 4,8 | 1,17 | > 54 |
| CV9 | 9 | 5,4 | 1,32 | > 54 |
| CV10 | 10 | 6 | 1,47 | > 72 |

Le temps de latence d'une solution d'amyline humaine à pH 7,4 (CV1), dépourvue de co-polyaminoacide, est inférieur à 0,02 h ; les solutions CV5 à CV10 selon l'invention, contenant des ratios molaires BB15 / amyline humaine supérieurs à 5 permettent d'obtenir des temps de latence supérieurs à une heure, un ratio molaire de 10 permettant d'obtenir des temps de latence supérieurs à 72 h.

### Exemple D2 : Stabilité de solutions d'amyline humaine à 0,6 mg/mL et d'insuline humaine 100 UI à pH 7,4 en présence de co-polyaminoacide BB15 à différentes concentrations.

**Tableau 23 : Mesure du temps de latence par ThT (40 µM) des solutions, CX6, CX10 et CX11.**

| Solution | Ratio BB15/amyline humaine | Concentration en co-polyaminoacide BB15 | | Temps de latence (h) |
|---|---|---|---|---|
| | mol/mol | mg/mL | mM | |
| CX1 | - | - | - | * |
| CX6 | 6 | 3,6 | 0,88 | > 0,1 |
| CX10 | 10 | 6 | 1,47 | > 0,5 |
| CX11 | 17,5 | 10,5 | 2,57 | > 5 |

| | | | | |
|---|---|---|---|---|
| ^{∗}Temps de latence non mesuré car solution turbide | | | | |

La solution d'amyline humaine et d'insuline humaine à pH 7,4 (CX1) est turbide. Le co-polyaminoacide BB15 permet d'obtenir une solution limpide contenant de l'amyline humaine en présence d'insuline humaine à pH 7,4 avec des temps de latence supérieurs à 0,1 heure à partir d'un ratio molaire BB15 / amyline humaine de 6, et supérieurs à 5 h pour un ratio molaire BB15 / amyline humaine de 17,5.

### Exemple D3 : Stabilité de solutions de pramlintide à 0,4 mg/ml à pH 7,4 en présence de co-polyaminoacide BB15 à différentes concentrations.

**Tableau 24 : Mesure du temps de latence par ThT (1 µM) des solutions CW1 à CW3.**

| Solution | Concentration en co-polyaminoacide BB15 | | Ratio BB15/pramlintide | Temps de latence (h) |
|---|---|---|---|---|
| | mg/mL | mM | mol/mol | |
| CW1 | - | | - | 0,7 |
| CW2 | 2,4 | 0,59 | 6 | > 40 |
| CW3 | 4 | 0,98 | 10 | > 63 |

La solution de pramlintide à pH 7,4 (CW1) dépourvu de co-polyaminoacide a un temps de latence court. Le co-polyaminoacide BB15 permet d'obtenir une solution contenant du pramlintide à pH 7,4 avec des temps de latence supérieurs à 40 h à partir d'un ratio molaire BB15 / pramlintide de 6.

### Exemple D4 : Stabilité de solutions de pramlintide à 0,9 mg/mL à pH 7,4 en présence de co-polyaminoacide BB15 à différentes concentrations.

**Tableau 25 : Mesure du temps de latence par ThT (2 µM) des solutions CY1 à CY7**

| Solution | Ratio BB15/pramlintide | Concentration en BB15 | | Temps de latence (h) |
|---|---|---|---|---|
| | mol/mol | mg/ml | mM | |
| CY1 | - | - | - | 0,7 |
| CY2 | 2 | 1,8 | 0,44 | > 0,8 |
| CY3 | 3 | 2,7 | 0,66 | > 4 |
| CY4 | 4 | 3,6 | 0,88 | > 30 |
| CY5 | 5 | 4,5 | 1,10 | >63 |
| CY6 | 6 | 5,4 | 1,22 | >63 |
| CY7 | 10 | 9 | 1,32 | >63 |

La solution de pramlintide à pH 7,4 (CY1) dépourvue de co-polyaminoacide présente un temps de latence court ; les temps de latence des solutions contenant un co-polyaminoacide sont supérieurs ou égaux aux temps de latence de la composition sans co-polyaminoacide à un ratio molaire de co-polyaminoacide BB15 / pramlintide de 2:1.

### Exemple D5 : Stabilité de solutions de pramlintide à 0,9 mg/ml à pH 7,4 en présence de différents co-polyaminoacides.

**Tableau 26 : Mesure du temps de latence par ThT (2 µM) des compositions CY1, CP2 à CP12.**

| Soluti on | Co-polya mino acide | Concentration en co-polyaminoacide | | Ratio co-polyaminoacide/praml intide | Temps de latence (h) |
|---|---|---|---|---|---|
| | | mg/m L | mM | mol/mol | |
| CY1 | - | - | | - | < 0,7 |
| CP2 | BB15 | 5 | 1,22 | 5,4 | > 63 |
| | | 10 | 2,45 | 10,8 | > 63 |
| CP3 | BB14 | 10 | 1,96 | 8,6 | > 15 |
| CP4 | AB17 | 10 | 1,22 | 9,8 | > 63 |
| CP10 | BB18 | 5 | 0,72 | 3,2 | > 63 |
| | | 10 | 1,44 | 6,3 | > 63 |
| CP11 | BB9 | 5 | 1 | 4,4 | > 50 |
| | | 10 | 2,01 | 8,8 | > 63 |
| CP12 | BB2 | 5 | 1,27 | 5,6 | > 10 |
| | | 10 | 2,54 | 11,2 | > 63 |

La solution de pramlintide à pH 7,4 (CY1) dépourvue de co-polyaminoacide présente un temps de latence court. Les co-polyaminoacides de l'invention permettent d'obtenir des temps de latence supérieurs à 10 h dans les conditions testées.

### Exemple D6 : Stabilité de solutions de pramlintide à 0,6 mg/mL à pH 6,6 en présence de co-polyaminoacide BB15 à différentes concentrations.

**Tableau 27 : Mesure du temps de latence par ThT (2 µM) des solutions CH1 et CH2 à CH8.**

| Solution | Ratio BB15/pramlintide | Concentration en co-polyaminoacide BB15 | | Temps de latence (h) |
|---|---|---|---|---|
| | mol/mol | mg/mL | mM | |
| CH1 | - | - | - | 1 |
| CH2 | 2 | 1,3 | 0,29 | >4 |
| CH3 | 3 | 2 | 0,45 | >10 |
| CH4 | 4 | 2,7 | 0,61 | >50 |
| CH5 | 6 | 4 | 0,90 | >50 |
| CH6 | 8 | 5,3 | 1,19 | >50 |
| CH7 | 10 | 6,7 | 1,50 | >50 |
| CH8 | 15 | 10 | 2,24 | >50 |

La solution de pramlintide à pH 6,6 (CH1) dépourvue de co-polyaminoacide présente un temps de latence court ; les temps de latence des solutions contenant un co-polyaminoacide sont supérieurs aux temps de latence de la composition sans co-polyaminoacide à un ratio molaire de co-polyaminoacide BB15/pramlintide de 2 :1.

### Exemple D7 : Stabilité de solutions de pramlintide à 0,6 mg/ml à pH 6,6 en présence de différents co-polyaminoacides.

**Tableau 28 : Mesure du temps de latence par ThT (2 µM) des compositions CI1 à CI14.**

| Solution | Co-polyaminoacide | Concentration en co-polyaminoacide | | Ratio co-polyaminoacide/pramlintide | Temps de latence (h) |
|---|---|---|---|---|---|
| | | mg/mL | mM | mol/mol | |
| CI1 | BB20 | 1,3 | 0,3 | 2 | >10 |
| | | 2,6 | 0,6 | 4 | >20 |
| CI2 | BB21 | 1,3 | 0,6 | 4 | >10 |
| CI3 | AB22 | 2,4 | 0,3 | 2 | >10 |
| CI4 | BB24 | 2,9 | 0,6 | 4 | >10 |
| CI5 | BB25 | 1,5 | 0,3 | 2 | >50 |
| | | 3 | 0,6 | 4 | >50 |
| CI6 | AB23 | 3,4 | 0,23 | 2 | >10 |
| CI7 | AB28 | 2,3 | 0,3 | 2 | >10 |
| | | 4,7 | 0,6 | 4 | >10 |
| CI8 | AB24 | 1,2 | 0,15 | 1 | >10 |
| | | 2,4 | 0,3 | 2 | >50 |
| CI9 | AB25 | 1,3 | 0,15 | 1 | >10 |
| | | 2,6 | 0,3 | 2 | >10 |
| CI10 | AB26 | 1,5 | 0,3 | 2 | >10 |
| CI11 | AB27 | 1,3 | 0,15 | 1 | >5 |
| | | 2,7 | 0,3 | 2 | >10 |
| CI12 | AB31 | 1,3 | 0,15 | 1 | >10 |
| | | 2,5 | 0,3 | 2 | >10 |
| CI13 | AB29 | 8,9 | 1,15 | 7,6 | >5 |
| CI14 | AB32 | 1,3 | 0,15 | 1 | >10 |
| | | 2,5 | 0,3 | 2 | >10 |

La solution de pramlintide à pH 6,6 (CH1) dépourvue de co-polyaminoacide présente un temps de latence court. Les co-polyaminoacides de l'invention permettent d'obtenir des temps de latence supérieurs à 5 h dans les conditions testées.

### Exemple D7A : Stabilité de solutions de pramlintide à 0,6 mg/ml à pH 6,6 en présence du co-polyaminoacide AB14 et de différentes teneurs en chlorure de sodium et en chlorure de zinc.

**Tableau 29 : Mesure du temps de latence par ThT (2 µM) des compositions BT1 à BT5**

| Solution | Co-polyaminoacide | Concentration en co-polyaminoacide | | [NaCl] | [ZnCl₂] | Temps de latence (h) |
|---|---|---|---|---|---|---|
| | | mg/mL | mM | (mM) | (mM) | |
| CT1 | AB14 | 6,3 | 1,87 | - | 0,75 | 0,6 |
| CT2 | AB14 | 6,3 | 1,87 | 50 | - | >2 |
| CT3 | AB14 | 6,3 | 1,87 | 100 | - | >5 |
| CT4 | AB14 | 6,3 | 1,87 | 50 | 0,75 | >5 |
| CT5 | AB14 | 6,3 | 1,87 | 100 | 0,75 | >20 |

La solution de pramlintide à pH 6,6 et de co-polyaminoacide AB14 présente un temps de latence plus important en présence de chlorure de sodium ou de chlorure de sodium et de zinc.

### Exemple D7B : Stabilité de solutions de pramlintide à 0,6 mg/ml à pH 6,6 en présence de différents co-polyaminoacides et de différentes teneurs en chlorure de sodium et en chlorure de zinc.

**Tableau 30 : Mesure du temps de latence par ThT (2 µM) des compositions BS1 à BS11**

| Solution | Co-polyaminoacide | Concentration en co-polyaminoacide | | [NaCl] | [ZnCl₂] | Temps de latence (h) |
|---|---|---|---|---|---|---|
| | | mg/mL | mM | (mM) | (mM) | |
| CS1 | AB15 | 7,8 | 1,6 | - | - | 3,5 |
| CS2 | AB15 | 11,7 | 2,3 | - | - | >30 |
| CS3 | AB15 | 3,9 | 0,8 | 50 | - | >10 |
| CS4 | AB15 | 6,3 | 1,3 | 50 | - | >50 |
| CS5 | AB15 | 7,8 | 1,6 | 50 | - | >30 |
| CS6 | AB15 | 3,9 | 0,8 | 100 | - | >50 |
| CS7 | AB16 | 12,4 | 1,5 | - | - | 8 |
| CS8 | AB16 | 16,7 | 2,1 | - | - | >50 |
| CS9 | AB16 | 7,4 | 0,9 | 50 | | >20 |
| CS10 | AB16 | 12,4 | 1,5 | 50 | | >50 |
| CS11 | AB16 | 7,4 | 0,9 | 50 | 1 | >30 |

Les solutions de pramlintide à pH 6,6 et de co-polyaminoacide AB15 et AB16 présentent un temps de latence plus important en présence de chlorure de sodium ou de chlorure de sodium et de zinc.

### Exemple D8 : Stabilité de solutions de pramlintide à 0,4 mg/ml et d'insuline humaine 100 UI/ml à pH 7,4 contenant du co-polyaminoacide BB15.

**Tableau 31 : Mesure du temps de latence par ThT (1 µM) des compositions CN1 à CN3.**

| Solution | Concentration en co-polyaminoacide BB15 | | Ratio BB15/pramlintide | Temps de latence (h) |
|---|---|---|---|---|
| | mg/mL | mM | mol/mol | |
| CN1 | - | | - | * |
| CN2 | 2,4 | 0,59 | 6 | >19 |
| CN3 | 4 | 0,98 | 10 | >19 |

| | | | | |
|---|---|---|---|---|
| ^{∗}Temps de latence non mesuré car solution turbide. | | | | |

La solution de pramlintide et d'insuline humaine à pH 7,4 (CN1) dépourvue de co-polyaminoacide est turbide.

Le co-polyaminoacide BB15 permet d'obtenir une solution limpide de pramlintide à 0,4 mg/ml et d'insuline humaine 100 UI/ml à pH 7,4 avec des temps de latence supérieurs à 19 h pour des ratios molaires BB15/pramlintide supérieurs à 6.

### Exemple D9 : Stabilité de solutions de pramlintide à 0,9 mg/ml et d'insuline humaine 100 UI/ml à pH 7,4 en présence de co-polyaminoacide BB15 à différentes concentrations.

**Tableau 32 : Mesure du temps de latence par ThT (2 µM) des solution CR1 à CR4 et CU3 à CU8.**

| Solution | Ratio BB15/pramlintide | Concentration en co-polyaminoacide BB15 | | Temps de latence (h) |
|---|---|---|---|---|
| | mol/mol | mg/ml | mM | |
| CR1 | - | - | - | * |
| CU3 | 2 | 1,8 | 0,44 | > 0,5 |
| CR2 | 3 | 2,7 | 0,66 | > 2 |
| CR3 | 4 | 3,6 | 0,88 | > 6 |
| CR4 | 5 | 4,5 | 1,10 | > 9 |
| CU7 | 6 | 5,4 | 1,32 | > 9 |
| CU8 | 10 | 9 | 2,20 | > 9 |

| | | | | |
|---|---|---|---|---|
| ^{∗}Temps de latence non mesuré car solution turbide. | | | | |

Une solution de pramlintide à 0,9 mg/ml et d'insuline humaine 100 UI/ml à pH 7,4 (BR1) dépourvue de co-polyaminoacide est turbide. Les solutions limpides de pramlintide à 0,9 mg/ml et d'insuline humaine 100 UI/ml à pH 7,4 en présence de co-polyaminoacide BB15 présentent des temps de latence supérieurs à 0,5 heure à ratio molaire BB15/pramlintide de 2, pouvant être supérieurs à 9 h pour des ratios molaires BB15/pramlintide supérieurs à 5.

### Exemple D10 : Stabilité de solutions de pramlintide à 0,9 mg/ml et d'insuline humaine 100 UI/ml à pH 7,4 en présence de différents co-polyaminoacides.

**Tableau 33 : Mesure du temps de latence par ThT (2 µM) des solutions CG2 à CG12.**

| Soluti on | Co-polyam inoacid e | Concentration en co-polyaminoacide | | Ratio co-polyaminoacide/pramlinti de | Temps de latence (h) |
|---|---|---|---|---|---|
| | | mg/mL | mM | mol/mol | |
| CR1 | | - | | - | * |
| CG2 | BB15 | 5 | 1,22 | 5,4 | > 9 |
| | | 10 | 2,45 | 10,8 | > 7 |
| CG3 | BB14 | 10 | 1,96 | 8,6 | > 9 |
| CG4 | AB17 | 5 | 1,11 | 4,9 | > 2 |
| | | 10 | 1,22 | 9,8 | > 5 |
| CG5 | AB15 | 10 | 1,99 | 8,8 | > 2 |
| CG10 | BB18 | 5 | 0,72 | 3,2 | > 1 |
| | | 10 | 1,44 | 6,3 | > 4 |
| CG11 | BB9 | 5 | 1 | 4,4 | > 4 |
| | | 10 | 2,01 | 8,8 | > 3 |
| CG12 | BB2 | 5 | 1,27 | 5,6 | > 5 |
| | | 10 | 2,54 | 11,2 | > 6 |

| | | | | | |
|---|---|---|---|---|---|
| ^{∗}Temps de latence non mesuré car solution turbide. | | | | | |

La solution de pramlintide et d'insuline humaine à pH 7,4 (CR1) est turbide. Les co-polyaminoacides permettent d'obtenir des temps de latence supérieurs à 1 heure dans les conditions testées.

### Exemple D11 : Stabilité de solutions de pramlintide à 0,9 mg/ml et d'insuline lispro 100 UI/ml à pH 7,4 en présence de co-polyaminoacide BB15 à différentes concentrations.

**Tableau 34 : Mesure du temps de latence par ThT (2 µM) des solutions CD1 et CD3 à CD8.**

| Solution | Ratio BB15/pramlintide | Concentration en co-polyaminoacide BB15 | | Temps de latence (h) |
|---|---|---|---|---|
| | mol/mol | mg/ml | mM | |
| CD1 | | - | - | * |
| CD3 | 2 | 1,8 | 0,44 | 0,8 |
| CD4 | 3 | 2,7 | 0,66 | > 2 |
| CD5 | 4 | 3,6 | 0,88 | > 7 |
| CD6 | 5 | 4,5 | 1,10 | > 9 |
| CD7 | 6 | 5,4 | 1,22 | > 9 |
| CD8 | 10 | 9 | 1,32 | > 9 |

| | | | | |
|---|---|---|---|---|
| ^{∗}Temps de latence non mesuré car solution turbide. | | | | |

La solution de pramlintide et d'insuline lispro à pH 7,4 (CD1) est turbide. Les co-polyaminoacides permettent d'obtenir des temps de latence supérieurs à 0,8 heure dans les conditions testées.

### Exemple D12 : Stabilité de solutions de pramlintide à 0,6 mg/ml et d'insuline humaine 100 UI/ml à pH 6,6 en présence de co-polyaminoacide BB15 à différentes concentrations,.

**Tableau 35 : Mesure du temps de latence par ThT (2 µM) des solutions CK1 et CK3 à CK8.**

| Solution | Ratio BB15/pramlintide | Concentration en co-polyaminoacide BB15 | | Temps de latence (h) |
|---|---|---|---|---|
| | mol/mol | mg/mL | mM | |
| CK1 | - | - | - | ∗ |
| CK3 | 3 | 2 | 0,45 | >0,5 |
| CK4 | 4 | 2,7 | 0,61 | >5 |
| CK5 | 6 | 4 | 0,90 | >5 |
| CK6 | 8 | 5,3 | 1,19 | >5 |
| CK7 | 10 | 6,7 | 1,50 | >5 |
| CK8 | 15 | 10 | 2,24 | >5 |

| | | | | |
|---|---|---|---|---|
| ^{∗}Temps de latence non mesuré car solution turbide. | | | | |

La solution de pramlintide et d'insuline humaine à pH 6,6 (CK1) est turbide. Les solutions limpides de pramlintide à 0,6 mg/mL et d'insuline humaine 100 UI/ml à pH 6,6 en présence de co-polyaminoacide BB15 présentent des temps de latence supérieurs à 0,5 h à ratio molaire BB15/pramlintide de 3, pouvant être supérieurs à 5 h à partir d'un ratio BB15/pramlintide de 4.

### Exemple D13 : Stabilité de solutions de pramlintide à 0,6 mg/ml et d'insuline humaine 100 UI/ml à pH 6,6 en présence de différents co-polyaminoacides.

**Tableau 36 : Mesure du temps de latence par ThT (2 µM) des solutions et CM1 à CM18.**

| Solution | Co-polyaminoacide | Concentration en co-polyaminoacide | | Ratio co-polyaminoacide/pramlintide | Temps de latence (h) |
|---|---|---|---|---|---|
| | | mg/mL | mM | mol/mol | |
| CM1 | BB20 | 2,6 | 0,61 | 4 | > 1 |
| | | 5,3 | 1,22 | 8 | >1 |
| CM2 | BB21 | 1,3 | 0,6 | 4 | >10 |
| CM3 | AB22 | 2,4 | 0,3 | 2 | >5 |
| CM4 | BB24 | 2,9 | 0,6 | 4 | >10 |
| CM5 | BB23 | 3 | 0,76 | 5 | >10 |
| CM6 | BB25 | 1,5 | 0,3 | 2 | >10 |
| CM7 | BB22 | 2,7 | 0,6 | 4 | >5 |
| CM8 | AB23 | 7,7 | 0,69 | 4,6 | >15 |
| CM9 | BB19 | 4,7 | 0,4 | 2,8 | > 1 |
| CM10 | AB28 | 2,3 | 0,3 | 2 | >10 |
| CM11 | AB24 | 2,4 | 0,3 | 2 | >5 |
| CM12 | AB25 | 2,6 | 0,3 | 2 | >5 |
| CM13 | AB26 | 1,5 | 0,3 | 2 | > 1 |
| | | 2,3 | 0,5 | 3 | >10 |
| CM14 | AB27 | 1,3 | 0,15 | 1 | > 1 |
| | | 2,7 | 0,3 | 2 | >5 |
| CM15 | AB30 | 1,2 | 0,15 | 1 | >1 |
| | | 2,3 | 0,3 | 2 | >10 |
| CM16 | AB31 | 1,3 | 0,15 | 1 | >1 |
| | | 2,5 | 0,3 | 2 | >10 |
| CM17 | AB29 | 5,9 | 0,8 | 5 | >1 |
| | | 8,9 | 1,15 | 7,6 | >5 |
| CM18 | AB32 | 2,5 | 0,3 | 2 | >1 |

La solution de pramlintide et d'insuline humaine à pH 6,6 (CK1) est turbide. Les co-polyaminoacides permettent d'obtenir des temps de latence supérieurs à 1 h dans les conditions testées.

### Exemple D13A : Stabilité de solutions de pramlintide à 0,6 mg/ml et d'insuline humaine 100 IU/ml à pH 6,6 en présence de différents co-polyaminoacides et de différentes teneurs en chlorure de sodium et en chlorure de zinc.

**Tableau 37 : Mesure du temps de latence par ThT (2 µM) des solutions BQ1 et BQ2 à BQ12**

| Solution | Co-polyaminoacide | Concentration en co-polyaminoacide | | [NaCl] | [ZnCl₂] | Temps de latence (h) |
|---|---|---|---|---|---|---|
| | | mg/mL | mM | (mM) | (mM) | |
| BQ1 | AB14 | 6,3 | 1,87 | 100 | 1 | >5 |
| BQ2 | AB15 | 7,8 | 1,6 | - | 0,23 | >2 |
| BQ3 | AB15 | 11,7 | 2,3 | - | 0,23 | >2 |
| BQ5 | AB15 | 6,3 | 1,3 | 50 | 0,23 | >2 |
| BQ6 | AB15 | 7,8 | 1,6 | 50 | 0,23 | >5 |
| BQ7 | AB15 | 3,9 | 0,8 | 100 | 0,23 | >2 |
| BQ8 | AB15 | 6,3 | 1,3 | 100 | 0,23 | >2 |
| BQ9 | AB15 | 7,8 | 1,6 | 100 | 0,23 | >5 |
| BQ10 | AB16 | 7,4 | 0,9 | 50 | 0,23 | >1 |
| BQ11 | AB16 | 12,4 | 1,5 | 50 | 0,23 | >2 |
| BQ12 | AB16 | 7,4 | 0,9 | 50 | 1 | >2 |

Les solutions de pramlintide et d'insuline humaine à pH 6,6 en présence des co-polyaminoacides de AB14, AB15 et AB16, de chlorure de sodium et de zinc présentent des temps de latence supérieurs à 1 h dans les conditions testées. L'ajout de chlorure de sodium ou de chlorure de sodium et de zinc permet d'augmenter les temps de latence.

### Exemple D14 : Stabilité de compositions présentant des concentrations pramlintide variables et de l'insuline humaine à 100 UI/mL en présence de co-polyaminoacide AB24, de m-crésol (29 mM), de glycérine (174 mM) et de chlorure de zinc (229 µM) à pH 6,6.

**Tableau 38 : Mesure du temps de latence par ThT (2 µM) des solutions CF2 à CF6.**

| Solution | Concentration en pramlintide | Concentration en co-polyaminoacide AB24 | | Ratio co-polyaminoacide/ pramlintide | Temps de latence (h) |
|---|---|---|---|---|---|
| | (mg/mL) | mg/mL | mM | mol/mol | |
| CF2 | 0,9 | 5,4 | 0,67 | 3 | >10 (12) |
| CF3 | 0,8 | 4,8 | 0,6 | 3 | >10 (14,1) |
| CF4 | 0,6 | 3,6 | 0,45 | 3 | >5 (5,5) |
| CF5 | 0,3 | 1,8 | 0,22 | 3 | >5 (5,6) |
| CF6 | 0,2 | 1 | 0,125 | 2,5 | >5 (5,4) |

Les solutions de pramlintide de concentration variables et d'insuline humaine à 100 UI/mL à pH 6,6 sont turbides (exemples CF1A-E). Les solutions de pramlintide de concentration variables et d'insuline humaine à 100 UI/mL à pH 6,6 en présence du co-polyaminoacide AB24 présentent des temps de latence supérieurs à 5 h dans les conditions testées.

### D II : Etude de la stabilité des compositions selon l'invention

### D II A : Préparation des compositions

Composition D1 : Préparation d'une solution de pramlintide à 0,9 mg/mL contenant du m-crésol (29 mM) et de la glycérine (174 mM) à pH 6,6.

Par un procédé similaire à celui utilisé à l'exemple CH1 une solution de pramlintide à 0,9 mg/mL contenant du m-crésol (29 mM) et de la glycérine (174 mM) à pH 6,6 est obtenue. La solution est limpide.
Composition D2 : Préparation d'une solution de pramlintide à 0,9 mg/mL contenant du co-polyaminoacide BB15, du m-crésol (29 mM) et de la glycérine (174 mM) à pH 6,6.

Par un procédé similaire à celui utilisé à l'exemple BH0 une solution de pramlintide à 0,9 mg/mL et de co-polyaminoacide BB15 à 10 mg/mL contenant du m-crésol (29 mM) et de la glycérine (174 mM) à pH 6,6 est obtenue. La solution est limpide.

### D II B : Procédure d'inspection visuelle :

Les vials ou cartouches de 3 mL remplis avec 1 mL de formulation sont inspectés visuellement afin de détecter l'apparition de particules visibles ou d'une turbidité. Cette inspection est réalisée selon les recommandations de la Pharmacopée Européenne (EP 2.9.20) : les vials sont soumises à un éclairage d'au moins 2000 Lux et sont observées face à un fond blanc et un fond noir. Le nombre de semaine ou de mois de stabilité correspond à la durée à partir de laquelle les solutions contiennent des particules visibles ou sont turbides.

Ces résultats sont en accord avec la pharmacopée US (USP <790>).

### D II C : Procédure de dosage des formulations :

La quantification de la pureté de pramlintide et d'insuline et du recouvrement en peptide natif est réalisée par HPLC en phase inverse équipée d'une colonne CA18 de dimension 4,6 x 150 mm avec une taille de particules de 3,5 µm. Le pramlintide est détecté à une longueur d'onde de 214 nm et l'insuline est détectée à une longueur d'onde de 276 nm. L'élution est réalisée dans une phase mobile aqueuse avec un gradient linéaire d'acétonitrile.

Le recouvrement de pramlintide ou d'insuline (%) à un temps t représente le rapport entre l'aire du pic de pramlintide ou l'aire du pic d'insuline au temps t et l'aire du pic de pramlintide initial.

La pureté de pramlintide et d'insuline (%) représente le rapport entre l'aire du pic d'absorbance du pramlintide ou d'isuline et l'aire totale de l'ensemble des pics incluant pramlintide et ses impuretés.

### D II D : Stabilité physique en cartouches à 37 °Cde solutions de pramlintide à 0,9 mg/ml en présence du co-polyaminoacide BB15, de m-crésol (29 mM) et de glycérine (174 mM) à pH 6,6 ou pH 7,4.

Les solutions D1, CY1, D2 et CY7 sont filtrées (0,22 µm). 1 mL de solution est introduit dans une cartouche en verre de 3 mL pour stylo auto-injecteur. Les cartouches sont placées dans une étuve à 37 °Cen statique. Les cartouches sont observées avec une fréquence hebdomadaire.

**Tableau 39 : Résultats des stabilités physiques à 37 °Cen cartouche des compositions de pramlinltide à 0,9 mg/mL en présence de co-polyaminoacide BB15.**

| Solution | Concentration en co-polyaminoacide BB15 | pH | Stabilité physique 37 °Cen cartouches (semaines) |
|---|---|---|---|
| | mg/mL | | |
| D1 | - | 6,6 | < 1 |
| CY1 | - | 7,4 | < 1 |
| D2 | 10 | 6,6 | > 4 |
| CY7 | 10 | 7,4 | > 4 |

Les solutions de pramlintide à 0,9 mg/mL à pH 6,6 et pH 7,4 présente une stabilité physique à 37 °Cen cartouche inférieure à une semaine.

Les solutions de pramlintide à 0,9 mg/ml à pH 6,6 et pH 7,4 en présence de co-polyaminoacide BB15 présentent une stabilité physique à 37 °Cen cartouches d'au moins 4 semaines.

### Exemple D II C : Stabilité chimique en cartouches à 37 °Cde solutions de pramlintide à 0,9 mg/mL en présence du co-polyaminoacide BB15, de m-crésol (29 mM) et de glycérine (174 mM) à pH 6,6 et 7,4.

Les solutions décrites à l'exemple D II D sont analysées par chromatographie RP-HPLC.

**Tableau 40 : Résultats des stabilités chimiques des compositions en pramlintide à 0,9 mg/mL en présence de co-polyaminoacide BB15.**

| Solution | Concentration en co-polyaminoacide BB15 | pH | Recouvrement pramlintide (%) 32 jours- 37 °C | Pureté pramlintide (%) | |
|---|---|---|---|---|---|
| | mg/mL | | | T0 | 32 jours 37 °C |
| D1 | - | 6,6 | <60 | 97,2 | <50 |
| CY1 | - | 7,4 | <20 | 94,7 | <50 |
| D2 | 10 | 6,6 | >90 | 97,8 | >60 |
| CY7 | 10 | 7,4 | >60 | 98,6 | >60 |

Les solutions de pramlintide à 0,9 mg/mL à pH 6,6 et pH 7,4 présentent un recouvrement en pramlintide inférieur à 60 % % et la pureté de pramlintide est inférieure à 50 % % après 32 jours à 37 °Cen cartouche.

Les solutions de pramlintide à 0,9 mg/ml à pH 6,6 et pH 7,4 en présence de co-polyaminoacide BB15 présentent un recouvrement supérieur à 65 % % et pouvant être supérieur à 90 % % à pH 6,6 après 32 jours à 37 °Cen cartouches. En présence de co-polyaminoacide BB15, la pureté de pramlintide est supérieure à 65 % et peut être supérieure à 85 % à pH 6,6.

### Exemple D II E : Stabilité physique en vial et cartouche à 30 °Cde solutions de pramlintide à 0,9 mg/mL et à 0,6 mg/mL en présence du co-polyaminoacide BB15, de m-crésol (29 mM) et de glycérine (174 mM) à pH 6,6.

Les solutions D1, CH1, D2 et CH8 sont filtrées (0,22 µm). 1 mL de solution est introduit dans des cartouches en verre de 3 mL pour stylo auto-injecteur et dans des vials en verre de 3 mL. Les cartouches et les vials sont placées dans une étuve à 30 °Cen statique puis sont observées toutes les 2 semaines.

**Tableau 41 : Résultats des stabilités physiques en vial et en cartouche à 30 °Cdes compositions en pramlintide à 0,9 et 0,6 mg/mL en présence de co-polyaminoacide BB15.**

| Solution | Concentration co-polyaminoacide BB15 | Concentration Pramlintide | pH | Stabilité physique 30 °Cen vial (semaines) | Stabilité physique 30 °Cen cartouche (semaines) |
|---|---|---|---|---|---|
| | mg/mL | (mg/mL) | | | |
| D1 | - | 0,9 | 6,6 | < 7 | < 2 |
| CH1 | - | 0,6 | 6,6 | < 7 | - |
| D2 | 10 | 0,9 | 6,6 | > 12 | >12 |
| CH8 | 10 | 0,6 | 6,6 | > 12 | >12 |

Les solutions de pramlintide à 0,9 mg/mL et 0,6 mg/mL à pH 6,6 présentent une stabilité physique en vial inférieure à 7 semaines à 30 °C. La stabilité physique en cartouche de la solution de pramlintide à 0,9 mg/mL pH 6,6 est inférieure à 2 semaines.

Les solutions de pramlintide à 0,9 mg/ml et 0,6 mg/mL à pH 6,6 en présence de co-polyaminoacide BB15 présentent une stabilité physique à 30 °Csupérieure à 12 semaines en vial et en cartouche.

### Exemple D II F : Stabilité chimique en vial à 30 °Cde solutions de pramlintide à 0,9 mg/mL et à 0,6 mg/mL en présence du co-polyaminoacide BB15, de m-crésol (29 mM) et de glycérine (174 mM) à pH 6,6.

Les solutions décrites à l'exemple D II E sont analysées par chromatographie RP-HPLC.

**Tableau 42 : Résultats des stabilités chimiques en vial à 30 °Cdes compositions en pramlintide à 0,9 et 0,6 mg/mL en présence de co-polyaminoacide BB15 à pH 6,6.**

| Solution | Concentration co-polyaminoacide BB15 | Concentration pramlintide | Recouvrement pramlintide (%) | Pureté pramlintide (%) | |
|---|---|---|---|---|---|
| | mg/mL | (mg/mL) | 5 semaines 30 °C | T0 | 5 semaines 30 °C |
| D1 | - | 0,9 | <70 | 97,2 | <60 |
| D2 | 10 | 0,9 | >95 | 97,8 | >90 |
| CH8 | 10 | 0,6 | >95 | 94,6 | >90 |

La solution de pramlintide à 0,9 mg/mL à pH 6,6 présente un recouvrement en pramlintide inférieur à 70% et la pureté de pramlintide est inférieure à 60 % après 5 semaines à 30 °Cen via.l.

Les solutions de pramlintide à 0,9 mg/ml et 0,6 mg/mL à pH 6,6 en présence de co-polyaminoacide BB15 présentent un recouvrement en pramlintide supérieur à 95 % et la pureté de pramlintide est supérieure à 90 % après 5 semaines à 30 °C.

### Exemple D II F : Stabilité physique en vial et cartouches à 30 °Cde solutions de pramlintide à 0,6 mg/mL et d'insuline 100 UI/mL à pH 6,6 en présence du co-polyaminoacide BB15, de m-crésol (29 mM), de glycérine (174 mM) et de zinc à pH 6,6.

La solution CK8 est filtrée (0,22 µM). 1 mL de solution est introduit dans des cartouches en verre de 3 mL pour stylo auto-injecteur et dans des vials en verre de 3 mL. Les cartouches et les vials sont placées dans une étuve à 30 °Cen statique puis sont observées toutes les 2 semaines.

**Tableau 43 : Résultats des stabilités physiques en vial et en cartouche à 30 °Cdes compositions de pramlintide à 0,6 mg/mL, d'insuline 100 UI/mL et en présence de co-polyaminoacide BB15 à pH 6,6.**

| Solution | Concentration co-polyaminoacide BB15 | Concentration pramlintide | Concentration Insuline | Stabilité physique 30 °Cen vial (semaines) | Stabilité physique 30 °Cen cartouche (semaines) |
|---|---|---|---|---|---|
| | mg/mL | (mg/mL) | (UI/mL) | | |
| CK1 | - | 0,6 | 100 | * | * |
| CK8 | 10 | 0,6 | 100 | > 3 | > 12 |

| | | | | | |
|---|---|---|---|---|---|
| ^{∗}solution turbide dès sa préparation. | | | | | |

La solution de pramlintide à 0,6 mg/mL et d'insuline à 100 UI/mL à pH 6,6 est turbide.

La solution de pramlintide à 0,6 mg/mL et d'insuline humaine à 100 UI/mL à pH 6,6 en présence de co-polyaminoacide BB15 présente une stabilité physique à 30 °Csupérieure à 3 semaines en vial et supérieure à 12 semaines en cartouche.

### Exemple D II G : Stabilité chimique en vial à 30 °Cd'une solution de pramlintide à 0,6 mg/ml et d'insuline 100 UI/mL à pH 6,6 en présence du co-polyaminoacide BB15, de m-crésol (29 mM), de glycérine (174 mM) et de zinc.

La solution décrite à l'exemple D II F est analysée par chromatographie RP-HPLC.

**Tableau 44 : Résultats de stabilité chimique en vial à 30 °Cd'une composition de pramlintide à 0,6 mg/mL, d'insuline 100 UI en présence de co-polyaminoacide BB15 à pH 6,6.**

| Solution | Recouvrement pramlintide (%) | Pureté pramlintide (%) | | Recouvrement Insuline (%) | Pureté Insuline (%) | |
|---|---|---|---|---|---|---|
| | 5 semaines 30 °C | T0 | 5 semaines 30 °C | 5 semaines 30 °C | T0 | 5 semaines 30 °C |
| CK8 | >90 | 96,8 | > 90 | > 90 | 97,9 | > 90 |

La solution de pramlintide à 0,6 mg/mL et d'insuline à 100 UI/mL à pH 6,6 en présence de co-polyaminoacide BB15 présente un recouvrement en pramlintide supérieur à 90 % et la pureté de pramlintide est supérieure à 90 % après 5 semaines à 30 °Cen vial. Le recouvrement en insuline est supérieur à 90 % et la pureté de l'insuline est supérieure à 90 % après 5 semaines à 30 °Cen vial.

### Exemple D II H : Stabilité physique en vial à 30 °Cet cartouche à 30 °C/37 °Cde solutions de pramlintide à 0,6 mg/mL et d'insuline 100 UI/mL à pH 6,6 en présence du co-polyaminoacide AB24 à 2,4 mg/mL, de m-crésol (29 mM), de glycérine (174 mM) et de zinc à pH 6,6.

La solution CM11 est filtrée (0,22 µM). 1 mL de solution est introduit dans des cartouches en verre de 3 mL pour stylo auto-injecteur et dans des vials en verre de 3 mL. Les cartouches et les vials sont placées dans une étuve à 30 °Cen statique puis sont observées toutes les 2 semaines. Des cartouches sont également placées dans une étuve à 37 °Cen statique puis sont observées toutes les semaines.

**Tableau 45 : Résultats des stabilités physiques en vial à 30 °Cet en cartouche à 30 et 37 °Cdes compositions de pramlintide à 0,6 mg/mL, d'insuline 100 UI/mL et en présence de co-polyaminoacide AB24 à pH 6,6.**

| Solution | Concentration co-polyaminoacid e AB24 | Concentration pramlintid e | Concentration Insuline | Stabilité physique 30 °Cen vial (semaines ) | Stabilité physique 30 °Cen cartouche (semaines ) | Stabilité physique 37 °Cen cartouche (semaines ) |
|---|---|---|---|---|---|---|
| | mg/mL | (mg/mL) | (UI/mL) | | | |
| CK1 | - | 0,6 | 100 | * | * | * |
| CM1 1 | 2,4 | 0,6 | 100 | > 9 | > 12 | > 9 |

| | | | | | | |
|---|---|---|---|---|---|---|
| ^{∗}solution turbide dès sa préparation. | | | | | | |

La solution de pramlintide à 0,6 mg/mL et d'insuline à 100 UI/mL à pH 6,6 est turbide.

La solution de pramlintide à 0,6 mg/mL et d'insuline humaine à 100 UI/mL à pH 6,6 en présence de co-polyaminoacide AB24 présente une stabilité physique à 30 °Csupérieure à 9 semaines en vial et supérieure à 12 semaines en cartouche. La stabilité physique à 37 °Cen cartouche est supérieure à 9 semaines.

### Exemple D III : Stabilité chimique en vial et cartouche à 30 °Cde solutions de pramlintide à 0,6 mg/mL et d'insuline 100 UI/mL à pH 6,6 en présence du co-polyaminoacide AB24 à 2,4 mg/mL, de m-crésol (29 mM), de glycérine (174 mM) et de zinc à pH 6,6.

La solution décrite à l'exemple D II H est analysée par chromatographie RP-HPLC.

**Tableau 46 : Résultats des stabilités chimiques en vial et cartouche à 30 °Cdes compositions de pramlintide à 0,6 mg/mL, d'insuline 100 UI en présence de co-polyaminoacide AB24 à pH 6,6.**

| Solution | Recouvrement pramlintide (%) | Pureté pramlintide (%) | | Recouvrement Insuline (%) | Pureté Insuline (%) | |
|---|---|---|---|---|---|---|
| CM11 | 9 semaines 30 °C | T0 | 9 semaines 30 °C | 9 semaines 30 °C | T0 | 9 semaines 30 °C |
| Vial | >88 | 96,7 | >90 | >90 | 97,4 | >90 |
| Cartouche | >88 | 96,9 | >85 | >90 | 97,7 | >90 |

La solution de pramlintide à 0,6 mg/mL et d'insuline humaine à 100 UI/mL à pH 6,6 en présence de co-polyaminoacide AB24 présente un recouvrement en pramlintide supérieur à 88 % et une pureté supérieure à respectivement 90 et 85 % après 9 semaines de stockage à 30 °Cen vial et en cartouche. Dans ces conditions le recouvrement en insuline est supérieur à 90 % et la pureté de l'insuline supérieure à 90 % en vial et en cartouche.

### Exemple D II J : Stabilité physique en cartouche à 4 °Cde solutions de pramlintide à 0,6 mg/mL à pH 6,6 en présence du co-polyaminoacide BB15, de m-crésol (29 mM), de glycérine (174 mM) à pH 6,6.

La solution CH8 est filtrée (0,22 µm). 1 mL de solution est introduit dans des cartouches en verre de 3 mL pour stylo auto-injecteur. Les cartouches sont placées dans un réfrigérateur à 4 °C.

**Tableau 47 : Résultats de la stabilité physique en cartouche à 4 °C dd'une composition de pramlintide à 0,6 mg/mL et en présence de co-polyaminoacide BB15 à pH 6,6.**

| Solution | Concentration co-polyaminoacide BB15 | Concentration pramlintide | pH | Stabilité physique 4 °Cen cartouche (mois) |
|---|---|---|---|---|
| | mg/mL | (mg/mL) | | |
| CH8 | 10 | 0,6 | 6,6 | > 6 |

La solution de pramlintide à 0,6 mg/mL et d'insuline humaine à 100 UI/mL à pH 6,6 en présence de co-polyaminoacide BB15 présente une stabilité physique en cartouche supérieure à 6 mois.

### Exemple D II K : Stabilité physique en cartouche à 4 °Cde solutions de pramlintide à 0,6 mg/mL à pH 6,6 et pH 7,4 en présence de DMPG à 4,5 mM.

Les solutions CZ0 et CZ1 sont filtrées (0,22 µM). 1 mL de solution est introduit dans des cartouches en verre de 3 mL pour stylo auto-injecteur. Les cartouches sont placées dans un réfrigérateur à 4 °C.

**Tableau 48 : Résultats des stabilités physiques en cartouche à 4 °Cdes compositions de pramlintide à 0,6 mg/mL et en présence de DMPG à pH 6,6 et 7,4.**

| Solution | DMPG (mM) | Concentration pramlintide (mg/mL) | pH | Excipients | Stabilité physique à 4 °Cen cartouches (mois) |
|---|---|---|---|---|---|
| CZ0 | 4,5 | 0,6 | 6,6 | m-crésol 29 mM | < 1,5 |
| | | | | Glycérine 174 mM | |
| CZ1 | 4,5 | 0,6 | 7,4 | phénol 30 mM | < 1,5 |
| | | | | Glycylglycine 8mM pH 7,4 | |
| | | | | Glycérine 174 mM | |

Les solutions de pramlintide à 0,6 mg/mL à pH 6,6 et à pH 7,4 présentent une stabilité physique à 4 °Cet en cartouches inférieure à 1,5 mois (solutions turbides).

### Exemple D II L : Stabilité en pompe de solutions de pramlintide à 0,6 mg/mL et d'insuline humaine à 100 UI/mL à pH 6,6 en présence du co-polyaminoacide AB24 à 3,6 mg/MI.

La solution CM11 composée de 0,6 mg/mL de pramlintide et de 100 UI/mL d'insuline humaine à pH 6,6 en présence du co-polyaminoacide AB24 à 3,6 mg/mL est filtrée (0,22 µm) et introduite dans un réservoir de 3 mL pour pompe à Insuline (Mlnlmed 530G system fabriqué par Medtronic). La pompe est équipée d'un set d'infusion (Quick set Paradigm 9/110 fabriqué par Medtronic).

La pompe à insuline est placée dans une étuve à 37 °Csur un agitateur orbtalaire réglé à une vitesse de 100 rpm. La pompe est réglée avec un débit basal de 0,8 UI/h. Des injections bolus de 6 UI sont réalisées 3 fois par jour pendant une durée totale de 8 jours.

Le tableau 49 présente les résultats des mesures de MFI (Micro-Flow Imaging) et les dosages réalisés par RP-HPLC sur les fractions collectées entre le 7^{ème} et le 8^{ème} jour de mise en stabilité.

**Tableau 49 : Résultats de la stabilité chimique en pompe à 37 °Cdes compositions de pramlintide à 0,6 mg/mL, d'insuline 100 UI/mL et en présence de co-polyaminoacide AB24 à pH 6,6.**

| Solution | Particules subvisible (Micro Flow Imaging, MFI) | Recouvre ment pramlintid e (%) | Pureté pramlintide (%) | | Recouvreme nt Insuline humaine (%) | Pureté Insuline (%) | |
|---|---|---|---|---|---|---|---|
| | 1 semaine 37°C | 1 semaine 37°C | T0 | 1 semain e 37°C | 1 semaine 37°C | T0 | 1 semaine 37°C |
| CM11 | particules> 10 µM < 6000 particules par contenant* | >95 | 97. 3 | >95 | >95 | 97. 4 | >95 |
| | particules> 25 µM < 600 particules par contenant* | | | | | | |

| | | | | | | | |
|---|---|---|---|---|---|---|---|
| ^{∗} *Norme USP <788> sur le nombre de particules sub-visibles dans les produits pour injections parentérales.* | | | | | | | |

Après une semaine de stabilté en pompe à 37 °C, la solution de pramlintide à 0,6 mg/mL et d'insuline humaine à 100 UI/mL à pH 6,6 en présence de co-polyaminoacide AB24 est limpide et présente un nombre de particules subvisibles conforme à la norme USP <788>. Dans ces conditions les recouvrement et les puretés de pramlintide et d'insuline sont supérieurs à 95 %.

### Exemple D II M : Stabilité en pompe de solutions de pramlintide à 0,6 mg/mL et d'insuline lispro à 100 UI/mL à pH 6,6 en présence du co-polyaminoacide AB24 à 3,6 mg/mL.

La solution CAS composée de 0,6 mg/mL de pramlintide et de 100 UI/mL d'insuline lispro à pH 6,6 en présence du co-polyaminoacide AB24 à 3,6 mg/mL est filtrée (0,22 µm) et soumise à un test de stabilité en pompe par un protocole identique à celui décrit à l'exemple DC11.

**Tableau 50 : Résultats de la stabilité chimique en pompe à 37 °Cdes compositions de pramlintide à 0,6 mg/mL, d'insuline lispro à 100 UI/mL et en présence de co-polyaminoacide AB24 à pH 6,6.**

| Solution | Particules subvisible (Micro Flow Imaging, MFI) | Recouvre ment pramlintid e (%) | Pureté pramlintide (%) | | Recouvrem ent lispro (%) | Pureté Insuline (%) | |
|---|---|---|---|---|---|---|---|
| | 1 semaine 37°C | 1 semaine 37°C | T0 | 1 semain e 37°C | 1 semaine 37°C | T0 | 1 semain e 37°C |
| CAS | particules> 1 0 µM < 6000 particules par contenant* | >95 | 97. 2 | >95 | >95 | 99,3 | >95 |
| | particules> 25 µM < 600 particules par contenant* | | | | | | |

| | | | | | | | |
|---|---|---|---|---|---|---|---|
| ^{∗} *Critère USP <788> sur le nombre de particules sub-visibles dans les produits pour injections parentérales.* | | | | | | | |

Après une semaine de stabilté en pompe à 37 °C, la solution de pramlintide à 0,6 mg/mL et d'insuline lispro à 100 UI/mL à pH 6,6 en présence de co-polyaminoacide AB24 est limpide et présente un nombre de particules subvisibles conforme à la norme USP <788>. Dans ces conditions les recouvrement et les puretés de pramlintide et d'insuline lispro sont supérieurs à 95 %.

### E. PHARMACODYNAMIE ET PHARMACOCINETIQUE

### E1: Protocole de mesure de la pharmacocinétique de formulations de pramlintide et d'insuline.

Des porcs domestiques d'environ 50 kg, préalablement cathétérisés au niveau de la jugulaire, sont mis à jeun 2,5 heures avant le début de l'expérience. Dans l'heure précédant l'injection des formulations de pramlintide et d'insuline, 3 prélèvements sanguins sont réalisés afin de déterminer le niveau basal de pramlintide.

L'injection des formulations à la dose de 1,125 µg/kg pour le pramlintide et 0,1875 UI/kg pour l'insuline est réalisée en sous-cutané au niveau du flanc de l'animal à l'aide d'un stylo à insuline (Novo, Sanofi ou Lilly) équipé d'une aiguille 31 G.

Des prélèvements sanguins sont ensuite réalisés toutes les 4 minutes pendant 20 minutes puis toutes les 10 à 60 minutes jusqu'à 3 heures. Après chaque prélèvement, le cathéter est rincé avec une solution diluée d'héparine.

Le sang ainsi prélevé est collecté dans un tube K2EDTA et est centrifugé pour isoler le plasma. Les taux de pramlintide dans les échantillons de plasma sont mesurés par la méthode immuno-enzymatique ELISA en sandwich pour chaque animal.

Les courbes de pharmacocinétique exprimées en delta du niveau basal sont ensuite tracées.

Les paramètres pharmacocinétiques suivants ont ensuite été déterminés par analyse non-compartimentale avec le logiciel Phoenix WinNonlin :
- tₘₐₓ pramlintide correspondant au temps nécessaire pour atteindre la concentration maximale de pramlintide dans le plasma ;
- AUC_{Pram 0-30min} correspondant à la surface sous la courbe des concentrations de pramlintide en fonction du temps entre 0 et 30 minutes post-administration ;
- AUC_{Pram 60-180min} correspondant à la surface sous la courbe des concentrations de pramlintide en fonction du temps entre 60 et 180 minutes post-administration ;
- Cₗₐₛₜ correspondant à la dernière concentration de pramlintide quantifiable dans le plasma ;
- tiast correspondant au temps auquel Cₗₐₛₜ est observée.

Le tₘₐₓ est couramment utilisé pour évaluer le démarrage de l'absorption. L'AUC_{Pram 0-30min} est couramment utilisé pour évaluer l'exposition précoce au pramlintide dans le plasma. L'AUC_{Pram 60-180min} permet quant à elle d'évaluer l'exposition tardive au pramlintide dans le plasma. Cₗₐₛₜ et tₗₐₛₜ permettent d'étudier les niveaux de concentration tardifs.

### E2: Résultats de pharmacocinétique du pramlintide des formulations de pramlintide et d'insuline des exemples CA2 et CA3

| Exempl e | Rh-Insulin e | co-polyaminoacide | Pramlintid e (mg/mL) | Nombre de porcs |
|---|---|---|---|---|
| CA1/CA2 (double injection) | 100 | - | 1 | 8 |
| CA3 | 100 | BB15 | 0.6 | 10 |

Les résultats de pharmacocinétique du pramlintide obtenus avec les compositions décrites dans les exemples CA1/CA2 et CA3 sont présentés à la Figure. 2. L'analyse de ces profils indique que la composition de l'exemple CA3 comprenant le co-polyaminoacide BB15, 100 UI/mL d'insuline et 0,6 mg/mL de pramlintide (courbe tracée avec les carrés correspondant à l'exemple CA3) permet d'obtenir une absorption du pramlintide plus lente que celle de la composition de l'exemple en double injection comprenant uniquement du pramlintide et de l'insuline (courbe tracée avec les triangles correspondant à l'exemple double injection CA1/CA2). Les paramètres de pharmacocinétique du pramlintide sont reportés dans le tableau suivant :

| Exemple | tₘₐₓ pramlintide (min) | AUC_{Pram 0-30min} (min*pmol/L ) | AUC_{Pram 60-180min} (min*pmol/L ) | Clast pramlintide (min) | tₗₐₛₜ pramlin-tide (min) |
|---|---|---|---|---|---|
| CA1/CA2 | 20 ± 10 | 2076 ± 1596 | 3286 ± 1951 | 12 ± 5 | 153 ± 40 |
| CA3 | 77 ± 44 | 1006 ± 885 | 5989 ± 3146 | 28 ± 24 | 168 ± 25 |

### E3: Résultats de pharmacocinétique du pramlintide des formulations de pramlintide et d'insuline des exemples CA1/CA2 et CA4.

| Exempl e | Rh-Insulin e | co-polyaminoacide | Pramlintid e (µg/mL) | Nombre de porcs |
|---|---|---|---|---|
| CA1/CA2 | 100 | - | - | 8 |
| CA4 | 100 | AB24 | 0.6 | 12 |

Les résultats de pharmacocinétique du pramlintide obtenus avec les compositions décrites dans les exemples CA1/CA2 et CA4 sont présentés à la Figure 3. L'analyse de ces profils indique que la composition de l'exemple CA4 comprenant le co-polyaminoacide AB24, 100 UI/mL d'insuline et 0,6 µg/mL de pramlintide (courbe tracée avec les carrés correspondant à l'exemple CA4) permet d'obtenir une absorption du pramlintide plus lente que celle de la composition de l'exemple en double injection comprenant uniquement du pramlintide et de l'insuline (courbe tracée avec les triangles correspondant à l'exemple double injection CA1/CA2). Les paramètres de pharmacocinétique du pramlintide sont reportés dans le tableau suivant :

| Exemple | tₘₐₓ pramlinti de (min) | AUC_{Pram 0-30mln} (min*pmol/L ) | AUCpram ₆₀₋₁₈₀ₘₗₙ (min*pmol/L ) | Cₗₐₛₜ pramlintid e (min) | tiast pramlintid e (min) |
|---|---|---|---|---|---|
| CA1/CA2 | 20 ± 10 | 2076 ± 1596 | 3286 ± 1951 | 12 ± 5 | 153 ± 40 |
| CA4 | 68 ± 23 | 749 ± 316 | 8064 ± 2963 | 34 ± 22 | 175 ± 17 |

## Revendications

1. Composition sous forme d'une solution aqueuse injectable, dont le pH est compris entre 6,0 et 8,0, comprenant au moins :
a) de l'amyline ou un analogue d'amyline ;
b) un co-polyaminoacide porteur de charges carboxylates et de radicaux hydrophobes Hy, ledit co-polyaminoacide étant constitué d'unités glutamiques ou aspartiques et lesdits radicaux hydrophobes Hy étant de formule I suivante : 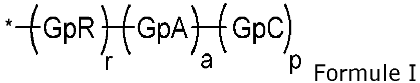 dans laquelle
- GpR est un radical de formules II, II' ou II" : 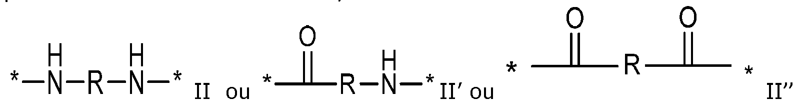
- GpA est un radical de formules III ou III' : 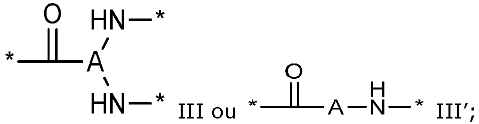
- GpC est un radical de formule IV : 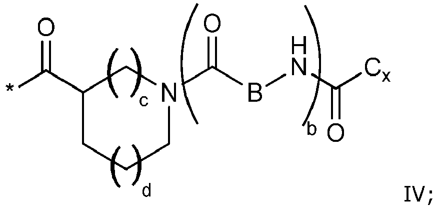
- les * indiquent les sites de rattachement des différents groupes liés par des fonctions amides ;
- a est un entier égal à 0 ou à 1 ;
- b est un entier égal à 0 ou à 1;
- p est un entier égal à 1 ou à 2 et
∘ si p est égal à 1 alors a est égal à 0 ou à 1 et GpA est un radical de formule III' et,
∘ si p est égal à 2 alors a est égal à 1, et GpA est un radical de formule III;
- c est un entier égal à 0 ou à 1, et si c est égal à 0 alors d est égal à 1 ou à 2;
- d est un entier égal à 0, à 1 ou à 2;
- r est un entier égal à 0 ou à 1, et
∘ si r est égal à 0 alors le radical hydrophobe de formule I est lié au co-polyaminoacide via une liaison covalente entre un carbonyl du radical hydrophobe et un atome d'azote en position N terminale du co-polyaminoacide, formant ainsi une fonction amide issue de la réaction d'une fonction amine en position N terminale du précurseur du co-polyaminoacide et une fonction acide portée par le précurseur du radical hydrophobe , et
∘ si r est égal à 1 alors le radical hydrophobe de formule I est lié au co-polyaminoacide :
▪ via une liaison covalente entre un atome d'azote du radical hydrophobe et un carbonyl du co-polyaminoacide, formant ainsi une fonction amide issue de la réaction d'une fonction amine du précurseur du radical hydrophobe et une fonction acide portée par le précurseur du co-polyaminoacide ou
▪ via une liaison covalente entre un carbonyl du radical hydrophobe et un atome d'azote en position N terminal du co-polyaminoacide, formant ainsi une fonction amide issue de la réaction d'une fonction acide du précurseur du radical hydrophobe et une fonction amine en position N terminale portée par le précurseur du co-polyaminoacide;
- R est un radical choisi dans le groupe constitué par :
∘ un radical alkyle divalent, linéaire ou ramifié, comprenant si GpR est un radical de formule II de 2 à 12 atomes de carbone ou si GpR est un radical de formule II' ou II"de 1 à 11 atomes de carbone ;
∘ un radical alkyle divalent, linéaire ou ramifié, comprenant, si GpR est un radical de formule II de 2 à 11 atomes de carbone ou si GpR est un radical de formule II' ou II"de 1 à 11 atomes de carbone, ledit radical alkyle portant une ou plusieurs fonctions -CONH₂, et
∘ un radical éther ou polyéther non substitué comprenant de 4 à 14 atomes de carbone et de 1 à 5 atomes d'oxygène ;
- A est un radical alkyle linéaire ou ramifié comprenant de 1 à 6 atomes de carbone;
- B est un radical alkyle linéaire ou ramifié, éventuellement comprenant un noyau aromatique, comprenant de 1 à 9 atomes de carbone;
- Cₓ est un radical alkyl monovalent linéaire ou ramifié, dans lequel x indique le nombre d'atomes de carbone et :
∘ si p est égal à 1, x est compris entre 11 et 25 (11 ≤ x ≤ 25) :
∘ si p est égal à 2, x est compris entre 9 et 15 (9 ≤ x ≤ 15),
- le ratio i entre le nombre de radicaux hydrophobes et le nombre d'unités glutamiques ou aspartiques étant compris entre entre 0 < i ≤ 0,5 ;
- lorsque plusieurs radicaux hydrophobes sont portés par un co-polyaminoacide alors ils sont identiques ou différents,
- le degré de polymérisation DP en unités glutamiques ou aspartiques est compris entre 10 et 250 ;
- les fonctions acides libres étant sous forme de sel de cation alkalin choisi dans le groupe constitué par Na⁺ et K⁺ ;
**caractérisée en ce que** la composition ne comprend pas d'une insuline basale dont le point isoélectrique pI est compris entre 5,8 et 8,5.

2. Composition selon la revendication 1, **caractérisée en ce que** lesdits radicaux hydrophobes sont choisis parmi les radicaux hydrophobes de formule I dans laquelle p = 1, représentée par la formule V suivante : 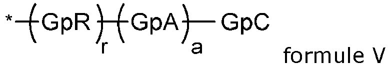 GpR, GpA, GpC, r et a ont les définitions données en revendication 1.

3. Composition selon la revendication 1, **caractérisée en ce que** lesdits radicaux hydrophobes sont choisis parmi les radicaux hydrophobes de formule I dans laquelle a = 1 et p = 2, représentée par la formule VI suivante : 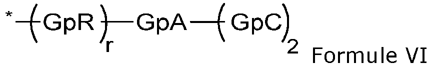 dans laquelle
GpR, GpA, GpC et r ont les définitions données en revendication 1.

4. Composition selon l'une quelconque des revendications précédentes, **caractérisée en ce que** le co-polyaminoacide porteur de charges carboxylates et de radicaux hydrophobes est choisi parmi les co-polyaminoacides de formule VII suivante : 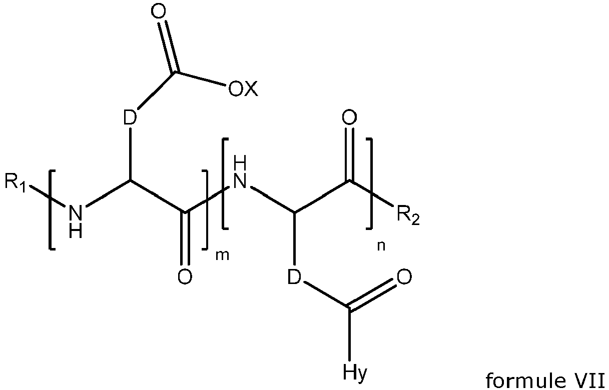 dans laquelle,
• D représente, indépendamment, soit un groupe -CH₂- (unité aspartique) soit un groupe -CH₂-CH₂- (unité glutamique),
• Hy est un radical hydrophobe choisi parmi les radicaux hydrophobes de formules I, V ou VI, dans lesquelles r = 1 et GpR est un radical de Formule II,
• R₁ est un radical hydrophobe choisi parmi les radicaux hydrophobes de formules I, V ou VI dans lesquelles r=0 ou r = 1 et GpR est un radical de Formule II', ou un radical choisi dans le groupe constitué par un H, un groupe acyle linéaire en C2 à C10, un groupe acyle ramifié en C3 à C10, un benzyle, une unité « acide aminé » terminale et un pyroglutamate,
• R₂ est un radical hydrophobe choisi parmi les radicaux hydrophobes de formules I, V ou VI dans lesquelles r = 1 et GpR est un radical de Formule II, ou un radical -NR'R", R' et R" identiques ou différents étant choisis dans le groupe constitué par H, les alkyles linéaires ou ramifiés ou cycliques en C2 à C10, le benzyle et lesdits R' et R" alkyles pouvant former ensemble un ou des cycles carbonés saturés, insaturés et/ou aromatiques et/ou pouvant comporter des hétéroatomes, choisis dans le groupe constitué par O, N et S,
• X représente un H ou une entité cationique choisie dans le groupe comprenant les cations métalliques ;
• n + m représente le degré de polymérisation DP du co-polyaminoacide, c'est-à-dire le nombre moyen d'unités monomériques par chaîne de co-polyaminoacide et 5 ≤ n + m ≤ 250.

5. Composition selon la revendication 4, **caractérisée en ce que** le co-polyaminoacide porteur de charges carboxylates et de radicaux hydrophobes est choisi parmi les co-polyaminoacides de formule VII, dans laquelle R₁ = R'₁ et R₂ = R'₂, de formule VIIa suivante : 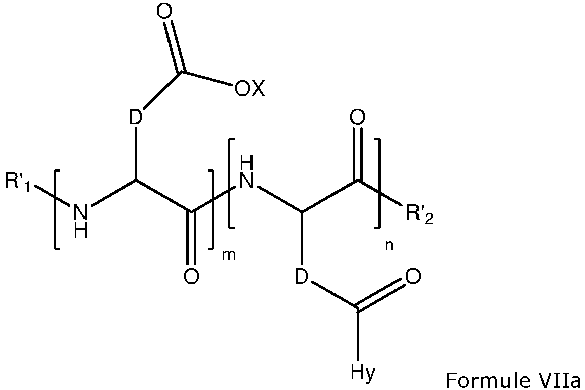 dans laquelle,
- m, n, X, D et Hy ont les définitions données en revendication 4,
- R'₁ est un radical choisi dans le groupe constitué par un H, un groupe acyle linéaire en C2 à C10, un groupe acyle ramifié en C3 à C10, un benzyle, une unité « acide aminé » terminale et un pyroglutamate,
- R'₂ est un radical -NR'R", R' et R" identiques ou différents étant choisis dans le groupe constitué par H, les alkyles linéaires ou ramifiés ou cycliques en C2 à C10, le benzyle et lesdits R' et R" alkyles pouvant former ensemble un ou des cycles carbonés saturés, insaturés et/ou aromatiques et/ou pouvant comporter des hétéroatomes, choisis dans le groupe constitué par O, N et S.

6. Composition selon la revendication 4, **caractérisée en ce que** le co-polyaminoacide porteur de charges carboxylates et de radicaux hydrophobes est choisi parmi les co-polyaminoacides de formule VII dans laquelle n = 0 de formule VIIb suivante : 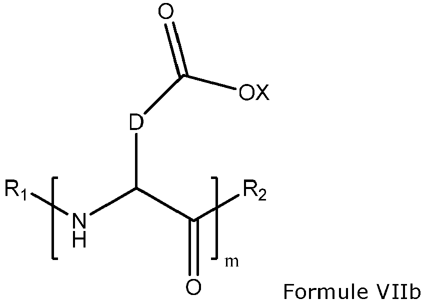 dans laquelle m, X, D, R₁ et R₂ ont les définitions données en revendication 4 et au moins R₁ ou R₂ est un radical hydrophobe de formule I, V ou VI.

7. Composition selon l'une quelconque des revendications précédentes, **caractérisée en ce que** ratio molaire co-polyaminoacide/amyline ou analogue d'amyline est supérieur ou égal à 1.

8. Composition selon l'une quelconque des revendications précédentes, **caractérisée en ce que** l'amyline ou analogue d'amyline est l'amyline.

9. Composition selon l'une quelconque des revendications précédentes, **caractérisée en ce que** l'amyline ou analogue d'amyline est le pramlintide.

10. Composition selon l'une quelconque des revendications précédentes, **caractérisée en ce qu'**elle comprend en outre une insuline prandiale.

11. Composition selon l'une quelconque des revendications précédentes, **caractérisée en ce que** ratio molaire co-polyaminoacide/insuline est supérieur ou égal à 1.

12. Composition selon l'une quelconque des revendications 1 à 9, **caractérisée en ce que** ladite composition présente une stabilité mesurée par ThT supérieure à celle d'une composition de référence comprenant de l'amyline ou un analogue d'amyline mais ne comprenant pas de co-polyaminoacide porteur de charges carboxylates et de radicaux hydrophobes Hy, ledit suivi ThT étant réalisé dans des conditions de stabilité accélérées : sous agitation et à 37 °C.

13. Utilisation d'un co-polyaminoacide porteur de charges carboxylates et de radicaux hydrophobes Hy selon l'une des revendications 1 à 6 pour stabiliser une composition comprenant de l'amyline ou un analogue d'amyline.

14. Formulations unidoses comprenant une composition selon l'une des revendications 1 à 12

## Patentansprüche

1. Zusammensetzung in Form einer wässrigen Injektionslösung, deren pH zwischen 6,0 und 8,0 beträgt, wenigstens umfassend:
a) Amylin oder ein Amylinanalogon;
b) eine Co-Polyaminosäure, die Carboxylat-Ladungen und hydrophobe Radikale Hy trägt, wobei die Co-Polyaminosäure aus Glutamin- oder Asparagineinheiten besteht und die hydrophoben Radikale Hy die folgende Formel I aufweisen: 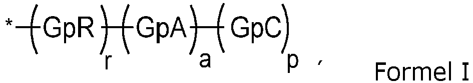 worin
- GpR ein Radikal gemäß den Formeln II, II' oder II" ist: 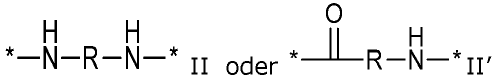 oder 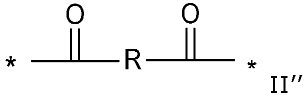
- GpA ein Radikal gemäß den Formeln III oder III' ist: 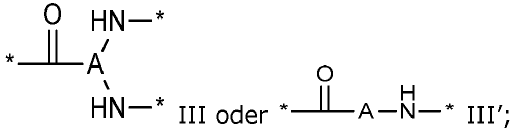
- GpC ein Radikal gemäß der Formel IV ist: 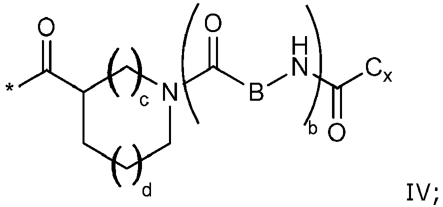
- die * die Bindungsstellen der verschiedenen, durch Amidfunktionen verbundenen Gruppen anzeigen;
- a eine ganze Zahl von 0 oder 1 ist;
- b eine ganze Zahl von 0 oder 1 ist;
- p eine ganze Zahl von 1 oder 2 ist und
o wenn p = 1 ist, a = 0 oder 1 ist und GpA ein Radikal gemäß Formel III' ist, und
o wenn p = 2 ist, a = 1 ist und GpA ein Radikal gemäß Formel III ist;
- c eine ganze Zahl von 0 oder 1 ist, und wenn c = 0 ist, d = 1 oder 2 ist;
- d eine ganze Zahl von 0, 1 oder 2 ist;
- r eine ganze Zahl von 0 oder 1 ist, und
o wenn r = 0 ist, das hydrophobe Radikal gemäß Formel I mit der Co-Polyaminosäure mittels einer kovalenten Bindung zwischen einem Carbonyl des hydrophoben Radikals und einem Stickstoffatom an N-terminaler Position der Co-Polyaminosäure verbunden ist, wodurch eine Amidfunktion gebildet ist als Ergebnis der Reaktion einer Aminfunktion an N-terminaler Position des Vorläufers der Co-Polyaminosäure und einer Säurefunktion, die von dem Vorläufer des hydrophoben Radikals getragen ist, und
∘wenn r = 1 ist, das hydrophobe Radikal gemäß Formel I mit der Co-Polyaminosäure verbunden ist:
▪ durch eine kovalente Bindung zwischen einem Stickstoffatom des hydrophoben Radikals und einem Carbonyl der Co-Polyaminosäure, wodurch eine Amidfunktion gebildet ist als Ergebnis der Reaktion einer Aminfunktion des Vorläufers des hydrophoben Radikals und einer Säurefunktion, die von dem Vorläufer der Co-Polyaminosäure getragen ist, oder
▪ durch eine kovalente Bindung zwischen einem Carbonyl des hydrophoben Radikals und einem Stickstoffatom an N-terminaler Position der Co-Polyamionsäure, wodurch eine Amidfunktion gebildet ist als Ergebnis der Reaktion einer Säurefunktion des Vorläufers des hydrophoben Radikals und einer Aminfunktion an N-terminaler Position, die von dem Vorläufer der Co-Polyaminosäure getragen ist;
- R ein Radikal ist, das ausgewählt ist aus der Guppe bestehend aus:
o einem divalenten, linearen oder verzweigten Alkylradikal umfassend, wenn GpR ein Radikal gemäß Formel II ist, 2 bis 12 Kohlenstoffatome oder, wenn GpR ein Radikal gemäß Formel II'oder II" ist, 1 bis 11 Kohlenstoffatome;
o einem divalenten, linearen oder verzweigten Alkylradikal umfasend, wenn GpR ein Radikal gemäß Formel II ist, 2 bis 11 Kohlenstoffatome oder, wenn GpR ein Radikal gemäß Formel II'oder II" ist, 1 bis 11 Kohlenstoffatome, wobei das Alkylradikal eine oder mehrere -CONH₂ - Funktionen trägt, und
o einem nicht-substituierten Ether- oder Polyether-Radikal umfassend 4 bis 14 Kohlenstoffatome und 1 bis 5 Sauerstoffatome;
- A ein lineares oder verzweigtes Alkylradikal ist umfassend 1 bis 6 Kohlenstoffatome;
- B ein lineares oder verzweigtes Alkylradikal ist, optional umfassend einen aromatischen Ring, umfassend 1 bis 9 Kohlenstoffatome;
- Cₓ ein monovalentes, lineares oder verzweigtes Alkylradikal ist, in dem x die Anzahl von Kohlenstoffatomen angibt, und:
o wenn p = 1 ist, x zwischen 11 und 25 beträgt (11 ≤ x ≤ 25),
o wenn p = 2 ist, x zwischen 9 und 15 beträgt (9 ≤ x ≤ 15),
- das Verhältnis i zwischen der Anzahl von hydrophoben Radikalen und der Anzahl der Glutamin- oder Asparagineinheiten zwischen 0 < i ≤ 0,5 beträgt;
- wenn mehrere hydrophobe Radikale von einer Co-Polyaminosäure getragen sind, diese identisch oder verschieden sind,
- der Polymerisationsgrad DP von Glutamin- oder Asparagineinheiten zwischen 10 und 250 beträgt;
- die freien Säurefunktionen in Form eines Salzes eines alkalischen Kations vorliegen, das ausgewählt ist aus der Gruppe bestehend aus Na⁺ und K⁺;
**dadurch gekennzeichnet, dass** die Zusammensetzung kein Basalinsulin umfasst, dessen isoelektrischer Punkt pI zwischen 5,8 und 8,5 beträgt.

2. Zusammensetzung nach Anspruch 1, **dadurch gekennzeichnet, dass** die hydrophoben Radikale ausgewählt sind aus den hydrophoben Radikalen gemäß Formel I, worin p = 1 ist, dargestellt durch die folgende Formel V: 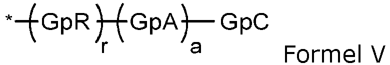 worin GpR, GpA, GpC, r und a wie in Anspruch 1 definiert sind.

3. Zusammensetzung nach Anspruch 1, **dadurch gekennzeichnet, dass** die hydrophoben Radikale ausgewählt sind aus den hydrophoben Radikalen gemäß Formel I, worin a = 1 und p =2 ist, dargestellt durch die folgenden Formel VI : 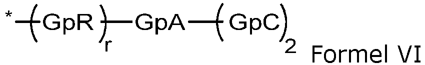 worin
GpR, GpA, GpC und r wie in Anspruch 1 definiert sind.

4. Zusammensetzung nach einem der vorangehenden Ansprüche, **dadurch gekennzeichnet, dass** die Carboxylat-Ladungen und hydrophobe Radikale tragende Co-Polyaminosäure ausgewählt ist aus den Co-Polyaminosäuren gemäß der folgenden Formel VII 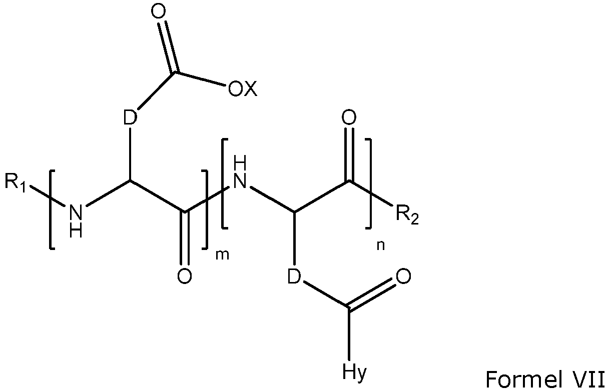 worin
• D, unabhängig, entweder eine -CH₂- -Gruppe (Asparagineinheit) oder eine -CH₂-CH₂- -Gruppe (Glutamineinheit) darstellt,
• Hy ein hydrophobes Radikal ist, das ausgewählt ist aus den hydrophoben Radikalen gemäß den Formeln I, V oder VI, worin r = 1 und GpR ein Radikal gemäß Formel II ist,
• R₁ ein hydrophobes Radikal ist, das ausgewählt ist aus den hydrophoben Radikalen gemäß den Formeln I, V oder VI, worin r = 0 oder r = 1 und GpR ein Radikal gemäß Formel II' ist, oder ein Radikal, das ausgewählt ist aus der Gruppe bestehend einem H, einer linearen C2- bis C10-Acylgruppe, einer verzweigen C3- bis C10-Acylgruppe, einem Benzyl, einer terminalen « Aminosäure »-Einheit und einem Pyroglutamat,
• R₂ ein hydrophobes Radikal ist, das ausgwählt ist aus den hydrophoben Radikalen gemäß den Formeln I, V oder VI, worin r = 1 und GpR ein Radikal gemäß Formel II ist, oder ein Radikal - NR'R" ist, wobei R' und R", die identisch oder verschieden sind, ausgewählt sind aus der Gruppe bestehend aus H, linearen oder verzweigten oder cyclischen C2- bis C10-Alkylen, Benzyl und die R'- und R"-Alkyle zusammen einen oder mehrere gesättigte, ungesättigen und/oder aromatische Kohlenstoffringe bilden können und/oder Heteroatome enthalten können, die aus der Gruppe ausgewählt sind, die aus O, N und S besteht,
• X ein H oder eine kationsiche Einheit darstellt, die ausgewählt ist aus der Gruppe umfassend metallische Kationen;
• n + m den Polymerisationsgrad DP der Co-Polyaminosäure darstellt, das heißt die durchschnittliche Anzahl von Monomereinheiten pro Kette der Co-Polyaminosäure, wobei 5 ≤ n + m ≤ 250.

5. Zusammensetzung nach Anspruch 4, **dadurch gekennzeichnet, dass** die Carboxylat-Ladungen und hydrophobe Radikale tragende Co-Polyaminosäure ausgewählt ist aus den Co-Polyaminosäuren gemäß Formel VII, worin R₁ = R'₁ und R₂ = R'₂ sind, gemäß der folgenden Formel VIIa: 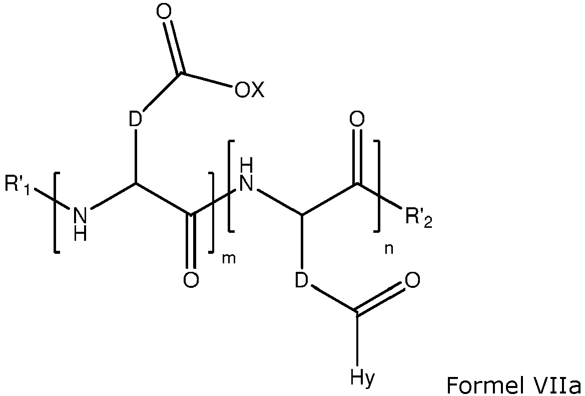 worin
- m, n, X, D und Hy wie in Anspruch 4 definiert sind,
- R'₁ ein Radikal ist, das ausgewählt ist aus der Gruppe bestehend aus H, einer linearen C2- bis C10-Acylgruppe, einer verzweigten C3- bis C10-Acylgruppe, einem Benzyl, einer terminalen « Aminosäure »-Einheit und einem Pyroglutamat,
- R'₂ ein Radikal -NR'R" ist, wobei R' und R", die identisch oder verschieden sind, ausgewählt sind aus der Gruppe bestehend aus H, linearen oder verzweigten oder zyklischen C2- bis C10-Alkylen, Benzyl und die R'- und R"-Alkyle zusammen einen oder mehrere gesättigte, ungesättigte und/oder aromatische Kohlenstoffringe bilden können und/oder Heteroatome enthalten können, die ausgewählt sind aus der Gruppe bestehend aus O, N und S.

6. Zusammensetzung nach Anspruch 4, **dadurch gekennzeichnet, dass** die Carboxylat-Ladungen und hydrophobe Radikale tragende Co-Polyaminosäure ausgewählt ist aus den Co-Polyaminosäuren gemäß Formel VII, worin n = 0 ist, mit der folgenden Formel VIIb: 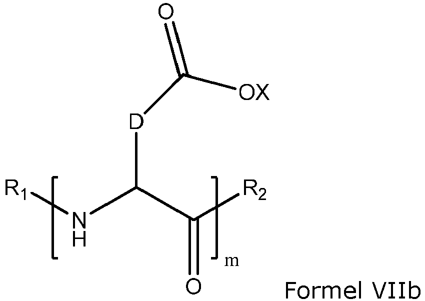 worin m, X, D, R₁ und R₂ wie in Anspruch 4 definifiert sind und wenigstens R₁ oder R₂ ein hydrophobes Radikal gemäß Formel I, V oder VI ist.

7. Zusammensetzung nach einem der vorangehenden Ansprüche, **dadurch gekennzeichnet, dass** das molare Verhältnis Co-Polyaminosäure/Amylin oder Amylinanalogon größer oder gleich 1 ist.

8. Zusammensetzung nach einem der vorangehenden Ansprüche, **dadurch gekennzeichnet, dass** das Amylin oder Amylinanalogon Amylin ist.

9. Zusammensetzung nach einem der vorangehenden Ansprüche, **dadurch gekennzeichnet, dass** das Amylin oder Amylinanlogon Pramlintid ist.

10. Zusammensetzung nach einem der vorangehenden Ansprüche, **dadurch gekennzeichnet, dass** sie zusätzlich prandiales Insulin umfasst.

11. Zusammensetzung nach einem der vorangehenden Ansprüche, daduch gekennzeichnet, dass das molare Verhältnis Co-Polyaminosäure/Insulin größer oder gleich 1 ist.

12. Zusammensetzung nach einem der Ansprüche 1 bis 9, **dadurch gekennzeichnet, dass** die Zusammensetzung eine mittels ThT gemessene Stabilität aufweist, die größer ist als die einer Referenzusammensetzung umfassend Amylin oder ein Amylinanalogon, aber nicht umfassend eine Carboxylat-Ladungen und hydrophobe Radikale Hy tragende Co-Polyaminosäure, wobei der ThT unter beschleunigten Stabilitätsbedingungen vorgenommen ist: unter Rühren und bei 37 °C.

13. Verwendung einer Carboxylat-Ladungen und hydrophobe Radikale Hy tragenden Co-Polyaminosäure nach einem der Ansprüche 1 bis 6, zum Stabilisieren einer Zusammensetzung umfassend Amylin oder ein Amylinanalogon.

14. Einzeldosisformulierung umfassend eine Zusammensetzung nach einem der Ansprüche 1 bis 12.

## Claims

1. A composition in the form of an injectable aqueous solution with a pH between 6.0 and 8.0, comprising at least:
a) amylin or an amylin analog;
b) a co-polyamino acid carrying carboxylate charges and Hy hydrophobic radicals, said co-polyamino acid consisting of glutamic or aspartic units, and said hydrophobic radicals Hy being of the following formula I: 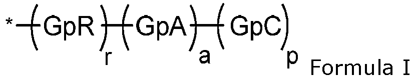 in which
- GpR is a radical of formula II, II', or II": 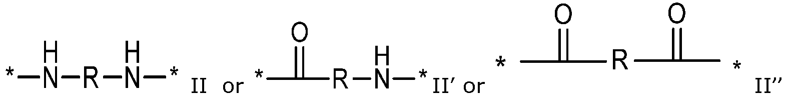
- GpA is a radical of formula III or III": 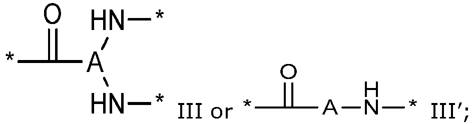
- GpC is a radical of formula IV: 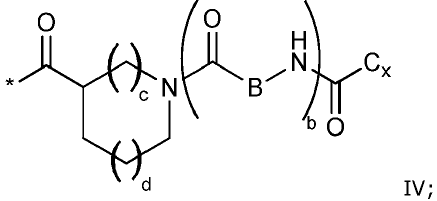
- the * indicating the attachment sites of the different groups linked by amide functions;
- a is an integer equal to 0 or 1;
- b is an integer equal to 0 or 1;
- p is an integer equal to 1 or 2; and
∘ if p is equal to 1, then a is equal to 0 or 1 and GpA is a radical of formula III', and
∘ if p is equal to 2, then a is equal to 1 and GpA is a radical of formula III;
- c is an integer equal to 0 or 1, and if c is equal to 0, then d is equal to 1 or 2;
- d is an integer equal to 0, 1, or 2;
- r is an integer equal to 0 or 1; and
∘ if r is equal to 0, then the hydrophobic radical of formula I is linked to the co-polyamino acid via a covalent bond between a carbonyl of the hydrophobic radical and a nitrogen atom in the N-terminal position of the co-polyamino acid, thus forming an amide function resulting from the reaction of an amine function in the N-terminal position of the precursor of the co-polyamino acid and an acid function carried by the precursor of the hydrophobic radical, and
∘ if r is equal to 1, then the hydrophobic radical of formula I is linked to the co-polyamino acid:
▪ via a covalent bond between a nitrogen atom of the hydrophobic radical and a carbonyl of the co-polyamino acid, thus forming an amide function resulting from the reaction of an amine function of the precursor of the hydrophobic radical and an acid function carried by the precursor of the co-polyamino acid, or
▪ via a covalent bond between a carbonyl of the hydrophobic radical and a nitrogen atom in the N-terminal position of the co-polyamino acid, thus forming an amide function resulting from the reaction of an acid function of the precursor of the hydrophobic radical and an amine function in the N-terminal position carried by the precursor of the co-polyamino acid;
- R is a radical selected from the group consisting of:
∘ a divalent, linear, or branched alkyl radical, comprising, if GpR is a radical of formula II, from 2 to 12 carbon atoms or, if GpR is a radical of formula II' or II", from 1 to 11 carbon atoms;
∘ a divalent, linear, or branched alkyl radical, comprising, if GpR is a radical of formula II, from 2 to 11 carbon atoms or, if GpR is a radical of formula II' or II", from 1 to 11 carbon atoms, said alkyl radical carrying one or more -CONH₂ functions, and
∘ an unsubstituted ether or polyether radical comprising from 4 to 14 carbon atoms and from 1 to 5 oxygen atoms;
- A is a linear or branched alkyl radical comprising from 1 to 6 carbon atoms;
- B is a linear or branched alkyl radical, optionally comprising an aromatic ring, comprising from 1 to 9 carbon atoms;
- Cₓ is a linear or branched monovalent alkyl radical, where x indicates the number of carbon atoms, and:
∘ if p is equal to 1, x is between 11 and 25 (11 ≤ x ≤ 25):
∘ if p is equal to 2, x is between 9 and 15 (9 ≤ x ≤ 15),
- the ratio i between the number of hydrophobic radicals and the number of glutamic or aspartic units being between 0 < i ≤ 0.5;
- when multiple hydrophobic radicals are carried by a co-polyamino acid, then they are identical or different,
- the degree of polymerization DP in glutamic or aspartic units is between 10 and 250;
- the free acid functions being in the form of a salt of an alkali metallic cation selected from the group consisting of Na⁺ and K⁺;
**characterized in that** the composition does not comprise a basal insulin whose isoelectric point pI is between 5.8 and 8.5.

2. The composition as set forth in claim 1, **characterized in that** said hydrophobic radicals are selected from among hydrophobic radicals of formula I in which p = 1, represented by the following formula V: 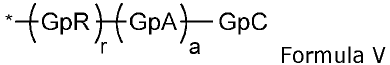 GpR, GpA, GpC, r, and a have the definitions given in claim 1.

3. The composition as set forth in claim 1, **characterized in that** said hydrophobic radicals are selected from among hydrophobic radicals of formula I in which a = 1 and p = 2, represented by the following formula VI: 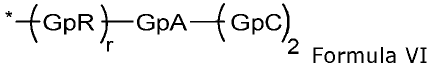 in which
GpR, GpA, GpC, and r have the definitions given in claim 1.

4. The composition as set forth in any one of the preceding claims, **characterized in that** the co-polyamino acid carrying carboxylate charges and hydrophobic radicals is selected from among co-polyamino acids of the following formula VII: 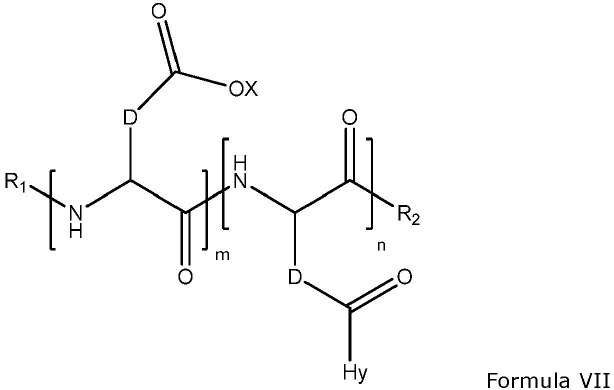 in which
• D represents, independently, either a -CH₂- group (aspartic unit) or a - CH₂-CH₂- group (glutamic unit),
• Hy is a hydrophobic radical selected from among hydrophobic radicals of formulas I, V, or VI in which r = 1 and GpR is a radical of formula II,
• R₁ is a hydrophobic radical selected from among hydrophobic radicals of formulas I, V, or VI in which r = 0 or r = 1 and GpR is a radical of formula II', or a radical selected from the group consisting of H, C₂-C₁₀ linear acyl, C₃-C₁₀ branched acyl, benzyl, a terminal "amino acid" unit, and pyroglutamate,
• R₂ is a hydrophobic radical selected from among hydrophobic radicals of formulas I, V, or VI in which r = 1 and GpR is a radical of formula II, or an -NR'R" radical, with R' and R" being identical or different and being selected from the group consisting of H, or linear or branched or cyclic C₂ to C₁₀ alkyls, it being possible for benzyl and said R' and R" alkyls to form together one or more saturated, unsaturated, and/or aromatic carbon rings and/or to comprise heteroatoms selected from the group consisting of O, N, and S,
• X represents an H or a cationic entity selected from the group comprising the metallic cations;
• n + m represents the degree of polymerization DP of the co-polyamino acid, i.e., the average number of monomeric units per co-polyamino acid chain and 5 ≤ n + m ≤ 250.

5. The composition as set forth in claim 4, **characterized in that** the the co-polyamino acid carrying carboxylate charges and hydrophobic radicals is selected from among co-polyamino acids of formula VII in which R₁ = R'₁ and R₂ = R'₂, of the following formula VIIa: 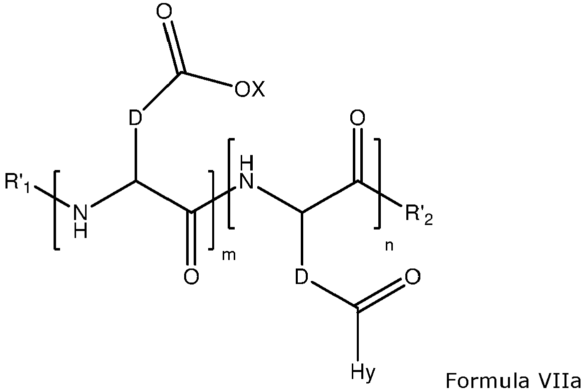 in which
- m, n, X, D, and Hy have the definitions given in claim 4,
- R'₁ is a radical selected from the group consisting of H, C₂-C₁₀ linear acyl, C₃-C₁₀ branched acyl, benzyl, a terminal "amino acid" unit, and pyroglutamate,
- R'₂ is an -NR'R" radical, with R' and R" being identical or different and being selected from the group consisting of H, or linear or branched or cyclic C₂ to C₁₀ alkyls, it being possible for benzyl and said R' and R" alkyls to form together one or more saturated, unsaturated, and/or aromatic carbon rings and/or to comprise heteroatoms selected from the group consisting of O, N, and S.

6. The composition as set forth in claim 4, **characterized in that** the co-polyamino acid carrying carboxylate charges and hydrophobic radicals is selected from among co-polyamino acids of formula VII in which n = 0 of the following formula VIIb: 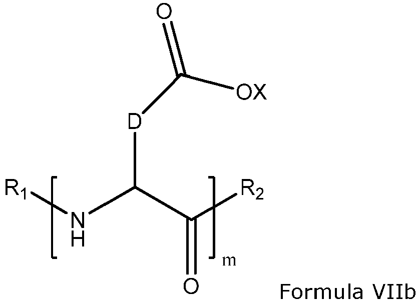 in which m, X, D, R₁, and R₂ have the definitions given in claim 4 and at least R₁ or R₂ is a hydrophobic radical of formula I, V, or VI.

7. The composition as set forth in any one of the preceding claims, **characterized in that** the molar ratio of co-polyamino acid to amyline or amyline analog is greater than or equal to 1.

8. The composition as set forth in any one of the preceding claims, **characterized in that** the amylin or amylin analog is amylin.

9. The composition as set forth in any one of the preceding claims, **characterized in that** the amylin or amylin analog is pramlintide.

10. The composition as set forth in any one of the preceding claims, **characterized in that** it further comprises a prandial insulin.

11. The composition as set forth in any one of the preceding claims, **characterized in that** the molar ratio of co-polyamino acid to insulin is greater than or equal to 1.

12. The composition as set forth in any one of claims 1 to 9, **characterized in that** said composition has a stability measured by ThT which is greater than that of a reference composition comprising amylin or an amylin analog but not comprising a co-polyamino acid carrying carboxylate charges and Hy hydrophobic radicals, said ThT monitoring being carried out under accelerated stability conditions: under stirring and at 37 °C.

13. Use of a co-polyamino acid carrying carboxylate charges and Hy hydrophobic radicals as set forth in any one of claims 1 to 6 to stabilize a composition comprising amylin or an amylin analog.

14. Single-dose formulations comprising a composition as set forth in any one of claims 1 to 12.
